(19) Europäisches Patentamt
European Patent Office
Office européen des brevets

(11) EP 4 234 566 B1

(12) EUROPEAN PATENT SPECIFICATION

(45) Date of publication and mention of the grant of the patent:
**15.04.2026 Bulletin 2026/16**

(21) Application number: **21882101.5**

(22) Date of filing: **21.10.2021**

(51) International Patent Classification (IPC):
***C07H 19/10*** *(2006.01)* ***C07H 19/20*** *(2006.01)*
***C12Q 1/68*** *(2018.01)*

(52) Cooperative Patent Classification (CPC):
**C07H 19/10; C07H 19/20;** C12Q 1/6869;
Y02P 20/55

(86) International application number:
**PCT/CN2021/125262**

(87) International publication number:
**WO 2022/083686 (28.04.2022 Gazette 2022/17)**

# (54) MODIFIED NUCLEOSIDE OR NUCLEOTIDE

MODIFIZIERTES NUKLEOSID ODER NUKLEOTID

NUCLÉOSIDE OU NUCLÉOTIDE MODIFIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2020 CN 202011134947**

(43) Date of publication of application:
**30.08.2023 Bulletin 2023/35**

(73) Proprietors:
- **BGI Shenzhen**
  **Shenzhen, Guangdong 518083 (CN)**
- **BGI Shenzhen Group Holdings Co., Limited**
  **Shenzhen, Guangdong 518083 (CN)**

(72) Inventors:
- **XU, Xun**
  **SHENZHEN, Guangdong 518083 (CN)**
- **TENG, Bo**
  **SHENZHEN, Guangdong 518083 (CN)**
- **ZHANG, Wenwei**
  **SHENZHEN, Guangdong 518083 (CN)**
- **CHEN, Ao**
  **SHENZHEN, Guangdong 518083 (CN)**
- **LI, Handong**
  **SHENZHEN, Guangdong 518083 (CN)**
- **YAN, Shengyi**
  **SHENZHEN, Guangdong 518083 (CN)**
- **ZHUO, Shitian**
  **SHENZHEN, Guangdong 518083 (CN)**
- **SHEN, Liang**
  **SHENZHEN, Guangdong 518083 (CN)**
- **ZHANG, Yonghui**
  **SHENZHEN, Guangdong 518083 (CN)**
- **GAO, Nanfeng**
  **SHENZHEN, Guangdong 518083 (CN)**
- **ZHAO, Jie**
  **SHENZHEN, Guangdong 518083 (CN)**
- **LIAO, Sha**
  **SHENZHEN, Guangdong 518083 (CN)**

(74) Representative: **Inspicos P/S**
**Strandvejen 60**
**2900 Hellerup (DK)**

(56) References cited:

| | |
|---|---|
| **WO-A1-2014/139596** | **WO-A1-2018/107350** |
| **WO-A1-2019/071474** | **WO-A1-2020/159447** |
| **WO-A1-2021/067970** | **WO-A1-94/23064** |
| **WO-A1-96/07669** | **WO-A2-02/29003** |
| **WO-A2-2008/037568** | **US-A1- 2016 010 150** |

- **CANARD BRUNO ET AL: "DNA polymerase fluorescent substrates with reversible 3'-tags", GENE 148 (1994), 2 June 1994 (1994-06-02), pages 1 - 6, XP093216361, DOI: https://doi.org/10.1016/0378-1119(94)90226-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **SHOSHANI ILANA, QIU HOWARD, JOHNSON FRANCIS, JOHNSON ROGER A.: "Synthesis of Iodo-aryl-azido Adenosine Analogs as Affinity Ligands for Adenylyl Cyclase", NUCLEOSIDES AND NUCLEOTIDES, vol. 13, no. 9, 1 September 1994 (1994-09-01), US , pages 1977 - 1989, XP009535854, ISSN: 0732-8311, DOI: 10.1080/15257779408010676**

## Description

Technical field

The invention relates to the field of nucleic acid sequencing. In particular, the present invention relates to modified nucleoside or nucleotide, wherein the 3'-OH of the modified nucleoside or nucleotide is reversibly blocked.

Background

The emergence of NGS sequencing overcomes the disadvantages of Sanger's high cost and time requirements, and greatly promotes the application of gene sequencing technology. At present, NGS sequencing has been deeply applied in fields such as prenatal screening, tumor diagnosis, tumor treatment, animal and plant breeding, and promotes the progress of science and medicine.

Deoxy-ribonucleoside triphosphate (dNTP) analogues carrying reversible blocking groups are key raw materials in NGS sequencing. Due to the incorporation of reversible blocking group, the 3'-OH group in dNTP can be retained, which overcomes the shortcomings of Sanger sequencing and ensures the accuracy of base recognition. It can be said that deoxy-ribonucleoside triphosphate (dNTP) analogue with reversible blocking group is the most critical technology in NGS sequencing.

Currently, many dNTP compounds carrying reversible blocking groups have been reported. WO2020159447 describes a method of synthesizing a single-stranded nucleotide sequence. WO0229003 describes massive parallel method for decoding DNA and RNA WO9423064 describes certain derivatives for use in nucleic acid sequencing. Canard Bruno et al Gene 148 (1994), 2 June 1994, pages 1-6 disclose certain DNA polymerase fluorescent substrates with reversible 3'-tags. WO9607669 describes the synthesis of oligonucleotides and other nucleic acid polymers using template independent enzymes. WO2019071474 describes certain modified nucleoside of nucleotide. Shoshani Ilana et al Nucleosides and Nucleotides, vol 13, no.9, 1 September 1994, pages 1977-1989 disclose the synthesis of iodo-aryl-azido adenosine analogs. WO2018107350 describes a method for purifying a reversibly blocked deoxyribonucleotide triphosphate. US20160010150 describes methods, compositions, and systems for nucleic acid sequencing. WO2008037568 describes certain reversible terminators WO2014139596 describes certain modified nucleoside of nucleotide.

The reversible blocking of dNTP is mainly realized through two kinds of ideas. The first kind of idea bases on the direct introduction of reversible blocking groups into 3'-OH of dNTP, and the advantage of such modified dNTP is that the blocking of 3'-OH ensures the blocking efficiency in sequencing. The other kind of idea bases on blocking the polymerase by base modification, rather than blocking 3'-OH, and the advantage of such idea is that the blocking groups may be selected from a wider range, rather than limited to polymerase.

In general, the two kinds of reversible blocking ideas have their own advantages and disadvantages, but the method of introducing group blocking directly into 3'- OH shows more reliability and significantly higher blocking efficiency. Therefore, such scheme is mainly used in currently marketed NGS sequencing.

Summary of the invention

The invention is defined in the appended claims. Any disclosure going beyond the scope of said claims is only intended for illustrative purposes and should not be construed as being par of the invention.

Azidomethyl is a kind of highly effective 3'-OH reversible blocking group possessing great advantages such as good stability, mild removal conditions and fast removal speed. The excision reaction of azido methyl on 3'-OH is essentially a cascade reaction, in that the azido group undergoes the Staudinger reaction under the action of phosphorous reagent to produce an intermediate with methylene amino group on 3'-OH, which then undergoes a rapid hydrolysis reaction to free the 3'-OH. During such cascade reaction, both Staudinger reaction and the hydrolysis reaction proceed very fast, so that the azidomethyl may be removed at very fast rate (as shown in Figure 1).

Azido group is a special chemical group with three nitrogen atoms in its chemical structure, forming a conjugated structure and in the same plane. The three nitrogen atoms are not positioned in a straight line but arranged with certain angle. The conjugated structure formed by the three nitrogen atoms in an azido group may be presented in several resonance modes, which contributes to its stability.

Azido group is generally stable. However, due to the iterative structure of the three nitrogen atoms, azido group may react and quickly release nitrogen under the appropriate reaction conditions, and thus the azide group has a very active reactivity and is prone to explosive risk. It is precisely based on the characteristic of azide group that it is widely used in various click reactions.

Based on such rapid reactivity of azido group, the inventor has designed a class of 3'-OH blocking groups carrying azido group that can undergo cascade reaction. The 3'-OH blocking group undergoes the Staudinger reaction to release

amine group, which attacks the ester/carbonate protection group on 3'-OH to free 3'-OH (as shown in Figure 2). Similarly, those groups capable of releasing a nucleophilic atom under appropriate conditions are all applicable to such design, such as-S-SR, -OCOR, - OCONHR and the like.

The invention intends to develop a class of dNTP analogues carrying ester and carbonate group on 3'-OH, which are useful in NGS sequencing. Such dNTP analogues have the general structural formula shown in Figure 3, with 3'-OH protected by reversible blocking group and a bulk structure comprising 2'-deoxyuridine triphosphate, 2'-deoxythymosine triphosphate, 2'-deoxycytidine triphosphate, 2'-deoxyadenosine triphosphate, 7-deaza-2'-deoxyadenosine triphosphate, 2'-deoxyguanosine triphosphate and 7-deaza-2'-deoxyguanosine triphosphate.

Therefore, in the first aspect of the invention, the present invention provides compound of formula (A) or a salt thereof,

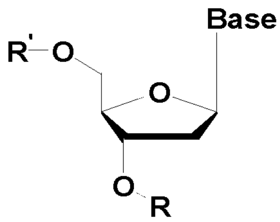

formula (A)

wherein:

R is a reversible blocking group, R is selected from

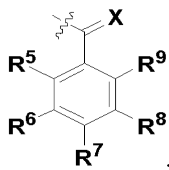

,

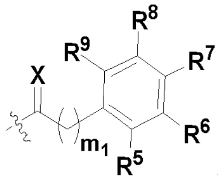

;

each X is independently selected from O, NH, S;

preferably, X is O;

$R^5$ is

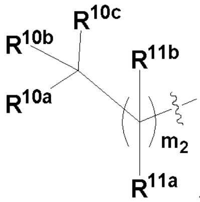

,

and $R^6$, $R^7$, $R^8$, and $R^9$ are each independently selected from H, nitro, $C_1$-$C_6$ alkyl, $C_1$-$C_6$ alkoxy, $C_1$-$C_6$ alkyl-C(=O)-$NH_2$-;

preferably $R^5$ is

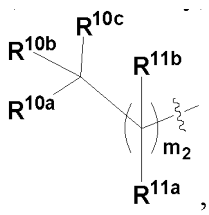,

and $R^6$, $R^7$, $R^8$, and $R^9$ are each independently selected from H, nitro, methoxy, acetamido;

most preferably, $R^5$ is

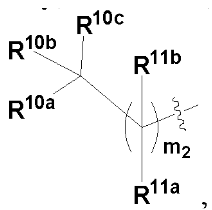,

$R^7$ is selected from H, nitro, methoxy, acetamido, and $R^6$, $R^8$, and $R^9$ are H;

any one of $R^{10a}$, $R^{10b}$, and $R^{10c}$ is $-N_3$ or -SS-$C_1$-$C_6$ alkyl (e.g., -SS-methyl, -SS- ethyl, -SS-isopropyl, -SS-tertbutyl or -SS-isobutyl), and the other two are each independently selected from H, $C_1$-$C_6$ alkyl, such as methyl, ethyl, isopropyl, tertbutyl.

In some embodiments, any one of $R^{10a}$, $R^{10b}$, and $R^{10c}$ is $-N_3$, -SS-methyl, -SS- ethyl, -SS-isopropyl, -SS-tertbutyl or -SS-isobutyl, and the other two are each independently selected from H, methyl.

$R^{11a}$ and $R^{11b}$ are each independently selected from H, or $C_1$-$C_6$ alkyl, such as methyl, ethyl, isopropyl, tertbutyl; preferably, $R^{11a}$ and $R^{11b}$ are H.

Each $m_1$ is independently selected from 1, 2, 3, 4, 5, and 6; preferably, $m_1$ is 1.

Each m2 is independently selected from 0, 1, 2, 3, 4, 5, and 6; preferably, each $m_2$ is independently selected from 0 or 1;

most preferably,

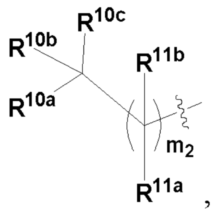,

as a whole, is selected from

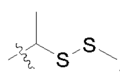,

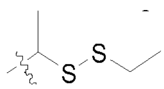, 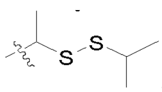, 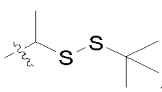, 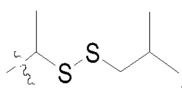, 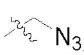, 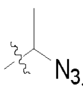, 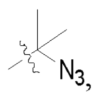,

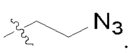.

R' is selected from H, monophosphate group

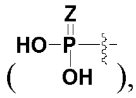

diphosphate group

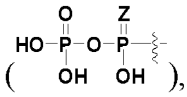

triphosphate group

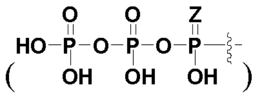

or tetraphosphate group

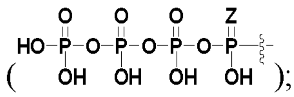

preferably R' is a triphosphate group

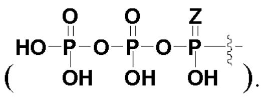

Each Z is independently selected from O, S, BH; preferably, Z is O.

Base is selected from a base, a deaza base or a tautomer thereof, for example, Base is selected from adenine, 7-deazaadenine, thymine, uracil, cytosine, guanine, 7-deazaguanine or a tautomer thereof;
preferably, the Base is selected from

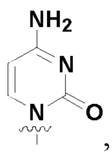,
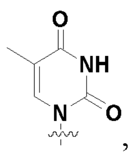,
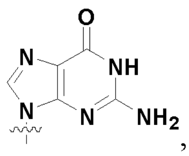,

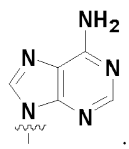.

The present invention provides the following compounds or salts thereof,

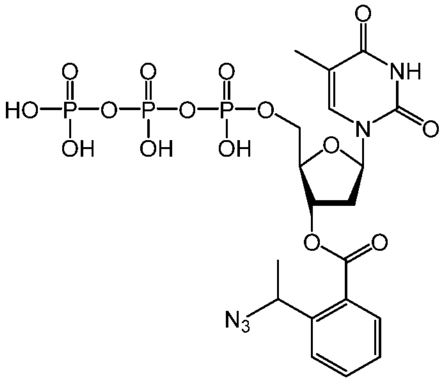

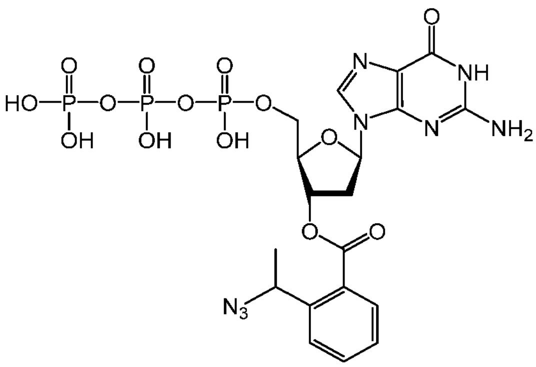

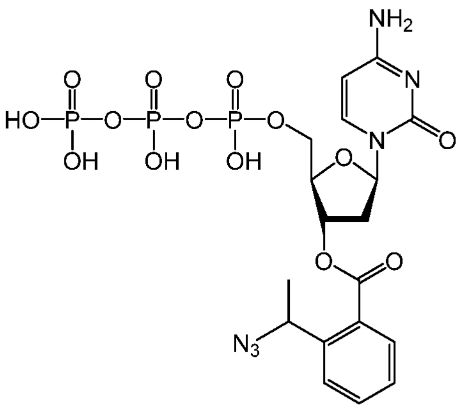

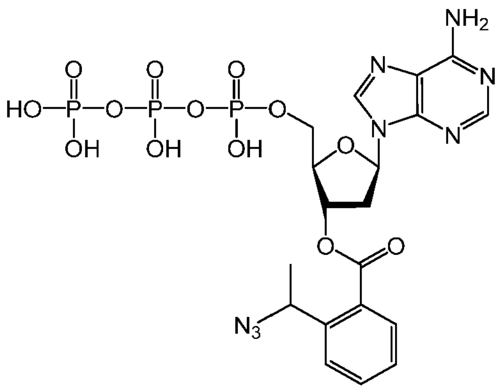

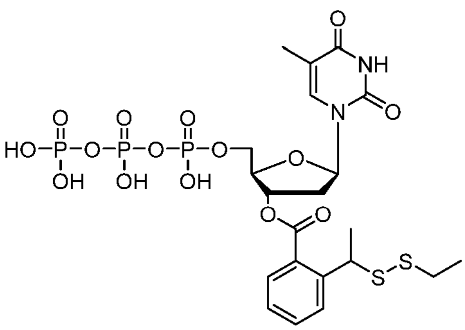

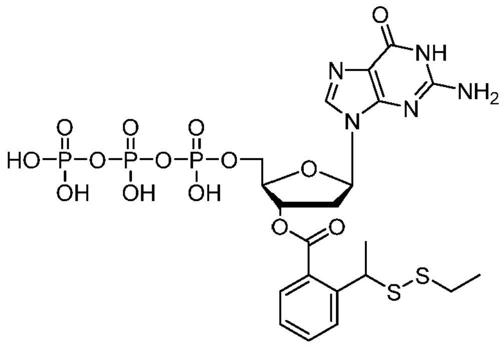

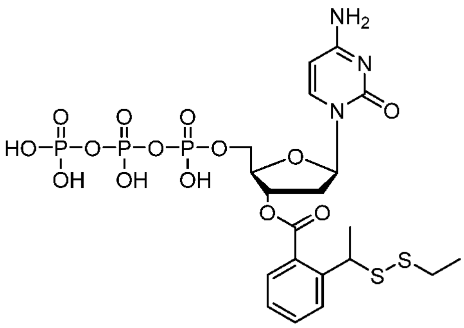

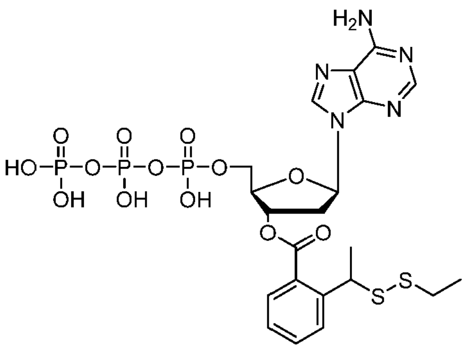

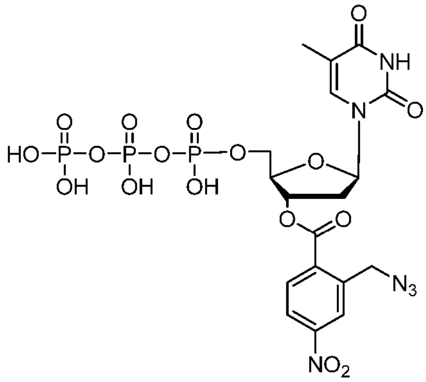

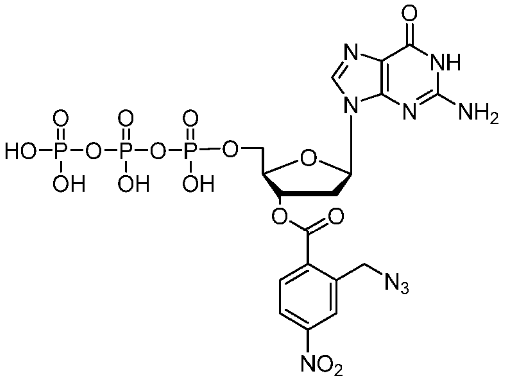

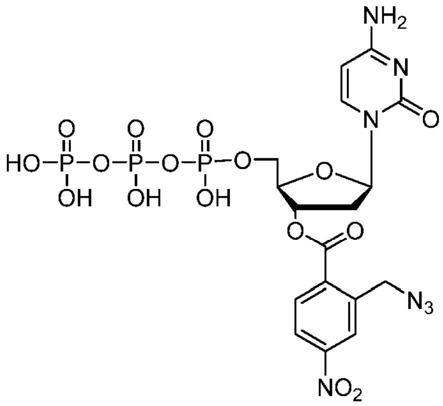

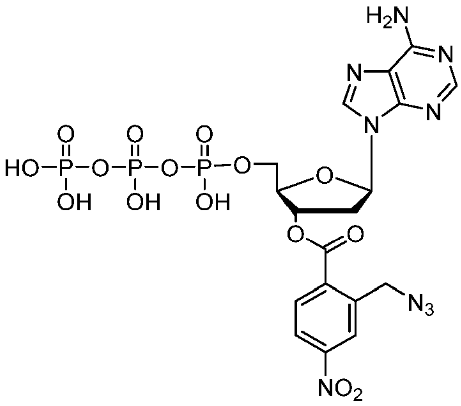

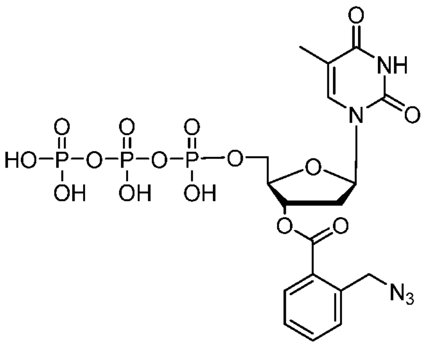

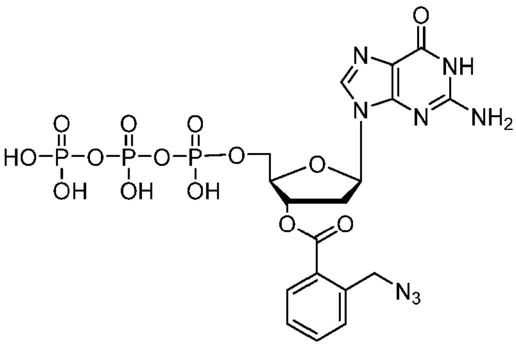

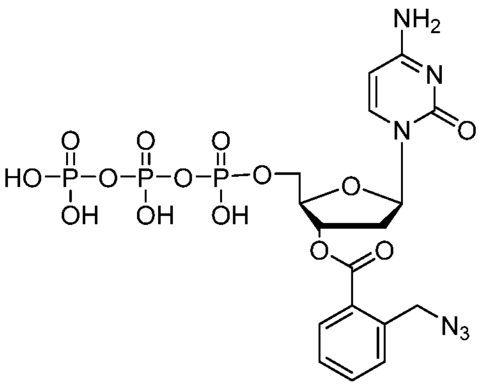

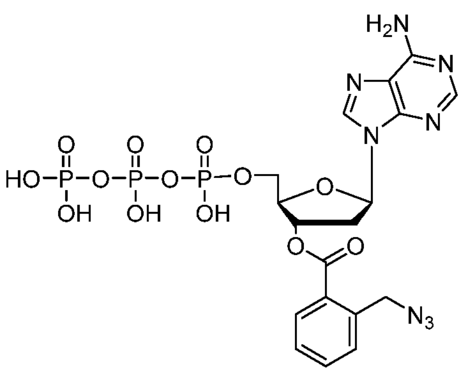

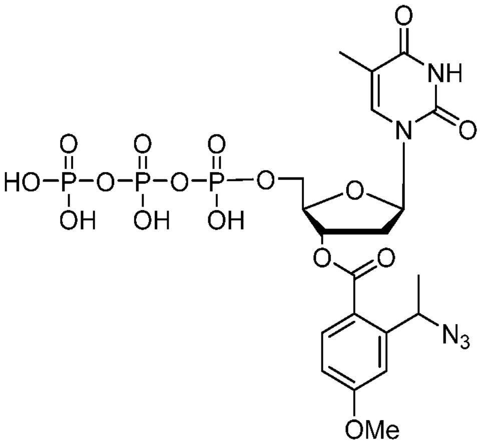

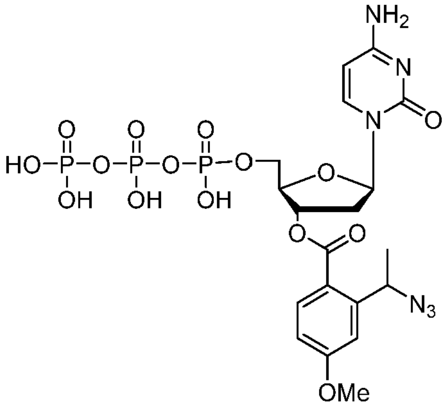

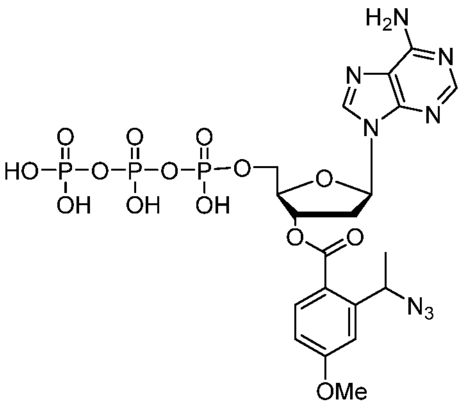

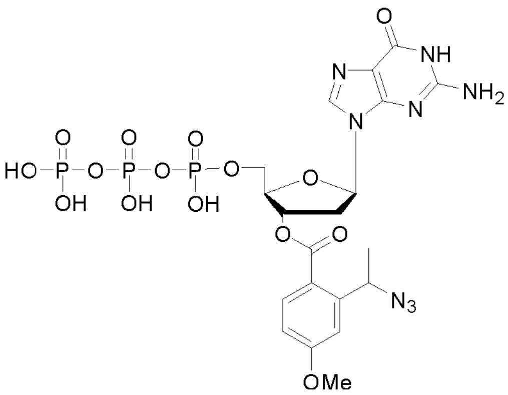

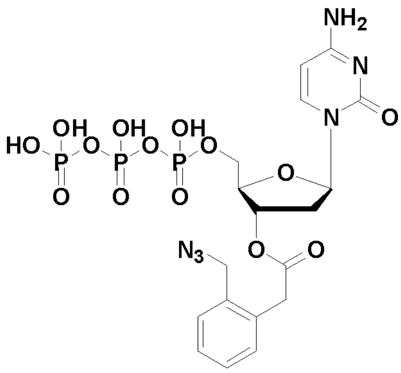

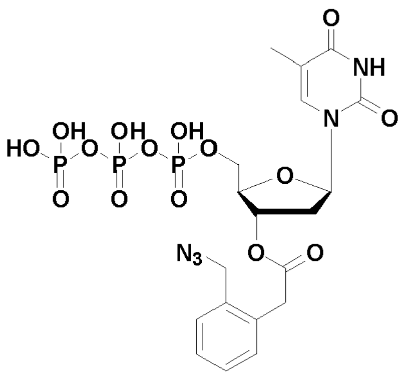

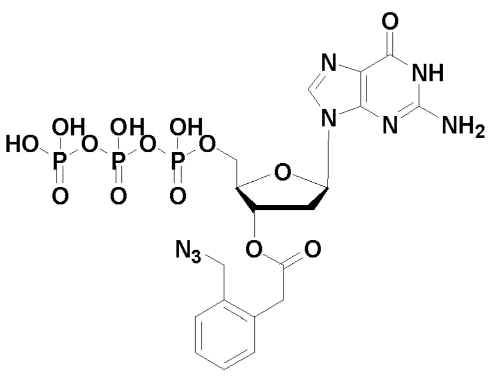

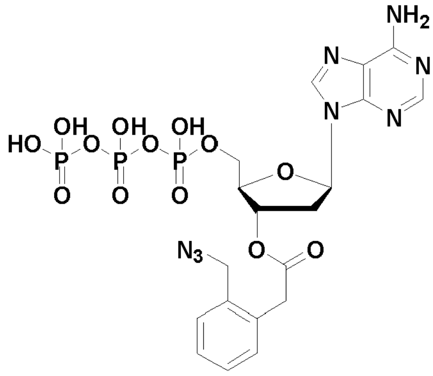

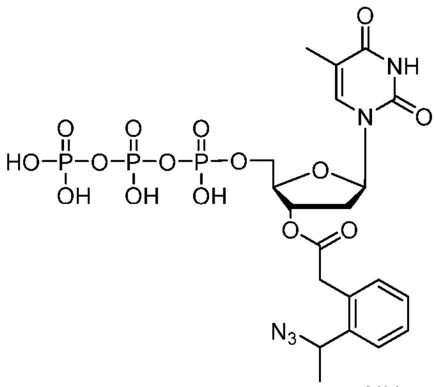

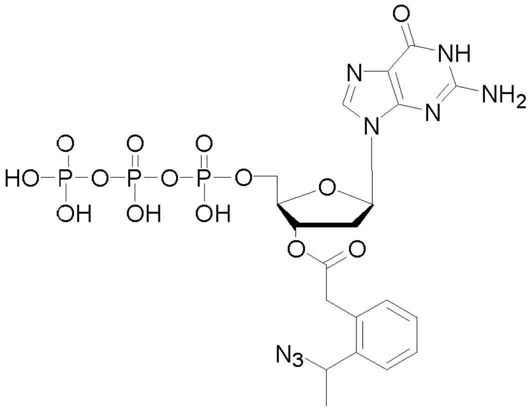

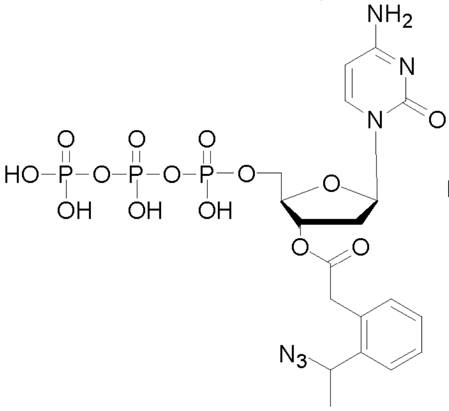

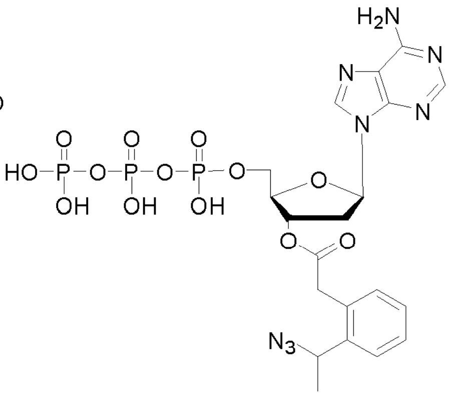

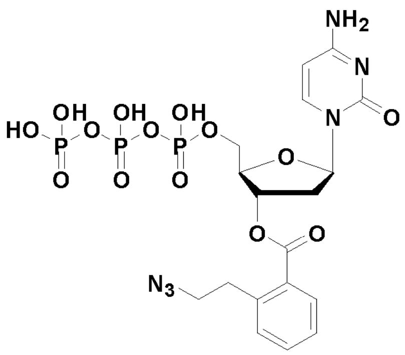

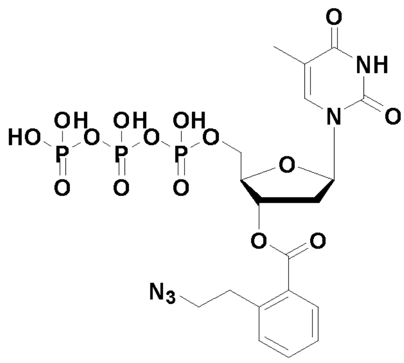

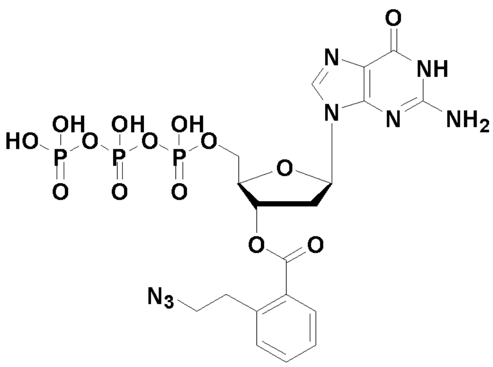

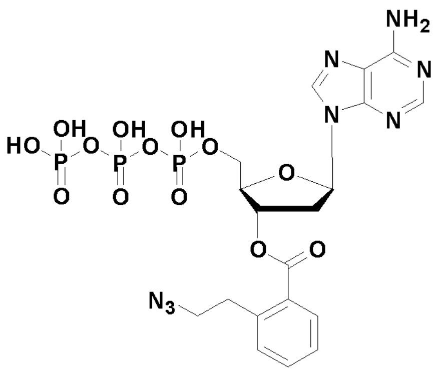

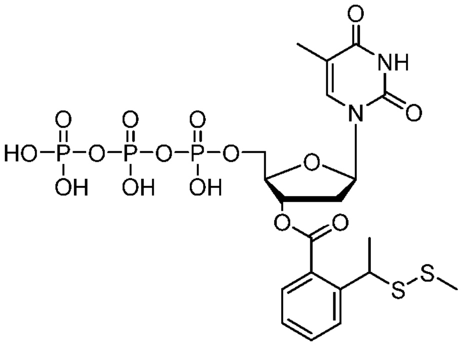

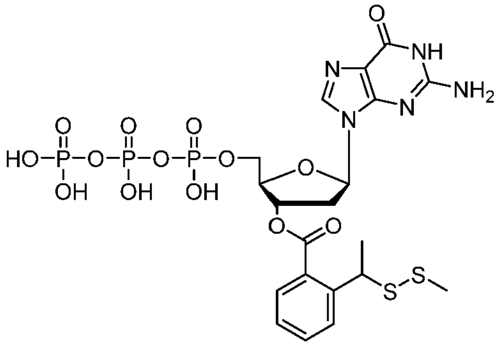

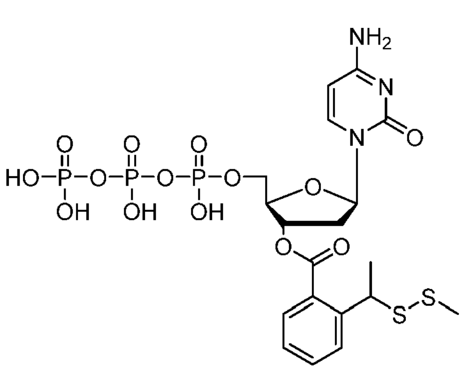

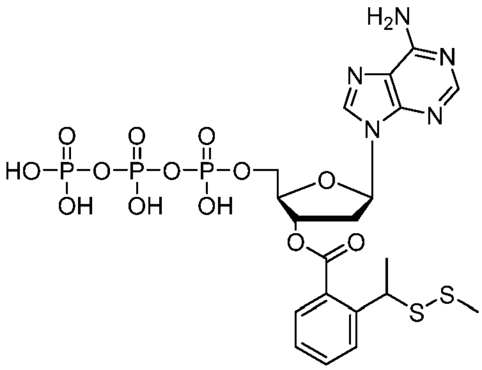

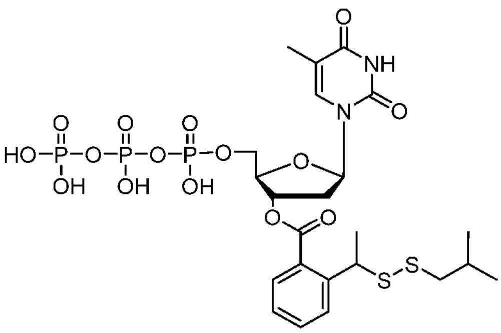

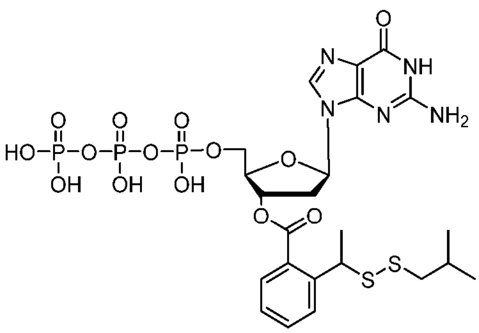

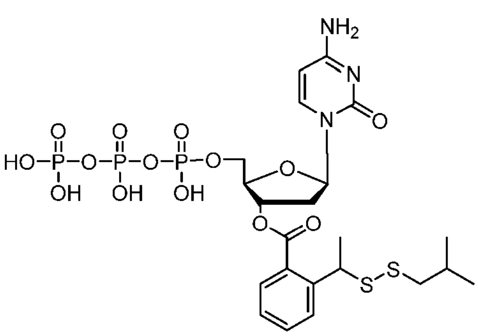

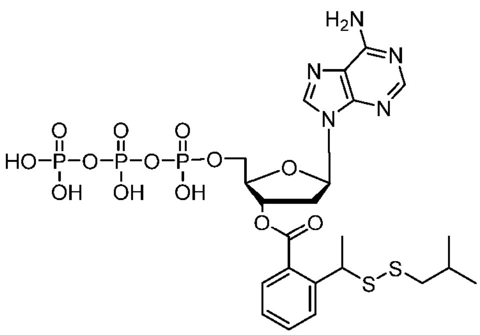

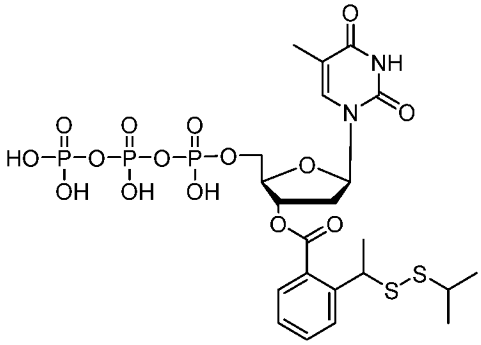

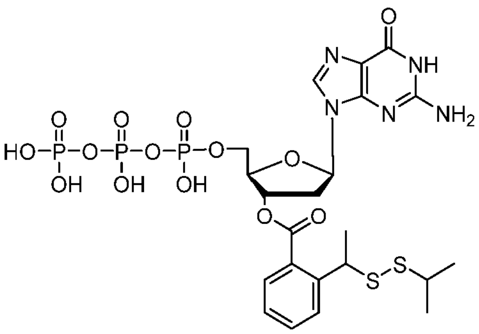

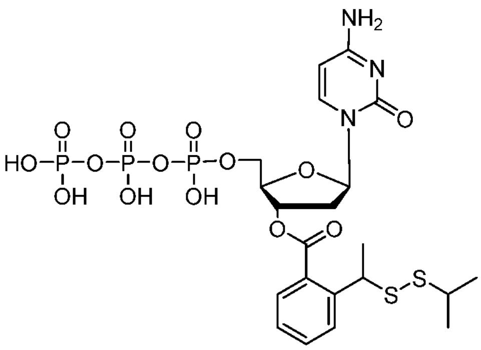

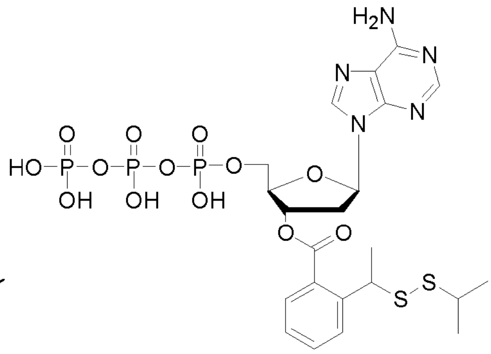

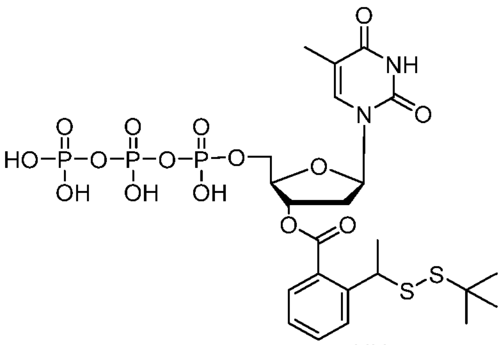

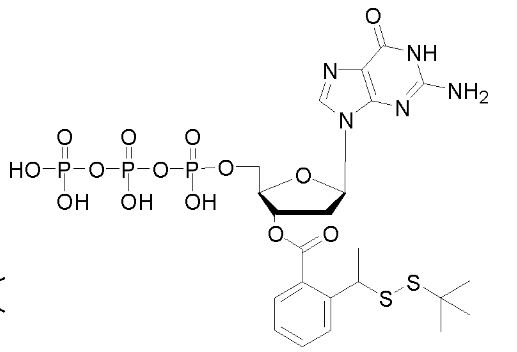

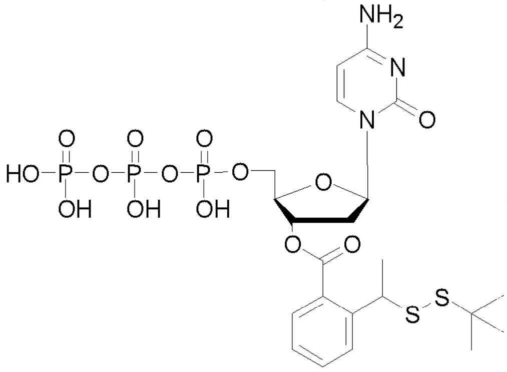

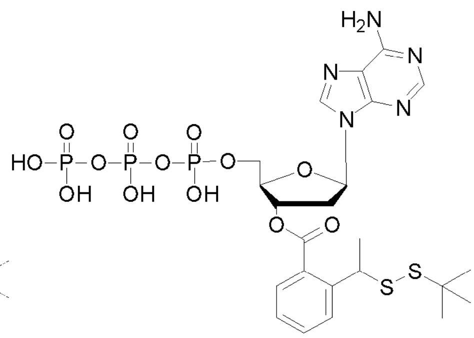

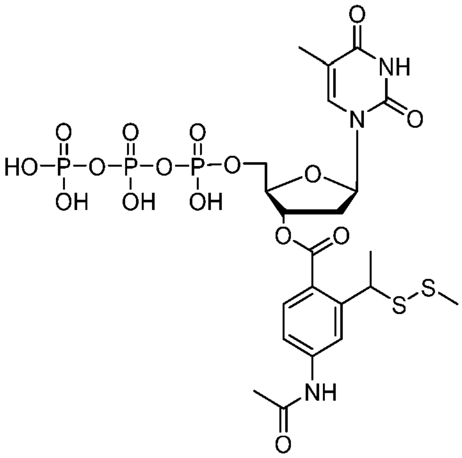

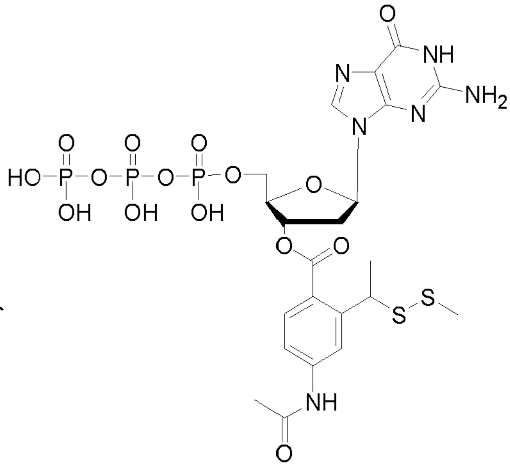

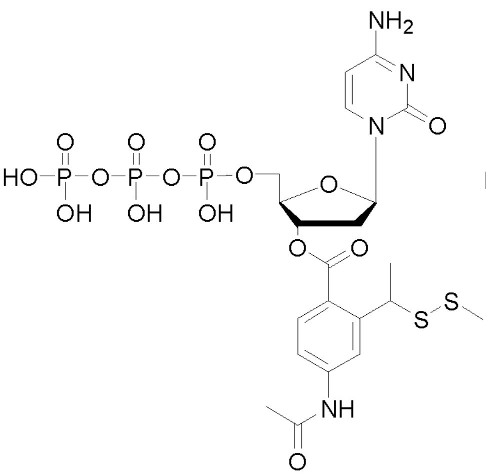

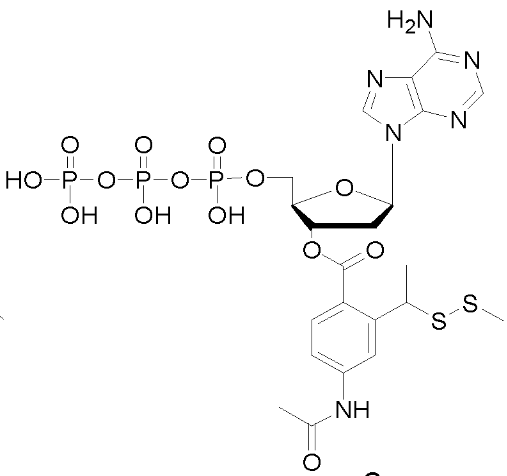

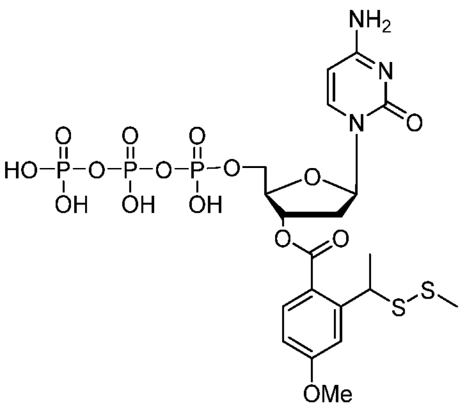

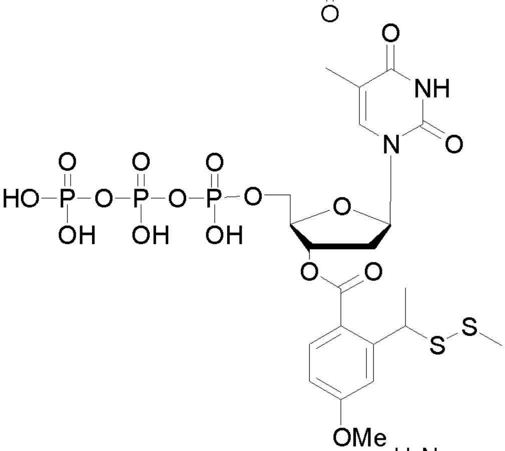

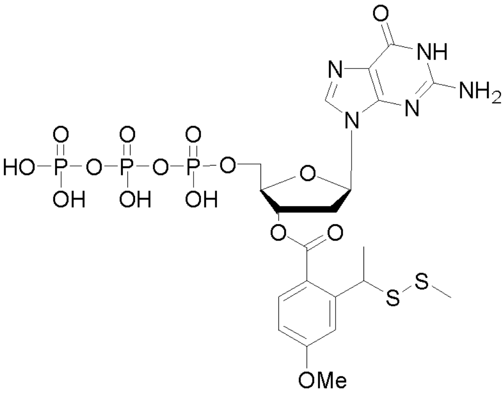

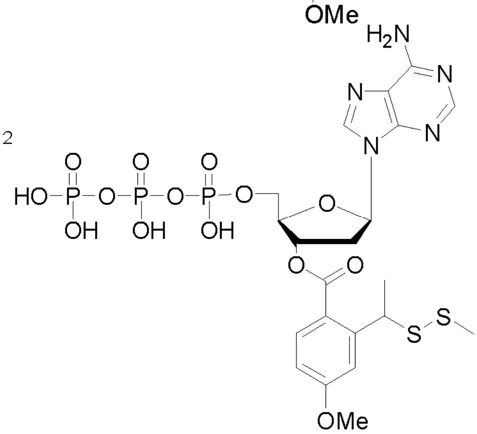

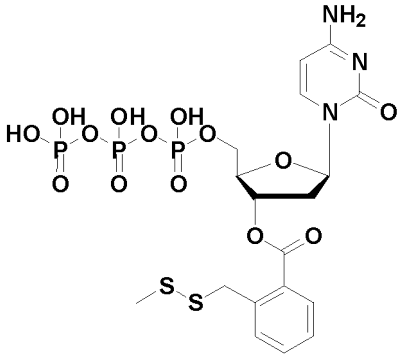

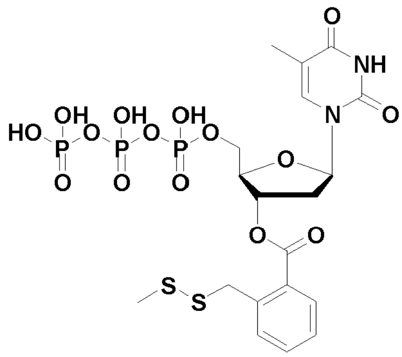

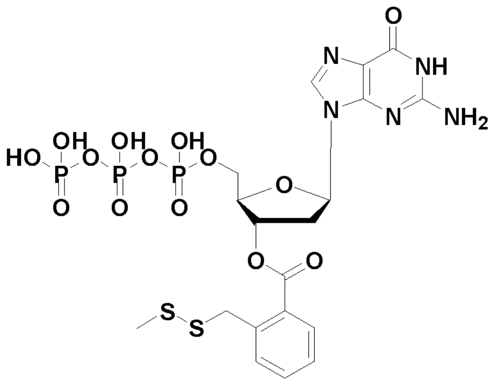

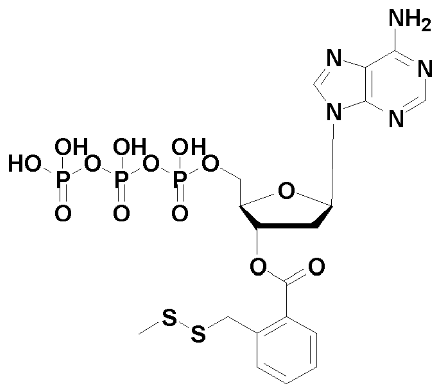

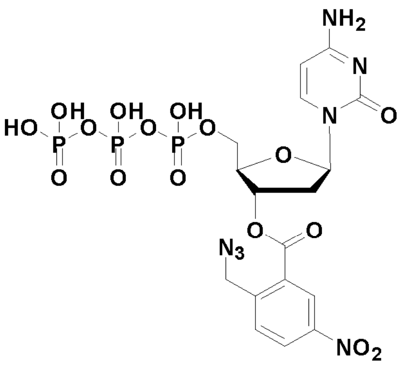

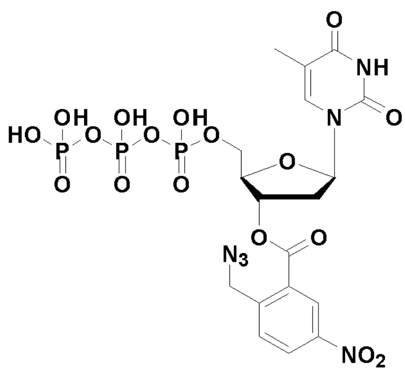

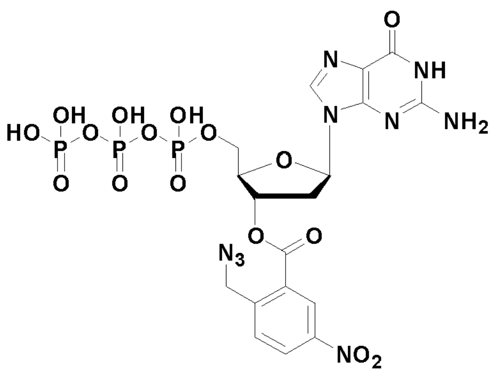

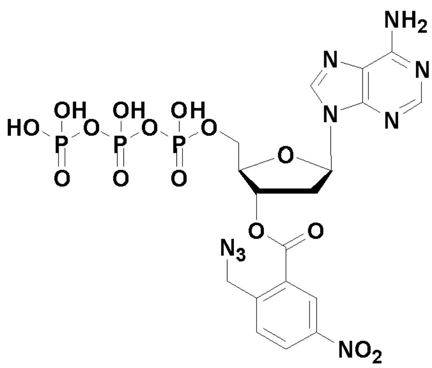

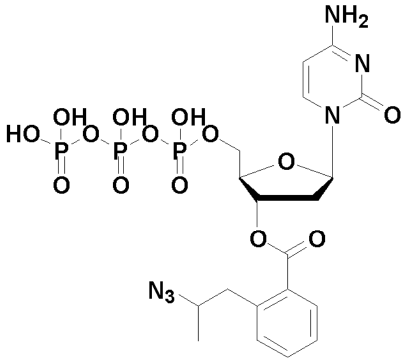

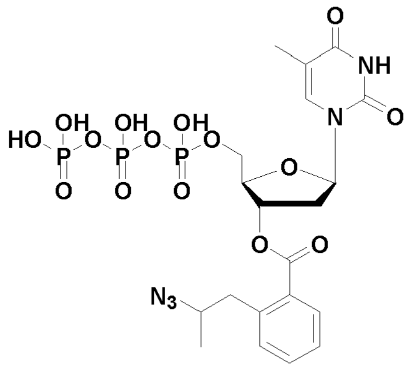

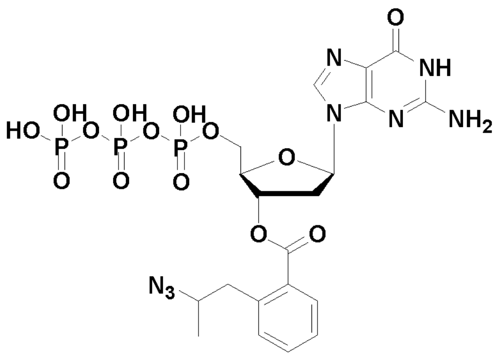

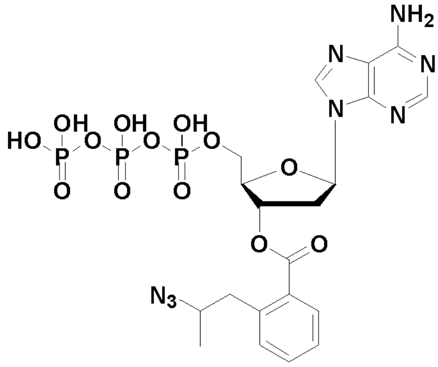

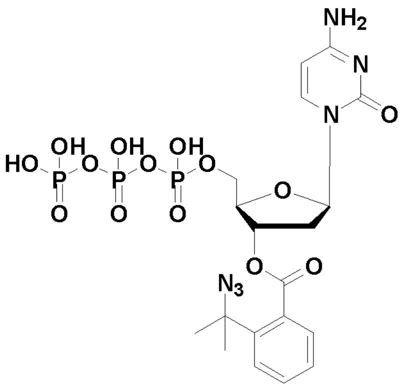

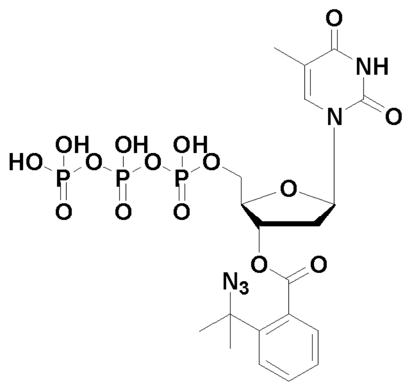

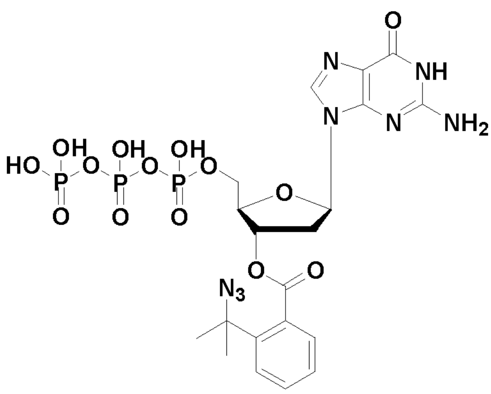

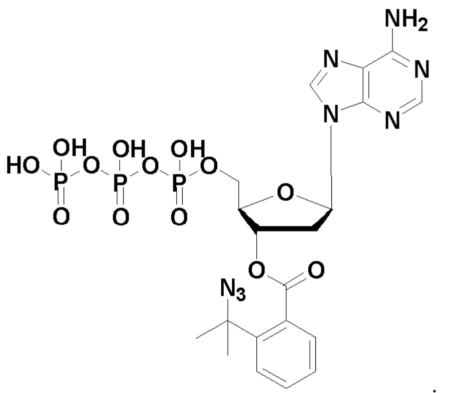

In some embodiments, the compound or a salt thereof as described above carries an additional detectable label (e.g., a fluorescent label).

In some embodiments, the additional detectable label carried by the compound or a salt thereof is introduced by

an affinity reagent (e.g., antibody, aptamer, Affimer, Knottin), wherein the affinity reagent carries the detectable label, and the affinity reagent can specifically recognize and bind to an epitope of the compound or a salt thereof.

In some embodiments, the additional detectable label (e.g., a fluorescent label) is linked to the compound or a salt thereof optionally via a linker.

In some embodiments, the additional detectable label (e.g., a fluorescent label) is linked to the Base of the compound or a salt thereof optionally via a linker.

In some embodiments, when the additional detectable label (e.g., a fluorescent label is linked to the Base of the compound or a salt thereof optionally via a linker,

the structure of Base is selected from the followings: 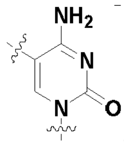, 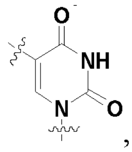,

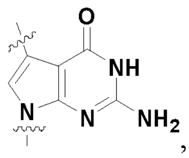, 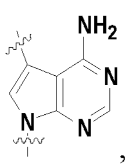, 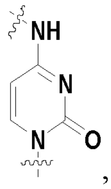, 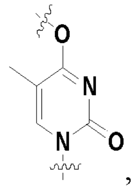, 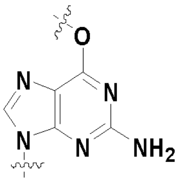 or 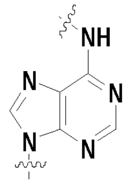,

preferably selected from

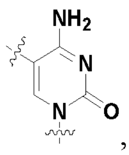, 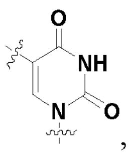, 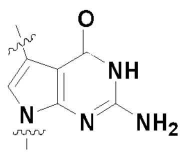 or 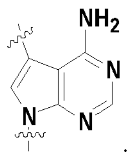.

**In** some embodiments, the linker is a cleavable linker or a non-cleavable linker.

In some embodiments, the cleavable linker is selected from a linker capable of being cleaved by electrophilic reaction, a linker capable of being cleaved by nucleophilic reaction, a linker capable of being cleaved by photolysis, a linker capable of being cleaved under reductive conditions, a linker capable of being cleaved under oxidative conditions, a safety-catch linker, a linker capable of being cleaved by elimination mechanisms, or any combination thereof.

In some embodiments, the linker has a structure of formula (B):

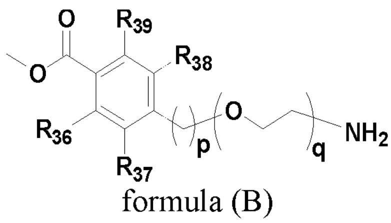

formula (B)

wherein:

- $R^{36}$, $R^{37}$, $R^{38}$, $R^{39}$ are each independently selected from H, $-N_3$, $N_3$-$C_1$-$C_6$ alkyl, nitro, amino, sulfo, carboxyl, aliphatic alkyl (e.g., $C_1$-$C_6$ alkyl), cycloalkyl (e.g., $C_3$-$C_6$ cycloalkyl), aromatic alkyl (e.g., phenyl $C_1$-$C_6$ alkyl), F, I, Br, Cl, alkoxy (e.g., $C_1$-$C_6$ alkoxy), the $C_1$-$C_6$ alkyl is optionally substituted by $C_1$-$C_6$ alkyl;
- p is selected from 1, 2, 3, 4, 5, or 6;
- q is selected from any integer between 1 and 12.

In some particular embodiments, any one of $R^{36}$, $R^{37}$, $R^{38}$, and $R^{39}$ is $N_3$-$C_1$-$C_6$ alkyl, and wherein the $C_1$-$C_6$ alkyl is optionally substituted by $C_1$-$C_6$ alkyl, the others are each independently selected from H, $-N_3$, nitro, amino, sulfo, carboxyl, aliphatic alkyl (e.g., $C_1$-$C_6$ alkyl), cycloalkyl (e.g., $C_3$-$C_6$ cycloalkyl), aromatic alkyl (e.g., phenyl $C_1$-$C_6$ alkyl), F, I, Br, Cl, alkoxy (e.g., $C_1$-$C_6$ alkoxy).

In some particular embodiments, $R^{36}$ is $N_3$-$C_1$-$C_6$ alkyl, and wherein the $C_1$-$C_6$ alkyl is optionally substituted by

$C_1$-$C_6$ alkyl, $R^{37}$, $R^{38}$, and $R^{39}$ are each independently selected from H, $C_1$-$C_6$ alkyl.

In some particular embodiments, $R^{36}$ is $N_3$-methyl optionally substituted by methyl, and $R^{37}$, $R^{38}$, and $R^{39}$ are H.

In some particular embodiments, p is selected from 1, 2, or 3.

In some particular embodiments, p is 1.

In some particular embodiments, q is selected from 2, 3, 4, 5, or 6.

In some particular embodiments, q is 4.

In some embodiments, methyl end of the linker of formula (B) is connected to the Base, and the amino end thereof is connected to the additional detectable label (e.g., a fluorescent label). For example,

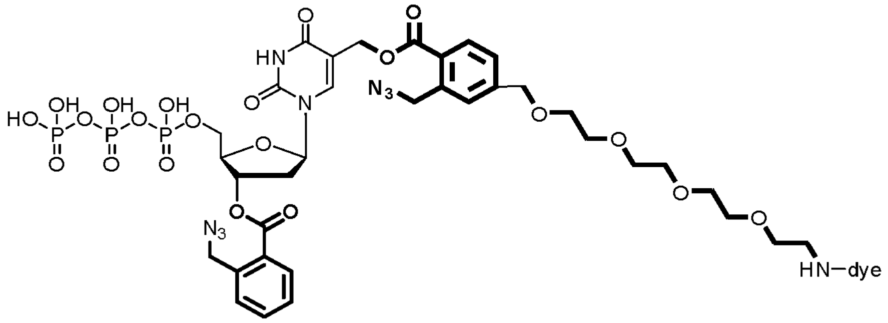

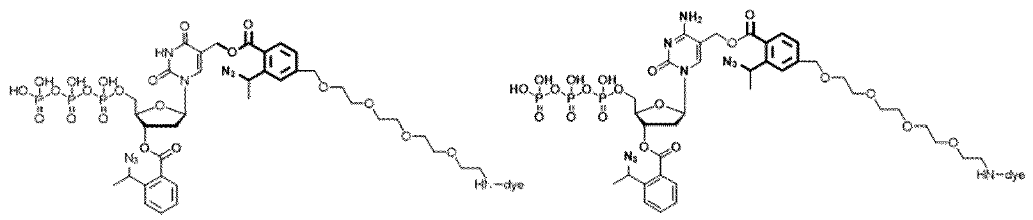

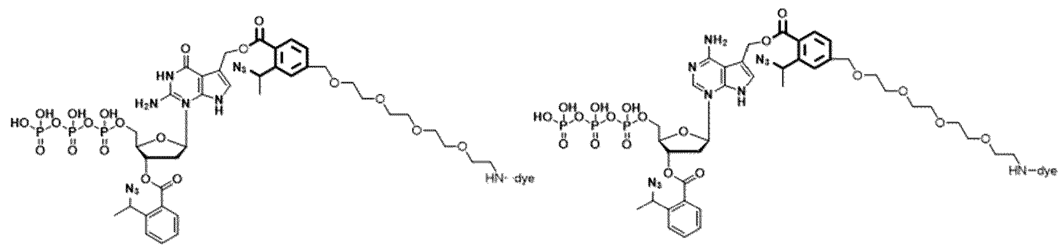

In some embodiments, the linker of formula (B) has the following structure

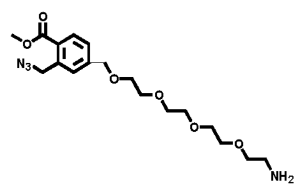
or
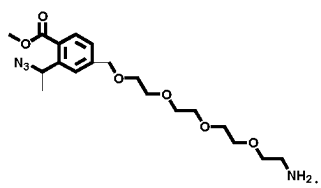

In some embodiments, the linker has the structure of formula (C) :

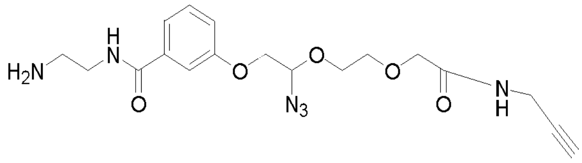

formula C.

In some embodiments, the alkynyl end of the linker of formula (C) is connected to the Base, and the amino end thereof is connected to the additional detectable label (e.g., a fluorescent label). For example,

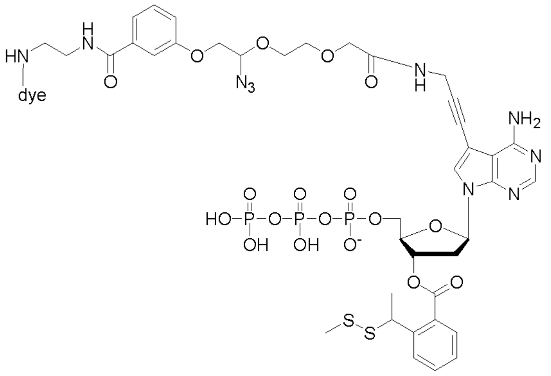

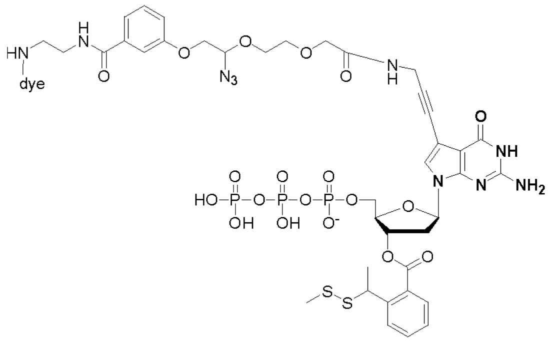

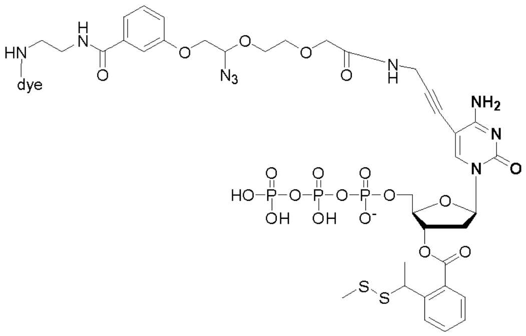

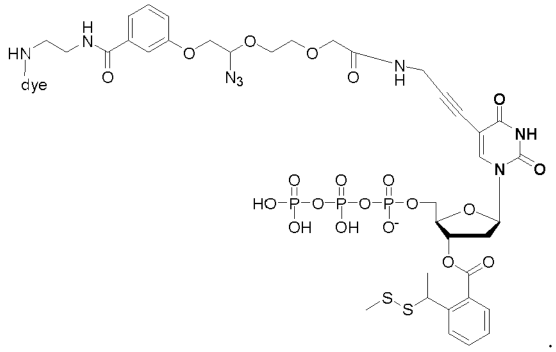
.

In some embodiments, the linker has the structure of formula (D) :

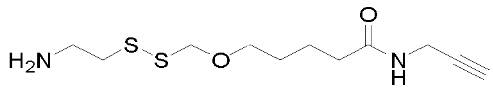

formula D.

In some embodiments, the alkynyl end of the linker of formula (D)is connected to the Base, and the amino end thereof is connected to the additional detectable label (e.g., a fluorescent label). For example,

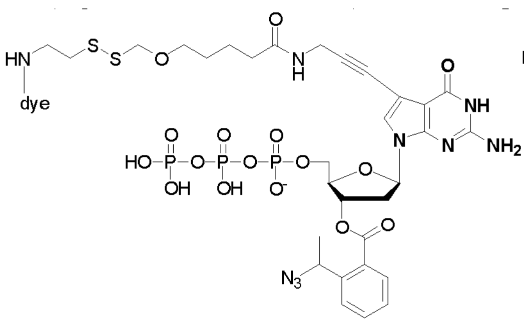

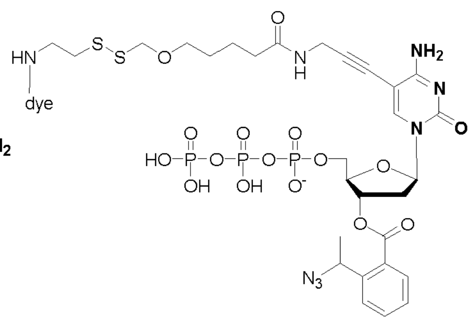

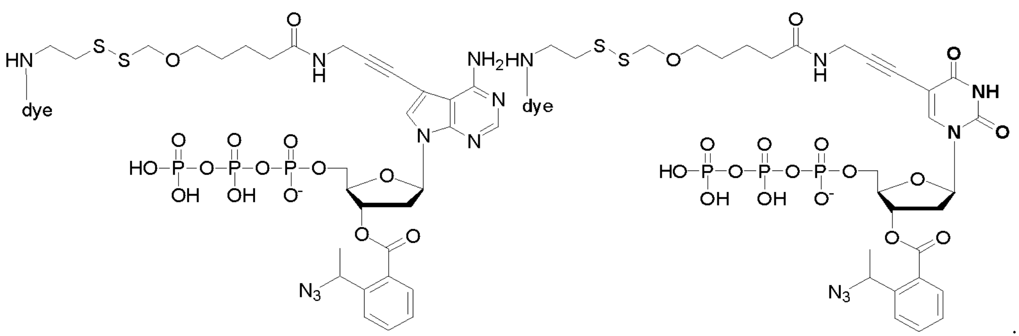
.

In some embodiments, the detectable label is selected from the followings:

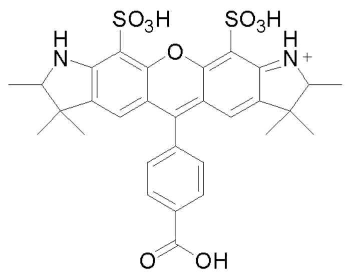

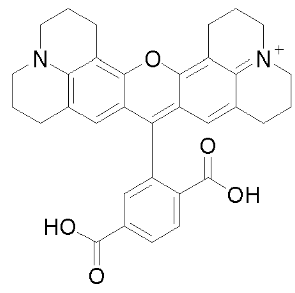

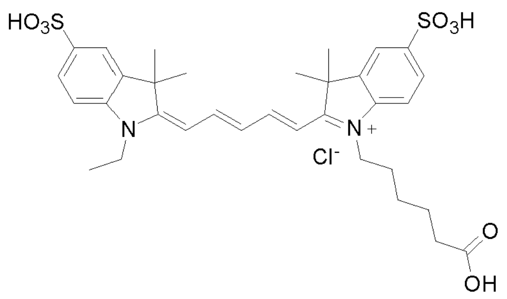

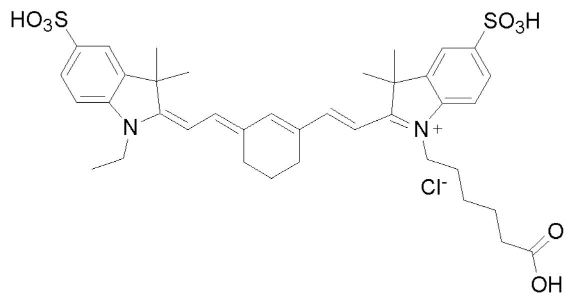

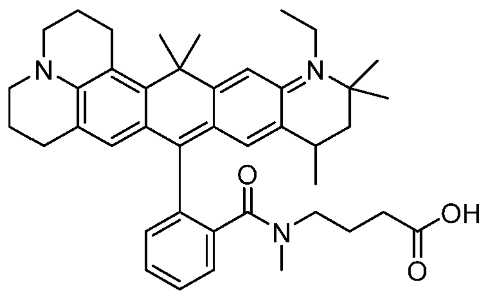

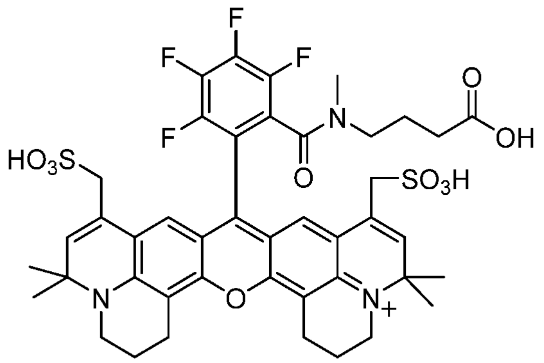

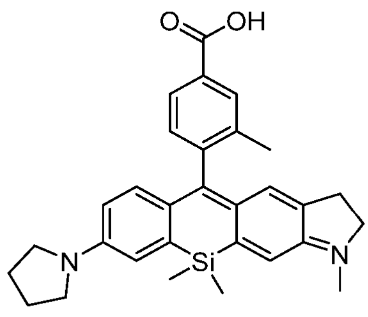

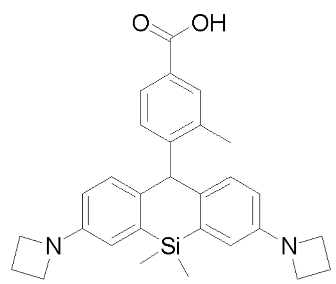

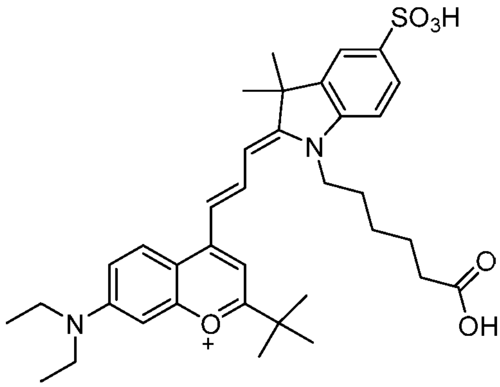

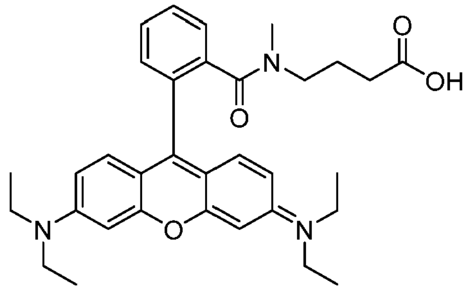

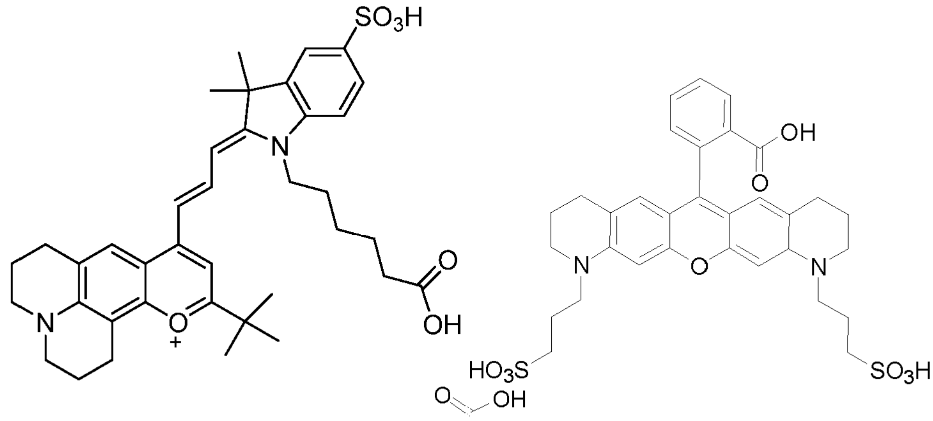

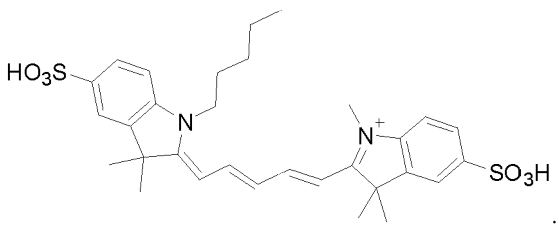

In some embodiments, if the Base is different, the detectable label (e.g., a fluorescent label) varies.

In some embodiments, the compound or a salt thereof carrying the additional detectable label has the following structure:

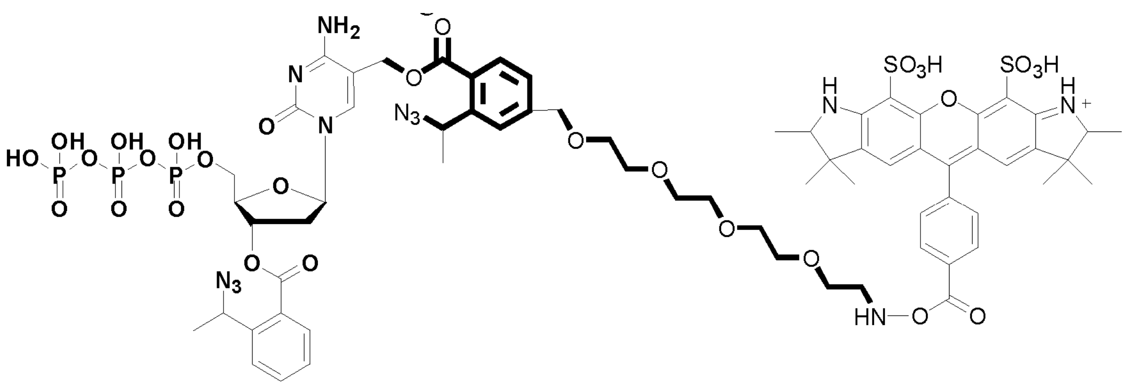

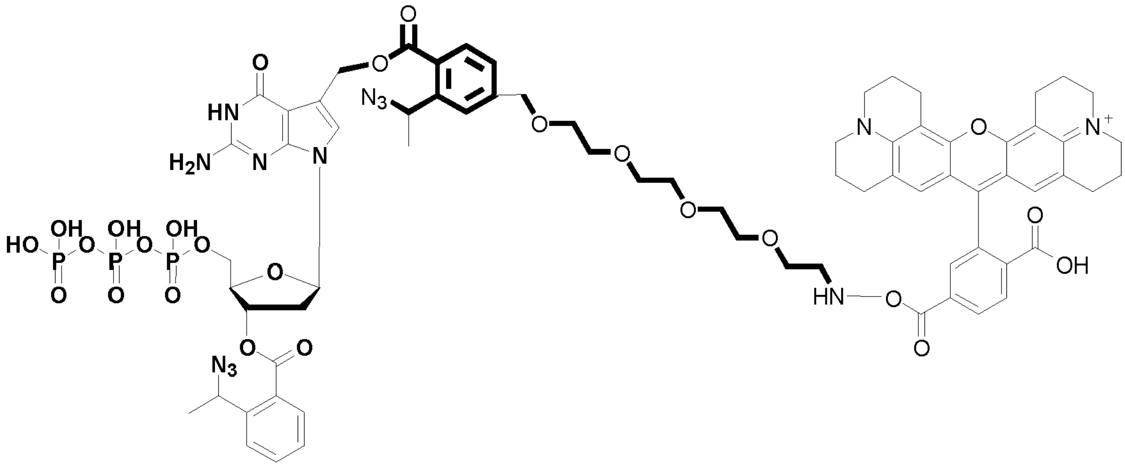

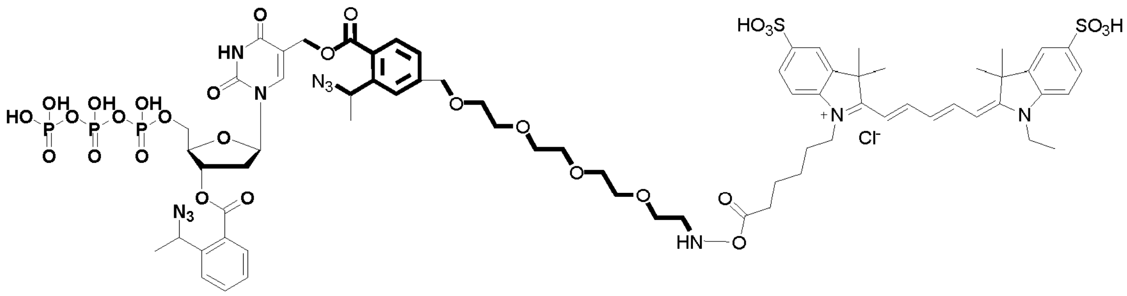

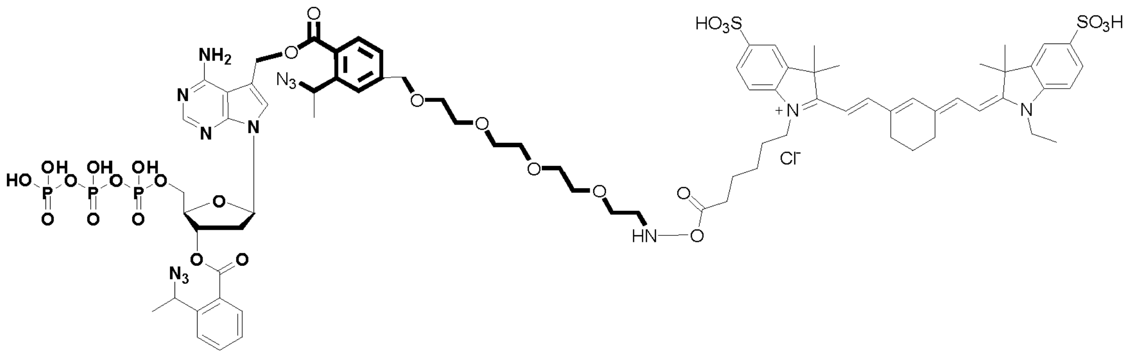

In some embodiments, the compound or a salt thereof carrying additional detectable label has the following structure:

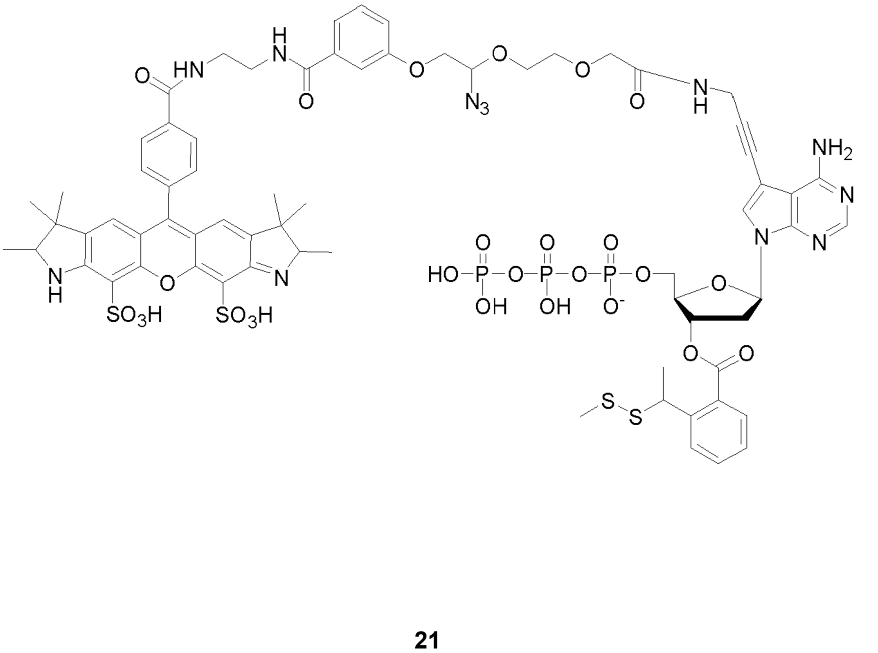

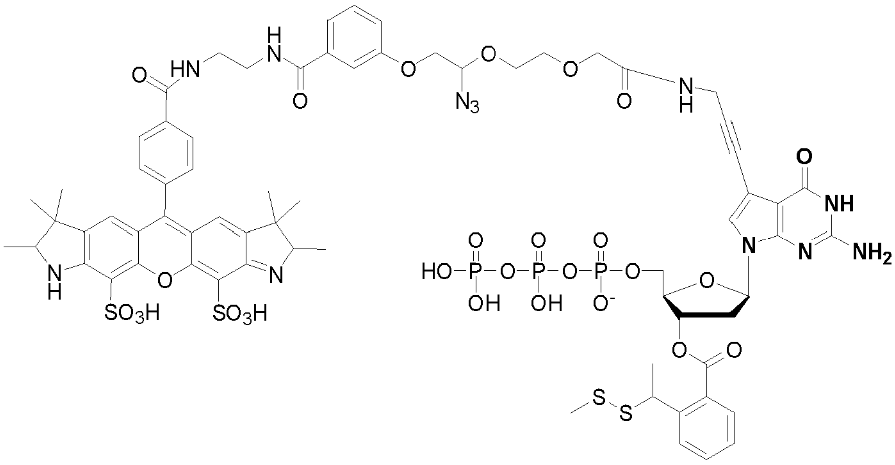

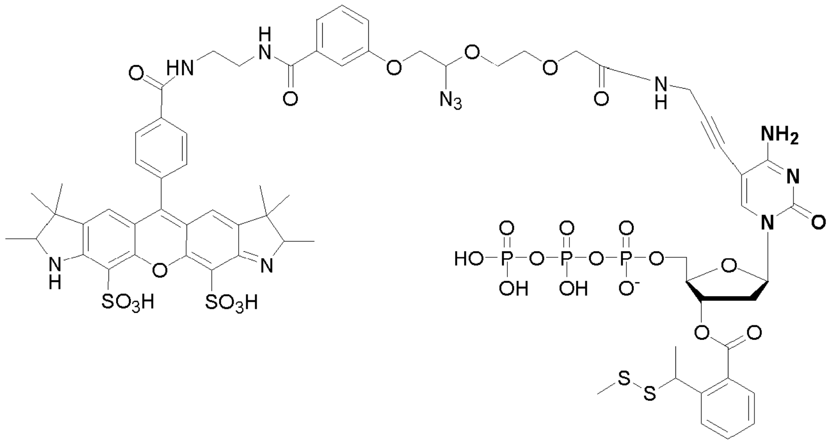

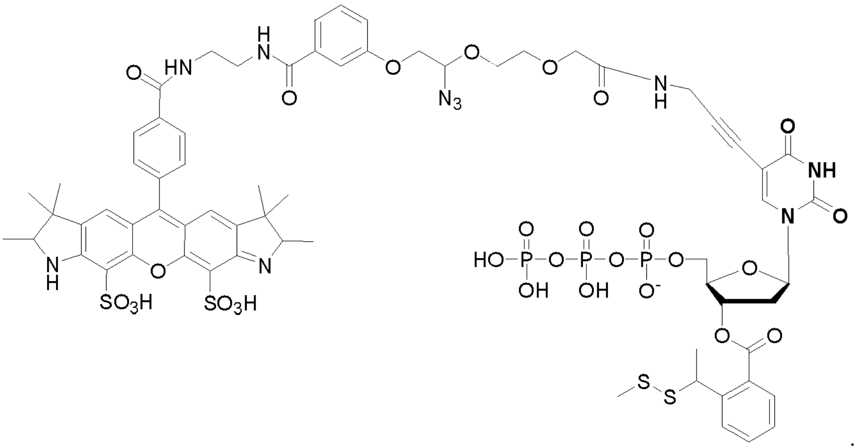

.

In some embodiments, the compound or a salt thereof carrying additional detectable label has the following structure:

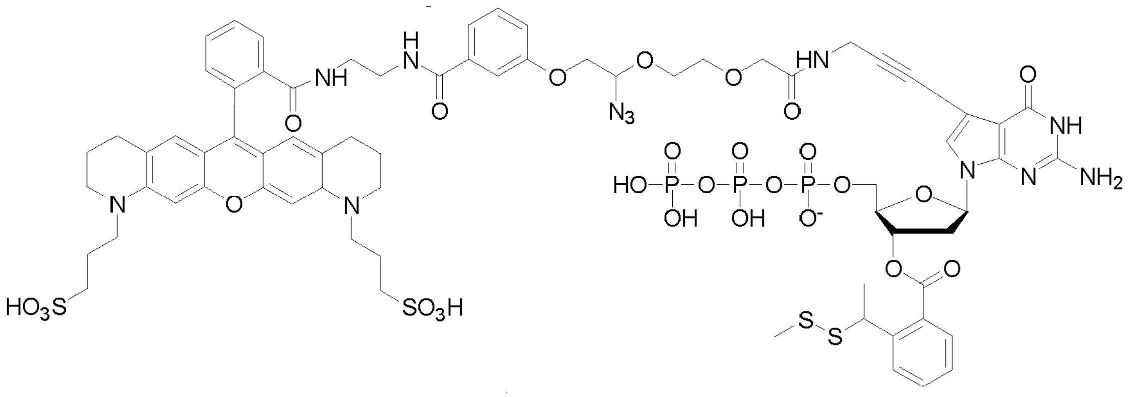

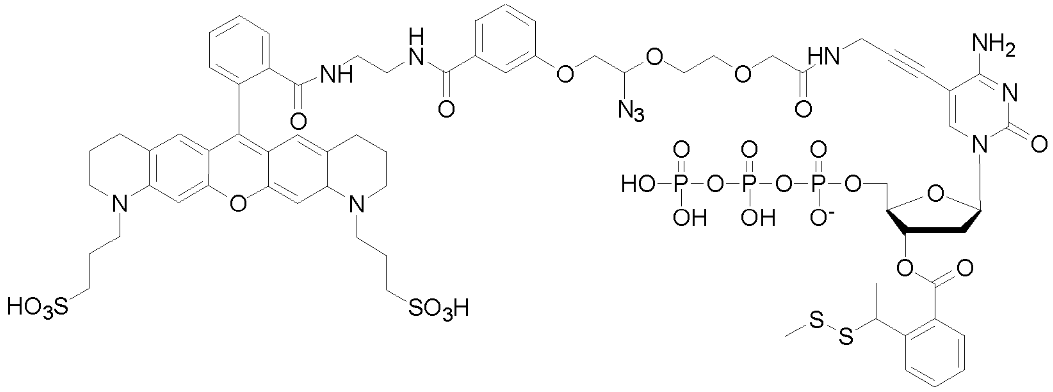

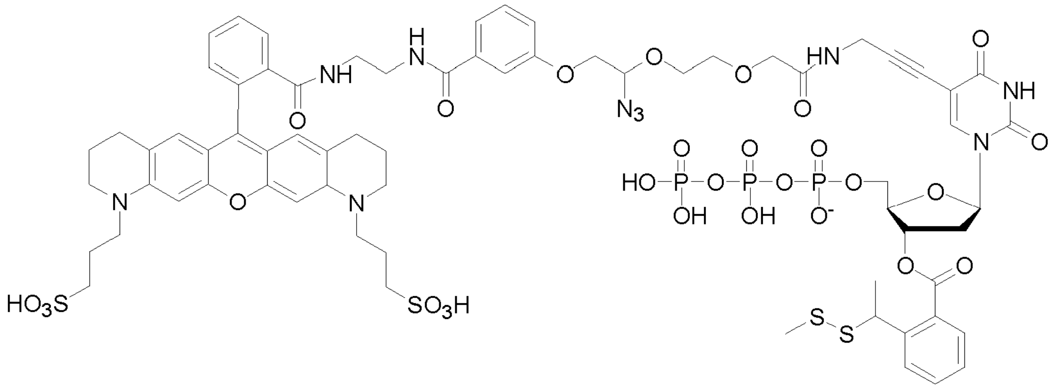

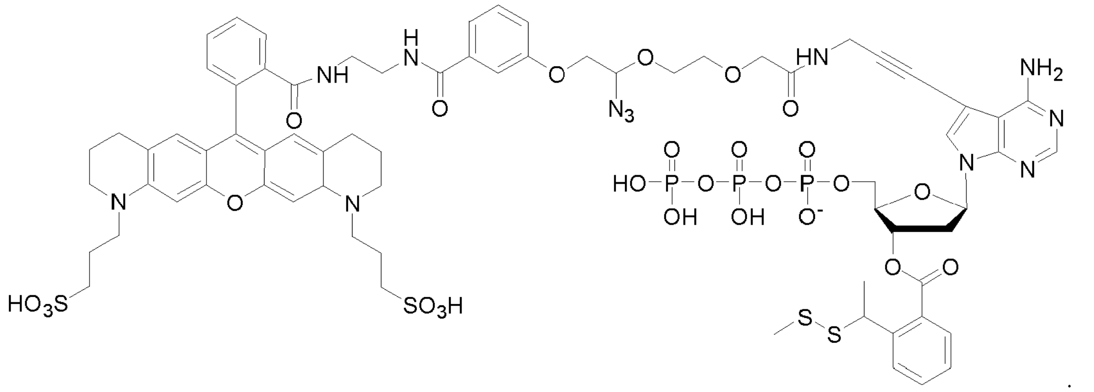

In some embodiments, the compound or a salt thereof carrying additional detectable label has the following structure:

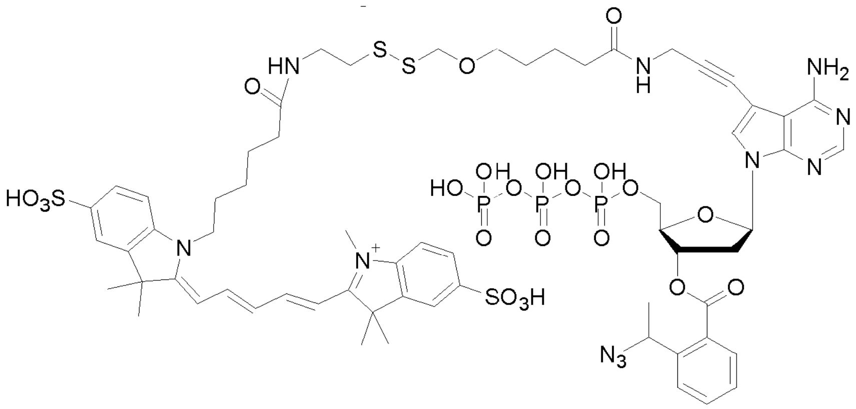

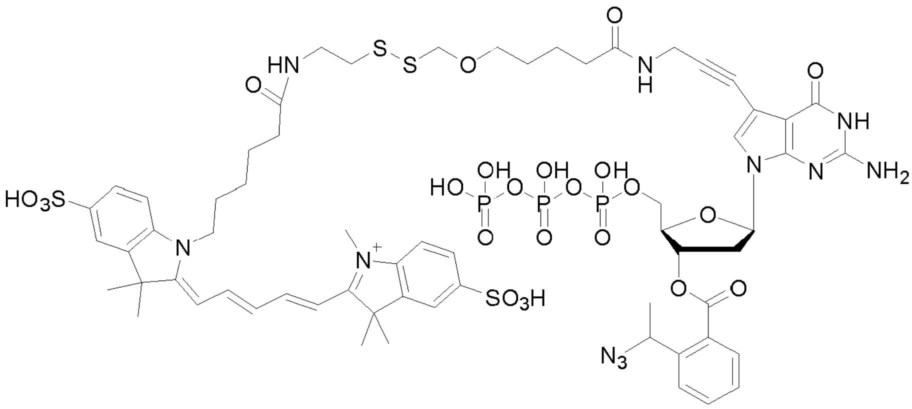

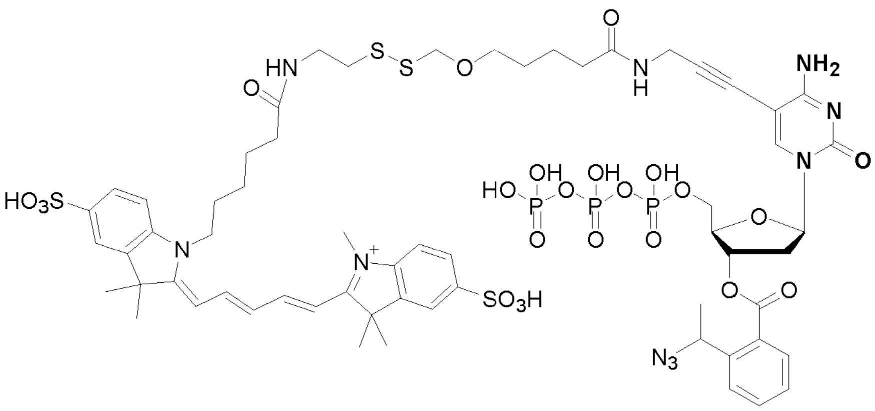

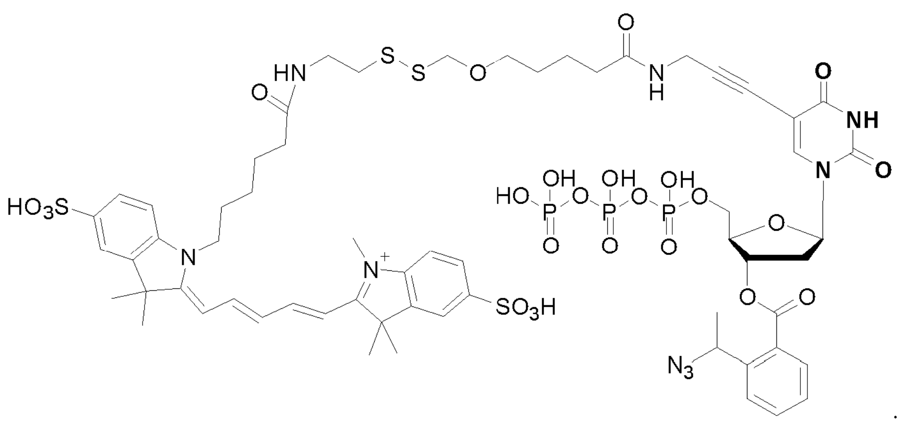

.

In the second aspect of the invention, the present invention provides a method for terminating a nucleic acid

synthesis, which comprises incorporating the compound or a salt thereof as described above into nucleic acid molecule to be terminated.

In some embodiments, the incorporation of the compound or a salt thereof is achieved by a terminal transferase, a terminal polymerase or a reverse transcriptase.

In some embodiments, the method comprises incorporating the compound or a salt thereof into the nucleic acid molecule to be terminated by using a polymerase.

In some embodiments, the method comprises performing the nucleotide polymerization reaction using a polymerase under conditions where the polymerase is allowed to carry out the nucleotide polymerization reaction, thereby incorporating the compound or a salt thereof into the 3' end of the nucleic acid molecule to be terminated.

In the third aspect of the invention, the present invention provides a method for preparing a growing polynucleotide complementary to a target single-stranded polynucleotide in a sequencing reaction, which comprises incorporating the compound or a salt thereof as described above into the growing complementary polynucleotide, wherein the incorporation of the compound or a salt thereof prevents any subsequent nucleotides from being introduced into the growing complementary polynucleotide.

In some embodiments, the incorporation of the compound or a salt thereof is achieved by a terminal transferase, a terminal polymerase or a reverse transcriptase.

In some embodiments, the method comprises incorporating compound or a salt thereof into the growing complementary polynucleotide by using a polymerase.

In some embodiments, the method comprises performing the nucleotide polymerization reaction using a polymerase under conditions where the polymerase is allowed to carry out the nucleotide polymerization reaction, thereby incorporating the compound or a salt thereof into the 3' end of the growing complementary polynucleotide.

In the fourth aspect of the invention, the present invention provides a method for determining the sequence of a target single-stranded polynucleotide, which comprises:

1) monitoring the sequential incorporation of nucleotides complementary to the target single-stranded polynucleotide in a growing nucleic acid strand, wherein at least one complementary nucleotide incorporated is the compound or a salt thereof as described above, and the compound or a salt thereof carries an additional detectable label (e.g., a fluorescent label), and,
2) detecting the detectable label.

In some embodiments, the additional detectable label (e.g., a fluorescent label) is linked to the compound or a salt thereof optionally via a linker.

In some embodiments, the linker is as described above.

In some embodiments, the additional detectable label is as described above.

In some embodiments, If the Base is different, the detectable label (e.g., a fluorescent label) carried by the compound or a salt thereof varies.

In some embodiments, prior to introducing a next complementary nucleotide, the reversible blocking group (R) and the detectable label in the compound or a salt thereof are removed.

In some embodiments, the reversible blocking group (R) and the detectable label are removed simultaneously.

In some embodiments, the reversible blocking group (R) and the detectable label are removed sequentially; for example, after the detectable label is removed, the reversible blocking group is removed, or, after the reversible blocking group is removed, the detectable label is removed.

In some embodiments, the method comprises the following steps:

(a) providing a plurality of different nucleotides, wherein the plurality of different nucleotides are the compound or a salt thereof as described above, wherein each nucleotide carries an additional detectable label that can be distinguished from an additional detectable label carried by another nucleotide during detection;
(b) incorporating the plurality of different nucleotides into sequence complementary to a target single-stranded polynucleotide;
(c) detecting the additional detectable labels carried by the nucleotides in step (b) to determine the types of nucleotides incorporated;
(d) removing the reversible blocking groups and the detectable labels carried by the nucleotides in step (b); and
(e) optionally repeating steps (b)-(d) one or more times;

whereby the sequence of the target single-stranded polynucleotide is determined.

In some embodiments, the method comprises the following steps:

(1)providing a first nucleotide, a second nucleotide, a third nucleotide and a fourth nucleotide, wherein at least one of

the four nucleotides is the compound or a salt thereof as described above, the Bases contained in the four nucleotides are different from each other, and the four nucleotides carry additional detectable label (e.g., a fluorescent label), preferably, the additional detectable label carried by the four nucleotides is introduced by an affinity reagent (e.g., antibody, aptamer, Affimer, Knottin), wherein the affinity reagent carries the detectable label, and the affinity reagent can specifically recognize and bind to an epitope of each nucleotide, or preferably, the four nucleotides are linked to the additional detectable label optionally via a linker, more preferably, the Base of the four nucleotides is linked to the additional detectable label optionally via a linker, most preferably, the additional detectable labels carried by the four nucleotides are different from each other;

(2)contacting the four nucleotides with a target single-stranded polynucleotide; removing the nucleotides that are not incorporated into the growing nucleic acid strand; detecting the detectable labels carried by the nucleotides that are incorporated into the growing nucleic acid strand; removing the reversible blocking groups and the detectable labels carried by the nucleotides that are incorporated into the growing nucleic acid strand;

optionally, it also includes (3): repeat the step (2) one or more times.

In some embodiments, the method comprises the following steps:

(a)providing a mixture comprising a duplex, at least one compound or a salt thereof as described above, polymerase and an excision reagent; wherein the duplex comprises growing nucleic acid strand and a nucleic acid strand to be sequenced; wherein the compound or a salt thereof carries an additional detectable label (e.g., a fluorescent label), preferably, the additional detectable label carried by the compound or a salt thereof is introduced by an affinity reagent (e.g., antibody, aptamer, Affimer, Knottin), wherein the affinity reagent carries the detectable label, and the affinity reagent can specifically recognize and bind to an epitope of the compound or a salt thereof, or preferably, the compound or a salt thereof is linked to the additional detectable label optionally via a linker, or more preferably, the Base of the compound or a salt thereof is linked to the additional detectable label optionally via a linker;

(b) carrying out a reaction comprising the following steps (i), (ii) and (iii), optionally, repeating the steps for one or more times:

step (i): incorporating the compound or a salt thereof into the growing nucleic acid strand using a polymerase to form a nucleic acid intermediate containing the reversible blocking group and the detectable label;

step (ii): detecting the detectable label contained in the nucleic acid intermediate;

step (iii): removing the reversible blocking group and/or the detectable label contained in the nucleic acid intermediate by using the excision reagent.

In some embodiments, the removal of the reversible blocking group and the removal of the detectable label are performed simultaneously, or, the removal of the reversible blocking group and the removal of the detectable label are performed sequentially (for example, the reversible blocking group is removed first, or the detectable label is removed first).

In some embodiments, the excision reagent used for the removal of the reversible blocking group is the same as that used for the removal of the detectable label.

In some embodiments, the excision reagent used for the removal of the reversible blocking group is different from that used for the removal of the detectable label.

In some embodiments, the duplex is linked to a support.

In some embodiments, the growing nucleic acid strand is a primer.

In some embodiments, the primer is annealed to the nucleic acid strand to be sequenced to form the duplex.

In some embodiments, the duplex, the compound or a salt thereof, and the polymerase together form a reaction system containing a solution phase and a solid phase.

In some embodiments, the Bases contained in the compounds or salts thereof are different from each other.

In some embodiments, the additional detectable label carried by the compound or a salt thereof are different from each other.

In some embodiments, the compound or a salt thereof is incorporated into the growing nucleic acid strand using a polymerase under conditions where the polymerase is allowed to carry out a nucleotide polymerization reaction, thereby forming a nucleic acid intermediate containing reversible blocking group and the detectable label.

In some embodiments, the polymerase is selected from KOD polymerase or its mutants thereof (e.g., KOD POL151, KOD POL157, KOD POL171, KOD POL174, KOD POL376, KOD POL391).

In some embodiments, before any step of detecting the detectable label contained in the nucleic acid intermediate, the solution phase of the reaction system in the previous step is removed, and the duplex linked to the support is retained.

In some embodiments, the excision reagent is in contact with the duplex or the growing nucleic acid strand in the

reaction system containing a solution phase and a solid phase.

In some embodiments, the excision reagent can remove the reversible blocking group and the additional detectable label carried by the compound that is incorporated into the growing nucleic acid strand, without affecting the phosphodiester bond on the backbone of the duplex.

In some embodiments, after any step of removing the reversible blocking group and/or additional detectable label contained in the nucleic acid intermediate, the solution phase of the reaction system in this step is removed.

In some embodiments, a washing operation is performed after any step comprising a removal operation.

In some embodiments, after step (ii), the method further comprises: determining the type of compound incorporated into the growing nucleic acid strand in step (i) according to the signal detected in step (ii), and determining the type of nucleotide at a corresponding position in the nucleic acid strand to be sequenced based on the principle of base complementary pairing.

In the fifth aspect of the invention, the present invention provides a kit comprising at least one compound or a salt thereof as described above.

In some embodiments, the kit comprises a first compound, a second compound, a third compound and a fourth compound, wherein the first, second, third and fourth compounds are each independently the compound or a salt thereof as described above.

In some embodiments, in the first compound, the Base is selected from adenine, 7-deazaadenine or a tautomer thereof (e.g.,

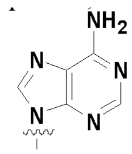

); in the second compound, the Base is selected from thymine, uracil or a tautomer thereof (e.g.,

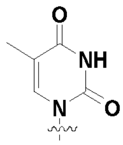

); in the third compound, the Base is selected from cytosine or a tautomer thereof (e.g.,

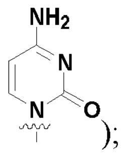

);

in the fourth compound, the Base is selected from guanine, 7-deazaguanine or a tautomer thereof (e.g.,

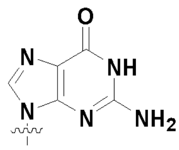

).

In some embodiments, the first, second, third and fourth compounds carry an additional detectable label.

In some embodiments, the additional detectable labels carried by the first, second, third and fourth compounds are introduced by an affinity reagent (e.g., antibody, aptamer, Affimer, Knottin), wherein the affinity reagent carries the detectable label, and the affinity reagent can specifically recognize and bind to the epitope of the first, second, third or fourth compound.

In some embodiments, the first, second, third and fourth compounds are linked to the additional detectable label optionally via a linker.

In some embodiments, the Base of the first, second, third or fourth compound is linked to the additional detectable

label optionally via a linker.

In some embodiments, the Bases contained in the first, second, third and fourth compounds are different from each other.

In some embodiments, the additional detectable labels carried by the first, second, third and fourth compounds are different from each other.

In some embodiments, the linker is as described above.

In some embodiments, the detectable label is as described above.

In some embodiments, the kit further comprises an reagent for pretreating the nucleic acid molecule; a support for linking nucleic acid molecule to be sequenced; a reagent for linking the nucleic acid molecule to be sequenced to the support (for example, covalently or non-covalently linking); a primer for initiating a nucleotide polymerization reaction; a polymerase for carrying out the nucleotide polymerization reaction; one or more buffer solutions; one or more washing solutions; or any combination thereof.

In the sixth aspect of the invention, the present invention provides the use of the compound or a salt thereof as described above or the kit as described above for determining the sequence of a target single-stranded polynucleotide.

Brief description of drawings

Figure 1 illustrates the reaction for removing azidomethyl, as an example of the present invention;

Figure 2 illustrates the nucleophilic cyclization cascade reaction for the reversible cleavage, as an example of the present invention;

Figure 3 illustrates reversible blocking nucleotide analogues with 3'-OH labels, as an example of the present invention.

Detailed description of the invention

The embodiments of the invention are described in detail below through specific embodiments, but in no case should they be interpreted as restrictions on the invention.

Unless otherwise specified, the above groups and substituents have common meanings in the field of pharmaceutical chemistry.

In various parts of the specification, the substituents of the compounds disclosed in the invention are disclosed according to the type or range of the group. It is particularly noted that the present invention includes each independent subcombination of each and every member of these group types and ranges. For example, the term "$C_1$-$C_6$ alkyl" particularly refers to independently disclosed methyl, ethyl, $C_3$ alkyl, $C_4$ alkyl, $C_5$ alkyl and $C_6$ alkyl.

In addition, it should be noted that, unless otherwise clearly pointed out, the expressions "each... independently is/are selected from" and "... are each independently is/are selected from", as used throughout the description, are interchangeable and both should be interpreted in a broad sense, i.e. it can mean that, in different groups, the specific options expressed by the same or different symbols are independent from each other, or it can mean that, in the same group, the specific options expressed by the same or different symbols are independent from each other.

The term "aliphatic alkyl " means any straight or branched saturated group containing from 1 to 20 carbon atoms, for example, $C_1$-$C_{12}$ alkyl, preferably $C_1$-$C_6$ alkyl.

The term "$C_1$-$C_6$ alkyl" means any straight or branched saturated group containing from 1 to 6 carbon atoms, such as, methyl, ethyl, n-propyl, isopropyl, n-butyl, isobutyl, tertbutyl, sec-butyl, n-amyl, tert-amyl, n-hexyl, etc.

The term "alkoxy" means any above-described alkyl (e.g., $C_1$-$C_6$ alkyl, etc.), which is connected to the rest of the molecule through the oxygen atom (-O-).

The term "cycloalkyl" means an saturated cyclic hydrocarbyl of 3-10 membered monocyclic ring system, for example, $C_3$-$C_8$ cycloalkyl, preferably $C_3$-$C_6$ cycloalkyl.

The term "$C_3$-$C_6$ cycloalkyl" means an saturated cyclic hydrocarbyl of 3-6 membered monocyclic ring system. $C_3$-$C_6$ cycloalkyl may be cyclopropyl, cyclobutyl, cyclopentyl, cyclohexyl, etc.

The term "aromatic alkyl" means an arylalkyl or heteroarylalkyl, wherein the alkyl is as defined above.

The term "heteroaryl" means an aromatic heterocycle, generally a 5-, 6-, 7-, or 8-memberd heterocycle containing from one to three heteroatoms selected from N, O or S; and the heteroaryl ring may optionally be further fused to an aromatic or nonaromatic carbon ring or heterocycle. The non-limiting examples of the heteroaryl are such as pyridyl, pyrazinyl, pyrimidinyl, pyridazinyl, indolyl, imidazolyl, thiazolyl, isothiazolyl, thioxazolyl, pyrrolyl, phenyl-pyrrolyl, furanyl, phenyl-furanyl, oxazolyl, isoxazolyl, pyrazolyl, thiophenyl, benzofuranyl, benzothiophenyl, benzo-1,3-dioxolane (benzodioxane), isodihydroindolyl, benzimidazolyl, indazolyl, quinolinyl, isoquinolinyl, 1,2,3-triazolyl, 1-phenyl-1,2,3-triazolyl, 2,3-dihydroindolyl, 2,3-dihydrobenzofuranyl, 2,3-dihydrobenzothiophenyl, benzopyranyl, 2,3-dihydrobenzoxazinyl, 2,3-dihydroquinoxalinyl, etc.

In the present invention,

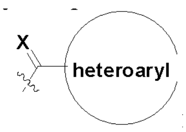

refers to group

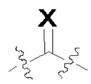

connected to a heteroaryl. Other similar structures can be correspondingly understood with reference to the above contents.

The term "aryl" means carbon-ring aromatic group having 6-14 carbon atoms, such as, $C_6$-$C_{10}$ aryl, preferably phenyl.

It is obvious to those skilled in the art from all the above descriptions that, any group having a combined name, such as "phenyl $C_1$-$C_6$ alkyl", should be understood as being conventionally constructed from the parts from which it is derived, such as constructed from $C_1$-$C_6$ alkyl substituted by phenyl, wherein the $C_1$-$C_6$ alkyl group is as defined above.

As used herein, the term " salt of the compound of formula (A)" may be exemplified by those organic addition salts of the organic acids forming the anion, including but not limiting to formate, acetate, propionate, benzoate, maleate, fumarate, succinate, tartrate, citrate, ascorbate, $\alpha$-ketoglutarate, $\alpha$-glycerophosphate, alkyl sulfonate or aryl sulfonate; preferably, the alkyl sulfonate is methanesulfonate or ethanesulfonate; the aryl sulfonate is benzenesulfonate or p-toluenesulfonate. Or alternatively, the term also means those inorganic salts, including but not limiting to hydrochloride, hydrobromide, hydroiodide, nitrate, bicarbonate and carbonate, sulfate or phosphate, etc.

For the compound of formula (B)

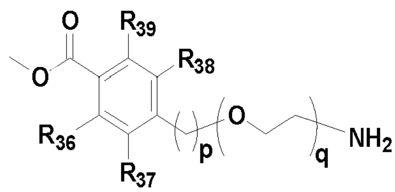

of the invention, the expression "$R^{36}$ is $N_3$-$C_1$-$C_6$ alkyl, and wherein the $C_1$-$C_6$ alkyl is optionally substituted by $C_1$-$C_6$ alkyl", it means that, $R^{36}$ is $N_3$-$C_1$-$C_6$ alkyl, and the hydrogen atoms on $C_1$-$C_6$ alkyl may further be replaced by other $C_1$-$C_6$ alkyl. For example, when $R^{36}$ is $N_3$-methyl, and the methyl is optionally substituted by methyl, the compound of formula (B) may have the following structure

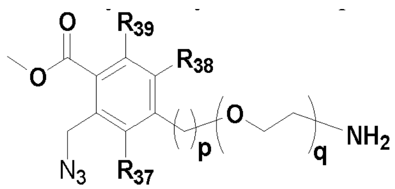

,

or

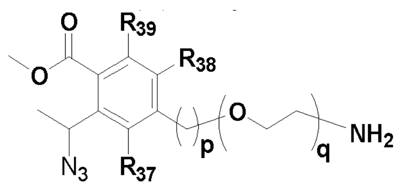

.

Other similar expressions may be correspondingly understood with reference to the above contents.

In the method of the invention, any substance composed of two chains, namely, the growing nucleic acid chain and the nucleic acid chain to be sequenced, may be called " a duplex ", regardless of the length of the growing nucleic acid chain or the nucleic acid chain to be sequenced, and wherein the nucleic acid chain to be sequenced may be longer than the length of the growing nucleic acid chain.

In the method of the invention, the nucleic acid molecule to be sequenced may be any target nucleic acid molecule. In some preferred embodiments, the nucleic acid molecules to be sequenced include deoxyribonucleotides, ribonucleotides, modified deoxyribonucleotides, modified ribonucleotides, or any combination thereof. In the method of the invention, the nucleic acid molecule to be sequenced is not limited to any type. In some preferred embodiments, the nucleic acid molecule to be sequenced is DNA or RNA. In some preferred embodiments, the nucleic acid molecules to be sequenced may be genomic DNA, mitochondrial DNA, chloroplast DNA, mRNA, cDNA, miRNA, or siRNA. In some preferred embodiments, the nucleic acid molecule to be sequenced is linear or circular. In some preferred embodiments, the nucleic acid molecule to be sequenced is double-stranded or single-stranded. For example, the nucleic acid molecule to be sequenced may be single-stranded DNA (ssDNA), double-stranded DNA (dsDNA), single-stranded RNA (ssRNA), double-stranded RNA (dsRNA), or a hybrid of DNA and RNA. In some preferred embodiments, the nucleic acid molecule to be sequenced is single-stranded DNA. In some preferred embodiments, the nucleic acid molecule to be sequenced is double-stranded DNA.

In the method of the invention, the nucleic acid molecule to be sequenced is not limited by its source. In some preferred embodiments, the nucleic acid molecules to be sequenced may be obtained from any source, such as any cell, tissue or organism (e.g., viruses, bacteria, fungi, plants and animals). In some preferred embodiments, the nucleic acid molecules to be sequenced originate from mammals (e.g., humans, non-human primates, rodents or canines), plants, birds, reptiles, fish, fungi, bacteria or viruses.

The methods for extracting or obtaining nucleic acid molecules from cells, tissues or organisms are well known to those skilled in the art. Suitable methods include but are not limited to such as ethanol precipitation and chloroform extraction. For a detailed description of such methods, see, for example, J. Sambrook et al., Molecular Cloning: A Laboratory Manual, 2nd Edition, Cold Spring Harbor Laboratory Press, 1989; and F.M. Ausubel et al., Short Protocols in Molecular Biology, 3rd Edition, John Wiley&Sons, Inc., 1995. In addition, various commercial kits may be used for extracting nucleic acid molecules from various sources (e.g., cells, tissues or organisms).

In the method of the invention, the nucleic acid molecule to be sequenced is not limited by its length. In some preferred embodiments, the length of the nucleic acid molecule to be sequenced may be at least 10bp, at least 20bp, at least 30bp, at least 40bp, at least 50bp, at least 100bp, at least 200bp, at least 300bp, at least 400bp, at least 500bp, at least 1000bp, or at least 2000bp. In some preferred embodiments, the length of nucleic acid molecules to be sequenced may be 10-20bp, 20-30bp, 30-40bp, 40-50bp, 50-100bp, 100-200bp, 200-300bp, 300-400bp, 400-500bp, 500-1000bp, 1000-2000bp, or more than 2000bp. In some preferred embodiments, the nucleic acid molecule to be sequenced can have a length of 10-1000bp, to facilitate high-throughput sequencing.

In the method for polynucleotides preparation or sequencing of the invention, suitable polymerase may be used for performing nucleotide polymerization reaction. In some exemplary embodiments, the polymerase can synthesize new DNA strands using DNA as a template (such as, a DNA polymerase) . In some exemplary embodiments, the polymerase can synthesize new DNA strands using RNA as a template (e.g., a reverse transcriptase). In some exemplary embodiments, the polymerase can synthesize new RNA chains using DNA or RNA as templates (e.g., a RNA polymerase). Therefore, in some preferred embodiments, the polymerase is selected from DNA polymerase, RNA polymerase, and reverse transcriptase. Appropriate polymerase can be selected according to actual needs to carry out nucleotide polymerization reaction. In some preferred embodiments, the polymerization reaction is a polymerase chain reaction (PCR). In some preferred embodiments, the polymerization reaction is a reverse transcription reaction.

In the method of the present invention, KOD polymerase or its mutants may be used for the nucleotide polymerization reaction. KOD polymerase or its mutants (e.g., KOD POL151, KOD POL157, KOD POL171, KOD POL174, KOD POL376, KOD POL391) may lead to acceptable Incorporation efficiency for the modified nucleoside or nucleotide of the invention. KOD POL391 and KOD POL171 lead to acceptable Incorporation efficiency for the modified nucleotides of the invention. In some embodiments, the Incorporation efficiency of KOD POL391 or KOD POL171 for the modified nucleotides of the invention is more than 70%, such as 70% - 80%, 80% - 90% or 90% - 100%.

In the method for polynucleotide preparation or sequencing of the invention, the polymerization reaction of nucleotides is carried out under suitable conditions. The suitable polymerization conditions include the composition of the solution phase, the concentration of each component, the pH of the solution phase, and the polymerization temperature. Polymerization is performed under suitable conditions to obtain acceptable and even higher Incorporation efficiency.

In the present invention, the hydroxyl (-OH) at 3'-position of the deoxyribose in the compound of formula (A) is protected (by R), thereby can terminate the polymerization caused by polymerase (such as DNA polymerase). For example, when the compound of formula (A) is introduced into the 3'-end of the growing nucleic acid chain, because there is no free hydroxyl (-OH) at the 3'-position of the deoxyribose of the compound, the polymerase cannot proceed to the next round of polymerization, and the polymerization is terminated. In such case, only one base will be incorporated into the

growing nucleic acid chain in each round of polymerization.

In addition, the protective group (R) of the hydroxyl (-OH) at the 3'-position of the deoxyribose in the compound of formula (A) can be removed to restore the free hydroxyl (-OH). Then the growing nucleic acid chain may undergo next round of polymerization reaction using polymerase and the compound of formula (A) to incorporate therein a base again.

That is, the hydroxyl (-OH) at the 3'-position of the deoxyribose in the compound of formula (A) is reversibly blocked: when the compound of formula (A) is incorporated into the 3' end of the growing nucleic acid chain, it will terminate the polymerization caused by the polymerase and stop any further extension of the growing nucleic acid chain; and, after the blocking group contained in the compound of formula (A) is removed, the polymerase can continue the polymerization of the growing nucleic acid chain to extend the nucleic acid chain.

Some embodiments described herein relate to the use of conventional detectable labels. Detection may be realized by any suitable method, including fluorescence spectroscopy or other optical means. Preferred label is a fluorescent label, namely a fluorophore, which emits radiation of certain wavelength after absorbing energy. Many suitable fluorescent labels are known in the art. For example, Welch et al., (Chem. Eur. J. 5(3):951-960,1999) discloses dansyl-functionalised fluorescent moieties that can be used in the present invention. Zhu et al. (Cytometry 28:206-211, 1997) describes the use of the fluorescent labels Cy3 and Cy5, which can also be used in the present invention. Labels suitable for use are also disclosed in Prober et al. (Science 238:336-341, 1987); Connell et al. (BioTechniques 5(4):342-384, 1987), Ansorge et al. (Nucl. Acids Res. 15(11):4593-4602, 1987) and Smith et al. (Nature 321:674, 1986). Other commercially available fluorescent labels include, but are not limited to, fluorescein, rhodamine (including TMR, texas red and Rox), alexa, BODIPY, acridine, coumarin, pyrene, benzanthracene and the cyanins.

Multiple labels can also be used in the present application, for example, bi-fluorophore FRET cassettes (Tet. Let. 46:8867-8871, 2000). Multi-fluor dendrimeric systems (J. Am. Chem. Soc. 123:8101-8108, 2001) can also be used. Although fluorescent labels are preferred, other forms of detectable labels will be apparent as useful to those of ordinary skill in the art. For example, microparticles, including quantum dots (Empodocles et al., Nature 399:126-130, 1999), gold nanoparticles (Reichert et al., Anal. Chem. 72:6025-6029, 2000) and microbeads (Lacoste et al., Proc. Natl. Acad. Sci USA 97(17):9461-9466, 2000) can all be used.

Multi-component labels can also be used in the present application. A multi-component label is one which is dependent on the interaction with a further compound for detection. The most common multi-component label used in biology is the biotin-streptavidin system. Biotin is used as the label attached to the nucleotide or modified nucleotide. Streptavidin is then added separately to enable detection to occur. Other multi-component systems can be used. For example, dinitrophenol has a commercially available fluorescent antibody that can be used for detection.

In some embodiments described herein, carrying the detectable labels described above on the modified nucleotides or nucleoside molecules can be achieved through the incorporation of affinity reagents (e.g., antibodies, aptamers, Affimer, and Knottin), wherein the affinity reagent can specifically recognize and bind to the epitopes of the modified nucleotides or nucleoside molecules. The specific principle is shown in WO2018129214A1.

In other embodiments described herein, the modified nucleotide or nucleoside molecule may be linked to a detectable label described above. In some such embodiments, the linker used may be cleaved. The use of cleavable linker ensures that the labels can be removed as needed after the detection, and thus avoiding any interference signals from any subsequently added labeled nucleotides or nucleosides.

In other embodiments, the linker used is not cleavable. Because in each circumstance where the labeled nucleotide of the present invention is incorporated, it is not necessary to subsequently incorporate any nucleotide, so it is not necessary to remove the label from the nucleotide.

Those skilled in the art will be aware of the utility of dideoxynucleoside triphosphates in Sanger sequencing methods, and related protocols (Sanger-type), which rely upon randomized chain-termination at particular type of nucleotide. The nucleotides of the present application, it will be recognized, may be of utility in Sanger methods and related protocols since the same effect achieved by using ddNTPs may be achieved by using the 3'-OH protecting groups described herein: both prevent incorporation of subsequent nucleotides. Similarly, the modified nucleosides and nucleotides of the present application may be of utility in high-throughput sequencing, especially in the high-throughput sequencing platform based on sequencing by synthesis. By using the reversible blocking nucleotides and analogues of the present application, the types of incorporated nucleotides may be determined one by one during the sequencing process.

Cleavable linkers are known in the art, and conventional chemistry can be applied to attach a linker to a nucleotide or modified nucleotide and a label. The linker can be cleaved by any suitable method, including exposure to acids, bases, nucleophiles, electrophiles, radicals, metals, reducing or oxidizing agents, light, temperature, enzymes etc. The linker as discussed herein may also be cleaved with the same catalyst used to cleave the 3'-O-protecting group bond. Suitable linkers can be adapted from standard chemical protecting groups, as disclosed in Greene & Wuts, Protective Groups in Organic Synthesis, John Wiley & Sons. Further suitable cleavable linkers used in solid-phase synthesis are disclosed in Guillier et al. (Chem. Rev. 100:2092-2157, 2000).

The use of the term "cleavable linker" is not meant to imply that the whole linker is required to be removed from, e.g., the nucleotide or modified nucleotide. Where the detectable label is attached to the nucleotide or modified nucleotide,

the nucleoside cleavage site can be located at a position on the linker that ensures that part of the linker remains attached to the nucleotide or modified nucleotide after cleavage.

Where the detectable label is attached to the nucleotide or modified nucleotide, the linker can be attached at any position on the nucleotide or modified nucleotide provided that Watson-Crick base pairing can still be carried out.

A. Linkers Capable Of Being Cleaved By Electrophilic Reaction

Linkers capable of being cleaved by electrophilic reaction are typically cleaved by protons and include cleavages sensitive to acids. Suitable linkers include the modified benzylic systems such as trityl, p-alkoxybenzyl esters and p-alkoxybenzyl amides. Other suitable linkers include tert-butyloxycarbonyl (Boc) groups and the acetal system.

The use of thiophilic metals, such as nickel, silver or mercury, in the cleavage of thioacetal or other sulfur-containing protecting groups can also be considered for the preparation of suitable linker molecules.

B. Linkers Capable Of Being Cleaved By Nucleophilic Reaction

Nucleophilic cleavage is also a well recognised method in the preparation of linker molecules. Groups such as esters that are labile in water (i.e., can be cleaved simply at basic pH) and groups that are labile to non-aqueous nucleophiles, can be used. Fluoride ions can be used to cleave silicon-oxygen bonds in groups such as triisopropyl silane (TIPS) or t-butyldimethyl silane (TBDMS).

C. Linker Capable Of Being Cleaved By Photolysis

Linkers capable of being cleaved by photolysis have been used widely in carbohydrate chemistry. It is preferable that the light required to activate cleavage does not affect the other components of the modified nucleotides. For example, if a fluorophore is used as the label, it is preferable if this absorbs light of a different wavelength to that required to cleave the linker molecule. Suitable linkers include those based on O-nitrobenzyl compounds and nitroveratryl compounds. Linkers based on benzoin chemistry can also be used (Lee et al., J. Org. Chem. 64:3454-3460, 1999).

D. Linkers Capable Of Being Cleaved Under Reductive Conditions

There are many linkers known that are susceptible to reductive cleavage. Catalytic hydrogenation using palladium-based catalysts has been used to cleave benzyl and benzyloxycarbonyl groups. Disulfide bond reduction is also known in the art.

E. Linkers Capable Of Being Cleaved Under Oxidative Conditions

Oxidation-based approaches are well known in the art. These include oxidation of p-alkoxybenzyl groups and the oxidation of sulfur and selenium linkers. The use of aqueous iodine to cleave disulfides and other sulfur or selenium-based linkers is also within the scope of the invention.

F. Safety-Catch linkers

Safety-catch linkers are those that cleave in two steps. In a preferred system the first step is the generation of a reactive nucleophilic center followed by a second step involving an intra-molecular cyclization that results in cleavage. For example, levulinic ester linkages can be treated with hydrazine or photochemistry to release an active amine, which can then be cyclised to cleave an ester elsewhere in the molecule (Burgess et al., J. Org. Chem. 62:5165-5168, 1997).

G. Linker Capable Of Being Cleaved By Elimination Mechanisms

Elimination reactions can also be used. For example, the base-catalysed elimination of groups such as Fmoc and cyanoethyl, and palladium-catalysed reductive elimination of allylic systems, can be used.

In some embodiments, the linker can comprise a spacer unit. The length of the linker is unimportant provided that the label is held a sufficient distance from the nucleotide so as not to interfere with any interaction between the nucleotide and an enzyme.

In some embodiments, the linker may consist of the similar functionality as the 3'-OH protecting group. This will make the deprotection and deprotecting process more efficient, as only a single treatment will be required to remove both the label and the protecting group. Particularly preferred linkers are phosphine-cleavable azide containing linkers.

The present invention will be further explained below in conjunction with specific examples. Unless otherwise

specified, the reagents used in the following examples can be commercially available. In addition, for each modification of 3'-OH, if one or more of the nucleotide analogues, dTTP, dATP, dCTP and dGTP, are prepared, those skilled in the art can undoubtedly prepare and obtain the remaining one or several nucleotide analogues.

I. Preparation Examples

Example 1

The structure of the target compound is:

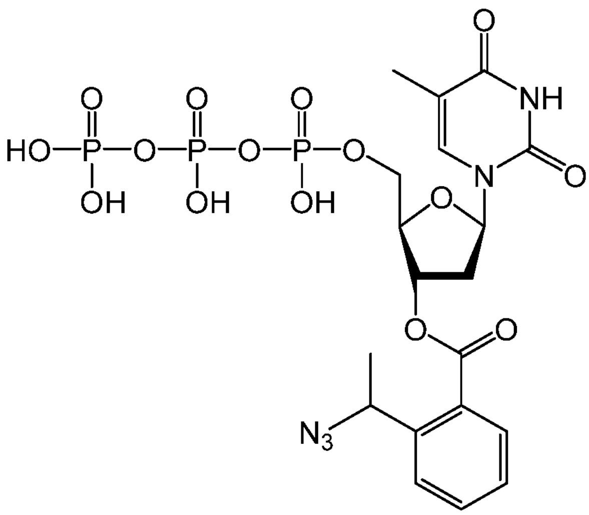

(1) Step 1

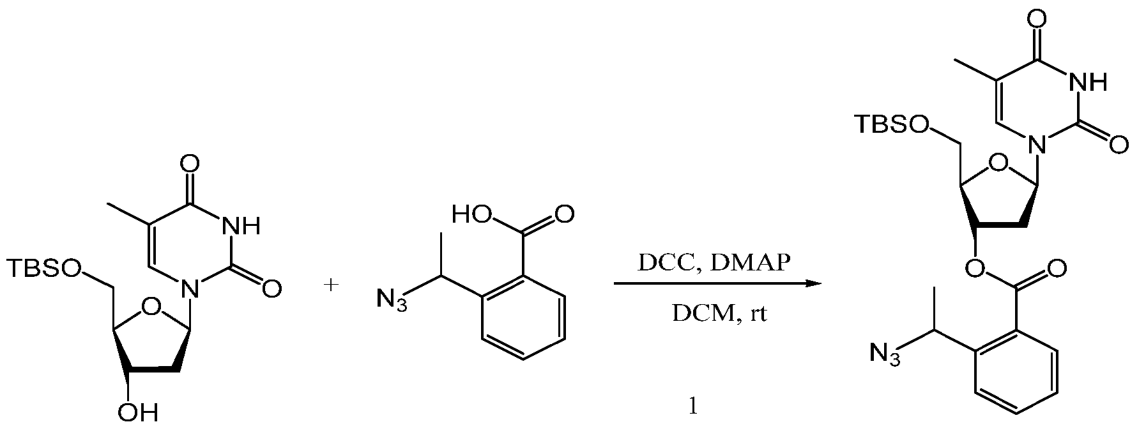


1

To a dried 100mL round bottom flask equipped with a magnetic stirrer and a rubber stopper with argon balloon, were added T-nucleoside (500mg, 1.4mmol, 1eq) (available from Beijing OKeanos Tech. Co., Ltd., cat. no. OK-N-18102) and azido protection group (268mg, 1.4mmol, 1eq) (available from Beijing OKeanos Tech. Co., Ltd., cat. no. OK-H-20001) separately, followed by adding with syringe 20mL of anhydrous methylene chloride as solvent. The reaction mixture was stirred at room temperature to facilitate dissolution, followed by adding DCC (435mg, 2. 1mmol, 1.5eq) and DMAP (20mg, 0.14mmol, 0.1eq) separately, and allowed to react under nitrogen balloon for providing protection atmosphere at room temperature for further 4h. The reaction was monitored with TLC at half an hour interval, until the starting materials were substantially depleted. The reaction mixture was concentrated with a rotary evaporator, followed by product separation by silica gel column chromatography (Hexane/EA=10/1~1/1, silica gel column diameter 3.5cm, filler height 10cm). The yield of this step was 95%.
$^1$H NMR (400 MHz, $CDCl_3$) δ 8.41 (1H, s); 7.97(1H, m); 7.61 (3H, m); 7.40 (1H, m); 7.27(1H, s); 6.45 (1H, m); 5.67 (1H, m); 5.50 (1H, m); 4.28 (1H, m); 4.02 (1H, m); 2.59 (1H, m); 2.24 (1H, m); 1.96 (3H, s); 1.55 (3H, d, $J$ = 6.8 Hz); 0.97 (9H, s); 0.10

(6H, s).

(2) Step 2

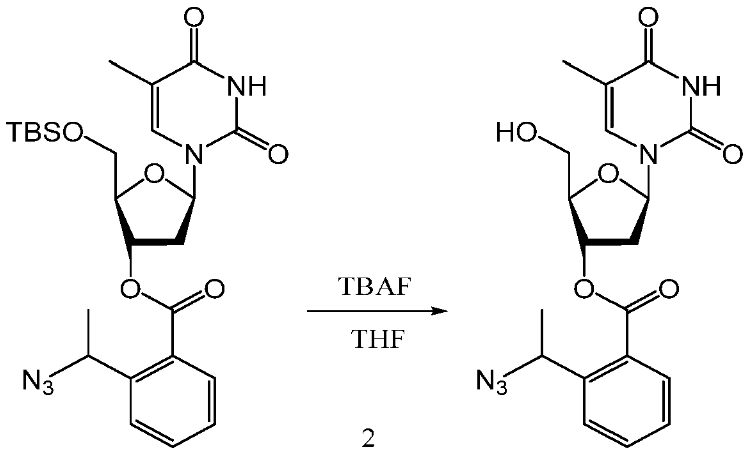


2

To a dried 100mL round bottom flask equipped with a magnetic stirrer and a rubber stopper with argon balloon was added the product from Step 1 (750mg, 1.4mmol, 1eq), followed by adding with syringe 20mL of anhydrous THF as solvent. The reaction mixture was stirred to facilitate dissolution, followed by slowly adding with syringe 1mol/L solution of TBAF (1.8mL, 1.8mmol, 1.3eq) dropwise while in ice bath. The flask was brough to room temperature and stirred continuously for about 2h. The reaction was monitored with TLC at half an hour interval, until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 70%.
$^1$H NMR (400 MHz, DMSO-$d_6$) δ 11.34 (1H, s); 7.89 (1H, m); 7.78 (1H, s); 7.70 - 7.62 (2H, m); 7.50- 7.46 (1H, m); 6.27 - 6.24 (1H, m); 5.58 -5.55 (1H, m), 5.26 (1H, t); 4.19 (1H, br.); 3.71 (2H, br.); 1.79 (3H, d); 1.49 (3H, d, $J$= 6.8 Hz).

(3) Step 3

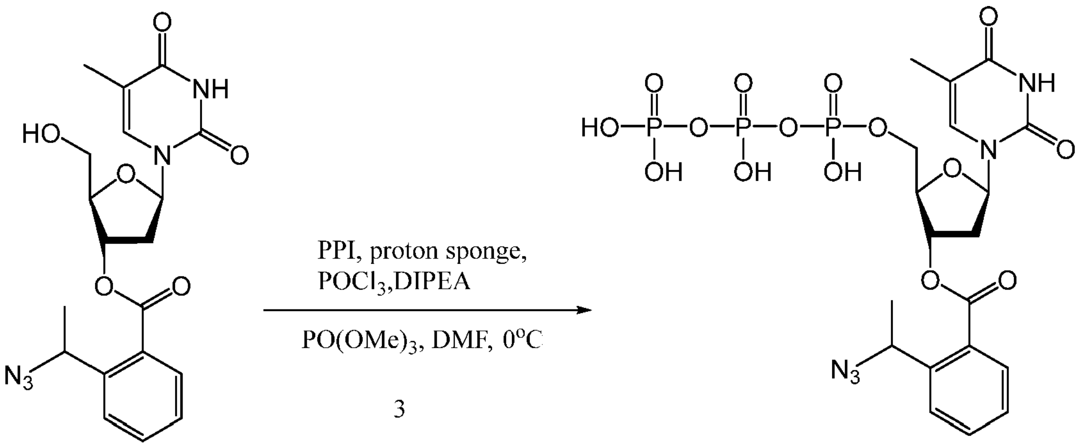


3

To a dried 100mL two-necked 1# flask equipped with a magnetic stirrer and a three-way valve with argon balloon, were added product from Step 2 (350mg, 0.85mmol, 1eq) and proton sponge (363.5mg, 1.7mmol, 2eq). To a dried 100mL two-necked 2# flask equipped with a magnetic stirrer and a three-way valve with argon balloon was added tributyl ammonium pyrophosphate (932.5mg, 1.7mmol, 2eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120μL , 1.3mmol, 1.5eq) and stirring continuously at 0°C for 1.5h. The two-necked 2# flask was filled with argon, followed by adding with syringe 2mL of anhydrous DMF and DIPEA (740μL, 4.25mmol, 5eq) with stirring at 0°C to facilitate dissolution. The liquid in 1# flask was transferred with syringe into the 2# flask. The mixture was maintained at 0°C and continuously stirred for 3h, followed by adding 20 mL of solution of TEAB (0.1mol/L) to quench the reaction. The reaction mixture was diluted with 50mL of deionized water, and then separated by DEAE resin column prepared-in-advance and eluted with gradient elution ($H_2O$:TEAB(1mol/L)=10/1~0/1, DEAE column diameter 4.5cm, filler height 8cm). Fractions were identified with HPLC and MS, and those fractions containing the triphosphorylated product were collected and concentrated under vacuum to 10mL, followed by preparative liquid chromatography separation with gradient elution ($CH_3CN$:TEAB(0.1mol/L)=2/98~98/2). The high-purity product solution recovered from the separation was concentrated with a rotary evaporator under vacuum, transferred to a plastic centrifuge tube, and then freeze-dried with a lyophilizer into a powder solid. MW=655. The yield of this step was 50%.

$^1$H NMR (400 MHz, DMSO-$d_6$) δ 11.32 (s, 1H), 7.97 (s, 1H), 7.93 - 7.88 (m, 1H), 7.72 - 7.60 (m, 2H), 7.52 - 7.46 (m, 1H), 6.40 - 6.29 (m, 1H), 5.64 - 5.54 (m, 2H), 4.33 (s, 1H), 4.17 - 4.01 (m, 2H), 2.54 - 2.50 (m, 1H), 2.45 - 2.36 (m, 1H), 1.85 (s, 3H), 1.49 (dd, *J*= 6.7, 1.0 Hz, 3H).

Example 2

The structure of the target compound is:

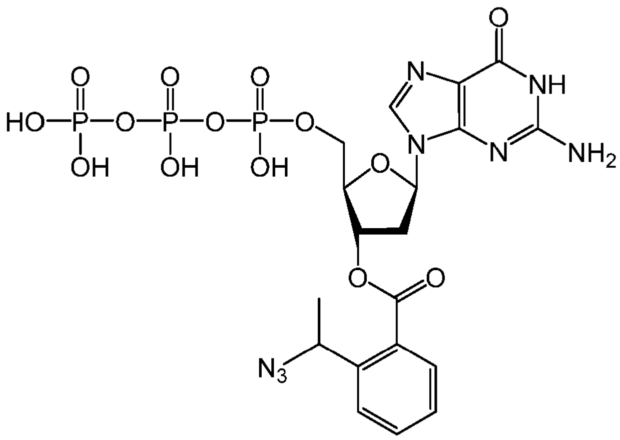

(1) Step 1

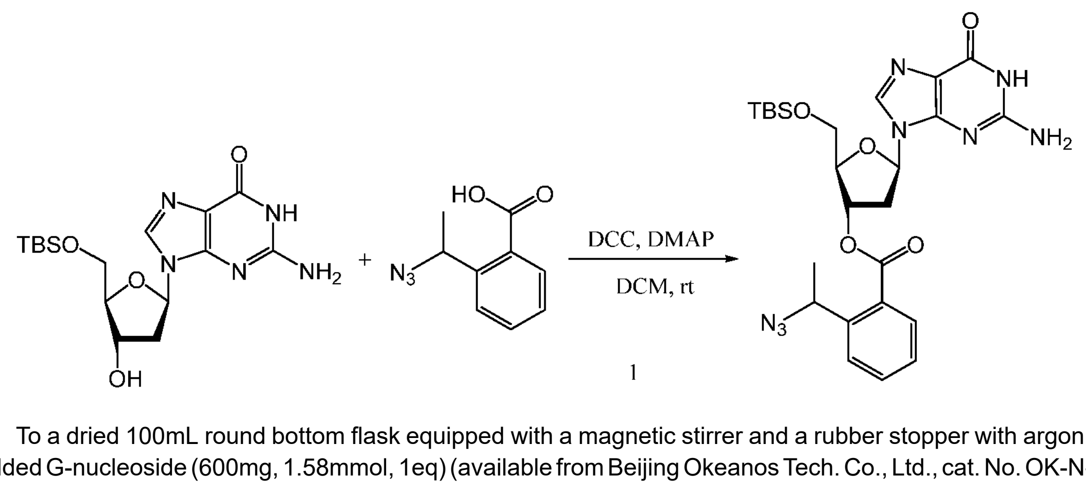

1

To a dried 100mL round bottom flask equipped with a magnetic stirrer and a rubber stopper with argon balloon were added G-nucleoside (600mg, 1.58mmol, 1eq) (available from Beijing Okeanos Tech. Co., Ltd., cat. No. OK-N-18103) and azido protection group (302mg, 1.58mmol, 1eq) (available from Beijing Okeanos Tech. Co., Ltd., cat. No. OK-H-20001) separately, followed by adding with syringe 20mL of anhydrous methylene chloride as solvent. The reaction mixture was stirred at room temperature to facilitate dissolution, followed by adding DCC (488mg, 2.37mmol, 1.5eq) and DMAP (20mg, 0.16mmol, 0.1eq) separately under nitrogen balloon for providing protection atmosphere, and allowed to react at room temperature for further 4h. The reaction was monitored with TLC at half an hour interval, until the starting

materials were substantially depleted. The reaction mixture was concentrated with a rotary evaporator, followed by product separation by silica gel column chromatography (Hexane/EA=10/1~1/1, silica gel column diameter 3.5cm, filler height 10cm). The yield of this step was 95%.
$^1$H NMR (500 MHz, DMSO-$d_6$) δ 10.65 (s, 1H), 7.99 - 7.84 (m, 2H), 7.79 - 7.61 (m, 2H), 7.57 - 7.45 (m, 1H), 6.47 (s, 2H), 6.20 (ddd, $J$ = 9.1, 5.8, 3.6 Hz, 1H), 5.65 - 5.49 (m, 2H), 4.27 (qd, $J$ = 4.8, 1.9 Hz, 1H), 3.86 (dd, $J$ = 4.6, 2.2 Hz, 2H), 2.99 - 2.80 (m, 1H), 2.78 - 2.61 (m, 1H), 1.50 (d, $J$ = 6.7 Hz, 3H), 0.88 (s, 9H), 0.07 (dd, $J$ = 4.5, 1.3 Hz, 6H).

(2) Step 2

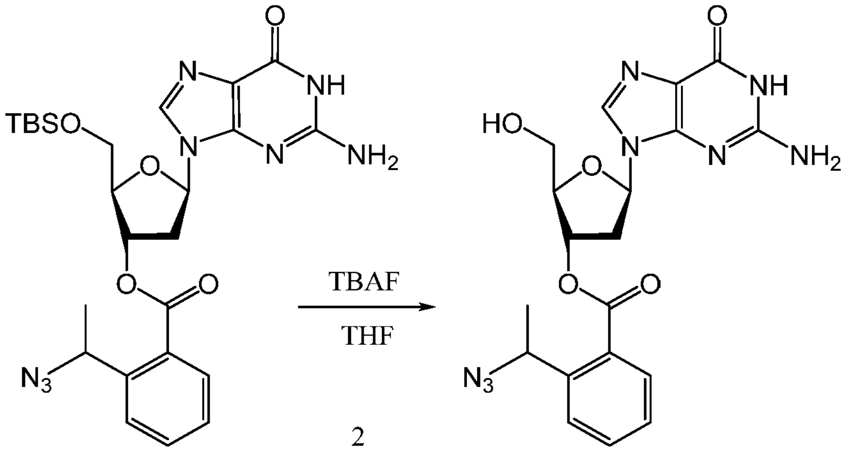


To a dried 100mL round bottom flask equipped with a magnetic stirrer and a rubber stopper with argon balloon was added the product from Step 1 (850mg, 1.54mmol, 1eq) followed by adding with syringe 20mL of anhydrous THF as solvent and stirring at room temperature to facilitate dissolution. The reaction mixture was cooled in ice bath and slowly added with syringe 1mol/L solution of TBAF (2.0mL, 2.0mmol, 1.3eq) dropwise. The flask was brough to room temperature and stirred continuously for about 2h. The reaction was monitored with TLC at half an hour interval, until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 70%.
$^1$H NMR (500 MHz, DMSO-$d_6$) δ 10.68(H, s), 7.99 (1H, s), 7.91 (1H, m), 7.67 (2H, m), 7.46(1H, m), 6.52 (2H, s), 6.17 (1H, m), 5.58 (2H, m), 5.25 (1H, m), 4.23 (1H, t), 3.66 (2H, t), 2.89 (1H, m), 2.61 (1H, m), 1.49 (3H, d, $J$ = 6.8 Hz).

(3) Step 3

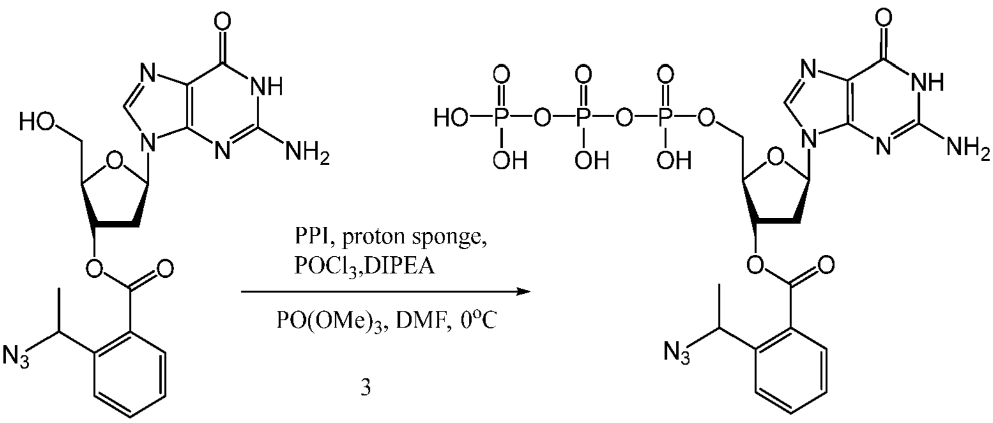


To a dried 100mL two-necked 1# flask equipped with a magnetic stirrer and a three-way valve with argon balloon were added product from Step 2 (375mg, 0.85mmol, 1eq) and proton sponge (363.5mg, 1.7mmol, 2eq). To a dried 100mL

two-necked 2# flask equipped with a magnetic stirrer and a three-way valve with argon balloon was added tributyl ammonium pyrophosphate (932.5mg, 1.7mmol, 2eq). The two-necked 1# flask was filled with argon, added with 15mL of trimethyl phosphate with stirring to facilitate dissolution. The reaction was brought to 0°C, followed by slowly adding with syringe phosphorus oxychloride (120μL, 1.3mmol, 1.5eq) and stirring continuously at 0°C for 1.5h. The two-necked 2# flask was filled with argon, followed by adding with syringe 5mL of anhydrous DMF and DIPEA (740μL, 4.25mmol, 5eq) with stirring at 0°C to facilitate dissolution. The liquid in 1# flask was transferred with syringe into the 2# flask. The mixture was maintained at 0°C and continuously stirred for 3h, followed by adding 20 mL of solution of TEAB (0.1mol/L) to quench the reaction. The reaction mixture was diluted with 50mL of deionized water, and then separated by DEAE resin column prepared-in-advance and eluted with gradient elution ($H_2O$/TEAB(1mol/L)=10/1~0/1, DEAE column diameter 4.5cm, filler height 8cm). The fractions containing the triphosphated product were collected and concentrated with a rotary evaporator under vacuum, followed by preparative liquid chromatography separation with gradient elution ($CH_3CN$:TEAB(0.1mol/L) =2/98~98/2). The high-purity product solution recovered from the separation was concentrated, transferred to a plastic centrifuge tube, and then freeze-dried with a lyophilizer into a powder solid. MW=680. The yield of this step was 45%. $^1H$ NMR (400 MHz, DMSO-$d_6$) $\delta$ 11.12 (s, 1H), 8.05 (d, $J$ = 3.1 Hz, 1H), 7.93 (d, $J$ = 7.6 Hz, 1H), 7.73 - 7.62 (m, 2H), 7.50 (t, $J$ = 7.6 Hz, 1H), 6.87 (s, 2H), 6.25 - 6.19 (m, 1H), 5.72 (d, $J$ = 4.9 Hz, 1H), 5.61 (q, $J$ = 6.6 Hz, 1H), 4.36 - 4.33 (m, 1H), 4.25 - 4.18 (m, 1H), 4.06 - 4.01 (m, 1H), 3.27 - 3.12 (m, 1H), 2.60 - 2.52 (m, 1H), 1.50 (dd, $J$ = 6.7, 2.0 Hz, 3H).

Example 3

The structure of the target compound is:

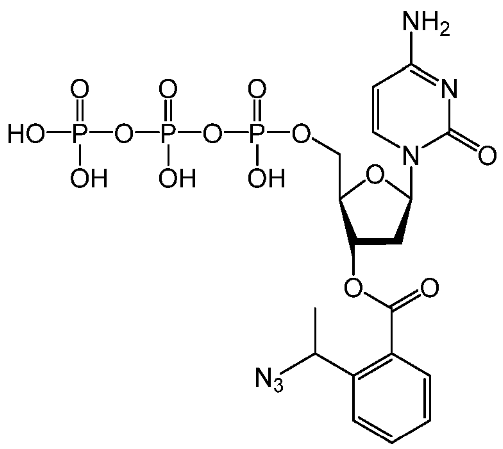

(1) Step 1

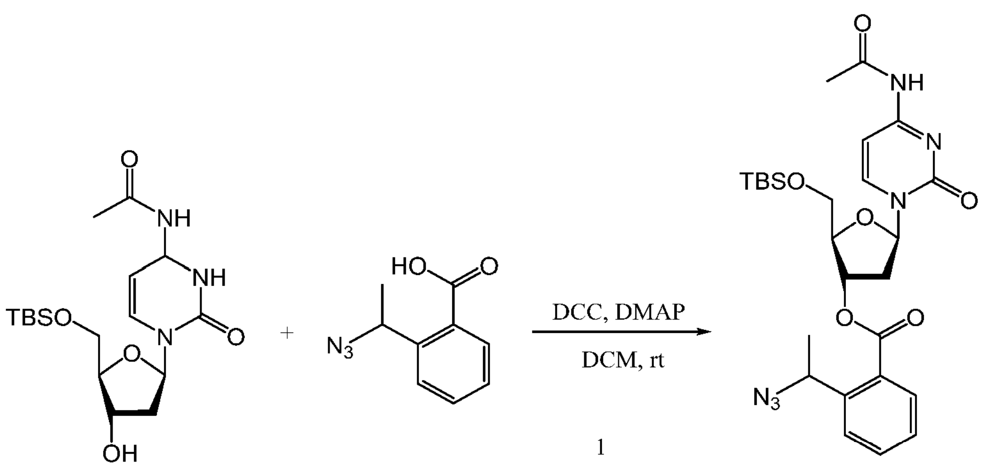


To a dried 100mL round bottom flask equipped with a magnetic stirrer and a rubber stopper with argon balloon were added C-nucleoside (600mg, 1.56mmol, 1eq) (available from Beijing Okeanos Tech. Co., Ltd., cat. No. OK-N-18104)

and azido protection group (297mg, 1.56mmol, 1eq) (available from Beijing Okeanos Tech. Co., Ltd., cat. No. : OK-H-20001) separately, followed by adding with syringe 20mL of anhydrous methylene chloride as solvent. The reaction mixture was stirred at room temperature to facilitate dissolution, followed by adding DCC (480mg, 2.34mmol, 1.5eq) and DMAP (20mg, 0.16mmol, 0.1eq) separately under nitrogen balloon for providing protection atmosphere, and allowed to react at room temperature for further 4h. The reaction was monitored with TLC at half an hour interval, until the starting materials were substantially depleted. The reaction mixture was concentrated with a rotary evaporator, followed by product separation by silica gel column chromatography (Hexane/EA=10/1~1/1, silica gel column diameter 3.5cm, filler height 10cm). The yield of this step was 95%.
$^1$H NMR (400 MHz, DMSO-$d_6$) δ 10.91 (1H, s); 8.23 (1H, d, *J* =7.52 Hz); 7.89 (1H, d, *J= 6.80* Hz); 7.66 (2H, m); 7.48 (1H, m); 7.23 (1H, d, J = 7.48); 6.19(1H, t), 5.55 (1H, m); 5.44 (1H, d, *J* =6.04 Hz), 4.42 (1H, t); 3.92 (2H, m); 2.74 (1H, m); 2.32(1H, m); 1.47 (3H, d, *J*= 6.7 Hz); 0.86 (9H, s); 0.09 (6H, s).

(2) Step 2

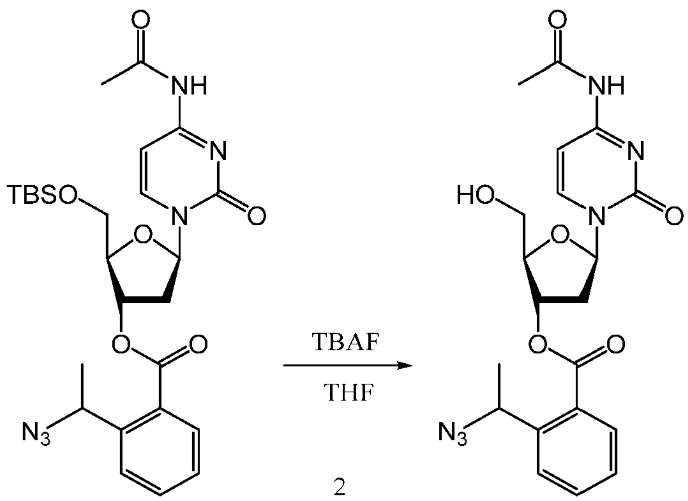


2

To a dried 100mL round bottom flask equipped with a magnetic stirrer and a rubber stopper with argon balloon was added the product from Step 1 (860mg, 1.54mmol, 1eq), followed by adding with syringe 20mL of anhydrous THF as solvent. The reaction mixture was stirred to facilitate dissolution, cooled in ice bath, followed by slowly adding with syringe 1mol/L solution of TBAF (2.0mL, 2.0mmol, 1.3eq), dropwise. The flask was brough to room temperature and stirred continuously for about 2h. The reaction was monitored with TLC at half an hour interval until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 70%.
$^1$H NMR (400 MHz, DMSO-$d_6$) δ 10.90 (1H, s); 8.34(1H, d, *J* =7.48 Hz); 7.89(1H, d, J=7.84 Hz); 7.66 (2H, m); 7.48(1H, m); 7.23(1H, d, J=7.48 Hz); 6.22(1H, t); 5.57(1H, m); 5.47(1H, d, *J* = 6.00 Hz); 5.25 (1H, s); 4.32 (1H, m); 3.72 (1H, s); 2.67 (1H, m); 2.31 (1H, m); 1.45 (3H, d, *J* = 6.7 Hz).

(3) Step 3

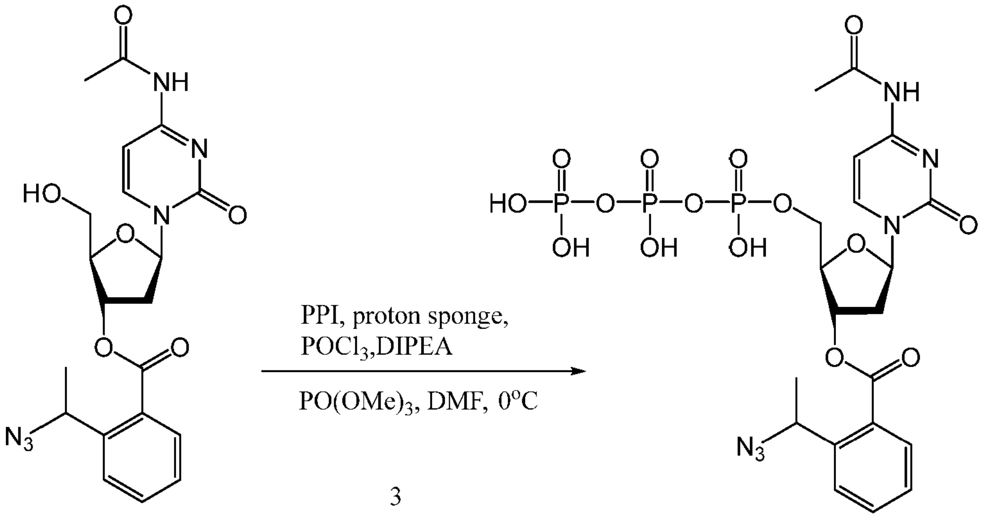


To a dried 100mL two-necked 1# flask equipped with a magnetic stirrer and a three-way valve with argon balloon were added product from Step 2 (377mg, 0.85mmol, 1eq) and proton sponge (363.5mg, 1.7mmol, 2eq). To a dried 100mL two-necked 2# flask equipped with a magnetic stirrer and a three-way valve with argon balloon was added tributyl ammonium pyrophosphate (932.5mg, 1.7mmol, 2eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120μL, 1.3mmol, 1.5eq) and stirring continuously at 0°C for 1.5h. The two-necked 2# flask was filled with argon, followed by adding with syringe 2mL of anhydrous DMF and DIPEA (740μL, 4.25mmol, 5eq) with stirring at 0°C to facilitate dissolution. The mixture was maintained at 0°C and continuously stirred for 3h, followed by adding 20mL of solution of TEAB (0.1mol/L) to quench the reaction. The reaction mixture was diluted with 50mL of deionized water, and then separated by DEAE resin column prepared-in-advance and eluted with gradient elution ($H_2O$:TEAB(1mol/L)=10/1~0/1, DEAE column diameter 4.5cm, filler height 8cm). The fractions containing the triphosphated product were collected and concentrated with a rotary evaporator, followed by preparative liquid chromatography separation with gradient elution ($CH_3CN$:TEAB(0.1mol/L)=2/98~98/2). The high-purity product solution recovered from the separation was concentrated with a rotary evaporator under vacuum, transferred to a plastic centrifuge tube, and then freeze-dried with a lyophilizer into a powder solid. MW=684. The yield of this step was 40%.

(4) Step 4

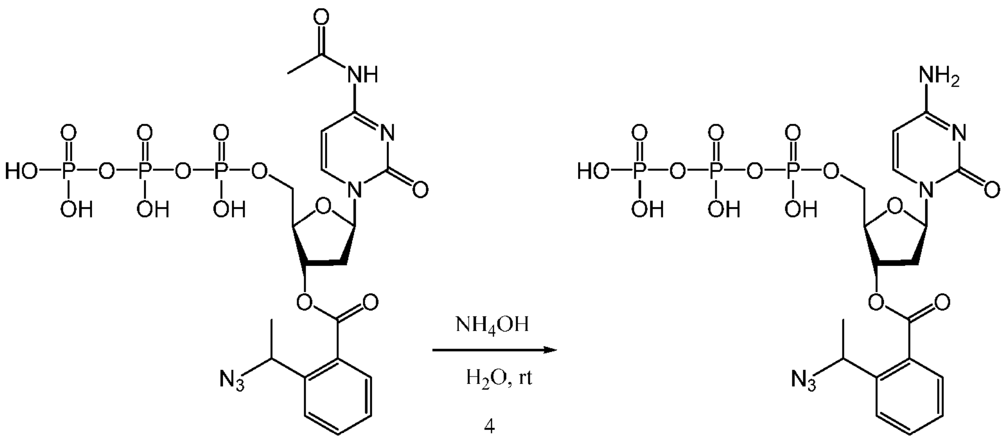


To a dried 100mL round bottom flask equipped with a magnetic stirrer were added the triphosphated product from Step 3(500mg, 0.73mmol, 1eq) and 5ml of deionized water. The mixture was stirred at room temperature to complete dissolution and slowly added with 25% aqueous ammonia (2.5g, 36.5mmol, 50eq), followed by stirring at room temperature for about 6h. Ammonia in the solution was removed by a rotary evaporator under vacuum and the residue was diluted with deionized water to about 10mL. After separation with preparative liquid chromatography with gradient

elution ($CH_3CN$:TEAB(0.1mol/L)=2/98~98/2), the resulted high-purity product solution was concentrated with a rotary evaporator under vacuum, transferred to a plastic centrifuge tube, and freeze-dried with a lyophilizer into a powder solid. MW=642. The yield of this step was 90%.

$^1$H NMR (400 MHz, DMSO-$d_6$) δ 7.99 (dd, $J$ = 7.5, 2.2 Hz, 1H), 7.90 (d, $J$ = 7.8 Hz, 1H), 7.71 - 7.63 (m, 2H), 7.54 - 7.42 (m, 1H), 7.30 (s, 1H), 7.10 (s, 1H), 6.41 - 6.28 (m, 1H), 5.81 (d, $J$ = 7.5 Hz, 1H), 5.65 - 5.50 (m, 2H), 4.33 (s, 1H), 4.18 - 3.96 (m, 2H), 2.46 - 2.26 (m, 2H), 1.49 (dd, $J$ = 6.7, 1.4 Hz, 3H).

Example 4

The structure of the target compound is:

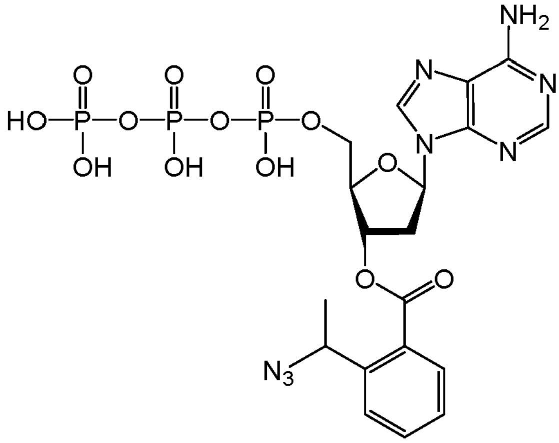

(1) Step 1

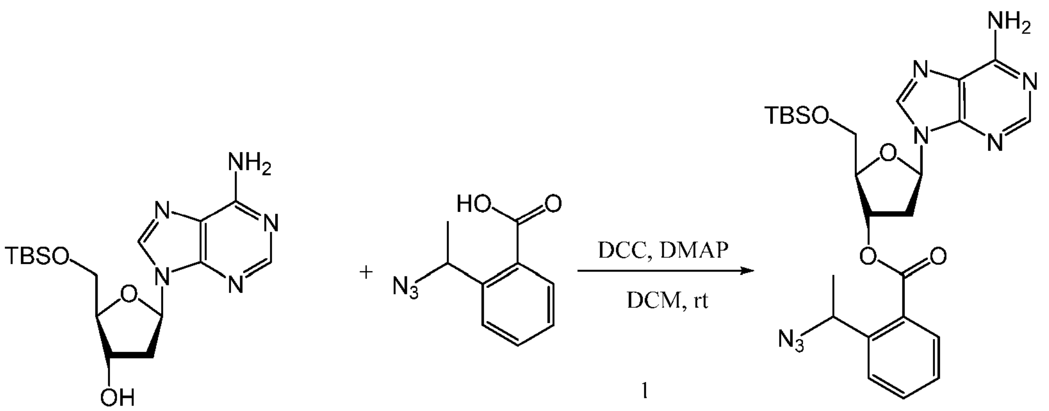


To a dried 100mL round bottom flask equipped with a magnetic stirrer and a rubber stopper with argon balloon were added A-nucleoside (600mg, 1.65mmol, 1eq) (available from Beijing Okeanos Tech. Co., Ltd., cat. No. OK-N-18101) and azido protection group (314mg, 1.65mmol, 1eq) (available from Beijing Okeanos Tech. Co., Ltd., cat. No. OK-H-20001) separately, followed by adding with syringe 20mL of anhydrous methylene chloride as solvent. The reaction mixture was stirred at room temperature to facilitate dissolution, followed by adding DCC (508mg, 2.5mmol, 1.5eq) and DMAP (24mg, 0.17mmol, 0.1eq) separately under nitrogen balloon for providing protection atmosphere, and allowed to react at room temperature for further 4h. The reaction was monitored with TLC at half an hour interval, until the starting materials were substantially depleted. The reaction mixture was concentrated with a rotary evaporator, followed by product separation by silica gel column chromatography (Hexane/EA=10/1~1/1, silica gel column diameter 3.5cm, filler height 10cm). The yield of this step was 95%.

$^1$H NMR (500 MHz, $CDCl_3$) δ 8.36 (1H, s); 8.28 (1H, d, $J$ = 4.4 Hz); 7.98 (1H, m); 7.65 (2H, m); 7.41 (1H, m); 6.60 (1H, t); 6.0 (2H, br.), 5.68 (2H, m); 4.40 (1H, br.); 4.01 (2H, br.); 1.93 (1H, m); 1.68 (1H, m); 1.56 (3H, d, $J$ = 6.7 Hz); 0.94 (9H, s); 0.15 (6H, s).

(2) Step 2

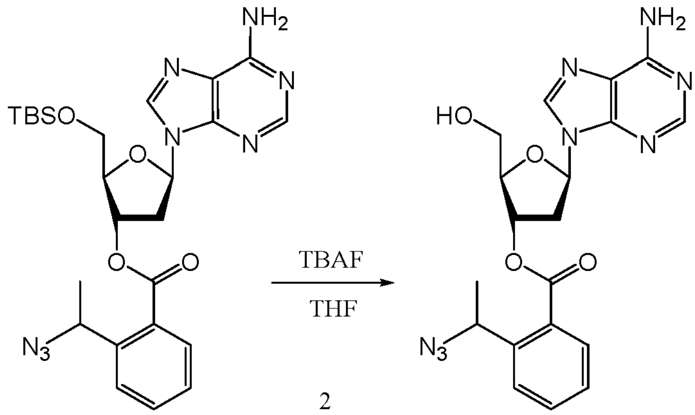


To a dried 100mL round bottom flask equipped with a magnetic stirrer and a rubber stopper with argon balloon was added the product from Step 1 (828mg, 1.54mmol, 1eq), followed by adding with syringe 20mL of anhydrous THF as solvent. The reaction mixture was stirred to facilitate dissolution, cooled in ice bath, followed by slowly adding with syringe 1mol/L solution of TBAF (2.0mL, 2.0mmol, 1.3eq) dropwise. The flask was brough to room temperature and stirred continuously for about 2h. The reaction was monitored with TLC at half an hour interval, until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 70%.
$^1$H NMR (500 MHz, DMSO-$d_6$) δ 10.66 (s, 1H), 8.00 (d, *J* = 1.5 Hz, 1H), 7.92 (ddd, *J* = 8.0, 3.0, 1.4 Hz, 1H), 7.82 - 7.59 (m, 2H), 7.51 (t, *J* = 7.5 Hz, 1H), 6.46 (s, 2H), 6.20 (dt, *J* = 9.5, 5.4 Hz, 1H), 5.58 (ddd, *J* = 15.6, 6.2, 2.5 Hz, 2H), 5.21 (td, *J* = 5.6, 1.8 Hz, 1H), 4.24 (dd, *J* = 4.3, 1.7 Hz, 1H), 3.77 - 3.55 (m, 2H), 2.91 (qd, *J* = 9.1, 5.8 Hz, 1H), 2.62 (dt, *J* = 13.7, 6.6 Hz, 1H), 1.51 (dd, *J* = 6.7, 1.7 Hz, 3H).

(3) Step 3

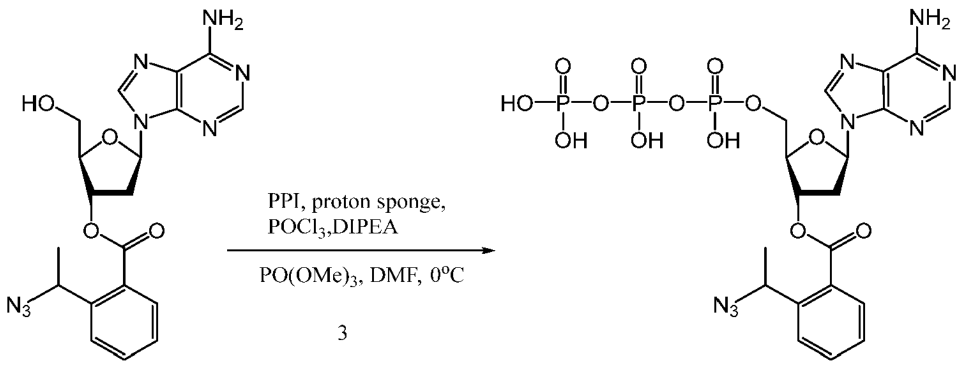


To a dried 100mL two-necked 1# flask equipped with a magnetic stirrer and a three-way valve with argon balloon were added product from Step 2 (360mg, 0.85mmol, 1eq) and proton sponge (363.5mg, 1.7mmol, 2eq). To a dried 100mL two-necked 2# flask equipped with a magnetic stirrer and a three-way valve with argon balloon was added tributyl ammonium pyrophosphate (932.5mg, 1.7mmol, 2eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120μL, 1.3mmol, 1.5eq) and stirring continuously at 0°C for 1.5h. The two-necked 2# flask was filled with argon, followed by adding with syringe 5mL of anhydrous DMF and DIPEA (740μL, 4.25mmol, 5eq) with stirring at 0°C to facilitate dissolution. The mixture was maintained at 0°C and continuously stirred for 3h, followed by adding 20 mL of solution of TEAB (0.1mol/L) to quench the reaction. The reaction mixture was diluted with 50mL of deionized water, and then separated by DEAE resin column prepared-in-advance and eluted with gradient elution

($H_2O$:TEAB(1mol/L)=10/1~0/1, DEAE column diameter 4.5cm, filler height 8cm). The fractions containing the triphosphated product were collected and concentrated with a rotary evaporator under vacuum, followed by preparative liquid chromatography separation with gradient elution ($CH_3CN$:TEAB(0.1mol/L)=2/98~98/2). The high-purity product solution recovered from the separation was concentrated with a rotary evaporator under vacuum, transferred to a plastic centrifuge tube, and then freeze-dried with a lyophilizer into a powder solid. MW=664. The yield of this step was 40%. $^1$H NMR (400 MHz, DMSO-$d_6$) δ 8.62 (d, $J$ = 3.6 Hz, 1H), 8.15 (d, $J$ = 1.5 Hz, 1H), 7.97 (d, $J$ = 7.8 Hz, 1H), 7.72 - 7.65 (m, 2H), 7.55 - 7.47 (m, 1H), 7.29 (s, 2H), 6.51 - 6.46 (m, 1H), 5.74 (d, $J$ = 5.3 Hz, 1H), 5.68 - 5.58 (m, 1H), 4.44 - 4.41 (m, 1H), 4.14 - 4.01 (m, 2H), 3.19 - 3.11 (m, 1H), 2.75 - 2.65 (m, 1H), 1.51 (dd, $J$ = 6.7, 1.5 Hz, 3H).

Example 5

The structure of the target compound is:

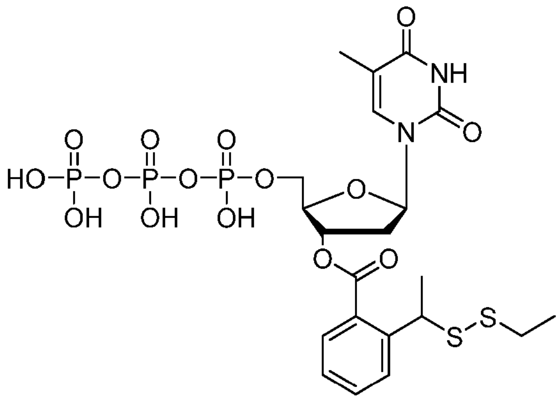

(1) Step 1

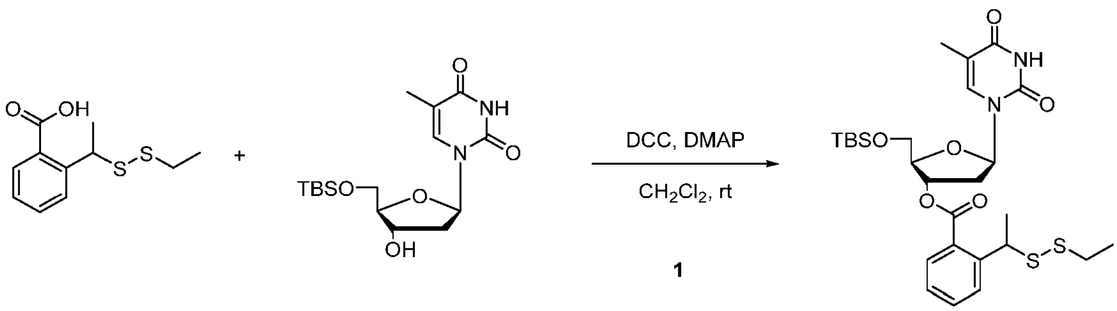


To a dried 100mL round bottom flask equipped with a magnetic stirrer and a rubber stopper with argon balloon were added T-nucleoside (500 mg, 1.40 mmol, 1 eq) (available from Beijing Okeanos Tech. Co., Ltd., cat. No. OK-N-18102) and disulfaneyl protection group (340 mg, 1.40 mmol, 1 eq) (available from Beijing Okeanos Tech. Co., Ltd., cat. No. : OK-H-21003) separately, followed by adding with syringe 20 mL of anhydrous methylene chloride as solvent. The reaction mixture was stirred at room temperature to facilitate dissolution, followed by adding DCC (433mg, 2.10mmol, 1.5eq) and DMAP (17 mg, 0.14 mmol, 0.1 eq) separately under nitrogen balloon for providing protection atmosphere, and allowed to react at room temperature for further 6h. The reaction was monitored with TLC at half an hour interval, until the starting materials were substantially depleted. The reaction mixture was concentrated with a rotary evaporator, followed by product separation by silica gel column chromatography (Hexane/EA=10/1~1/1, silica gel column diameter 3.5cm, filler height 10cm), yield 85%. $^1$H NMR (400 MHz, $CDCl_3$) δ 8.61 (d, $J$ = 4.7 Hz, 1H), 7.84 (t, $J$ = 8.3 Hz, 1H), 7.63 - 7.57 (m, 2H), 7.52 (t, $J$ = 7.6 Hz, 1H), 7.33 (dd, $J$ = 11.0, 4.1 Hz, 1H), 6.47 - 6.40 (m, 1H), 5.52 - 5.45 (m, 1H), 5.31 - 5.20 (m, 1H), 4.30 (d, $J$ = 21.8 Hz, 1H), 4.07 - 3.94 (m, 2H), 3.51 - 3.44 (m, 1H), 2.63 - 2.53 (m, 1H), 2.29 - 2.20 (m, 2H), 1.94 (s, 3H), 1.69 (d, $J$ = 7.0 Hz, 3H), 1.12 - 1.07 (m, 3H), 0.96 (s, 9H), 0.17 (s, 6H).

(2) Step 2

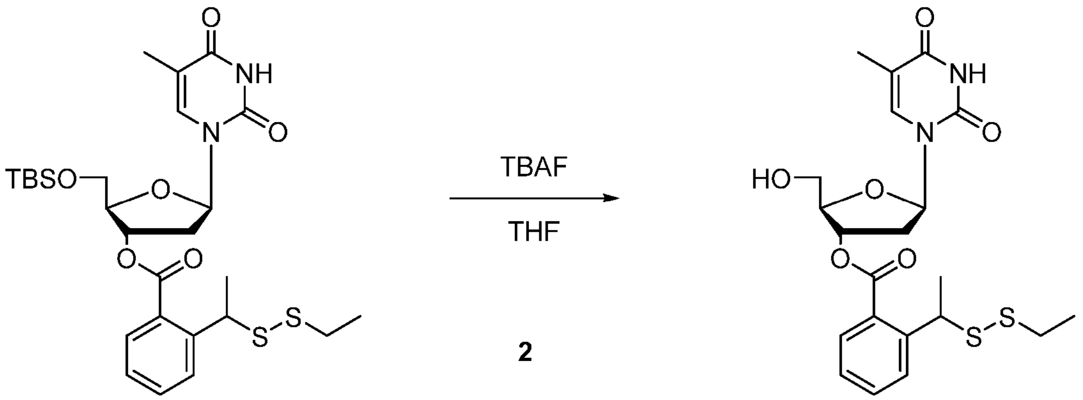


2

To a dried 100mL round bottom flask equipped with a magnetic stirrer and a rubber stopper with argon balloon was added the product from Step 1 (690 mg, 1.19 mmol, 1eq), followed by adding with syringe 20mL of anhydrous THF as solvent. The reaction mixture was stirred at room temperature to facilitate dissolution, cooled in ice bath, followed by slowly adding with syringe 1mol/L solution of TBAF (1.6 mL, 1.6 mmol, 1.3eq) dropwise. The flask was brough to room temperature and stirred continuously for about 2h. The reaction was monitored with TLC at half an hour interval, until the dot of starting material on TLC plate disappeared. The reaction mixture was concentrated with a rotary evaporator, followed by product separation by silica gel column chromatography (Hexane/EA=5/1~1/1 to methylene chloride/anhydrous methanol=100/1~10/1, silica gel column diameter 3.5cm, filler height 10cm). The yield of this step was 80%.
$^1H$ NMR (500 MHz, $CDCl_3$) δ 8.57 (s, 1H), 7.87 - 7.80 (m, 1H), 7.60 (dd, *J* = 7.8, 1.9 Hz, 1H), 7.57 - 7.50 (m, 2H), 7.39 - 7.29 (m, 1H), 6.35 - 6.25 (m, 1H), 5.64 - 5.56 (m, 1H), 5.23 (p, *J* = 7.1 Hz, 1H), 4.29 (dd, *J* = 14.3, 2.4 Hz, 1H), 4.02 (dd, *J* = 4.9, 2.5 Hz, 2H), 2.59 - 2.55 (m, 2H), 2.29 - 2.19 (m, 2H), 1.94 (d, *J* = 0.6 Hz, 3H), 1.70 (d, *J* = 7.0 Hz, 3H), 1.11 (td, *J* = 7.4, 2.6 Hz, 3H).

(3) Step 3

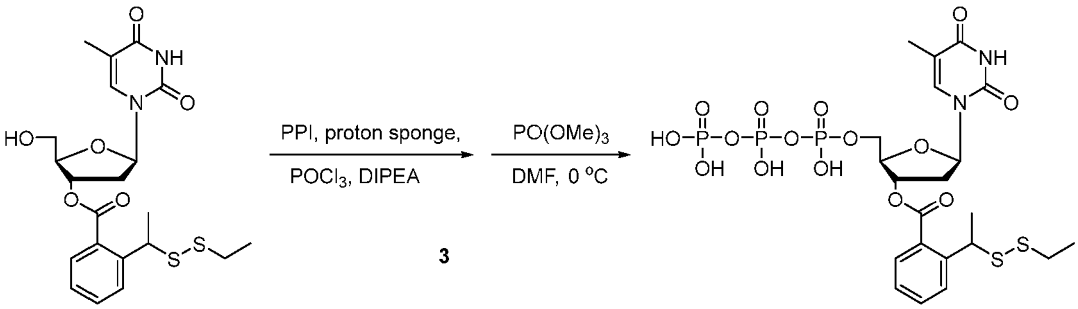


3

To a dried 100mL two-necked 1# flask equipped with a magnetic stirrer and a three-way valve with argon balloon were added product from Step 2 (396 mg, 0.85 mmol, 1 eq) and proton sponge (363.5 mg, 1.7 mmol, 2 eq). To a dried 100mL two-necked 2# flask equipped with a magnetic stirrer and a three-way valve with argon balloon was added tributyl ammonium pyrophosphate (932.5 mg, 1.7 mmol, 2 eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120 μL, 1.3 mmol, 1.5 eq) and stirring continuously at 0°C for 1.5h. The two-necked 2# flask was filled with argon, followed by adding with syringe 5mL of anhydrous DMF and DIPEA (740 μL, 4.25 mmol, 5 eq) at 0°C with stirring to facilitate dissolution. The mixture was maintained at 0°C and continuously stirred for 3h, followed by adding 20 mL of solution of TEAB (0.1mol/L) to quench the reaction. The reaction mixture was diluted with 50mL of deionized water, and then separated by DEAE resin column prepared-in-advance and eluted with gradient elution ($H_2O$:TEAB(1mol/L)=10/1~0/1, DEAE column diameter 4.5cm, filler height 8cm). The fractions containing the triphosphated product were collected and concentrated with a rotary evaporator under vacuum, followed by preparative liquid chromatography separation with gradient elution ($CH_3CN$:TEAB(0.1mol/L)=2/98~98/2). The high-purity product

solution recovered from the separation was concentrated with a rotary evaporator under vacuum, transferred to a plastic centrifuge tube, and then freeze-dried with a lyophilizer into a powder solid. MW = 706. The yield of this step was 40%. $^1$H NMR (400 MHz, DMSO-$d_6$) δ 11.30 (s, 1H), 7.96 (dd, $J$ = 6.0, 1.0 Hz, 1H), 7.86 (d, $J$ = 7.7 Hz, 1H), 7.68 - 7.56 (m, 2H), 7.46 - 7.41 (m, 1H), 6.38 - 6.27 (m, 1H), 5.60 (d, $J$ = 5.3 Hz, 1H), 5.16 - 5.11 (m, 1H), 4.31 (d, $J$ = 20.8 Hz, 1H), 4.18 - 4.00 (m, 2H), 2.58 - 2.51 (m, 1H), 2.44 - 2.22 (m, 3H), 1.85 (s, 3H), 1.65 (d, $J$ = 7.0 Hz, 3H), 1.09 - 1.03 (m, 3H).

Example 6

The structure of the target compound is:

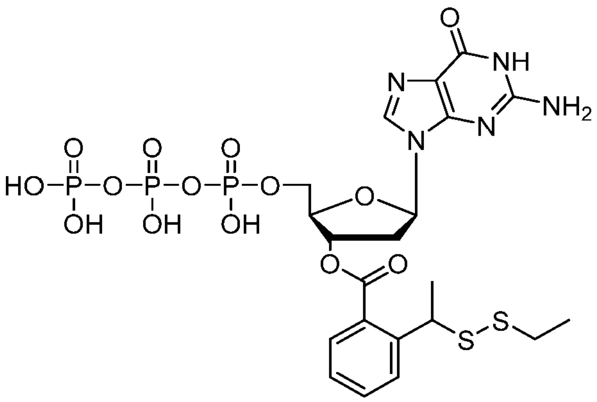

(1) Step 1

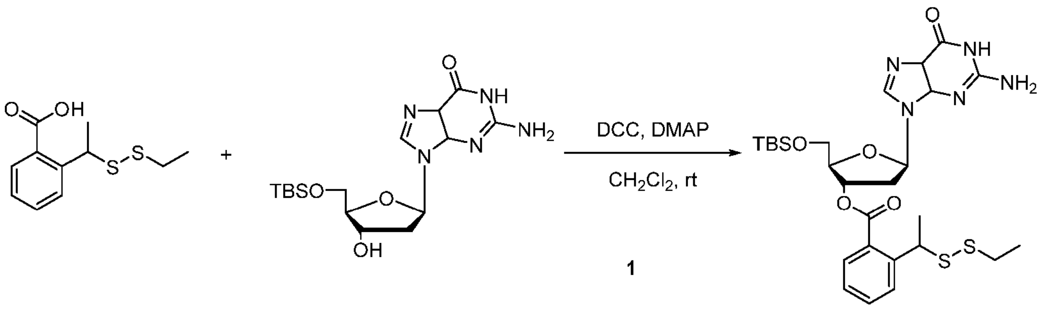


To a dried 100mL round bottom flask equipped with a magnetic stirrer and a rubber stopper with argon balloon were added G-nucleoside (600 mg, 1.58 mmol, 1 eq) (available from Beijing Okeanos Tech. Co., Ltd., cat. No. OK-N-18103) and disulfaneyl protection group (605 mg, 1.58 mmol, 1 eq) (available from Beijing Okeanos Tech. Co., Ltd., cat. No. OK-H-21003) separately, followed by adding with syringe 20mL of anhydrous methylene chloride as solvent. The reaction mixture was stirred at room temperature to facilitate dissolution, followed by adding DCC (488mg, 2.37mmol, 1.5eq) and DMAP (20mg, 0.16mmol, 0.1eq) separately under nitrogen balloon for providing protection atmosphere, and allowed to react at room temperature for further 4h. The reaction was monitored with TLC at half an hour interval, until the starting materials were substantially depleted. The reaction mixture was concentrated with a rotary evaporator, followed by product separation by silica gel column chromatography (Hexane/EA=10/1~1/2, silica gel column diameter 3.5cm, filler height 10cm). The yield of this step was 80%.
$^1$H NMR (500 MHz, $CDCl_3$) δ 12.00 (s, 1H), 7.93 (t, $J$ = 9.2 Hz, 1H), 7.88 - 7.83 (m, 1H), 7.59 (dd, $J$ = 13.6, 8.0 Hz, 1H), 7.53 (t, $J$ = 7.6 Hz, 1H), 7.33 (t, $J$ = 7.5 Hz, 1H), 6.37 - 6.33 (m, 2H), 5.62 (s, 1H), 5.30 - 5.22 (m, 1H), 4.36 (dd, $J$ = 10.9, 1.5 Hz, 1H), 4.01 - 3.89 (m, 2H), 3.51 - 3.40 (m, 1H), 2.78 - 2.66 (m, 2H), 2.33 - 2.22 (m, 2H), 1.70 (d, $J$ = 6.9 Hz, 3H), 1.13 (d, $J$ = 2.5 Hz, 3H), 0.92 (s, 9H), 0.12 (s, 6H).

(2) Step 2

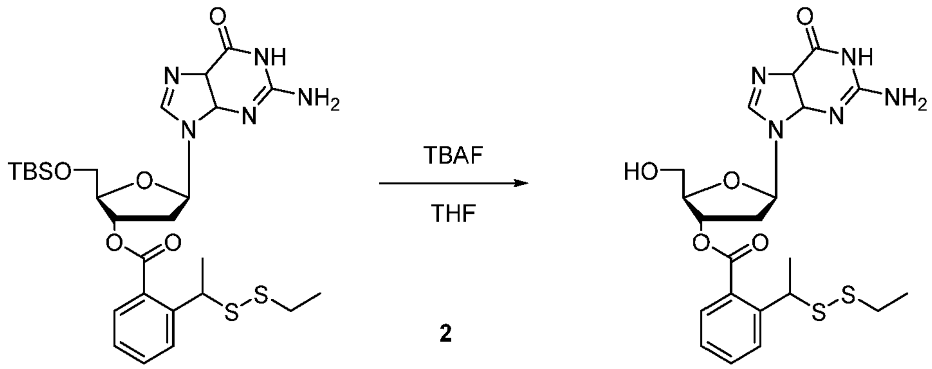


2

To a dried 100mL round bottom flask equipped with a magnetic stirrer and a rubber stopper with argon balloon was added the product from Step 1 (932 mg, 1.54 mmol, 1 eq), followed by adding with syringe 20mL of anhydrous THF as solvent. The reaction mixture was stirred to facilitate dissolution, cooled in ice bath, followed by slowly adding with syringe 1mol/L solution of TBAF (2.0 mL, 2.0 mmol, 1.3 eq) dropwise. The flask was brough to room temperature and stirred continuously for about 2h. The reaction was monitored with TLC at half an hour interval, until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 70%.
$^1$H NMR (400 MHz, $CDCl_3$) δ 11.71 (s, 1H), 7.97 (d, *J* = 7.8 Hz, 1H), 7.62 (d, *J* = 7.9 Hz, 1H), 7.52 (t, *J* = 7.5 Hz, 2H), 7.32 (t, *J* = 7.2 Hz, 1H), 6.36 - 6.02 (m, 1H), 5.41 - 5.22 (m, 2H), 3.03 - 2.97 (m, 3H), 2.34 (dd, *J* = 14.6, 7.2 Hz, 2H), 2.26 - 2.20 (m, 1H), 2.06 - 1.97 (m, 1H), 1.69 (d, *J* = 6.9 Hz, 3H), 1.14 (t, *J* = 7.3 Hz, 3H).

(3) Step 3

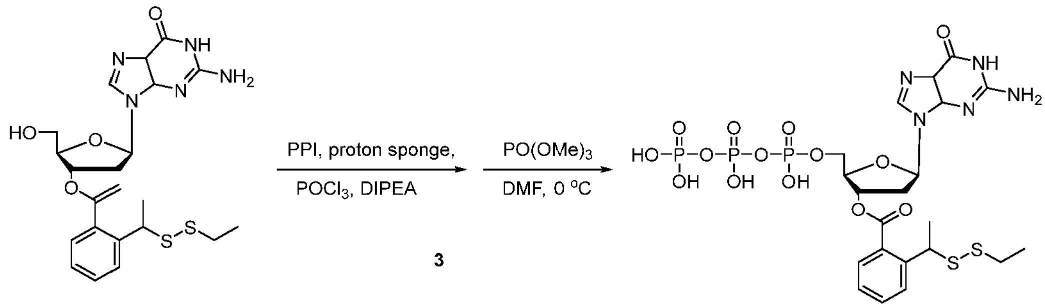


3

To a dried 100mL two-necked 1# flask equipped with a magnetic stirrer and a three-way valve with argon balloon were added product from Step 2 (417 mg, 0.85 mmol, 1 eq) and proton sponge (363.5 mg, 1.7 mmol, 2 eq). To a dried 100mL two-necked 2# flask equipped with a magnetic stirrer and a three-way valve with argon balloon was added tributyl ammonium pyrophosphate (932.5 mg, 1.7 mmol, 2 eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120 μL, 1.3 mmol, 1.5 eq) and stirring continuously at 0°C for 1.5h. The two-necked 2# flask was filled with argon, followed by adding with syringe 5mL of anhydrous DMF and DIPEA (740 μL, 4.25 mmol, 5 eq) with stirring at 0°C to facilitate dissolution. The mixture was maintained at 0°C and continuously stirred for 3h, followed by adding 20 mL of solution of TEAB (0.1mol/L) to quench the reaction. The reaction mixture was continuously stirred for 0.5h, diluted with 50mL of deionized water, and then separated by DEAE resin column prepared-in-advance and eluted with gradient elution ($H_2O$:TEAB(1mol/L)=10/1~0/1, DEAE column diameter 4.5cm, filler height 8cm). The fractions containing the triphosphated product were collected and concentrated under vacuum to 10mL, followed by preparative liquid chromatography separation with gradient elution ($CH_3CN$:TEAB(0.1mol/L)=2/98~98/2). The high-purity product solution recovered from the separation was concentrated with a rotary evaporator under vacuum, transferred to a plastic centrifuge tube, and then freeze-dried with a lyophilizer into a powder solid. MW = 706. The yield of this step was 40%.
$^1$H NMR (400 MHz, DMSO-$d_6$) δ 10.82 (s, 1H), 8.03 (d, *J* = 5.5 Hz, 1H), 7.88 (d, *J* = 7.8 Hz, 1H), 7.69 - 7.58 (m, 2H), 7.46 - 7.42 (m, 1H), 6.72 (s, 2H), 6.25 - 6.18 (m, 1H), 5.70 (t, *J* = 5.0 Hz, 1H), 5.22 - 5.10 (m, 1H), 4.40 - 4.27 (m, 1H), 4.21 (dd, *J* = 11.0, 5.0 Hz, 1H), 4.01 (dd, *J* = 7.0, 3.7 Hz, 1H), 2.59 - 2.52 (m, 1H), 2.48 - 2.30 (m, 3H), 1.65 (d, *J* = 7.0 Hz, 3H), 1.07 (t, *J* =

5.1 Hz, 3H).

Example 7

The structure of the target compound is:

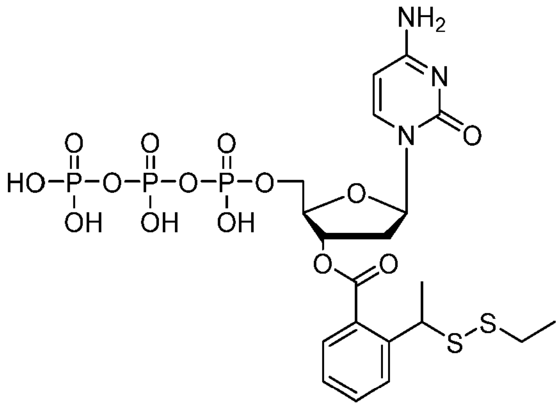

(1) Step 1

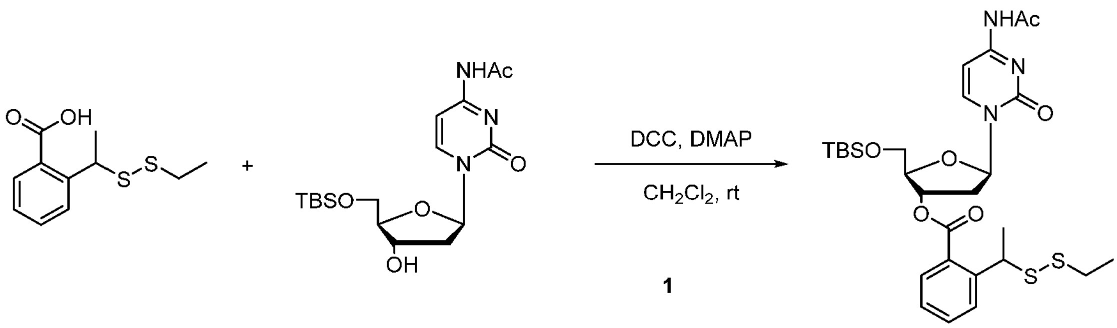


To a dried 100mL round bottom flask equipped with a magnetic stirrer and a rubber stopper with argon balloon were added C-nucleoside (608 mg, 1.58 mmol, 1 eq) (available from Beijing Okeanos Tech. Co., Ltd., cat. No. OK-N-18104) and disulfaneyl protection group (605 mg, 1.58 mmol, 1 eq) (available from Beijing Okeanos Tech. Co., Ltd., cat. No. : OK-H-21003) separately, followed by adding with syringe 20mL of anhydrous methylene chloride as solvent. The reaction mixture was stirred at room temperature to facilitate dissolution, followed by adding DCC (488mg, 2.37mmol, 1.5eq) and DMAP (20mg, 0.16mmol, 0.1eq) separately under nitrogen balloon for providing protection atmosphere, and allowed to react at room temperature for further 4h. The reaction was monitored with TLC at half an hour interval, until the starting materials were substantially depleted. The reaction mixture was concentrated with a rotary evaporator, followed by product separation by silica gel column chromatography (Hexane/EA=10/1~1/2, silica gel column diameter 3.5cm, filler height 10cm). The yield of this step was 90%.

$^1$H NMR (500 MHz, $CDCl_3$) δ 9.53 (s, 1H), 8.35 (d, *J* = 6.0 Hz, 1H), 7.87 (ddd, *J* = 7.8, 4.4, 1.3 Hz, 1H), 7.59 (t, *J* = 7.9 Hz, 1H), 7.54 - 7.50 (m, 1H), 7.41 (d, *J* = 6.0 Hz, 1H), 7.32 (td, *J* = 7.7, 1.2 Hz, 1H), 6.44 - 6.38 (m, 1H), 5.50 - 5.48 (m, 1H), 5.29 - 5.21 (m, 1H), 4.45 - 4.36 (m, 1H), 4.01 (s, 2H), 3.50 - 3.44 (m, 1H), 2.93 - 2.86 (m, 1H), 2.24 - 2.12 (m, 2H), 1.69 (dd, *J* = 7.0, 0.6 Hz, 3H), 1.11 (td, *J* = 7.4, 3.3 Hz, 3H), 0.93 (s, 9H), 0.13 (s, 6H).

(2) Step 2

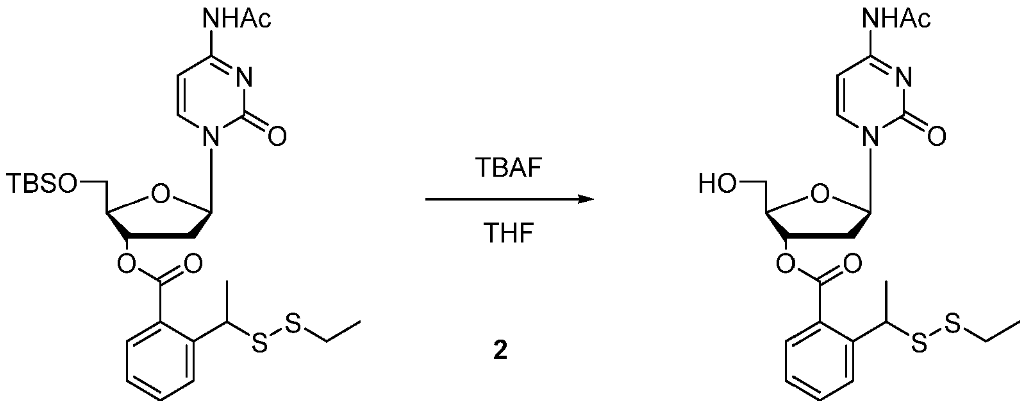


2

To a dried 100mL round bottom flask equipped with a magnetic stirrer and a rubber stopper with argon balloon was added the product from Step 1 (938 mg, 1.54 mmol, 1 eq), followed by adding with syringe 20mL of anhydrous THF as solvent. The reaction mixture was stirred to facilitate dissolution, cooled in ice bath, followed by slowly adding with syringe 1mol/L solution of TBAF (2.0 mL, 2.0 mmol, 1.3 eq) dropwise . The flask was brough to room temperature and stirred continuously for about 2h. The reaction was monitored with TLC at half an hour interval until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 80%.
$^1$H NMR (400 MHz, $CDCl_3$) δ 9.50 (s, 1H), 8.39 (s, 1H), 7.85 (t, *J* = 7.2 Hz, 1H), 7.61 - 7.51 (m, 3H), 7.34 (t, *J* = 7.3 Hz, 1H), 6.30 (s, 1H), 5.63 (d, *J* = 2.8 Hz, 1H), 5.27 - 5.19 (m, 1H), 4.40 (d, *J* = 15.5 Hz, 1H), 4.15 - 3.94 (m, 2H), 3.40 (s, 1H), 2.83 (td, *J* = 14.3, 4.8 Hz, 1H), 2.60 - 2.45 (m, 1H), 2.27 - 2.14 (m, 2H), 1.69 (d, *J* = 6.8 Hz, 3H), 1.10 (q, *J* = 6.8 Hz, 3H).

(3) Step 3

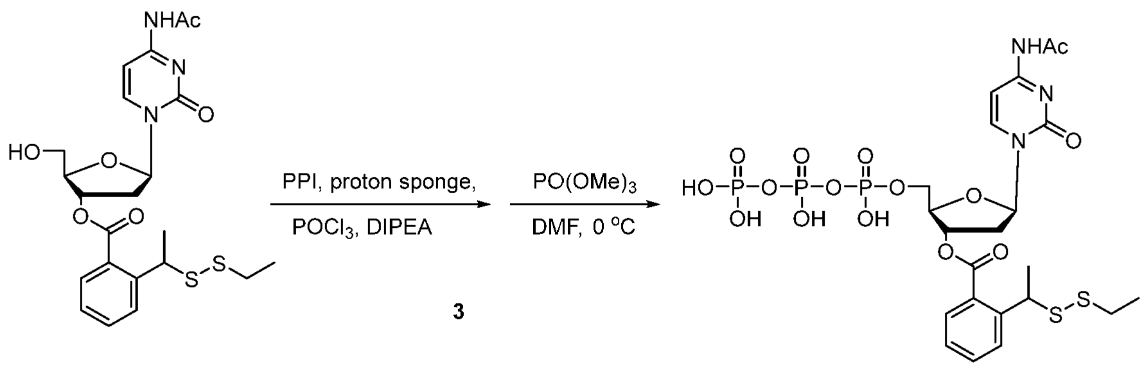


3

To a dried 100mL two-necked 1# flask equipped with a magnetic stirrer and a three-way valve with argon balloon were added product from Step 2 (421 mg, 0.85 mmol, 1 eq) and proton sponge (363.5 mg, 1.7 mmol, 2 eq). To a dried 100mL two-necked 2# flask equipped with a magnetic stirrer and a three-way valve with argon balloon was added tributyl ammonium pyrophosphate (932.5 mg, 1.7 mmol, 2 eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120 μL, 1.3 mmol, 1.5 eq) and stirring continuously at 0°C for 1.5h. The two-necked 2# flask was filled with argon, followed by adding with syringe 5mL of anhydrous DMF and DIPEA (740 μL, 4.25 mmol, 5 eq) with stirring at 0°C to facilitate dissolution. The mixture was maintained at 0°C and continuously stirred for 3h, followed by adding 20 mL of solution of TEAB (0.1mol/L) to quench the reaction. The reaction mixture was continuously stirred for 0.5h. The reaction mixture was diluted with 50mL of deionized water, and then separated by DEAE resin column prepared-in-advance and eluted with gradient elution ($H_2O$:TEAB(1mol/L)=10/1~0/1, DEAE column diameter 4.5cm, filler height 8cm). The fractions containing the triphosphated product were collected and concentrated with a rotary evaporator under vacuum, followed by preparative liquid chromatography separation with gradient elution ($CH_3CN$:TEAB(0.1mol/L) =2/98~98/2). The high-purity product solution recovered from the separation was concentrated with a rotary evaporator under vacuum, transferred to a plastic centrifuge tube, and then freeze-dried with a lyophilizer into a powder solid. MW =

733. The yield of this step was 45%.

(4) Step 4

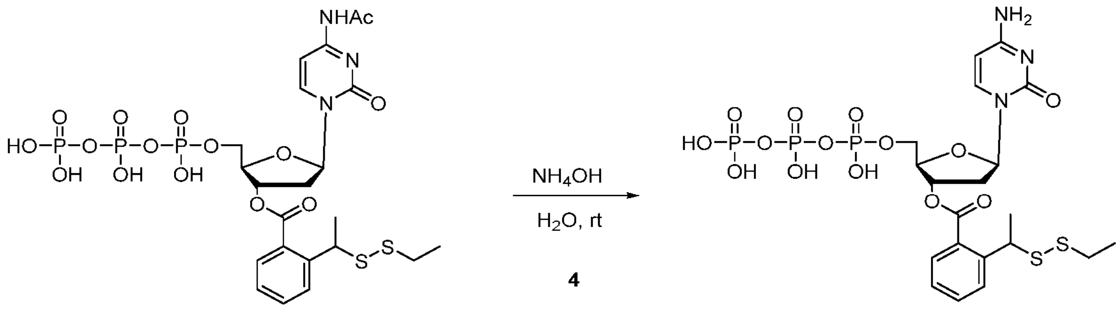


To a dried 100mL round bottom flask equipped with a magnetic stirrer were added the triphosphated product from Step 3(537 mg, 0.73 mmol, 1 eq) and 5ml of deionized water. The mixture was stirred at room temperature to complete dissolution, followed by adding 25% aqueous ammonia (2.5 g, 36.5 mmol, 50 eq) and stirring at room temperature for about 2h. Ammonia in the solution was removed by a rotary evaporator under vacuum and the residue was diluted with deionized water to about 10mL. After separation with preparative liquid chromatography with gradient elution ($CH_3CN$:TEAB(0.1mol/L)=2/98~98/2). The high-purity product solution recovered from the separation was concentrated with a rotary evaporator under vacuum, transferred to a plastic centrifuge tube, and then freeze-dried with a lyophilizer into a powder solid. MW=691. The yield of this step was 90%.
$^1$H NMR (400 MHz, DMSO-$d_6$) $\delta$ 7.97 (dd, $J$ = 7.4, 6.2 Hz, 1H), 7.85 (d, $J$ = 8.7 Hz, 1H), 7.66 - 7.58 (m, 2H), 7.45 - 7.41 (m, 1H), 7.31 (s, 1H), 7.10 (s, 1H), 6.39 - 6.29 (m, 1H), 5.81 (d, $J$ = 7.4 Hz, 1H), 5.53 (d, $J$ = 5.0 Hz, 1H), 5.17 - 5.10 (m, 1H), 4.31 (d, $J$ = 19.8 Hz, 1H), 4.13 - 4.01 (m, 2H), 2.46 - 2.23 (m, 4H), 1.65 (d, $J$ = 7.0 Hz, 3H), 1.09 - 1.03 (m, 3H).

Example 8

The structure of the target compound is:

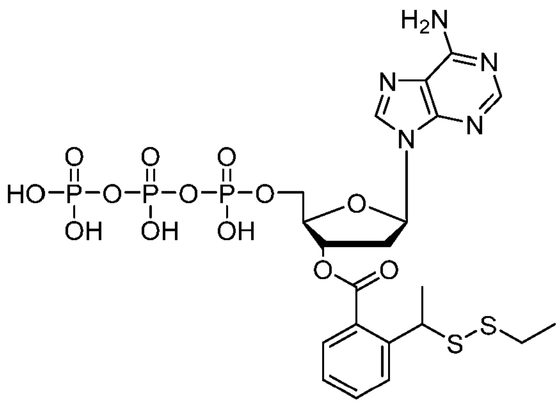

(1) Step 1

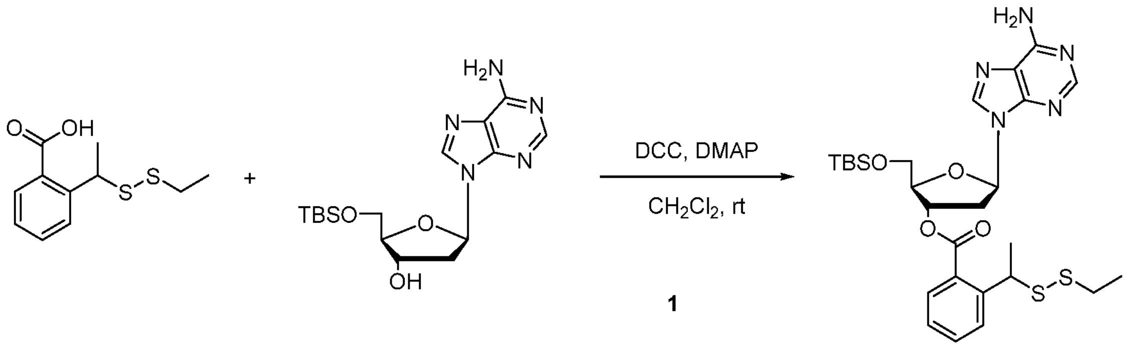


1

To a dried 100mL round bottom flask equipped with a magnetic stirrer and a rubber stopper with argon balloon were added A-nucleoside (577 mg, 1.58 mmol, 1 eq) (available from Beijing Okeanos Tech. Co., Ltd., cat. No. OK-N-18101) and disulfaneyl protection group (605 mg, 1.58 mmol, 1 eq) (available from Beijing Okeanos Tech. Co., Ltd., cat. No. OK-H-21003) separately, followed by adding with syringe 20mL of anhydrous methylene chloride as solvent. The reaction mixture was stirred at room temperature to facilitate dissolution, followed by adding DCC (488mg, 2.37mmol, 1.5eq) and DMAP (20mg, 0.16mmol, 0.1eq) separately under nitrogen balloon for providing protection atmosphere, and allowed to react at room temperature for further 4h. The reaction was monitored with TLC at half an hour interval until the starting materials were substantially depleted. The reaction mixture was concentrated with a rotary evaporator, followed by product separation by silica gel column chromatography (Hexane/EA=10/1~1/2, silica gel column diameter 3.5cm, filler height 10cm). The yield of this step was 85%.

$^1$H NMR (500 MHz, $CDCl_3$) δ 8.34 (s, 1H), 8.24 (d, *J* = 11.2 Hz, 1H), 7.87 (ddd, *J* = 7.8, 4.4, 1.3 Hz, 1H), 7.60 (d, *J* = 7.9 Hz, 1H), 7.52 (t, *J* = 7.6 Hz, 1H), 7.32 (td, *J* = 7.7, 1.2 Hz, 1H), 6.58 (t, *J* = 7.1 Hz, 1H), 6.21 (s, 2H), 5.68 - 5.61 (m, 1H), 5.27 (p, *J* = 7.0 Hz, 1H), 4.45 - 4.36 (m, 1H), 4.04 - 3.95 (m, 2H), 2.83 - 2.73 (m, 2H), 2.31 - 2.18 (m, 2H), 1.69 (dd, *J*= 7.0, 0.6 Hz, 3H), 1.11 (td, *J* = 7.4, 3.3 Hz, 3H), 0.93 (s, 9H), 0.13 (t, *J* = 1.7 Hz, 6H).

(2) Step 2

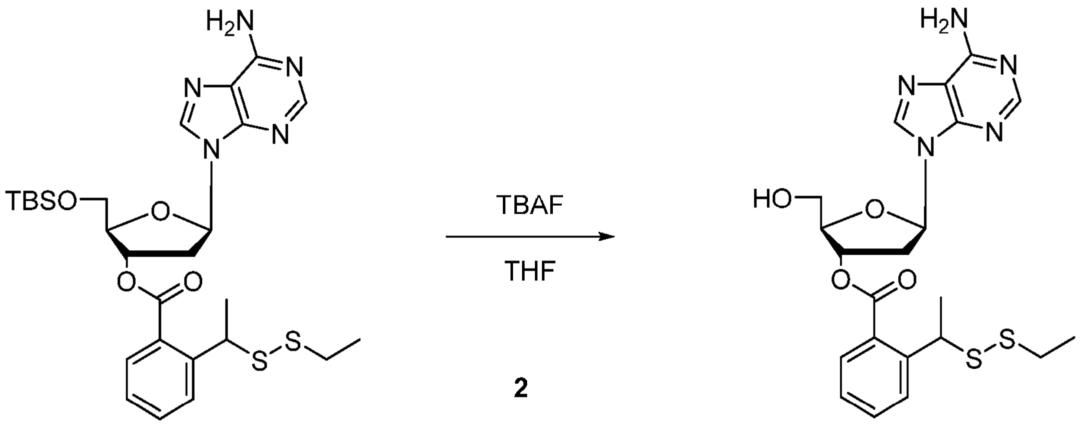


2

To a dried 100mL round bottom flask equipped with a magnetic stirrer and a rubber stopper with argon balloon was added the product from Step 1 (907 mg, 1.54 mmol, 1 eq), followed by adding with syringe 20mL of anhydrous THF as solvent. The reaction mixture was stirred to facilitate dissolution, cooled in ice bath, followed by slowly adding with syringe 1mol/L solution of TBAF (2.0 mL, 2.0 mmol, 1.3 eq) dropwise. The flask was brough to room temperature and stirred continuously for about 2h. The reaction was monitored with TLC at half an hour interval until the dot of starting material on TLC plate disappeared. The reaction mixture was concentrated with a rotary evaporator, followed by product separation by silica gel column chromatography (Hexane/EA=10/1~1/1 to methylene chloride/anhydrous methanol=100/1~10/1, silica gel column diameter 3.5cm, filler height 10cm). The yield of this step was 80%.

$^1$H NMR (500 MHz, $CDCl_3$) δ 8.37 (s, 1H), 7.93 (s, 1H), 7.89 - 7.85 (m, 1H), 7.65 - 7.61 (m, 1H), 7.56 (td, *J* = 7.7, 1.3 Hz, 1H), 7.39 - 7.33 (m, 1H), 6.37 (dd, *J* = 9.3, 5.3 Hz, 1H), 6.02 (s, 2H), 5.83 (t, *J* = 5.5 Hz, 1H), 5.31 - 5.23 (m, 1H), 4.48 (d, *J* = 14.2 Hz, 1H), 4.09 - 3.97 (m, 2H), 3.33 - 3.28 (m, 1H), 2.63 (dd, *J* = 14.0, 4.9 Hz, 1H), 2.41 - 2.31 (m, 2H), 1.73 (d, *J* = 7.0 Hz, 3H), 1.17 (td, *J* = 7.4, 2.3 Hz, 3H).

(3) Step 3

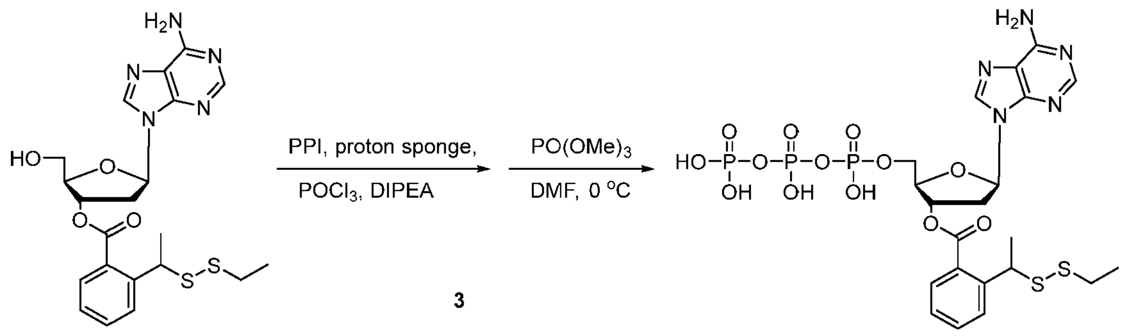


To a dried 100mL two-necked 1# flask equipped with a magnetic stirrer and a three-way valve with argon balloon were added product from Step 2 (404 mg, 0.85 mmol, 1 eq) and proton sponge (363.5 mg, 1.7 mmol, 2 eq). To a dried 100mL two-necked 2# flask equipped with a magnetic stirrer and a three-way valve with argon balloon was added tributyl ammonium pyrophosphate (932.5 mg, 1.7 mmol, 2 eq). The two-necked 1# flask was filled with argon, followed by adding 15mL of trimethyl phosphate with stirring to facilitate dissolution. The reaction was brought to 0°C, followed by slowly adding with syringe phosphorus oxychloride (120 μL, 1.3 mmol, 1.5 eq) and stirring continuously at 0°C for 1.5h. The two-necked 2# flask was filled with argon, followed by adding with syringe 5mL of anhydrous DMF and DIPEA (740 μL, 4.25 mmol, 5 eq) with stirring at 0°C to facilitate dissolution. The mixture was maintained at 0°C and continuously stirred for 3h, followed by adding 20 mL of solution of TEAB (0.1mol/L) to quench the reaction. The reaction mixture was continuously stirred for 0.5h, diluted with 50mL of deionized water, and then separated by DEAE resin column prepared-in-advance and eluted with gradient elution ($H_2O$:TEAB(1mol/L)=10/1~0/1, DEAE column diameter 4.5cm, filler height 8cm). The fractions containing the triphosphated product were collected and concentrated with a rotary evaporator under vacuum, followed by preparative liquid chromatography separation with gradient elution ($CH_3CN$:TEAB(0.1mol/L)=2/98~98/2). The high-purity product solution recovered from the separation was concentrated with a rotary evaporator under vacuum, transferred to a plastic centrifuge tube, and then freeze-dried with a lyophilizer into a powder solid. MW = 715. The yield of this step was 40%.
$^1$H NMR (400 MHz, DMSO-$d_6$) δ 8.60 (s, 1H), 8.15 (s, 1H), 8.05 (d, *J* = 6.5 Hz, 1H), 7.69 (t, *J* = 8.2 Hz, 1H), 7.60 - 7.54 (m, 2H), 7.27 (s, 2H), 6.25 - 6.18 (m, 1H), 5.70 (t, *J* = 5.0 Hz, 1H), 5.22 - 5.10 (m, 1H), 4.40 - 4.27 (m, 1H), 4.21 (dd, *J* = 11.0, 5.0 Hz, 1H), 4.01 (dd, *J* = 7.0, 3.7 Hz, 1H), 2.59 - 2.52 (m, 1H), 2.48 - 2.30 (m, 3H), 1.65 (d, *J* = 7.0 Hz, 3H), 1.07 (t, *J* = 5.1 Hz, 3H).

Example 9 (Not according to the invention)

(1) Step 1

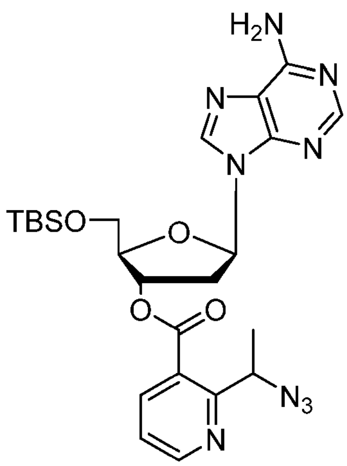

The product was synthesized similarly to Example 4 step 1, wherein 2-(1-azidoethyl)benzoic acid was replaced by 2-(1-azidoethyl) nicotinic acid (available from Beijing Okeanos Tech. Co., Ltd., cat. No. : OK-H-20002), 85% yield, MW = 539.

$^1$H NMR (400 MHz, DMSO-$d_6$) δ 8.86 (dd, *J* = 4.7, 1.7 Hz, 1H), 8.38 (dt, *J* = 7.9, 1.7 Hz, 1H), 8.33 (s, 1H), 8.16 (d, *J* = 0.9 Hz, 1H), 7.58 (dd, *J* = 7.9, 4.7 Hz, 1H), 7.32 (s, 2H), 6.47 (ddd, *J* = 8.5, 6.1, 2.9 Hz, 1H), 5.66 (dq, *J* = 6.2, 2.0 Hz, 1H), 5.41 - 5.24 (m, 1H), 4.44 - 4.23 (m, 1H), 4.05 - 3.77 (m, 2H), 3.13 (ddd, *J* = 14.3, 8.3, 6.3 Hz, 1H), 2.87 - 2.68 (m, 1H), 1.61 (d, *J* = 6.7 Hz, 3H), 0.86 (s, 9H), 0.04 (d, *J* = 3.9 Hz, 6H).

(2) Step 2

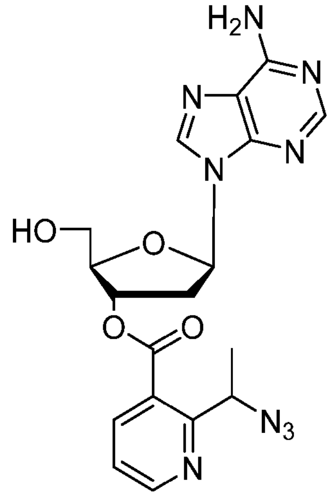

The product is synthesized similarly to Example 4 Step 2, wherein 2-(1-azidoethyl)benzoic acid was replaced by 2-(1-azidoethyl) nicotinic acid (available from Beijing Okeanos Tech. Co., Ltd., cat. No. : OK-H-20002), 75% yield, MW = 425.

$^1$H NMR (500 MHz, DMSO-$d_6$) δ 8.86 (dd, *J* = 4.8, 1.8 Hz, 1H), 8.45 - 8.31 (m, 2H), 8.16 (d, *J* = 1.1 Hz, 1H), 7.59 (dd, *J* = 7.9, 4.7 Hz, 1H), 7.38 (s, 2H), 6.47 (ddd, *J* = 9.4, 5.8, 4.2 Hz, 1H), 5.71 - 5.62 (m, 1H), 5.57 (ddd, *J* = 7.5, 4.8, 2.8 Hz, 1H), 5.33 (p, *J* = 6.7 Hz, 1H), 4.34 (ddt, *J* = 7.6, 4.1, 2.2 Hz, 1H), 3.83 - 3.60 (m, 2H), 3.09 (ddd, *J* = 14.5, 9.0, 5.9 Hz, 1H), 2.85 - 2.65 (m, 1H), 1.61 (dd, *J* = 6.7, 2.7 Hz, 3H).

(3) Step 3

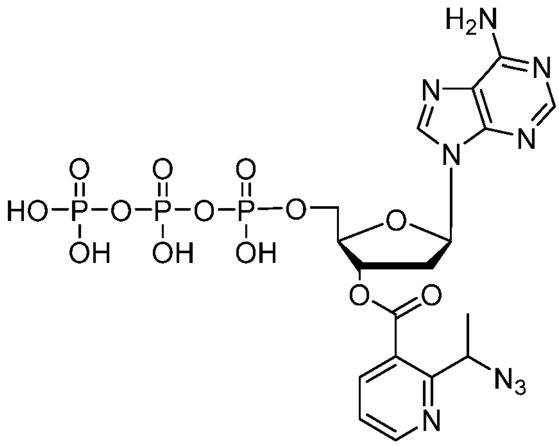

The product was synthesized similarly to Example 4 step 3, wherein 2-(1-azidoethyl)benzoic acid was replaced by 2-(1-azidoethyl) nicotinic acid (available from Beijing Okeanos Tech. Co., Ltd., cat. No. : OK-H-20002), 40% yield, MW =

665.

$^1$H NMR (400 MHz, DMSO-$d_6$) δ 8.85 (d, $J$ = 3.6 Hz, 1H), 8.66 (d, $J$ = 3.4 Hz, 1H), 8.40 (d, $J$ = 7.3 Hz, 1H), 8.15 (s, 1H), 7.58 (dd, $J$ = 7.9, 4.8 Hz, 1H), 7.31 (s, 2H), 6.54 - 6.45 (m, 1H), 5.77 (d, $J$ = 5.1 Hz, 1H), 5.37 - 5.28 (m, 1H), 4.44 (d, $J$ = 4.5 Hz, 1H), 4.14 - 4.02 (m, 2H), 3.23 - 3.12 (m, 1H), 2.76 - 2.66 (m, 1H), 1.61 (dd, $J$ = 6.7, 2.1 Hz, 3H).

Example 10 (Not according to the invention)

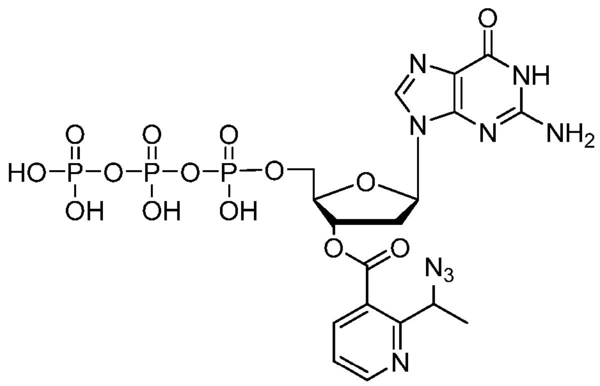

The product was synthesized similarly to Example 2 Steps 1-3, wherein 2-(1-azidoethyl)benzoic acid was replaced by 2-(1-azidoethyl) nicotinic acid (available from Beijing Okeanos Tech. Co., Ltd., cat. No. : OK-H-20002)), yield for the last step was 40%, MW = 681.

$^1$H NMR (400 MHz, DMSO-$d_6$) δ 10.77 (s, 1H), 8.84 (d, $J$ = 4.6 Hz, 1H), 8.39 - 8.29 (m, 1H), 8.02 (d, $J$ = 5.5 Hz, 1H), 7.57 (dd, $J$ = 7.8, 4.8 Hz, 1H), 6.71 (s, 2H), 6.28 - 6.16 (m, 1H), 5.73 (d, $J$ = 4.9 Hz, 1H), 5.35 - 5.28 (m, 1H), 4.36 (dd, $J$= 10.4, 6.0 Hz, 1H), 4.24 - 4.15 (m, 1H), 4.01 - 3.97 (m, 1H), 3.25 - 3.13 (m, 1H), 2.61 - 2.55 (m, 1H), 1.60 (dd, $J$ = 6.6, 2.6 Hz, 3H).

Example 11 (Not according to the invention)

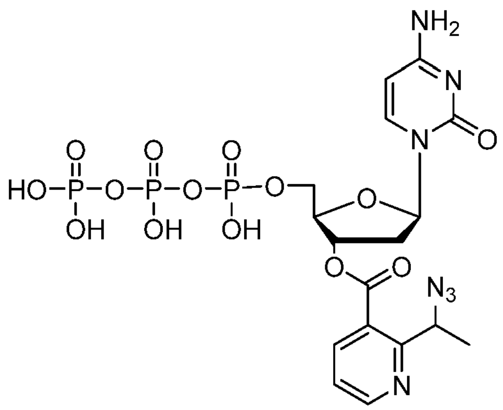

The product was synthesized similarly to Example 3 Steps 1-4, wherein 2-(1-azidoethyl)benzoic acid was replaced by 2-(1-azidoethyl) nicotinic acid (available from Beijing Okeanos Tech. Co., Ltd., cat. No. : OK-H-20002), yield for the last two steps was 30%, MW = 641.

$^1$H NMR (400 MHz, DMSO-$d_6$) δ 8.83 (d, $J$ = 3.5 Hz, 1H), 8.33 (d, $J$ = 7.8 Hz, 1H), 7.95 (dd, $J$ = 7.4, 2.9 Hz, 1H), 7.55 (dd, $J$ = 7.9, 4.7 Hz, 1H), 7.30 (s, 1H), 7.08 (s, 1H), 6.37 - 6.34 (m, 1H), 5.82 (d, $J$ = 7.4 Hz, 1H), 5.54 (d, $J$ = 4.9 Hz, 1H), 5.39 - 5.20 (m, 1H), 4.39 - 4.32 (m, 1H), 4.14 - 3.98 (m, 2H), 2.47 - 2.41 (m, 1H), 2.36 - 2.24 (m, 1H), 1.59 (dd, $J$ = 6.7, 1.7 Hz, 3H).

Example 12 (Not according to the invention)

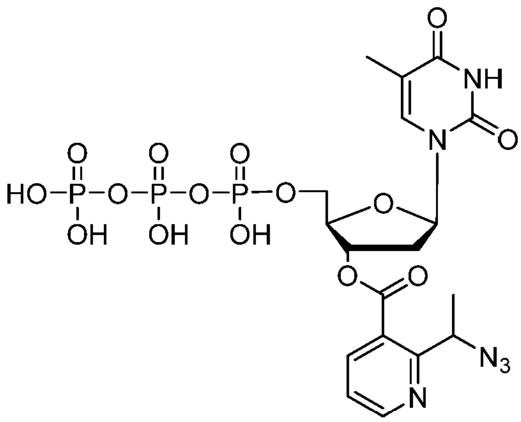

The product was synthesized similarly to Example 1 Steps 1-3, wherein 2-(1-azidoethyl)benzoic acid was replaced by 2-(1-azidoethyl) nicotinic acid (available from Beijing Okeanos Tech. Co., Ltd., cat. No. : OK-H-20002), yield for the last step was 45%, MW = 656.

$^1$H NMR (400 MHz, DMSO-$d_6$) δ 11.31 (s, 1H), 8.84 (dd, *J* = 4.6, 1.1 Hz, 1H), 8.34 (d, *J* = 7.9 Hz, 1H), 7.98 (s, 1H), 7.56 (dd, *J* = 7.9, 4.8 Hz, 1H), 6.39 - 6.32 (m, 1H), 5.63 (d, *J* = 5.2 Hz, 1H), 5.33 - 5.26 (m, 1H), 4.35 (s, 1H), 4.15 - 4.04 (m, 2H), 2.55 - 2.52 (m, 1H), 2.46 - 2.38 (m, 1H), 1.84 (s, 3H), 1.58 (dd, *J* = 6.6, 1.3 Hz, 3H).

Example 13

(1) Step 1

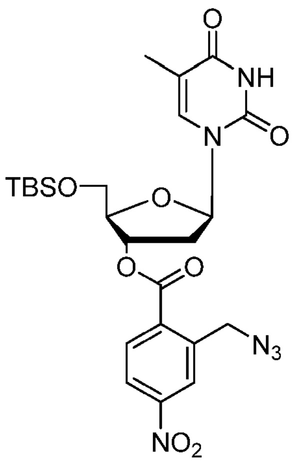

The product was synthesized similarly to Example 1 step 1, wherein 2-(1-azidoethyl)benzoic acid was replaced by 2-(azidomethyl)-4-nitrobenzoic acid (available from Beijing Okeanos Tech. Co., Ltd., cat. No. : OK-H-21004), the yield of this step was 85%, MW = 560.

$^1$H NMR (500 MHz, $CDCl_3$) δ 8.43 (d, *J* = 2.3 Hz, 1H), 8.34 - 8.23 (m, 2H), 8.20 (d, *J* = 8.6 Hz, 1H), 7.57 (d, *J* = 1.2 Hz, 1H), 6.45 (dd, *J* = 9.3, 5.2 Hz, 1H), 5.54 (d, *J* = 5.9 Hz, 1H), 5.04 - 4.84 (m, 2H), 4.28 (q, *J* = 1.8 Hz, 1H), 4.12 - 3.92 (m, 2H), 3.48 (s, 1H), 2.60 (dd, *J* = 14.0, 5.3 Hz, 1H), 2.28 (ddd, *J* = 14.0, 9.3, 6.1 Hz, 2H), 1.95 (d, *J* = 1.2 Hz, 3H), 0.97 (s, 9H), 0.18 (d, *J* = 1.1 Hz, 6H).

(2) Steps 2-3

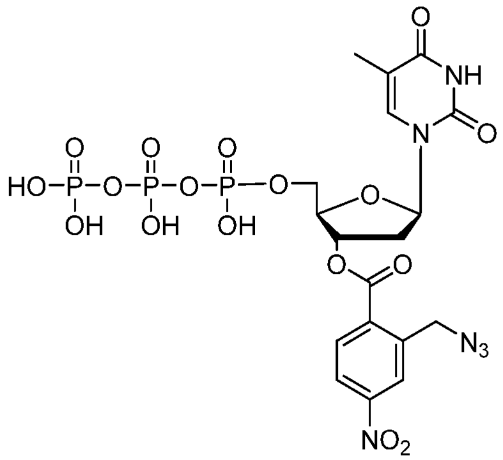

The product was synthesized similarly to Example 1 Steps 2-3, wherein 2-(1-azidoethyl)benzoic acid was replaced by 2-(azidomethyl)-4-nitrobenzoic acid (available from Beijing Okeanos Tech. Co., Ltd., cat. No. : OK-H-21004), yield for the last step was 40%, MW = 686.

$^1$H NMR (400 MHz, DMSO-$d_6$) δ 11.30 (s, 1H), 8.41 (d, *J* = 2.0 Hz, 1H), 8.33 (dd, *J* = 8.6, 2.2 Hz, 1H), 8.23 (d, *J* = 8.6 Hz, 1H), 7.95 (s, 1H), 6.36 (dd, *J* = 9.0, 6.0 Hz, 1H), 5.65 (d, *J* = 5.2 Hz, 1H), 4.97 (s, 2H), 4.37 (s, 1H), 4.15 - 4.04 (m, 2H), 2.56 - 2.52 (m, 1H), 2.44 (dd, *J* = 14.0, 5.9 Hz, 1H), 1.84 (s, 3H).

Example 14

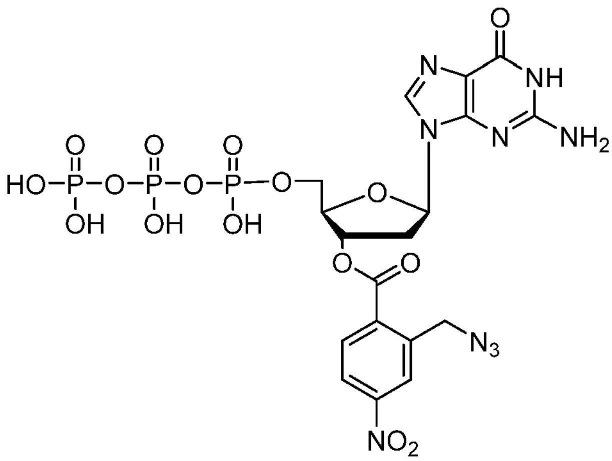

The product was synthesized similarly to Example 2 Steps 1-3, wherein 2-(1-azidoethyl)benzoic acid was replaced by 2-(azidomethyl)-4-nitrobenzoic acid (available from Beijing Okeanos Tech. Co., Ltd., cat. No. : OK-H-21004), yield for the last step was 40%, MW = 711.

$^1$H NMR (400 MHz, DMSO-$d_6$) δ 11.00 (s, 1H), 8.42 (d, *J* = 2.3 Hz, 1H), 8.34 (dd, *J* = 8.6, 2.3 Hz, 1H), 8.25 (d, *J* = 8.6 Hz, 1H), 8.03 (s, 1H), 6.82 (s, 2H), 6.23 (dd, *J* = 9.2, 5.9 Hz, 1H), 5.76 (d, *J* = 4.2 Hz, 1H), 4.99 (s, 2H), 4.39 (t, *J* = 4.8 Hz, 1H), 4.25 - 4.00 (m, 2H), 3.31 - 3.14 (m, 1H), 2.59 (dd, *J* = 14.0, 6.4 Hz, 1H).

Example 15

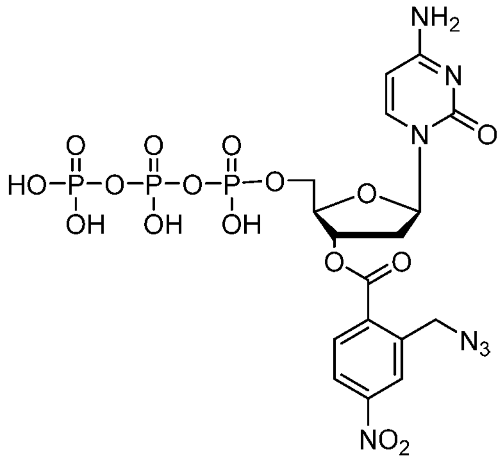

The product was synthesized similarly to Example 3 Steps 1-4, wherein 2-(1-azidoethyl)benzoic acid was replaced by 2-(azidomethyl)-4-nitrobenzoic acid (available from Beijing Okeanos Tech. Co., Ltd., cat. No. : OK-H-21004), yield for the last two steps was 30%, MW = 671.
$^1$H NMR (400 MHz, DMSO-$d_6$) δ 8.41 (d, $J$ = 2.1 Hz, 1H), 8.33 (dd, $J$ = 8.5, 2.3 Hz, 1H), 8.23 (d, $J$ = 8.6 Hz, 1H), 7.95 (d, $J$ = 7.5 Hz, 1H), 7.28 (s, 1H), 7.07 (s, 1H), 6.37 (dd, $J$ = 9.1, 5.6 Hz, 1H), 5.81 (d, $J$ = 7.5 Hz, 1H), 5.56 (d, $J$ = 5.4 Hz, 1H), 4.98 (s, 2H), 4.37 (s, 1H), 4.12 - 3.99 (m, 2H), 2.47 - 2.40 (m, 1H), 2.36 - 2.26 (m, 1H).

Example 16

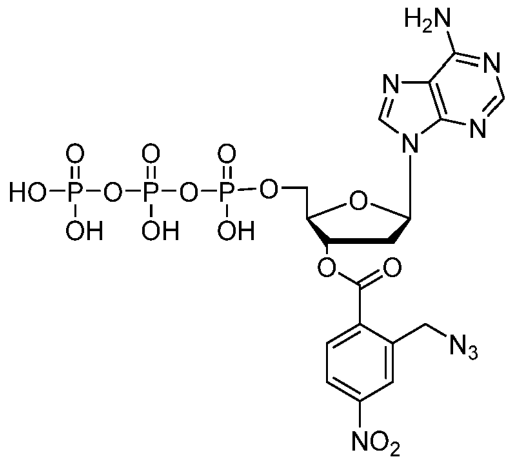

The product was synthesized similarly to Example 4 Steps 1-3, wherein 2-(1-azidoethyl)benzoic acid was replaced by 2-(azidomethyl)-4-nitrobenzoic acid (available from Beijing Okeanos Tech. Co., Ltd., cat. No. : OK-H-21004), yield for the last step was 40%, MW = 695.
$^1$H NMR (400 MHz, DMSO-$d_6$) δ 8.58 (s, 1H), 8.42 (d, $J$ = 1.9 Hz, 1H), 8.34 (dd, $J$ = 8.5, 2.2 Hz, 1H), 8.29 (d, $J$ = 8.6 Hz, 1H), 8.15 (s, 1H), 7.29 (s, 2H), 6.51 (dd, $J$ = 9.0, 6.1 Hz, 1H), 5.77 (d, $J$ = 5.3 Hz, 1H), 5.02 (s, 2H), 4.47 (s, 1H), 4.14 - 4.00 (m, 2H), 3.17 - 3.08 (m, 1H), 2.75 - 2.70 (m, 1H).

Example 17

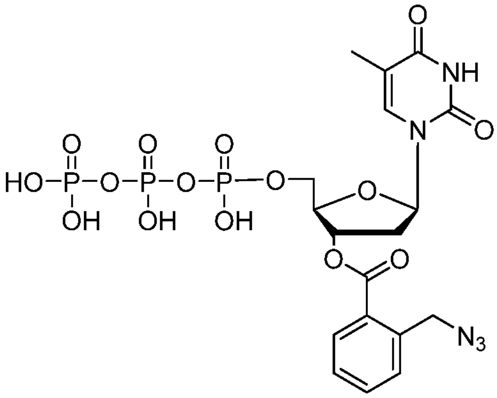

The product was synthesized similarly to Example 1 Steps 1-3, wherein 2-(1-azidoethyl)benzoic acid was replaced by 2-(azidomethyl)benzoic acid (available from Beijing Okeanos Tech. Co., Ltd., cat. No. : OK-H-20003), yield for the last step was 45%, MW = 641.
$^1H$ NMR (400 MHz, DMSO-$d_6$) δ 11.30 (s, 1H), 7.99 (dd, *J* = 9.6, 8.7 Hz, 2H), 7.68 (td, *J* = 7.6, 1.1 Hz, 1H), 7.61 - 7.50 (m, 2H), 6.35 (dd, *J* = 9.3, 5.7 Hz, 1H), 5.60 (d, *J* = 5.3 Hz, 1H), 4.80 (s, 2H), 4.31 (s, 1H), 4.18 - 4.00 (m, 2H), 2.57 - 2.50 (m, 1H), 2.39 (dd, *J* = 13.9, 5.7 Hz, 1H), 1.84 (s, 3H).

Example 18

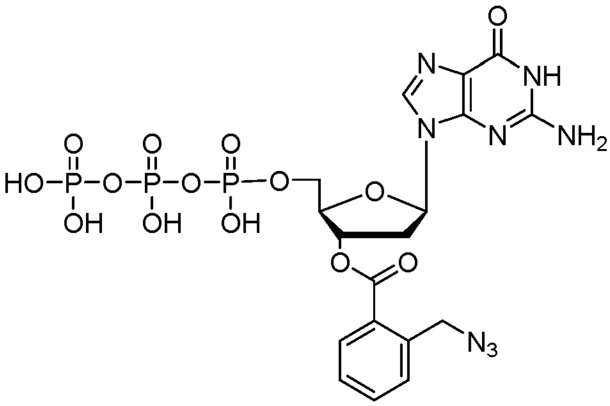

The product was synthesized similarly to Example 2 Steps 1-3, wherein 2-(1-azidoethyl)benzoic acid was replaced by 2-(azidomethyl)benzoic acid (available from Beijing Okeanos Tech. Co., Ltd., cat. No. : OK-H-20003), yield for the last step was 40%, MW = 666.
$^1H$ NMR (400 MHz, DMSO-$d_6$) δ 10.70 (s, 1H), 8.03 (d, *J* = 9.8 Hz, 2H), 7.69 (td, *J* = 7.6, 1.2 Hz, 1H), 7.60 - 7.51 (m, 2H), 6.67 (s, 2H), 6.23 (dd, *J* = 9.4, 5.8 Hz, 1H), 5.68 (d, *J* = 4.9 Hz, 1H), 4.82 (s, 2H), 4.40 - 4.30 (m, 1H), 4.21 - 4.16 (m, 1H), 4.05 - 3.96 (m, 1H), 3.22 - 3.10 (m, 1H), 2.60 - 2.51 (m, 1H).

Example 19

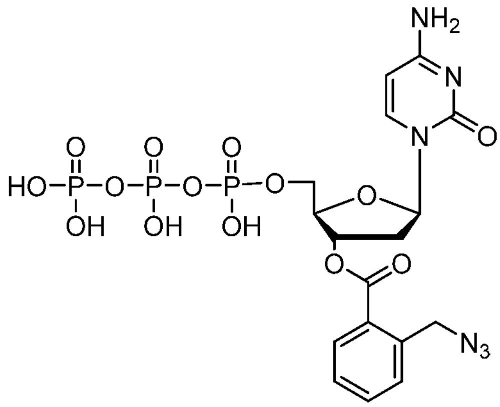

The product was synthesized similarly to Example 3 Steps 1-4, wherein 2-(1-azidoethyl)benzoic acid was replaced by 2-(azidomethyl)benzoic acid (available from Beijing Okeanos Tech. Co., Ltd., cat. No. : OK-H-20003), yield for the last two steps was 30%, MW = 626.

$^1$H NMR (400 MHz, DMSO-$d_6$) δ 8.00 (dd, *J* = 7.8, 1.1 Hz, 1H), 7.95 (d, *J* = 7.5 Hz, 1H), 7.68 (td, *J* = 7.6, 1.4 Hz, 1H), 7.61 - 7.50 (m, 2H), 7.29 (s, 1H), 7.07 (s, 1H), 6.37 (dd, *J* = 9.2, 5.5 Hz, 1H), 5.82 (d, *J* = 7.5 Hz, 1H), 5.51 (d, *J* = 5.4 Hz, 1H), 4.81 (s, 2H), 4.32 (s, 1H), 4.13 - 3.97 (m, 2H), 2.41 (dd, *J* = 13.8, 5.6 Hz, 1H), 2.30 (ddd, *J* = 14.4, 7.5, 4.6 Hz, 1H).

Example 20

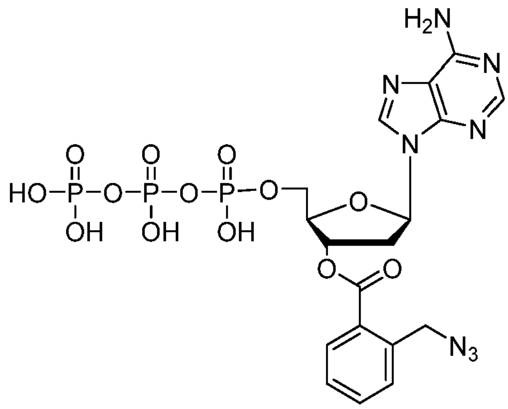

The product was synthesized similarly to Example 4 Steps 1-3, wherein 2-(1-azidoethyl)benzoic acid was replaced by 2-(azidomethyl)benzoic acid (available from Beijing Okeanos Tech. Co., Ltd., cat. No. : OK-H-20003), yield for the last step was 40%, MW = 650.

$^1$H NMR (400 MHz, DMSO-$d_6$) δ 8.60 (s, 1H), 8.15 (s, 1H), 8.05 (d, *J* = 6.5 Hz, 1H), 7.69 (t, *J* = 8.2 Hz, 1H), 7.60 - 7.54 (m, 2H), 7.27 (s, 2H), 6.49 (dd, *J* = 8.8, 5.8 Hz, 1H), 5.71 (d, *J* = 5.6 Hz, 1H), 4.83 (s, 2H), 4.42 - 4.34 (m, 1H), 4.02 - 3.80 (m, 2H), 2.72 - 2.63 (m, 1H), 2.35 - 2.30 (m, 1H).

Example 21

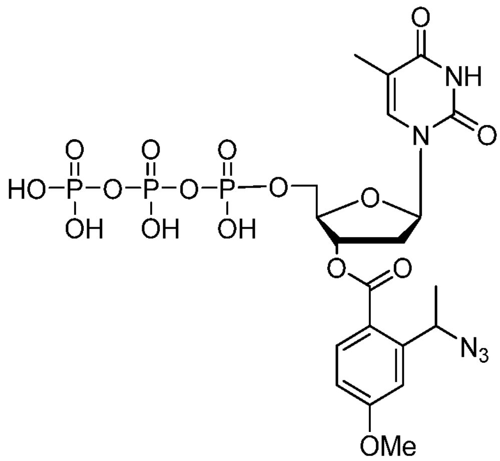

The product was synthesized similarly to Example 1 Steps 1-3, wherein 2-(1-azidoethyl)benzoic acid was replaced by 2-(1-azidoethyl)-4-methoxy benzoic acid (available from Beijing Okeanos Tech. Co., Ltd., cat. No. : OK-H-21005), yield for the last step was 45%, MW = 685.
$^1$H NMR (400 MHz, DMSO-$d_6$) δ 11.30 (s, 1H), 8.00 - 7.90 (m, 2H), 7.11 (d, *J* = 2.6 Hz, 1H), 7.04 (dd, *J* = 8.8, 2.6 Hz, 1H), 6.39 - 6.29 (m, 1H), 5.74 - 5.68 (m, 1H), 5.55 (d, *J* = 5.1 Hz, 1H), 4.30 (s, 1H), 4.16 - 3.98 (m, 2H), 3.86 (s, 3H), 2.48 - 2.30 (m, 2H), 1.84 (s, 3H), 1.48 (dd, *J* = 6.7, 1.4 Hz, 3H).

Example 22

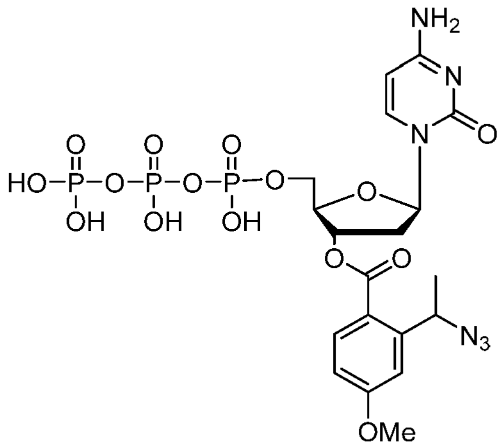

The product was synthesized similarly to Example 3 Steps 1-3, wherein 2-(1-azidoethyl)benzoic acid was replaced by 2-(1-azidoethyl)-4-methoxy benzoic acid (available from Beijing Okeanos Tech. Co., Ltd., cat. No. : OK-H-21005), yield for the last two steps was 30%, MW = 670.
$^1$H NMR (400 MHz, DMSO-$d_6$) δ 8.00 - 7.89 (m, 2H), 7.32 (s, 1H), 7.11 (d, *J* = 2.5 Hz, 2H), 7.05 (dd, *J* = 8.8, 2.6 Hz, 1H), 6.41 - 6.31 (m, 1H), 5.82 (d, *J* = 7.5 Hz, 1H), 5.74 - 5.68 (m, 1H), 5.49 (d, *J* = 4.5 Hz, 1H), 4.30 (s, 1H), 4.10 - 4.00 (m, 2H), 3.86 (s, 3H), 2.42 - 2.36 (m, 1H), 2.33 - 2.23 (m, 1H), 1.48 (dd, *J* = 6.6, 1.5 Hz, 3H).

Example 23

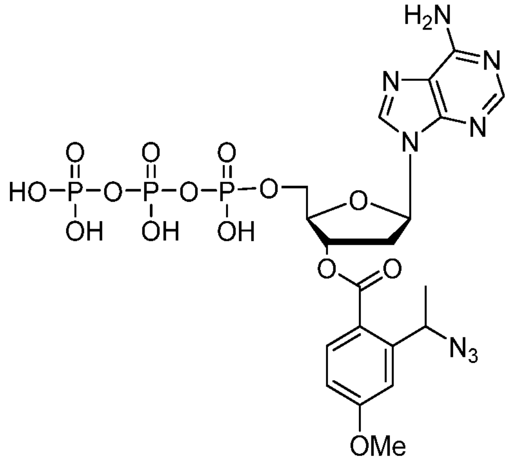

The product was synthesized similarly to Example 4 Steps 1-3, wherein 2-(1-azidoethyl)benzoic acid was replaced by 2-(1-azidoethyl)-4-methoxy benzoic acid (available from Beijing Okeanos Tech. Co., Ltd., cat. No. : OK-H-21005), the yield for the last step was 40%, MW = 694.
$^1$H NMR (400 MHz, DMSO-$d_6$) δ 8.61 (d, $J$ = 3.4 Hz, 1H), 8.16 (d, $J$ = 1.4 Hz, 1H), 8.02 (dd, $J$ = 8.7, 1.7 Hz, 1H), 7.30 (s, 2H), 7.12 (d, $J$ = 2.5 Hz, 1H), 7.07 (dd, $J$ = 8.8, 2.6 Hz, 1H), 6.51 - 6.46 (m, 1H), 5.78 - 5.72 (m, 1H), 5.68 (d, $J$ = 5.2 Hz, 1H), 4.43 - 4.36 (m, 1H), 4.14 - 4.00 (m, 2H), 3.87 (s, 3H), 3.18 - 3.08 (m, 1H), 2.71 - 2.62 (m, 1H), 1.50 (dd, $J$ = 6.6, 2.0 Hz, 3H).

Example 24 (Not according to the invention)

(1) Steps 1-2

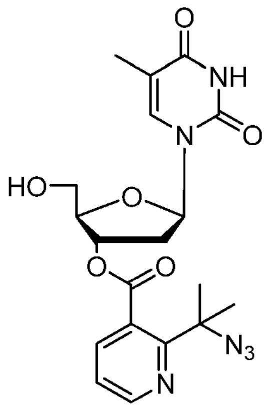

The product was synthesized similarly to Example 1 steps 1-2, wherein 2-(1-azidoethyl)benzoic acid was replaced by 2-(2-azidopropan-2-yl) nicotinic acid (available from Beijing Okeanos Tech. Co., Ltd., cat. No. : OK-H-20004), yield for the last step was 90%, MW = 430.
$^1$H NMR (400 MHz, $CDCl_3$) δ 9.12 (s, 1H), 8.31 (dd, $J$ = 4.8, 1.7 Hz, 1H), 7.39 (dd, $J$ = 7.7, 1.6 Hz, 1H), 6.96 (dd, $J$ = 7.3, 5.2 Hz, 1H), 5.97 (t, $J$ = 7.2 Hz, 1H), 5.33 (dd, $J$ = 5.1, 2.7 Hz, 1H), 4.02 (d, $J$ = 2.3 Hz, 1H), 3.70 (d, $J$ = 2.4 Hz, 2H), 2.72 (s, 1H), 2.25 (dd, $J$ = 10.7, 5.1 Hz, 2H), 1.60 (s, 3H), 1.45 (s, 6H).

(2) Step 3

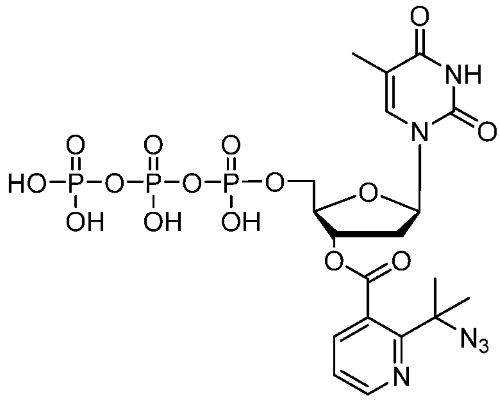

The product was synthesized similarly to Example 1 step 3, wherein 2-(1-azidoethyl)benzoic acid was replaced by 2-(2-azidopropan-2-yl) nicotinic acid (available from Beijing Okeanos Tech. Co., Ltd., cat. No. : OK-H-20004), the yield of this step was 35%, MW = 670.

$^1$H NMR (400 MHz, DMSO-$d_6$) δ 11.30 (s, 1H), 8.67 (dd, $J$ = 4.7, 1.4 Hz, 1H), 8.02 (dd, $J$ = 7.7, 1.4 Hz, 1H), 7.96 (s, 1H), 7.47 (dd, $J$ = 7.7, 4.8 Hz, 1H), 6.29 (dd, $J$ = 9.2, 5.7 Hz, 1H), 5.61 (d, $J$ = 5.3 Hz, 1H), 4.31 (s, 1H), 4.19 - 4.03 (m, 2H), 2.56 - 2.52 (m, 1H), 2.36 (dd, $J$ = 13.9, 5.6 Hz, 1H), 1.84 (s, 3H), 1.70 (d, $J$ = 3.8 Hz, 6H).

Example 25

(1) Steps 1-2

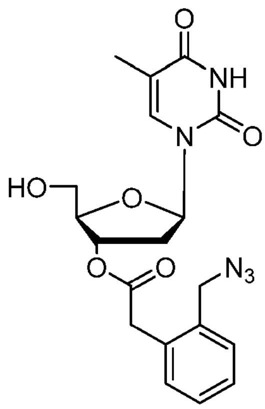

The product was synthesized similarly to Example 1 steps 1-2, wherein 2-(1-azidoethyl)benzoic acid was replaced by 2-(2-(azidomethyl)phenyl)acetic acid (available from Beijing Okeanos Tech. Co., Ltd., cat. No. : OK-H-20005), yield for the last step was 90%, MW = 415.

$^1$H NMR (500 MHz, $CDCl_3$) δ 8.08 (s, 1H), 7.46 (d, $J$ = 1.1 Hz, 1H), 7.39 - 7.32 (m, 3H), 7.30 (d, $J$ = 6.8 Hz, 1H), 6.20 (dd, $J$ = 8.5, 5.9 Hz, 1H), 5.37 - 5.35 (m, 1H), 4.40 (s, 2H), 4.08 (q, $J$ = 2.4 Hz, 1H), 3.90 (qd, $J$ = 11.8, 2.6 Hz, 2H), 3.76 (s, 2H), 2.48 - 2.33 (m, 2H), 1.92 (d, $J$ = 1.0 Hz, 3H).

(2) Step 3

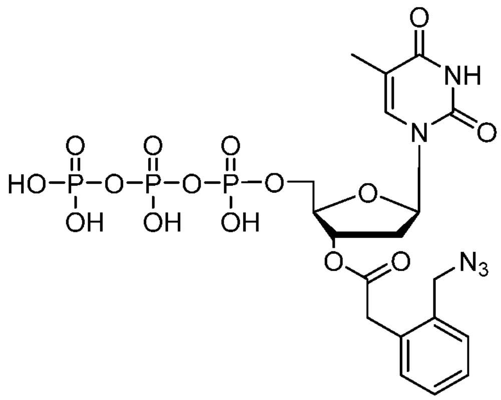

The product was synthesized similarly to Example 1 step 3, wherein 2-(1-azidoethyl)benzoic acid was replaced by 2-(2-(azidomethyl)phenyl)acetic acid (available from Beijing Okeanos Tech. Co., Ltd., cat. No. : OK-H-20005), the yield of this step was 40%, MW = 655.
$^1$H NMR (400 MHz, DMSO-$d_6$) δ 11.33 (s, 1H), 7.91 (s, 1H), 7.43 - 7.28 (m, 4H), 6.26 (dd, *J* = 9.4, 5.6 Hz, 1H), 5.34 (d, *J* = 5.4 Hz, 1H), 4.50 (s, 2H), 4.09 (s, 1H), 4.05 - 3.95 (m, 2H), 3.84 (s, 2H), 2.44 - 2.31 (m, 1H), 2.18 (dd, *J* = 13.9, 5.5 Hz, 1H), 1.82 (s, 3H).

Example 26

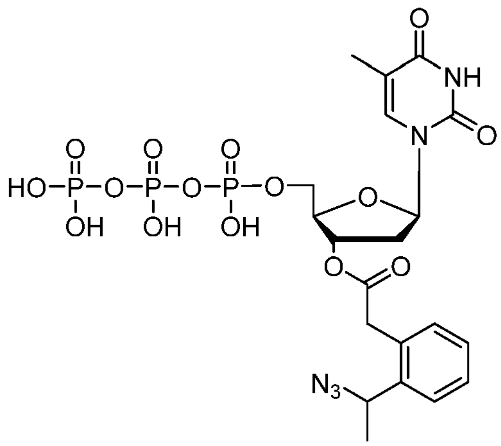

The product was synthesized similarly to Example 1 Steps 1-3, wherein 2-(1-azidoethyl)benzoic acid was replaced by 2-(2-(1-azidoethyl)phenyl)acetic acid (available from Beijing Okeanos Tech. Co., Ltd., cat. No. : OK-H-20006), yield for the last step was 40%, MW = 669.
$^1$H NMR (400 MHz, DMSO-$d_6$) δ 11.29 (s, 1H), 7.87 (s, 1H), 7.44 (d, *J* = 7.6 Hz, 1H), 7.35 (dt, *J* = 7.9, 4.2 Hz, 1H), 7.30 (d, *J* = 4.0 Hz, 2H), 6.26 (dd, *J* = 9.5, 5.6 Hz, 1H), 5.33 (d, *J* = 5.4 Hz, 1H), 5.05 (q, *J* = 6.7 Hz, 1H), 4.10 (s, 1H), 4.02 - 3.94 (m, 2H), 3.90 (d, *J* = 2 Hz, 2H), 2.42 - 2.33 (m, 1H), 2.21 - 2.12 (m, 1H), 1.81 (s, 3H), 1.45 (dd, *J* = 6.7, 1.3 Hz, 3H).

Example 27

(1) Steps 1-2

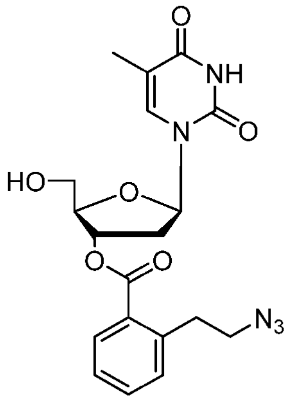

The product was synthesized similarly to Example 1 steps 1-2, wherein 2-(1-azidoethyl)benzoic acid was replaced by 2-(2-azidoethyl)benzoic acid (available from Beijing Okeanos Tech. Co., Ltd., cat. No. : OK-H-21006), yield for the last step was 90%, MW = 415.
$^1$H NMR (500 MHz, $CDCl_3$) δ 8.40 (s, 1H), 7.98 (d, *J* = 7.8 Hz, 1H), 7.57 - 7.49 (m, 2H), 7.39 - 7.30 (m, 2H), 6.36 - 6.29 (m, 1H), 5.64 - 5.56 (m, 1H), 4.26 (d, *J* = 2.4 Hz, 1H), 4.02 (d, *J* = 2.5 Hz, 2H), 3.54 (t, *J* = 7.0 Hz, 2H), 3.27 (t, *J* = 7.1 Hz, 2H), 2.60 - 2.50 (m, 2H), 1.95 (s, 3H).

(2) Step 3

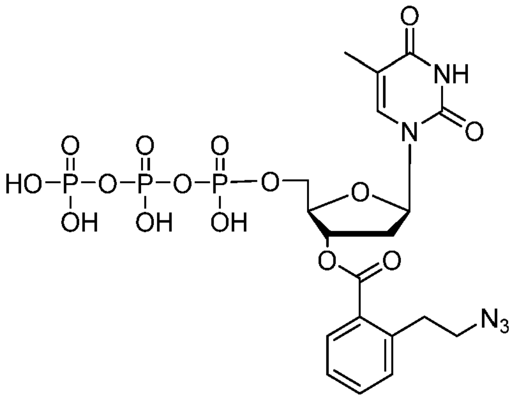

The product was synthesized similarly to Example 1 step 3, wherein 2-(1-azidoethyl)benzoic acid was replaced by 2-(2-azidoethyl)benzoic acid (available from Beijing Okeanos Tech. Co., Ltd., cat. No. : OK-H-21006), the yield of this step was 40%, MW = 655.
$^1$H NMR (400 MHz, DMSO-$d_6$) δ 11.28 (s, 1H), 7.98 - 7.85 (m, 2H), 7.57 (t, *J* = 7.4 Hz, 1H), 7.42 (t, *J* = 7.7 Hz, 2H), 6.34 (dd, *J* = 9.3, 5.7 Hz, 1H), 5.57 (d, *J* = 5.3 Hz, 1H), 4.30 (s, 1H), 4.08 - 4.02 (m, 2H), 3.57 (t, *J* = 6.9 Hz, 2H), 3.21 (t, *J* = 6.9 Hz, 2H), 2.55 - 2.52 (m, 1H), 2.43 - 2.27 (m, 1H), 1.84 (s, 3H).

Example 28 (Not according to the invention)

(1) Steps 1-2

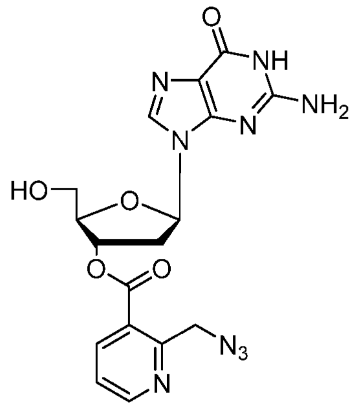

The product was synthesized similarly to Example 2 steps 1-2, wherein 2-(1-azidoethyl)benzoic acid was replaced by 2-(azidomethyl) nicotinic acid (available from Beijing Okeanos Tech. Co., Ltd., cat. No. : OK-H-21007), yield for the last step was 90%, MW = 427.
$^1$H NMR (400 MHz, $CDCl_3$) δ 8.52 (s, 2H), δ = 8.20 (d, 1H), δ = 7.90 (d, 1H), δ = 7.58 (s, 1H), δ = 7.35 (dd, 1H), δ = 6.26 (t, 1H), δ = 5.59 (dq, 1H), δ = 5.11 (s, 2H), δ = 4.61 (dt, 1H), δ = 3.90 (m, 2H), δ = 2.38 (m, 2H).

(2) Step 3

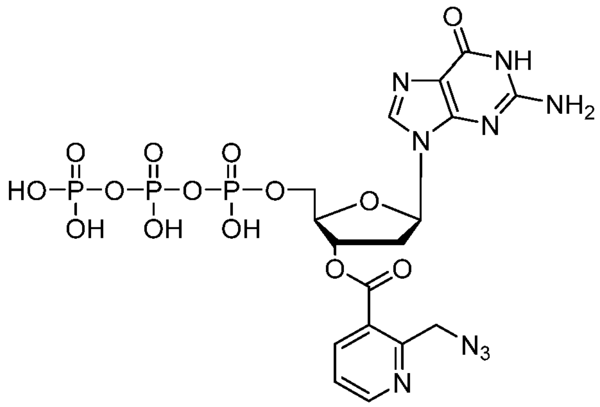

The product was synthesized similarly to Example 2 step 3, wherein 2-(1-azidoethyl)benzoic acid was replaced by 2-(azidomethyl) nicotinic acid (available from Beijing Okeanos Tech. Co., Ltd., cat. No. : OK-H-21007), the yield of this step was 30%, MW = 667.
$^1$H NMR (400 MHz, DMSO-$d_6$) δ 10.76 (s, 1H), 8.83 (dd, $J$ = 4.8, 1.5 Hz, 1H), 8.40 (dd, $J$ = 7.9, 1.7 Hz, 1H), 8.00 (d, $J$ = 6.6 Hz, 1H), 7.59 (dd, $J$ = 7.9, 4.8 Hz, 1H), 6.73 (s, 2H), 6.23 (dd, $J$ = 9.3, 5.8 Hz, 1H), 5.72 (d, $J$ = 4.8 Hz, 1H), 4.85 (s, 2H), 4.36 (d, $J$ = 4.9 Hz, 1H), 4.27 - 4.16 (m, 1H), 4.01 - 3.93 (m, 1H), 3.26 - 3.18 (d, $J$ = 14.3 Hz, 1H), 2.56 (dd, $J$ = 13.7, 5.3 Hz, 1H).

Example 29 (Not according to the invention)

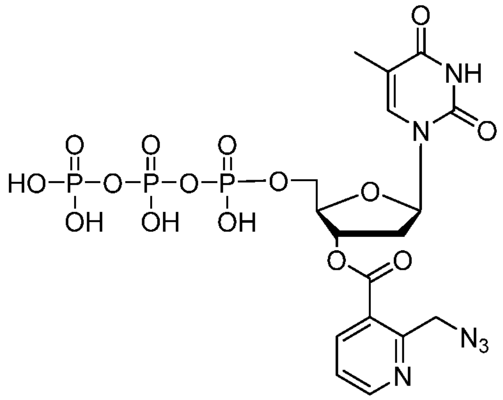

The product was synthesized similarly to Example 1 Steps 1-3, wherein 2-(1-azidoethyl)benzoic acid was replaced by 2-(azidomethyl) nicotinic acid (available from Beijing Okeanos Tech. Co., Ltd., cat. No. : OK-H-21007), yield for the last step was 40%, MW = 642.
$^1$H NMR (400 MHz, DMSO-$d_6$) $\delta$ 11.29 (s, 1H), 8.81 (dd, $J$ = 4.8, 1.7 Hz, 1H), 8.39 (dd, $J$ = 7.9, 1.7 Hz, 1H), 7.89 (d, $J$ = 4.2 Hz, 1H), 7.58 (dd, $J$ = 7.9, 4.8 Hz, 1H), 6.35 (dd, $J$ = 9.0, 6.0 Hz, 1H), 5.59 (d, $J$ = 4.8 Hz, 1H), 4.83 (s, 2H), 4.35 (s, 1H), 4.14 - 3.97 (m, 2H), 2.46 - 2.36 (m, 2H), 1.83 (d, $J$ = 2.7 Hz, 3H).

Example 30

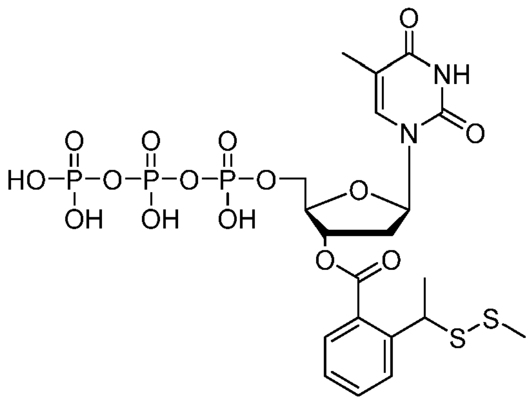

The product was synthesized similarly to Example 5 Steps 1-3, wherein 2-(1-(ethyldisulfaneyl)ethyl)benzoic acid was replaced by 2-(1-(methyldisulfaneyl)ethyl)benzoic acid (available from Beijing Okeanos Tech. Co., Ltd., cat. No. : OK-H-20007), yield for the last step was 30%, MW = 692.
$^1$H NMR (400 MHz, $D_2O$) $\delta$ 7.90 - 7.82 (m, 2H), 7.69 - 7.58 (m, 2H), 7.46 - 7.39 (m, 1H), 6.47 - 6.35 (m, 1H), 5.73 (s, 1H), 5.10 - 5.01 (m, 1H), 4.57 (dd, $J$ = 13.6, 3.0 Hz, 1H), 4.41 - 4.24 (m, 2H), 2.65 - 2.52 (m, 2H), 1.98 (d, $J$ = 15.2 Hz, 3H), 1.94 (s, 3H), 1.68 (d, $J$ = 7.2 Hz, 3H).

Example 31

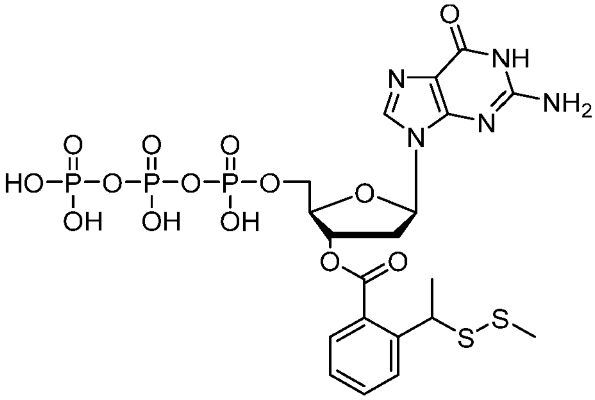

The product was synthesized similarly to Example 6 Steps 1-3, wherein 2-(1-(ethyldisulfaneyl)ethyl)benzoic acid was replaced by 2-(1-(methyldisulfaneyl)ethyl)benzoic acid (available from Beijing Okeanos Tech. Co., Ltd., cat. No. : OK-H-20007), yield for the last step was 30%, MW = 717.
$^1$H NMR (400 MHz, $D_2O$) δ 8.14 (t, *J* = 3.1 Hz, 1H), 7.82 (dd, *J* = 8.1, 3.1 Hz, 1H), 7.55 (q, *J* = 3.2 Hz, 2H), 7.38 - 7.34 (m, 1H), 6.31 - 6.22 (m, 1H), 5.76 (d, *J* = 4.1 Hz, 1H), 5.10 - 5.00 (m, 1H), 4.65 - 4.55 (m, 1H), 4.36 - 4.21 (m, 2H), 3.02 - 2.94 (m, 1H), 2.71 - 2.61 (m, 1H), 1.94 (dd, *J* = 22.2, 3.2 Hz, 3H), 1.62 (dd, *J* = 7.2, 3.3 Hz, 3H).

Example 32

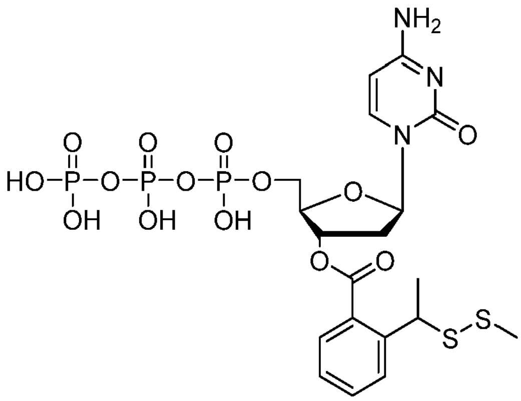

The product was synthesized similarly to Example 7 Steps 1-4, wherein 2-(1-(ethyldisulfaneyl)ethyl)benzoic acid was replaced by 2-(1-(methyldisulfaneyl)ethyl)benzoic acid (available from Beijing Okeanos Tech. Co., Ltd., cat. No. : OK-H-20007), yield for the last two steps was 28%, MW = 677.
$^1$H NMR (400 MHz, $D_2O$) δ 8.08 - 8.00 (m, 1H), 7.91 - 7.84 (m, 1H), 7.72 - 7.59 (m, 2H), 7.49 - 7.40 (m, 1H), 6.48 - 6.40 (m, 1H), 6.19 (dd, *J* = 7.7, 2.3 Hz, 1H), 5.73 - 5.64 (m, 1H), 5.13 - 5.02 (m, 1H), 4.60 (d, *J* = 12.6 Hz, 1H), 4.36 - 4.27 (m, 2H), 2.71 - 2.64 (m, 1H), 2.55 - 2.44 (m, 1H), 2.00 (dd, *J* = 9.5, 2.2 Hz, 3H), 1.69 (dd, *J* = 7.2, 2.1 Hz, 3H).

Example 33

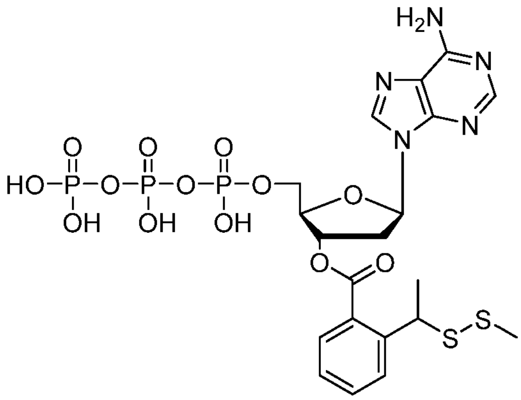

The product was synthesized similarly to Example 8 Steps 1-3, wherein 2-(1-(ethyldisulfaneyl)ethyl)benzoic acid was replaced by 2-(1-(methyldisulfaneyl)ethyl)benzoic acid (available from Beijing Okeanos Tech. Co., Ltd., cat. No. : OK-H-20007), yield for the last step was 35%, MW = 701.
$^1$H NMR (400 MHz, $D_2O$) δ 8.53 (dd, $J$ = 5.2, 1.8 Hz, 1H), 8.18 - 8.10 (m, 1H), 7.83 (d, $J$ = 7.8 Hz, 1H), 7.55 - 7.51 (m, 2H), 7.38 - 7.34 (m, 1H), 6.49 - 6.40 (m, 1H), 5.81 - 5.73 (m, 1H), 5.07 - 5.00 (m, 1H), 4.67 - 4.57 (m, 1H), 4.39 - 4.18 (m, 2H), 3.06 - 2.95 (m, 1H), 2.75 (ddd, $J$ = 21.2, 14.2, 5.6 Hz, 1H), 1.93 (dd, $J$ = 25.1, 1.9 Hz, 3H), 1.60 (dd, $J$ = 6.9, 4.0 Hz, 3H).

Example 34

(1) Step 1-

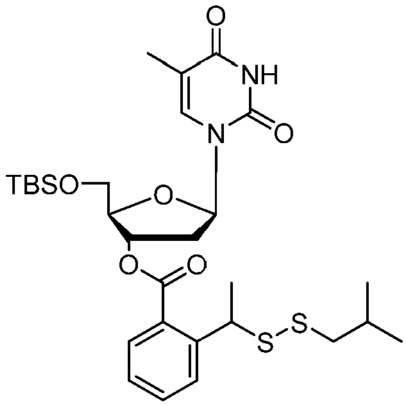

The product was synthesized similarly to Example 5 step 1, wherein 2-(1-(ethyldisulfaneyl)ethyl)benzoic acid was replaced by 2-(1-(isobutyldisulfaneyl)ethyl)benzoic acid (available from Beijing Okeanos Tech. Co., Ltd., cat. No. : OK-H-21008), the yield of this step was 79%, MW = 608.
$^1$H NMR (400 MHz, $CDCl_3$) δ 8.66 - 8.39 (m, 1H), 7.89 - 7.80 (m, 1H), 7.63 - 7.47 (m, 3H), 7.33 (td, $J$ = 8.8, 4.5 Hz, 1H), 6.47 - 6.40 (m, 1H), 5.52 - 5.40 (m, 1H), 5.30 - 4.92 (m, 1H), 4.36 - 4.21 (m, 1H), 4.07 - 3.92 (m, 2H), 2.60 - 2.48 (m, 1H), 2.31 - 2.16 (m, 1H), 1.94 (s, 3H), 1.72 - 1.66 (m, 2H), 1.63 - 1.51 (m, 1H), 1.00 (d, $J$ = 6.8 Hz, 2H), 0.98 - 0.93 (m, 9H), 0.87 - 0.81 (m, 4H), 0.19 - 0.13 (m, 6H).

(2) Steps 2-3

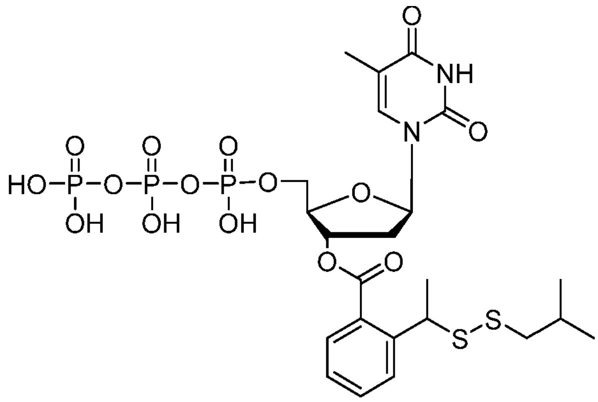

The product was synthesized similarly to Example 5 Steps 2-3, wherein 2-(1-(ethyldisulfaneyl)ethyl)benzoic acid was replaced by 2-(1-(isobutyldisulfaneyl)ethyl)benzoic acid (available from Beijing Okeanos Tech. Co., Ltd., cat. No. : OK-H-21008), yield for the last step was 32 %, MW = 734.

$^1$H NMR (400 MHz, DMSO-$d_6$) δ 11.31 (s, 1H), 7.96 (dd, *J* = 8.2, 0.9 Hz, 1H), 7.85 (d, *J* = 8.3 Hz, 1H), 7.62 (d, *J* = 3.9 Hz, 2H), 7.46 - 7.41 (m, 1H), 6.33 (td, *J* = 9.7, 5.6 Hz, 1H), 5.63 - 5.55 (m, 1H), 5.18 - 5.08 (m, 1H), 4.30 (d, *J* = 23.4 Hz, 1H), 4.18 - 4.02 (m, 2H), 2.57 - 2.51 (m, 1H), 2.43 - 2.29 (m, 1H), 2.12 - 1.93 (m, 2H), 1.84 (s, 3H), 1.70 - 1.56 (m, 4H), 0.85 - 0.71 (m, 6H).

Example 35

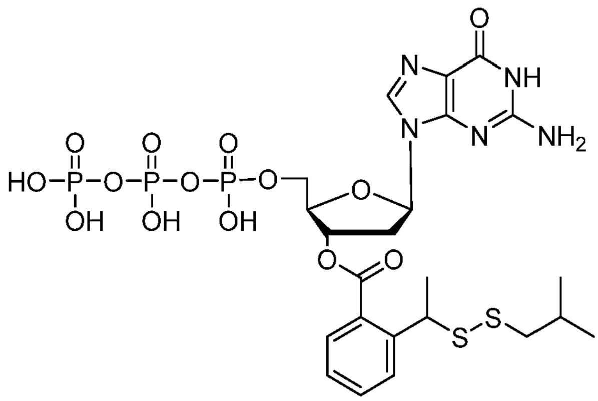

The product was synthesized similarly to Example 6 Steps 1-3, wherein 2-(1-(ethyldisulfaneyl)ethyl)benzoic acid was replaced by 2-(1-(isobutyldisulfaneyl)ethyl)benzoic acid (available from Beijing Okeanos Tech. Co., Ltd., cat. No. : OK-H-21008), the yield for the last step was 40%, MW = 759.

$^1$H NMR (400 MHz, DMSO-$d_6$) δ 10.73 (s, 1H), 8.03 (d, *J* = 6.9 Hz, 1H), 7.89 (d, *J*= 7.6 Hz, 1H), 7.63 (d, *J* = 2.8 Hz, 2H), 7.49 - 7.38 (m, 1H), 6.66 (s, 2H), 6.27 - 6.15 (m, 1H), 5.69 (dd, *J* = 8.2, 4.0 Hz, 1H), 5.16 (q, *J* = 6.9 Hz, 1H), 4.39 - 4.26 (m, 1H), 4.19 (dd, *J* = 10.6, 5.1 Hz, 1H), 4.01 (dd, *J* = 11.4, 5.9 Hz, 1H), 2.57 - 2.52 (m, 1H), 2.48 - 2.41 (m, 1H), 2.24 - 2.12 (m, 2H), 1.70 (dd, *J* = 13.2, 6.6 Hz, 1H), 1.65 (d, *J* = 6.8 Hz, 3H), 0.85 - 0.74 (m, 6H).

Example 36

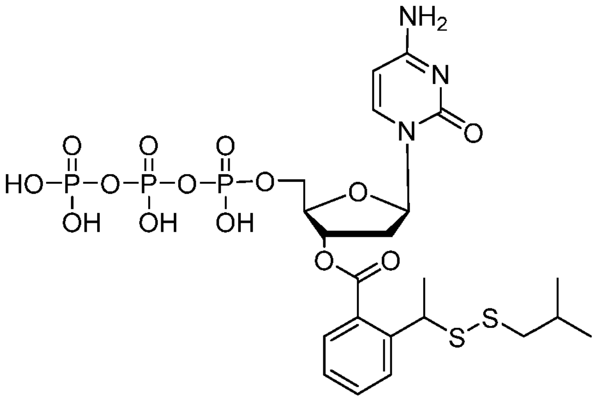

The product was synthesized similarly to Example 7 Steps 1-4, wherein 2-(1-(ethyldisulfaneyl)ethyl)benzoic acid was replaced by 2-(1-(isobutyldisulfaneyl)ethyl)benzoic acid (available from Beijing Okeanos Tech. Co., Ltd., cat. No. : OK-H-21008 (available from Beijing Okeanos Tech. Co., Ltd., cat. No. : OK-H-21008), the yield for the last two steps was 30%, MW = 719.
$^1$H NMR (400 MHz, DMSO-$d_6$) δ 7.98 (dd, *J* = 9.4, 7.6 Hz, 1H), 7.85 (d, *J* = 7.8 Hz, 1H), 7.62 (d, *J* = 4.1 Hz, 2H), 7.46 - 7.42 (m, 1H), 7.33 (s, 1H), 7.12 (s, 1H), 6.40 - 6.30 (m, 1H), 5.82 (dd, *J* = 7.5, 1.0 Hz, 1H), 5.53 (d, *J* = 4.4 Hz, 1H), 5.17 - 5.11 (m, 1H), 4.30 (d, *J* = 21.7 Hz, 1H), 4.16 - 4.01 (m, 1H), 2.46 - 2.27 (m, 2H), 2.15 - 1.98 (m, 2H), 1.71 - 1.58 (m, 4H), 0.80 (d, *J* = 2.5 Hz, 3H), 0.78 (d, *J* = 2.6 Hz, 3H).

Example 37

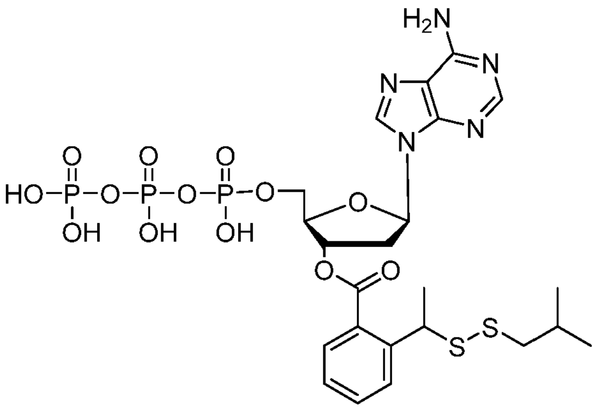

The product was synthesized similarly to Example 8 Steps 1-3, wherein 2-(1-(ethyldisulfaneyl)ethyl)benzoic acid was replaced by 2-(1-(isobutyldisulfaneyl)ethyl)benzoic acid (available from Beijing Okeanos Tech. Co., Ltd., cat. No. : OK-H-21008 (available from Beijing Okeanos Tech. Co., Ltd., cat. No. : OK-H-21008), the yield for the last step was 40%, MW = 743.
$^1$H NMR (400 MHz, DMSO-$d_6$) δ 8.67 (d, *J* = 8.3 Hz, 1H), 8.14 (d, *J* = 5.9 Hz, 1H), 7.94 - 7.87 (m, 1H), 7.64 (d, *J* = 3.9 Hz, 2H), 7.46 (dt, *J* = 8.1, 4.2 Hz, 1H), 7.32 (s, 2H), 6.51 - 6.44 (m, 1H), 5.75 (s, 1H), 5.18 (q, *J* = 7.0 Hz, 1H), 4.45 - 4.33 (m, 1H), 4.16 - 4.04 (m, 2H), 3.25 - 3.12 (m, 1H), 2.71 - 2.57 (m, 1H), 2.19 - 2.01 (m, 2H), 1.73 - 1.59 (m, 4H), 0.81 (d, *J* = 6.7 Hz, 3H), 0.78 (d, *J* = 6.7 Hz, 3H).

Example 38

(1) Step 1

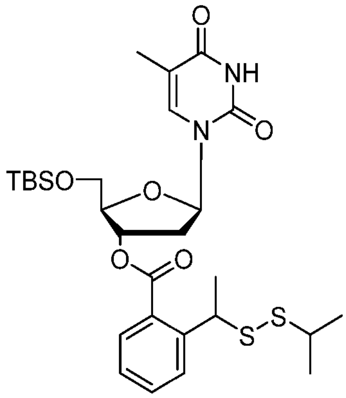

The product was synthesized similarly to Example 5 step 1, wherein 2-(1-(ethyldisulfaneyl)ethyl)benzoic acid was replaced by 2-(1-(isopropyldisulfaneyl)ethyl)benzoic acid (available from Beijing Okeanos Tech. Co., Ltd., cat. No. : OK-H-21009), the yield of this step was 80%, MW = 594.
$^1$H NMR (500 MHz, $CDCl_3$) δ 8.51 (s, 1H), 7.85 (ddd, *J* = 12.8, 7.8, 1.2 Hz, 1H), 7.64 - 7.57 (m, 2H), 7.57 - 7.50 (m, 1H), 7.37 - 7.29 (m, 1H), 6.45 (ddd, *J* = 20.4, 9.2, 5.3 Hz, 1H), 5.50 (t, *J* = 5.3 Hz, 1H), 5.28 - 5.15 (m, 1H), 4.32 (dd, *J* = 36.3, 1.3 Hz, 1H), 4.07 - 3.96 (m, 2H), 2.59 (td, *J* = 13.5, 5.3 Hz, 1H), 2.47 - 2.16 (m, 2H), 1.95 (s, 3H), 1.70 (dd, *J* = 7.0, 1.0 Hz, 3H), 1.17 - 1.06 (m, 6H), 0.97 (s, 9H), 0.18 (s, 6H).

(2) Step 2

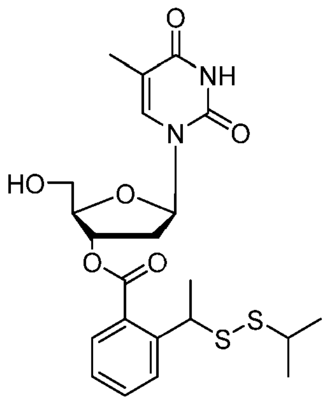

The product was synthesized similarly to Example 5 Step 2, wherein 2-(1-(ethyldisulfaneyl)ethyl)benzoic acid was replaced by 2-(1-(isopropyldisulfaneyl)ethyl)benzoic acid (available from Beijing Okeanos Tech. Co., Ltd., cat. No. : OK-H-21009), the yield of this step was 90%, MW = 594.
$^1$H NMR (500 MHz, $CDCl_3$) δ 8.84 (s, 1H), 7.84 (ddd, *J* = 7.4, 5.8, 1.3 Hz, 1H), 7.65 - 7.49 (m, 3H), 7.33 (td, *J* = 7.7, 1.2 Hz, 1H), 6.37 - 6.27 (m, 1H), 5.61 (tt, *J* = 4.7, 2.5 Hz, 1H), 5.22 (dq, *J* = 13.9, 7.0 Hz, 1H), 4.30 (dq, *J* = 19.8, 2.4 Hz, 1H), 4.03 (dd, *J* = 5.7, 2.7 Hz, 2H), 2.60 - 2.31 (m, 3H), 1.95 (d, *J* = 1.1 Hz, 3H), 1.70 (d, *J* = 7.0 Hz, 3H), 1.15 - 1.07 (m, 6H).

(3) Step 3

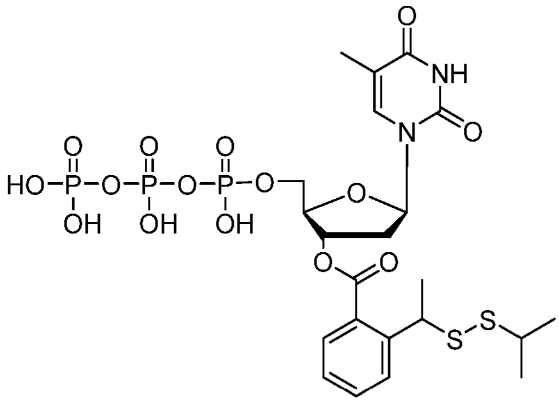

The product was synthesized similarly to Example 5 step 3, wherein 2-(1-(ethyldisulfaneyl)ethyl)benzoic acid was replaced by 2-(1-(isopropyldisulfaneyl)ethyl)benzoic acid (available from Beijing Okeanos Tech. Co., Ltd., cat. No. : OK-H-21009), the yield of this step was 40%, MW = 720.

$^{1}$H NMR (400 MHz, DMSO-$d_6$) δ 11.29 (s, 1H), 7.98 (dd, *J* = 6.6, 0.9 Hz, 1H), 7.85 (dd, *J* = 7.7, 2.4 Hz, 1H), 7.61 (t, *J* = 6.4 Hz, 2H), 7.45 - 7.40 (m, 1H), 6.36 - 6.31 (m, 1H), 5.60 (d, *J* = 5.3 Hz, 1H), 5.11 (q, *J* = 6.9 Hz, 1H), 4.30 (d, *J* = 20.0 Hz, 1H), 4.12 - 4.04 (m, 2H), 2.58 - 2.51 (m, 1H), 2.46 - 2.29 (m, 2H), 1.84 (s, 3H), 1.64 (d, *J* = 7.0 Hz, 3H), 1.06 (d, *J* = 5.7 Hz, 6H).

Example 39

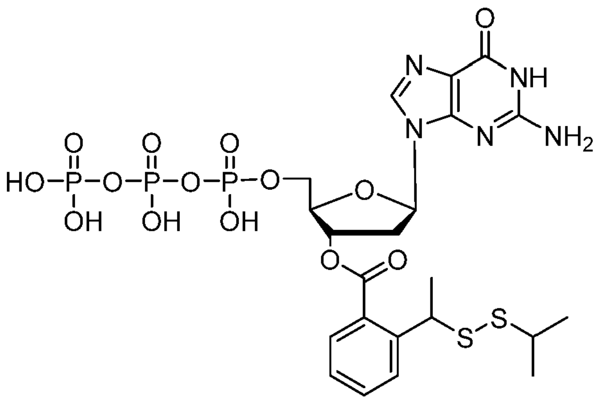

The product was synthesized similarly to Example 6 Steps 1-3, wherein 2-(1-(ethyldisulfaneyl)ethyl)benzoic acid was replaced by 2-(1-(isopropyldisulfaneyl)ethyl)benzoic acid (available from Beijing Okeanos Tech. Co., Ltd., cat. No. : OK-H-21009), yield for the last step was 40%, MW = 745.

$^{1}$H NMR (400 MHz, DMSO-$d_6$) δ 10.57 (s, 1H), 7.98 (d, *J* = 7.9 Hz, 1H), 7.88 (d, *J* = 7.5 Hz, 1H), 7.63 (d, *J* = 3.3 Hz, 2H), 7.47 - 7.40 (m, 1H), 6.63 (d, *J* = 25.9 Hz, 2H), 6.27 - 6.16 (m, 1H), 5.68 (t, *J* = 5.3 Hz, 1H), 5.32 (t, *J* = 4.7 Hz, 1H), 5.18 - 5.10 (m, 1H), 4.37 - 4.28 (m, 1H), 4.02 - 3.96 (m, 1H), 2.68 - 2.66 (m, 1H), 2.61 - 2.56 (m, 1H), 2.33 (dt, *J* = 3.5, 1.7 Hz, 1H), 1.65 (d, *J* = 6.9 Hz, 3H), 1.13 (d, *J* = 7.9 Hz, 6H).

Example 40

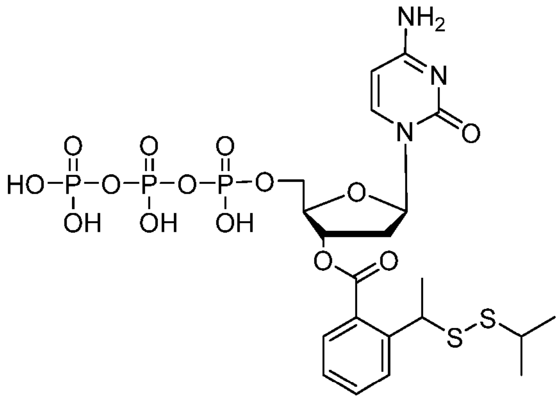

The product was synthesized similarly to Example 7 Steps 1-4, wherein 2-(1-(ethyldisulfaneyl)ethyl)benzoic acid was replaced by 2-(1-(isopropyldisulfaneyl)ethyl)benzoic acid (available from Beijing Okeanos Tech. Co., Ltd., cat. No. : OK-H-21009), yield for the last two steps was 30%, MW = 705.
$^1$H NMR (400 MHz, DMSO-$d_6$) δ 8.00 - 7.93 (m, 1H), 7.84 (dd, *J* = 7.6, 3.3 Hz, 1H), 7.62 (d, *J* = 3.7 Hz, 2H), 7.47 - 7.38 (m, 1H), 7.28 (s, 1H), 7.07 (s, 1H), 6.38 - 6.33 (m, 1H), 5.81 (d, *J* = 7.3 Hz, 1H), 5.52 (d, *J* = 5.2 Hz, 1H), 5.11 (q, *J* = 7.0 Hz, 1H), 4.30 (d, *J* = 19.7 Hz, 1H), 4.14 - 3.97 (m, 2H), 2.47 - 2.38 (m, 2H), 2.34 - 2.31 (m, 1H), 1.64 (d, *J* = 7.0 Hz, 3H), 1.10 (d, *J* = 2.1 Hz, 6H).

Example 41

(1) Step 1

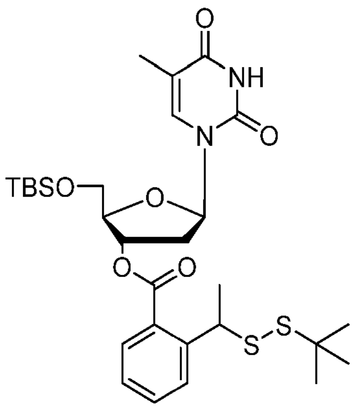

The product was synthesized similarly to Example 5 step 1, wherein 2-(1-(ethyldisulfaneyl)ethyl)benzoic acid was replaced by 2-(1-(tert-butyldisulfaneyl)ethyl)benzoic acid (available from Beijing Okeanos Tech. Co., Ltd., cat. No. : OK-H-21010), the yield of this step was 80%, MW = 608.
$^1$H NMR (400 MHz, $CDCl_3$) δ 8.73 (d, *J* = 5.8 Hz, 1H), 7.84 (ddd, *J* = 7.8, 3.5, 1.5 Hz, 1H), 7.70 - 7.45 (m, 3H), 7.32 (td, *J* = 7.6, 1.4 Hz, 1H), 6.46 (td, *J* = 9.5, 5.3 Hz, 1H), 5.57 - 5.35 (m, 1H), 5.16 (p, *J* = 7.1 Hz, 1H), 4.28 (dd, *J* = 17.9, 1.7 Hz, 1H), 4.13 - 3.90 (m, 2H), 2.58 (ddd, *J* = 14.7, 9.8, 5.3 Hz, 1H), 2.23 (dtd, *J* = 16.2, 6.2, 2.7 Hz, 1H), 1.98 - 1.92 (m, 3H), 1.69 (d, *J* = 6.7 Hz, 3H), 1.22 (d, *J* = 11.2 Hz, 8H), 0.96 (s, 9H), 0.17 (s, 6H).

(2) Step 2

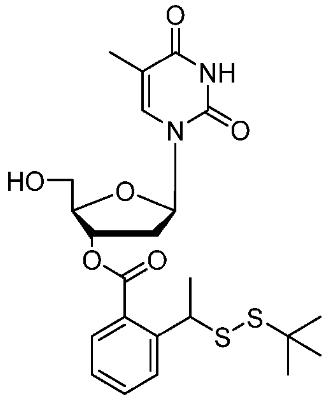

The product was synthesized similarly to Example 5 Step 2, wherein 2-(1-(ethyldisulfaneyl)ethyl)benzoic acid was replaced by 2-(1-(tert-butyldisulfaneyl)ethyl)benzoic acid (available from Beijing Okeanos Tech. Co., Ltd., cat. No. : OK-H-21010), the yield of this step was 90%, MW = 494.

$^1$H NMR (400 MHz, $CDCl_3$) δ 8.90 (s, 1H), 7.83 (dt, *J* = 7.9, 1.6 Hz, 1H), 7.62 (d, *J* = 7.9 Hz, 1H), 7.55 (dtd, *J* = 7.8, 4.0, 1.4 Hz, 2H), 7.32 (td, *J* = 7.5, 1.4 Hz, 1H), 6.31 (ddd, *J* = 8.4, 6.0, 2.8 Hz, 1H), 5.59 (dt, *J* = 5.4, 2.5 Hz, 1H), 5.14 (dq, *J* = 18.4, 7.0 Hz, 1H), 4.27 (dt, *J* = 8.4, 2.5 Hz, 1H), 4.01 (d, *J* = 2.6 Hz, 2H), 2.65 - 2.48 (m, 2H), 2.35 (s, 2H), 1.94 (d, *J* = 1.2 Hz, 3H), 1.68 (dd, *J* = 6.9, 1.4 Hz, 3H), 1.22 (d, *J* = 2.9 Hz, 9H).

(3) Step 3

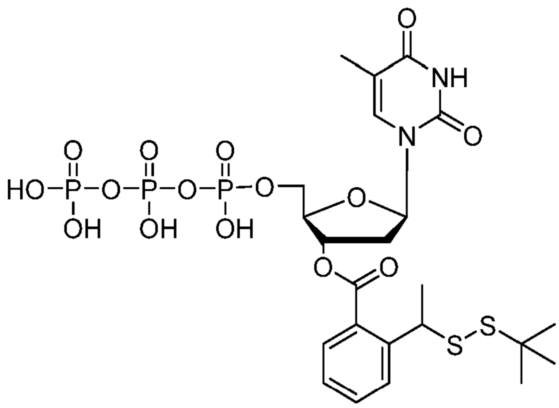

The product was synthesized similarly to Example 5 step 3, wherein 2-(1-(ethyldisulfaneyl)ethyl)benzoic acid was replaced by 2-(1-(tert-butyldisulfaneyl)ethyl)benzoic acid (available from Beijing Okeanos Tech. Co., Ltd., cat. No. : OK-H-21010), the yield of this step was 40%, MW = 734.

$^1$H NMR (400 MHz, DMSO-$d_6$) δ 11.30 (s, 1H), 7.91 (d, *J* = 7.2 Hz, 1H), 7.84 (d, *J* = 8.0 Hz, 1H), 7.62 (d, *J* = 4.6 Hz, 2H), 7.44 - 7.39 (m, 1H), 6.37 - 6.29 (m, 1H), 5.56 (t, *J* = 5.0 Hz, 1H), 5.07 - 4.98 (m, 1H), 4.29 (d, *J* = 28.0 Hz, 1H), 4.14 - 3.99 (m, 2H), 2.39 (dd, *J* = 13.9, 5.9 Hz, 1H), 2.31 (dd, *J* = 14.2, 5.7 Hz, 1H), 1.83 (s, 3H), 1.63 (dd, *J* = 7.0, 1.1 Hz, 3H), 1.19 (d, *J* = 2.5 Hz, 9H).

Example 42

(1) Step 1

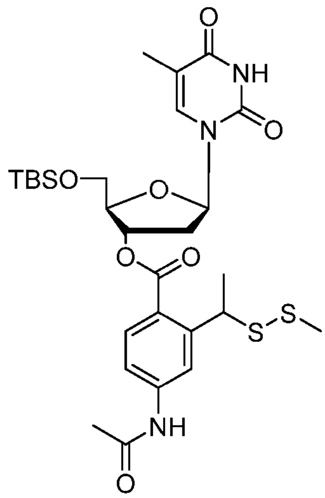

The product was synthesized similarly to Example 5 step 1, wherein 2-(1-(ethyldisulfaneyl)ethyl)benzoic acid was replaced by 4-acetamido-2-(1-(methyldisulfaneyl)ethyl)benzoic acid (available from Beijing Okeanos Tech. Co., Ltd., cat. No. : OK-H-21002), the yield of this step was 80%, MW = 623.
$^1$H NMR (400 MHz, $CDCl_3$) δ 8.29 (s, 1H), 7.88 (d, *J* = 8.6 Hz, 1H), 7.68 (s, 1H), 7.64 - 7.58 (m, 2H), 7.46 (s, 1H), 6.42 (dd, *J* = 9.3, 5.1 Hz, 1H), 5.46 (d, *J* = 5.9 Hz, 1H), 5.42 - 5.36 (m, 1H), 4.29 (d, *J* = 1.8 Hz, 1H), 4.04 - 3.95 (m, 2H), 2.57 (dd, *J* = 13.8, 5.3 Hz, 1H), 2.29 - 2.23 (m, 1H), 2.22 (s, 3H), 2.06 (s, 3H), 1.94 (d, *J* = 1.2 Hz, 3H), 1.68 (d, *J* = 7.0 Hz, 3H), 0.95 (s, 9H), 0.17 (s, 6H).

(2) Step 2

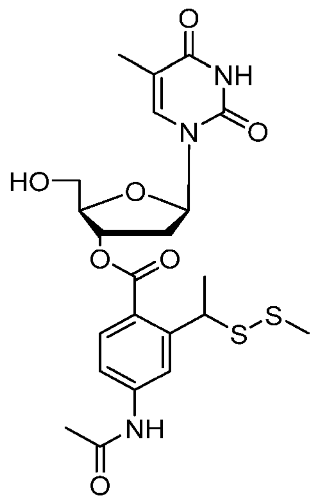

The product was synthesized similarly to Example 5 Step 2, wherein 2-(1-(ethyldisulfaneyl)ethyl)benzoic acid was replaced by 4-acetamido-2-(1-(methyldisulfaneyl)ethyl)benzoic acid (available from Beijing Okeanos Tech. Co., Ltd., cat. No. : OK-H-21002), the yield of this step was 75%, MW = 509.
$^1$H NMR (400 MHz, $CDCl_3$) δ 8.08 (s, 1H), 7.89 (dd, *J* = 8.5, 5.6 Hz, 1H), 7.66 (d, *J*= 9.5 Hz, 2H), 7.54 (d, *J* = 7.2 Hz, 1H), 7.45 (s, 1H), 6.29 (t, *J* = 7.1 Hz, 1H), 5.59 - 5.56 (m, 1H), 5.41 - 5.35 (m, 1H), 4.29 - 4.25 (m, 1H), 4.01 (d, *J* = 2.2 Hz, 2H), 3.01 - 2.93 (m, 1H), 2.58 - 2.55 (m, 1H), 2.23 (s, 3H), 2.09 (d, *J* = 5.6 Hz, 3H), 1.95 (*d, J* = 1.2 Hz, 3H), 1.68 (s, 3H).

(3) Step 3

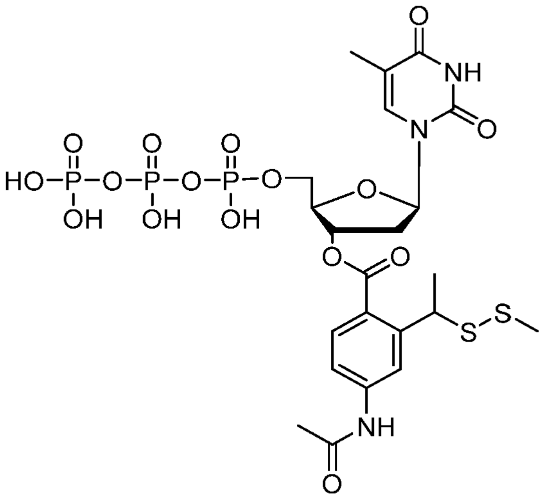

The product was synthesized similarly to Example 5 step 3, wherein 2-(1-(ethyldisulfaneyl)ethyl)benzoic acid was replaced by 4-acetamido-2-(1-(methyldisulfaneyl)ethyl)benzoic acid (available from Beijing Okeanos Tech. Co., Ltd., cat. No. : OK-H-21002), the yield of this step was 35%, MW = 749.
$^1$H NMR (400 MHz, DMSO-$d_6$) δ 11.30 (s, 1H), 10.52 (d, $J$ = 3.1 Hz, 1H), 7.97 (d, $J$ = 4.1 Hz, 1H), 7.92 - 7.82 (m, 2H), 7.69 (d, $J$ = 8.8 Hz, 1H), 6.37 - 6.26 (m, 1H), 5.55 (d, $J$ = 5.3 Hz, 1H), 5.34 - 5.25 (m, 1H), 4.27 (d, $J$ = 16.7 Hz, 1H), 4.12 - 4.05 (m, 2H), 2.49 - 2.46 (m, 1H), 2.35 (td, $J$ = 13.9, 5.7 Hz, 1H), 2.11 (d, $J$ = 12.7 Hz, 3H), 2.08 (s, 3H), 1.84 (s, 3H), 1.61 (d, $J$ = 6.9 Hz, 3H).

Example 43

(1) Step 1

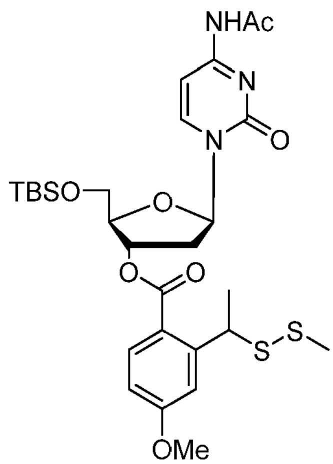

The product was synthesized similarly to Example 7 step 1, wherein 2-(1-(ethyldisulfaneyl)ethyl)benzoic acid was replaced by 4-methoxy-2-(1-(methyldisulfaneyl)ethyl)benzoic acid (available from Beijing Okeanos Tech. Co., Ltd., cat. No. : OK-H-21001), the yield of this step was 80%, MW = 623.
$^1$H NMR (500 MHz, DMSO-$d_6$) δ 10.92 (s, 1H), 8.24 (d, $J$ = 7.5 Hz, 1H), 7.92 (dd, $J$ = 8.7, 4.0 Hz, 1H), 7.24 (d, $J$ = 7.5 Hz, 1H), 7.10 (d, $J$ = 2.6 Hz, 1H), 7.00 (ddd, $J$ = 8.8, 2.6, 1.4 Hz, 1H), 6.19 (q, $J$ = 6.3 Hz, 1H), 5.57 (d, $J$ = 8.0 Hz, 3H), 5.46 - 5.37 (m, 1H), 5.37 - 5.27 (m, 1H), 4.37 (dd, $J$ = 21.7, 2.9 Hz, 1H), 3.95 (dd, $J$ = 11.3, 3.2 Hz, 1H), 3.85 (s, 3H), 2.78 - 2.62 (m, 1H), 2.38 - 2.26 (m, 1H), 2.14 - 2.09 (m, 6H), 0.86 (s, 9H), 0.09 (s, 6H).

(2) Step 2

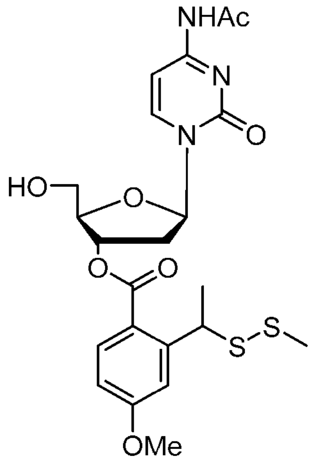

The product was synthesized similarly to Example 7 Step 2, wherein 2-(1-(ethyldisulfaneyl)ethyl)benzoic acid was replaced by 4-methoxy-2-(1-(methyldisulfaneyl)ethyl)benzoic acid (available from Beijing Okeanos Tech. Co., Ltd., cat. No. : OK-H-21001), the yield of this step was 85%, MW = 509.
$^1$H NMR (500 MHz, DMSO-$d_6$) δ 10.91 (s, 1H), 8.35 (d, *J* = 7.5 Hz, 1H), 7.92 (dd, *J* = 8.8, 4.1 Hz, 1H), 7.24 (d, *J* = 7.5 Hz, 1H), 7.10 (d, *J* = 2.7 Hz, 1H), 7.00 (ddd, *J* = 8.8, 2.7, 1.3 Hz, 1H), 6.22 (dt, *J* = 7.9, 6.0 Hz, 1H), 5.43 (dt, *J* = 6.6, 2.1 Hz, 1H), 5.39 - 5.30 (m, 1H), 5.26 (t, *J* = 5.3 Hz, 1H), 4.29 (dd, *J* = 18.4, 2.7 Hz, 1H), 3.85 (s, 3H), 3.72 (dd, *J* = 5.5, 3.2 Hz, 2H), 2.68 - 2.55 (m, 1H), 2.34 (dt, *J* = 14.1, 7.0 Hz, 1H), 2.16 - 2.08 (m, 6H), 1.65 (d, *J* = 7.0 Hz, 3H).

(3) Steps 3-4

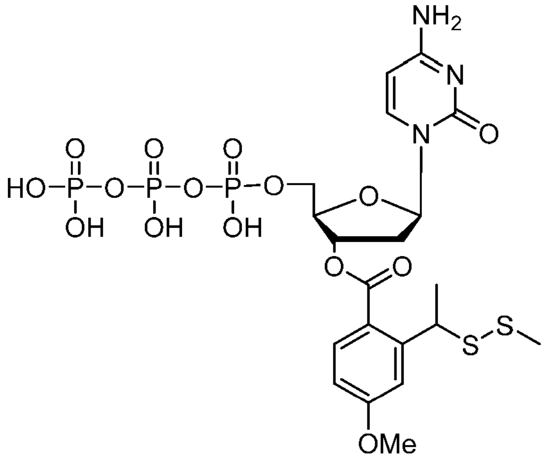

The product was synthesized similarly to Example 7 steps 3-4, wherein 2-(1-(ethyldisulfaneyl)ethyl)benzoic acid was replaced by 4-methoxy-2-(1-(methyldisulfaneyl)ethyl)benzoic acid (available from Beijing Okeanos Tech. Co., Ltd., cat. No. : OK-H-21001), total yield of these two steps was 30%, MW = 707.
$^1$H NMR (400 MHz, DMSO) δ 7.96 - 7.90 (m, 2H), 7.29 (s, 1H), 7.09 (d, *J* = 2.3 Hz, 2H), 7.00 (dd, *J* = 8.8, 2.5 Hz, 1H), 6.34 (dd, *J* = 15.3, 6.6 Hz, 1H), 5.81 (d, *J* = 7.5 Hz, 1H), 5.47 (d, *J* = 4.3 Hz, 1H), 5.33 (q, *J* = 7.0 Hz, 1H), 4.28 (d, *J* = 17.7 Hz, 1H), 4.13 - 3.97 (m, 2H), 3.85 (s, 3H), 2.43 - 2.22 (m, 2H), 2.13 (d, *J* = 10.6 Hz, 3H), 1.64 (d, *J* = 7.0 Hz, 3H).

Example 44 (Not according to the invention)

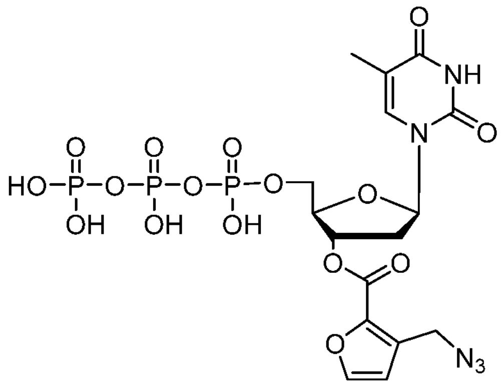

The product was synthesized similarly to Example 5 Steps 1-3, wherein 2-(1-(ethyldisulfaneyl)ethyl)benzoic acid was replaced by 3-(azidomethyl)furan-2-carboxylic acid (available from Beijing Okeanos Tech. Co., Ltd., cat. No. : OK-H-21011), yield for the last step was 45%, MW = 631.
$^1$H NMR (400 MHz, DMSO-$d_6$) δ 11.31 (s, 1H), 8.01 (d, $J$ = 1.6 Hz, 1H), 7.91 (d, $J$ = 22.7 Hz, 1H), 6.83 (d, $J$ = 1.6 Hz, 1H), 6.33 (dd, $J$ = 9.4, 5.7 Hz, 1H), 5.63 - 5.56 (m, 1H), 4.65 (s, 2H), 4.25 (s, 1H), 4.10 - 3.99 (m, 2H), 2.48 - 2.46 (m, 1H), 2.37 - 2.32 (m, 1H), 1.83 (s, 3H).

Example 45 (Not according to the invention)

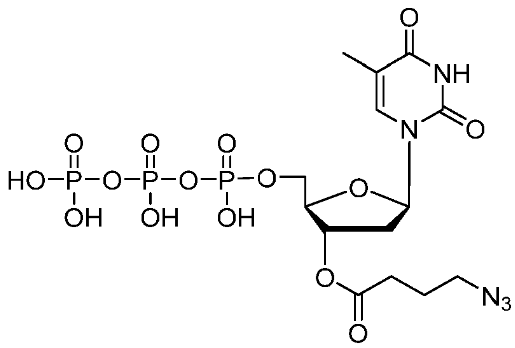

The product was synthesized similarly to Example 5 Steps 1-3, wherein 2-(1-(ethyldisulfaneyl)ethyl)benzoic acid was replaced by 4-azidobutanoic acid (available from Beijing Okeanos Tech. Co., Ltd., cat. No. : OK-H-20008), yield for the last step was 20%, MW = 593.
$^1$H NMR (400 MHz, DMSO-$d_6$) δ 11.28 (s, 1H), 7.90 (d, $J$ = 0.9 Hz, 1H), 6.24 (dd, $J$ = 9.5, 5.6 Hz, 1H), 5.33 (d, $J$ = 5.5 Hz, 1H), 4.09 (s, 1H), 4.04 - 3.95 (m, 2H), 3.38 (t, $J$ = 6.8 Hz, 2H), 2.44 (t, $J$ = 7.3 Hz, 2H), 2.41 - 2.33 (m, 1H), 2.18 (dd, $J$ = 13.8, 5.6 Hz, 1H), 1.82 (s, 3H), 1.81 - 1.75 (m, 2H).

II. Testing Examples

The inventor found that the nucleotide analogues of the present application led to both excellent blocking effect and excellent polymerization effect. Sequencing performed with these nucleotide analogues resulted in excellent effects, with both high mapping rate and low error rate.

Specifically, the inventor evaluated and tested the nucleotide analogues prepared in the above preparation examples on a high-throughput sequencer.

1. Evaluation of Blocking effect

Nucleotide substrate: fluorescent labeled standard hot dNTP (four kinds) and standard cold dNTP (four kinds), with structures showed below, all available from MGISEQ-2000RS high-throughput sequencing kit (FCL SE50, MGI Tech Co., Ltd., cat. No. 1000012551). For each test, only one of the nucleotide analogues cold dNTP of the invention (dTTP, dATP, dCTP, and dGTP) was used. For ease of presentation, only testing results for dTTP were provided in the following table 1 for each kind of 3'-OH modification.

Sequencing was conducted according to the operation protocol of MGISEQ2000 sequencer, using the above nucleotide substrates and MGISEQ-2000RS high-throughput sequencing kit (FCL SE50).

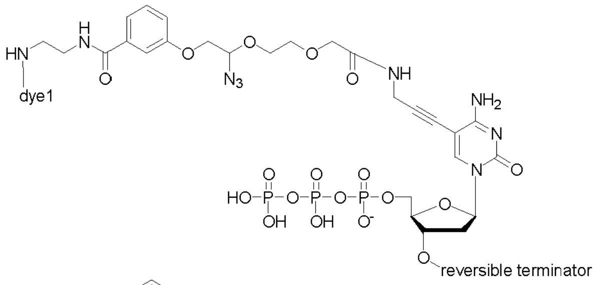

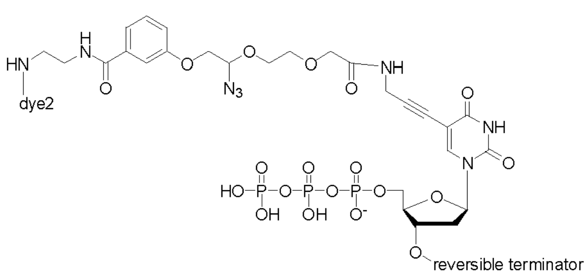

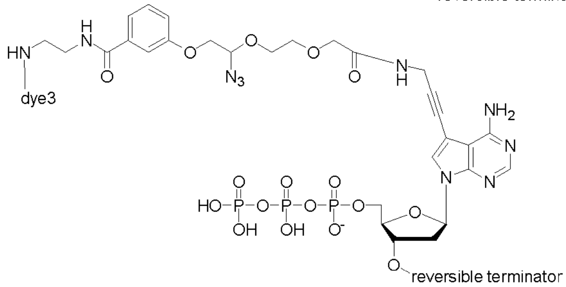

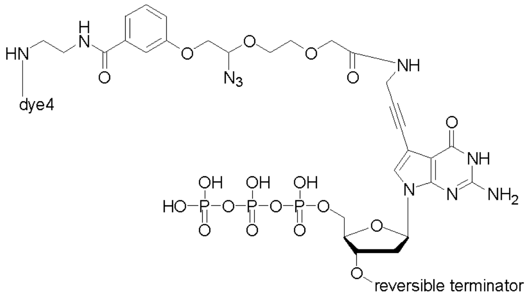

standard hot dNTP

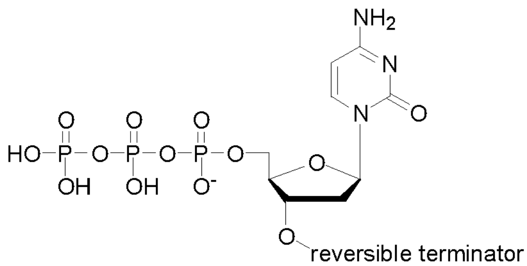

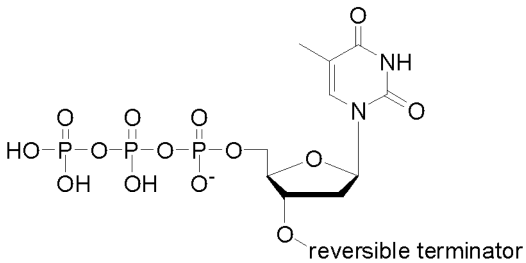

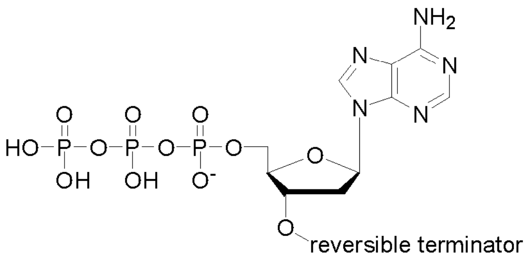

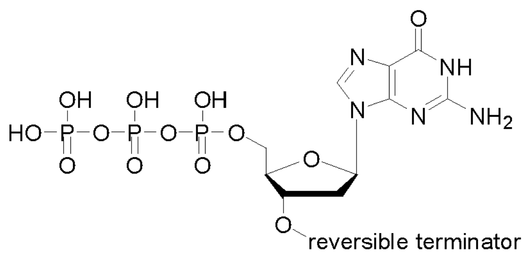

standard cold dNTP

(1) Preparing DNA nanospheres by using Ecoli sequencing library;
(2) Loading DNA nanospheres onto the MGISEQ2000 sequencing chip;
(3) Amounting the loaded sequencing chip onto the MGISEQ2000 sequencer and set the sequencing process;
(4) Performing Test Round 1: incorporating standard hot dNTP, taking photos for recording signal value, and then cleaving the blocking group with thpp reagent, 65°C Imin.
(5) Performing Test Round 2: incorporating standard cold dNTP, then incorporating standard hot dNTP, taking photos for recording signal value, and then cleaving blocking group with thpp reagent, 65°C 1min.
(6) Performing Test Round 3: incorporating standard hot dNTP, taking photos for recording signal value, and then cleaving blocking group with thpp reagent, 65°C Imin.
(7) Performing Test Round 4: incorporating nucleotide analogues cold dNTP of the invention (only one kind of cold dNTP incorporated for each test), then incorporating standard hot dNTP, taking photos for recording signal value, and then cleaving blocking group with thpp reagent, 65°C 1min.
(8) Performing Test Round 5: incorporating standard hot dNTP, taking photos for recording signal value.

(9) Evaluating Incorporation efficiency and Cleavage efficiency. The results were showed in Table 1.

Equation for calculating Incorporation efficiency:

$$E_{\mathrm{I}} = \frac{c_3 - c_4}{c_1 - c_2}$$

wherein:

EI (Incorporation efficiency) is the ratio of Incorporation efficiency values for testing nucleotide and comparative nucleotide;
C1 is signal value of Test Round 1;
C2 is signal value of Test Round 2;
C3 is signal value of Test Round 3;
C4 is signal value of Test Round 4;
Equation for calculating Cleavage efficiency:

$$E_c = 1 - \frac{\Sigma_{CGT}\left(\frac{c_3 - c_5}{c_3}\right)}{3EI} * 4$$

wherein:

Ec (Cleavage efficiency) is the ratio of Cleavage efficiency values for testing nucleotide and comparative nucleotide;
EI is the ratio of Incorporation efficiency values for testing nucleotide and comparative nucleotide;
C3 is signal value of Test Round 3;
C5 is signal value of Test Round 5;
CGT is the signals of base C, base G and base T in Test Round 3.

Table 1. Incorporation efficiency and Cleavage efficiency of nucleotide analogues (only those of dTTP illustrated)

| | |
|---|---|
| 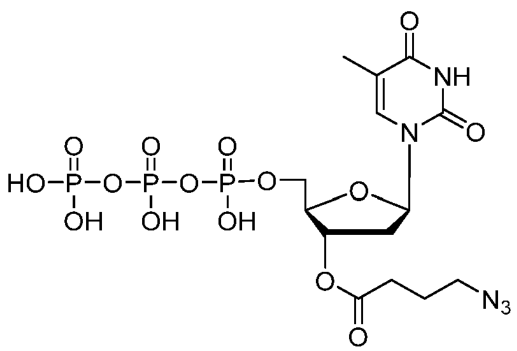 | Incorporation efficiency ($E_I$) 95% |
| | Cleavage efficiency (Ec) 36% |
| | Incorporation efficiency ($E_I$) 101% |

(continued)

| | |
|---|---|
| 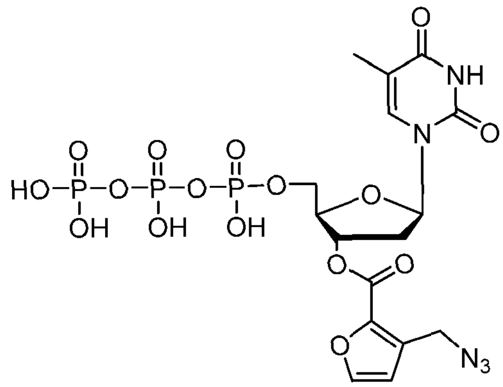 | Cleavage efficiency (Ec) 5% |
| 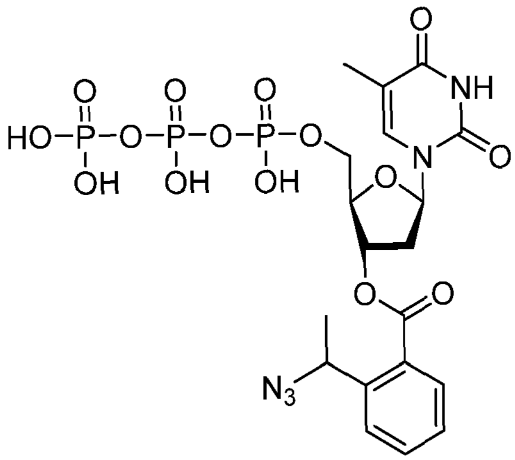 | Incorporation efficiency ($E_I$) 101%<br>Cleavage efficiency (Ec) 105% |
| 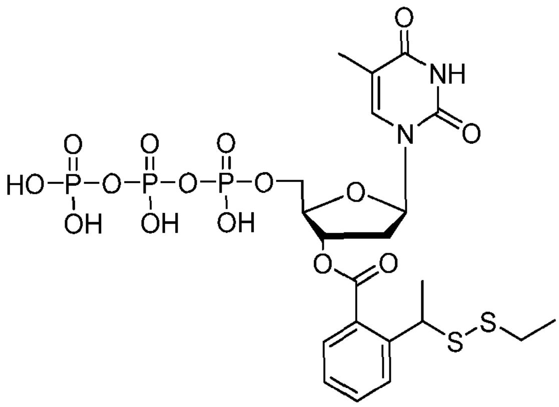 | Incorporation efficiency ($E_I$) 100%<br>Cleavage efficiency (Ec) 110% |
| 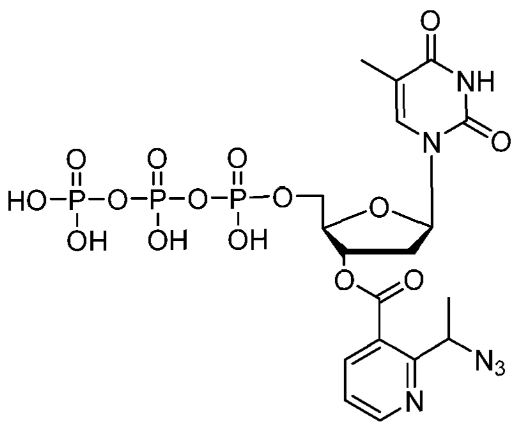 | Incorporation efficiency ($E_I$) 100%<br>Cleavage efficiency (Ec) 115% |
| | Incorporation efficiency ($E_I$) |

(continued)

| | |
|---|---|
| | 101% |
| 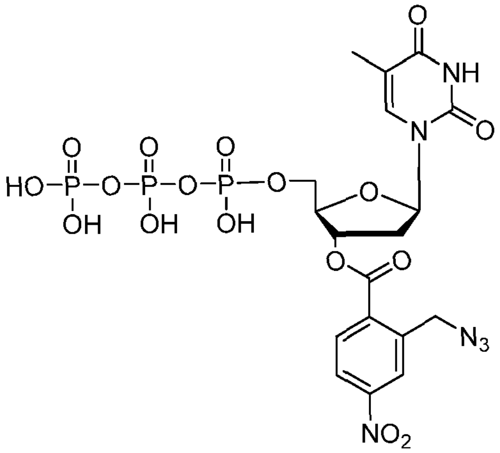 | Cleavage efficiency (Ec)<br>112% |
| 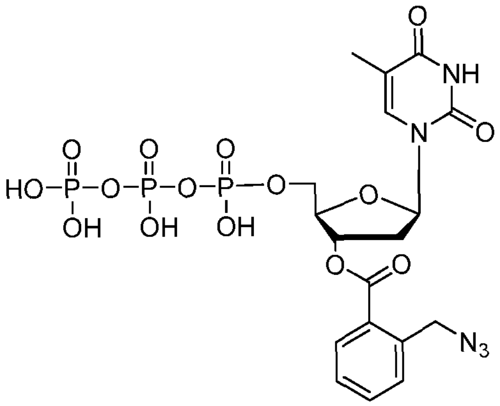 | Incorporation efficiency ($E_I$)<br>98%<br>Cleavage efficiency (Ec)<br>91% |
| 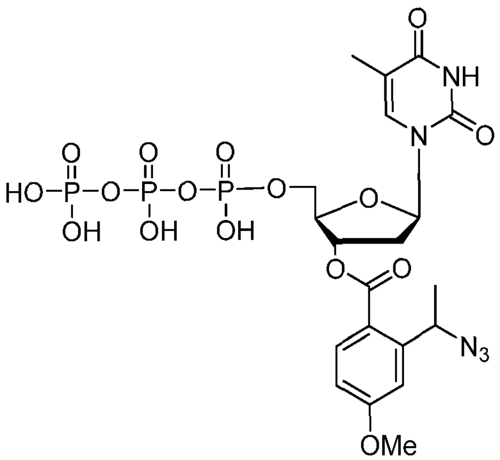 | Incorporation efficiency ($E_I$)<br>101%<br>Cleavage efficiency ($E_C$)<br>98% |

(continued)

| | |
|---|---|
| 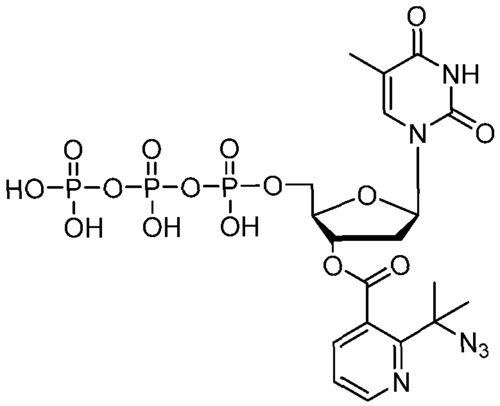 | Incorporation efficiency ($E_I$) 95% |
| | Cleavage efficiency (Ec) 110% |
| | Incorporation efficiency ($E_I$) 95% |
| 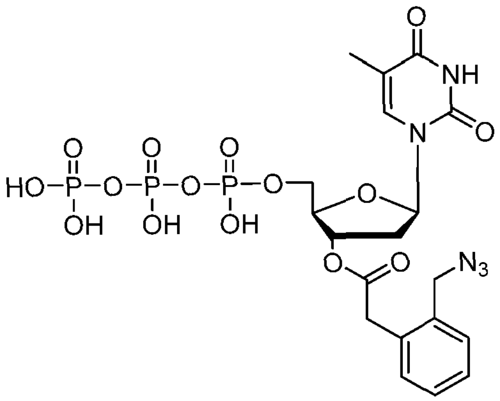 | Cleavage efficiency (Ec) 89% |
| 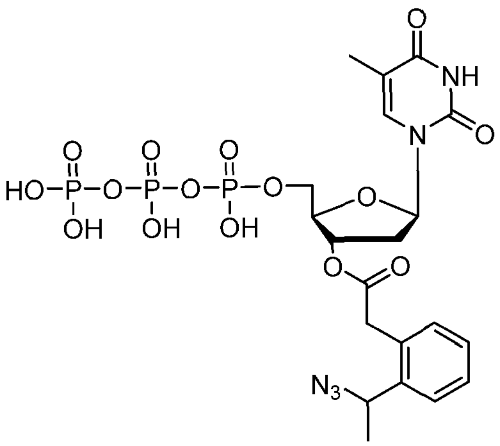 | Incorporation efficiency ($E_I$) 98% |
| | Cleavage efficiency (Ec) 91% |

(continued)

| | |
|---|---|
| 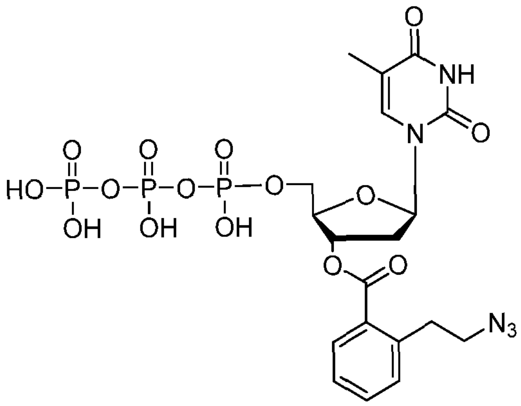 | Incorporation efficiency ($E_I$)<br>100% |
| | Cleavage efficiency (Ec)<br>100% |
| 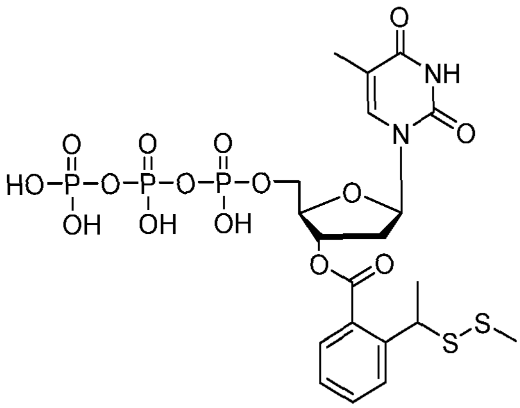 | Incorporation efficiency ($E_I$)<br>100% |
| | Cleavage efficiency (Ec)<br>111% |
| | Incorporation efficiency ($E_I$)<br>90% |
| 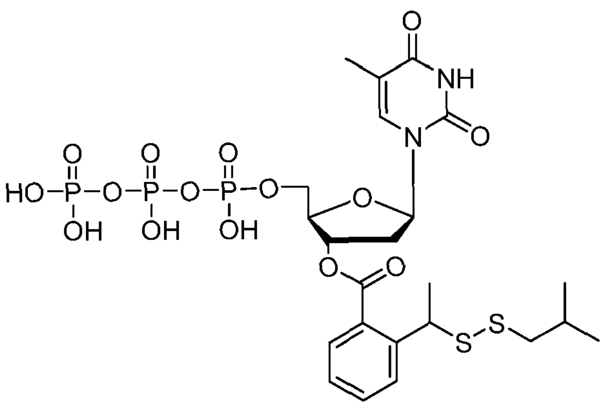 | Cleavage efficiency (Ec)<br>98% |
| 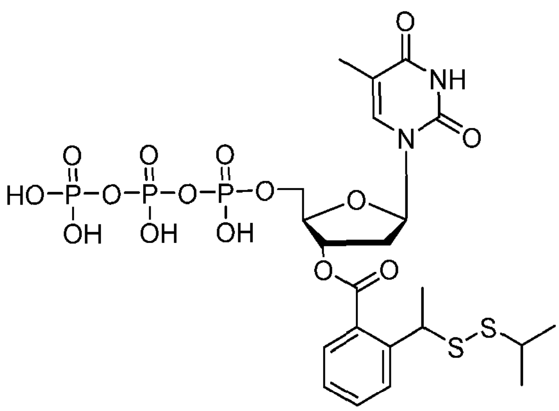 | Incorporation efficiency ($E_I$)<br>95% |
| | Cleavage efficiency (Ec)<br>103% |

(continued)

| | |
|---|---|
| 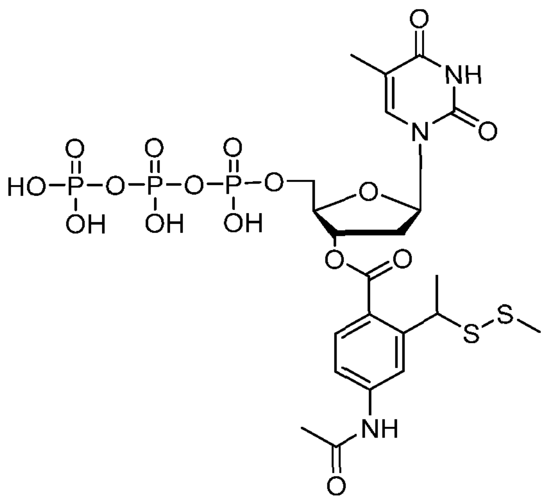 | Incorporation efficiency ($E_I$) 100%<br>Cleavage efficiency (Ec) 98% |
| 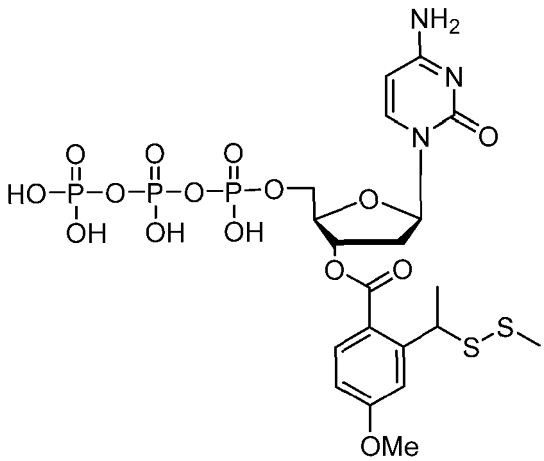 | Incorporation efficiency ($E_I$) 104%<br>Cleavage efficiency (Ec) 101% |

2. Evaluation of sequencing effect

Sequencing Example 1

Nucleotide substrates: fluorescent labeled standard hot dNTP (four kinds, all available from MGISEQ-2000RS high-throughput sequencing kit (FCL SE50), MGI Tech Co., Ltd., cat. No. 1000012551); and nucleotide analogues cold dNTP of the invention (four kinds, named as AEB) with structures showed below.

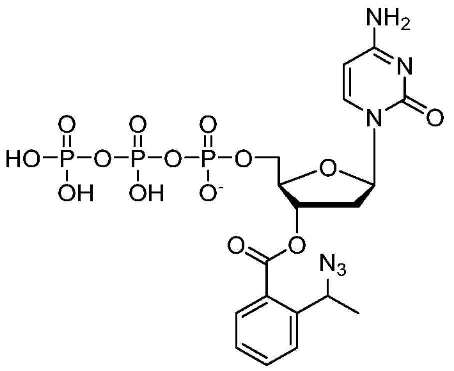

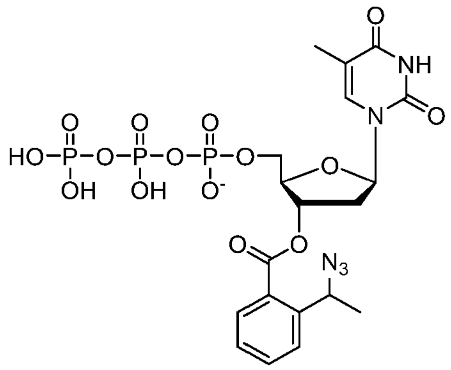

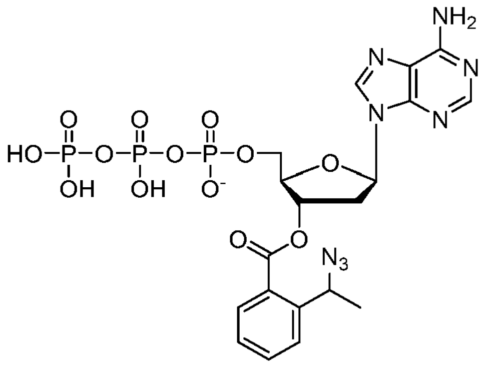

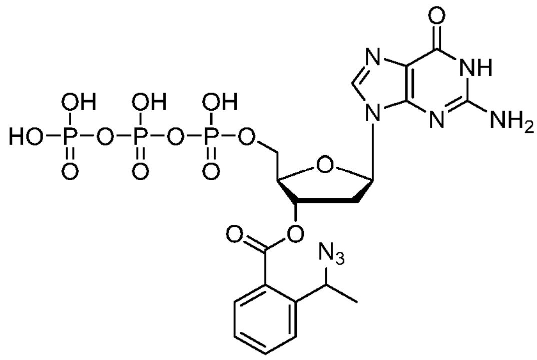

cold dNTP(AEB)

Sequencing was conducted according to the operation protocol of MGISEQ2000 sequencer, using the above nucleotide substrates and MGISEQ-2000RS high-throughput sequencing kit (FCL SE50).

(1) Preparing DNA nanospheres by using Ecoli sequencing library;
(2) Loading DNA nanospheres onto the MGISEQ2000 sequencing chip;
(3) Amounting the loaded sequencing chip onto the MGISEQ2000 sequencer and set the sequencing process, hot dNTP incorporation : 60°C 2min; cold dNTP incorporation : 60°C 2min; signal acquisition; cleavage of blocking group: 65°C 2min;
(4) Conducting Basecall analysis on offline data and output results of sequencing index including mapping rate, error rate, Q30, etc. The results were showed in Table 2.

Table 2. Results of Basecall analysis

| Nucleotide analogues cold dNTP Mix | **AEB** |
|---|---|
| Reference | Ecoli.fa |
| Cycle Number | 50 |
| Q30(%) | 84.61 |
| Lag (1%) | 0.18 |
| Lag (2%) | 0.23 |
| Runon (1%) | 0.3 |
| Runon (2%) | 0.37 |
| ESR (%) | 78.17 |
| Mapping Rate, % | 98.8 |
| AvgError Rate, % | 0.84 |
| AvgError Rate!N, % | 0.81 |

Sequencing Example 2

Nucleotide substrates: fluorescent labeled standard hot dNTP (four kinds, all available from MGISEQ-2000RS high-throughput sequencing kit (FCL SE50), MGI Tech Co., Ltd., cat. No. 1000012551); nucleotide analogues cold dNTP of the invention (four kinds, named as **SSEB**) with structures showed below.

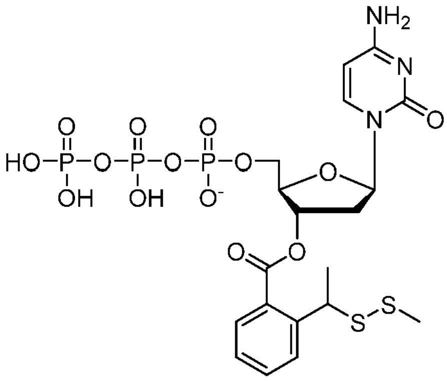

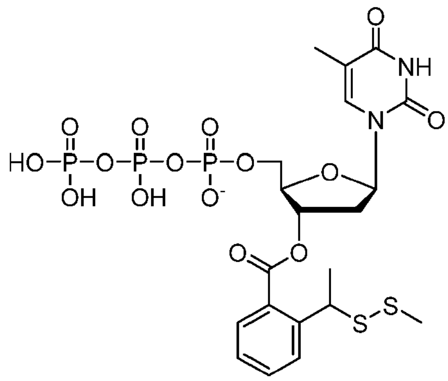

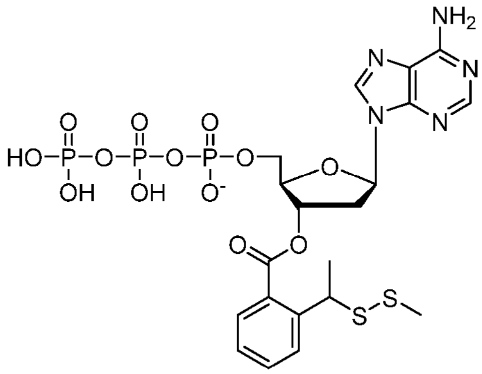

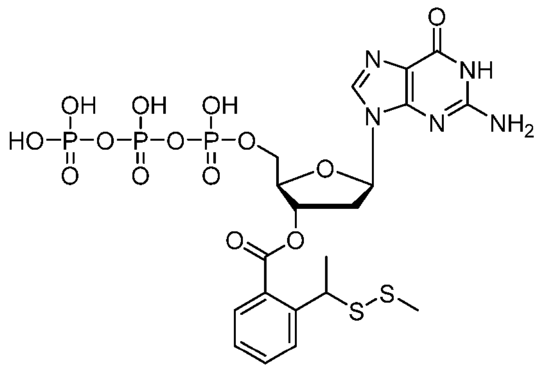

cold dNTP(SSEB)

Sequencing procedure was same as that above in Sequencing Example 1. Results of Basecall analysis were showed in Table 3.

Table 3. Results of Basecall analysis

| Nucleotide analogues cold dNTP Mix | **SSEB** |
|---|---|
| Reference | Ecoli.fa |
| Cycle Number | 50 |
| Q30(%) | 91.89 |
| Lag(%) | 0.11 |
| Runon(%) | 0.23 |
| ESR(%) | 90.33 |
| Mapping Rate, % | 98.59 |
| AvgError Rate, % | 0.13 |

Sequencing Example 3

Nucleotide substrates: fluorescent labeled standard hot dNTP (four kinds, all available from MGISEQ-2000RS high-throughput sequencing kit (FCL SE50), MGI Tech Co., Ltd., cat. No. 1000012551); nucleotide analogues cold dNTP of the invention (four kinds, named as **AZBN**) with structures showed below.

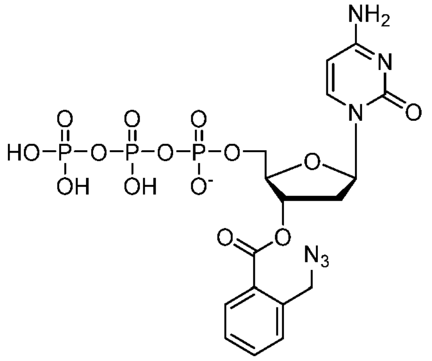

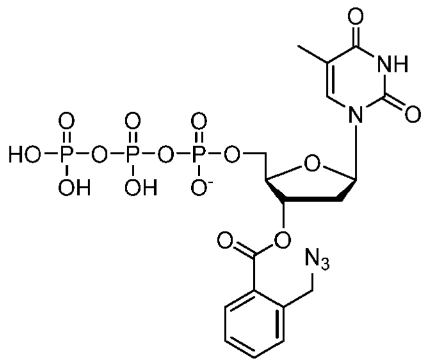

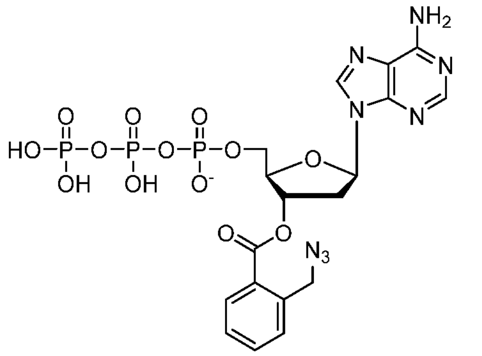

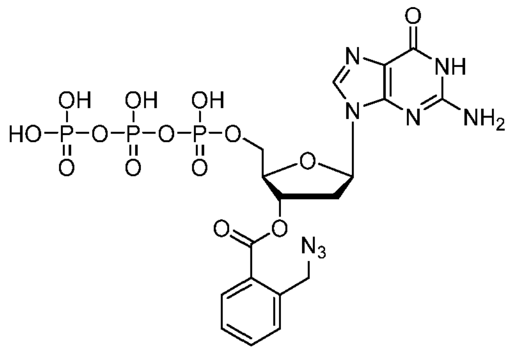

cold dNTP Mix (**AZBN**)

Sequencing procedure was same as that above in Sequencing Example 1. Results of Basecall analysis were showed in Table 4.

Table 4. Results of Basecall analysis

| Nucleotide analogues cold dNTP Mix | **AZBN** |
|---|---|
| Reference | Ecoli.fa |
| Cycle Number | 50 |
| Q30(%) | 61.39 |
| Lag(%) | 0.03 |
| Runon(%) | 0.12 |
| ESR(%) | 62.17 |
| Mapping Rate, % | 5.75 |
| AvgError Rate, % | 4.94 |
| AvgError Rate!N, % | 4.89 |

Sequencing Example 4: Sequencing with hot T-SSEB

1. Synthesis of nucleotide analogues dTTP carrying fluorescent group (named as hot T-SSEB)

(1) Step 1

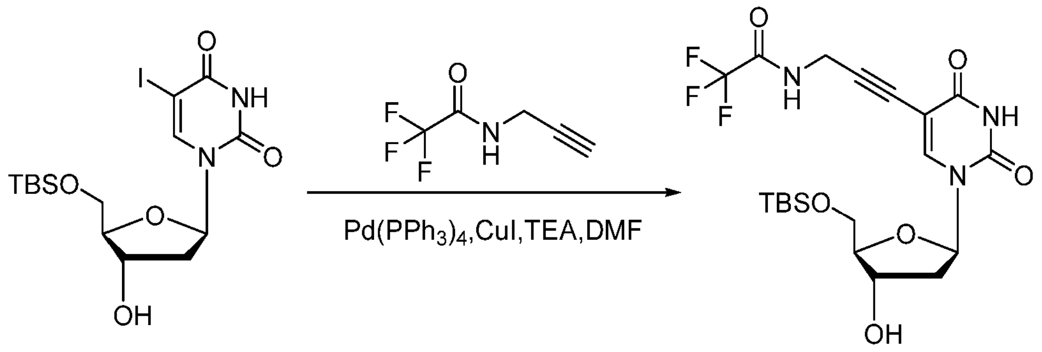


Iodinated nucleoside substrate T (Okeanos Tech, cat. No. OK-N-16001, 1g) was dissolved in DMF, followed by adding Pd(PPh3)4 (10 mol%), CuI (15 mol%), triethylamine (3 eq) and substrate propargylamine (Okeanos Tech, cat. No. OK20A410, 1.5eq). The mixture was allowed to react at 60°C for 12h. The reaction was quenched with water, extracted with DCM, concentrated, and purified by column chromatography to provide the product 1.1 g, as a white solid. MS[ES(-)],m/z 491.1. $^1$H NMR (400 MHz, DMSO-d6) δ 11.67 (s, 1H), 10.00 (t, J = 5.5 Hz, 1H), 7.94 (s, 1H), 6.12 (dd, J = 7.6, 5.9 Hz, 1H), 5.28 (d, J = 4.1 Hz, 1H), 4.26 - 4.12 (m, 3H), 3.88 (q, J = 2.8 Hz, 1H), 3.81 (dd, J = 11.5, 2.6 Hz, 1H), 3.73 (dd, J = 11.5, 3.1 Hz, 1H), 2.17 (ddd, J = 13.2, 6.0, 2.8 Hz, 1H), 2.05 (ddd, J = 13.3, 7.7, 5.8 Hz, 1H), 0.87 (s, 9H), 0.08 (d, J = 1.8 Hz, 6H).

(2) Step 2

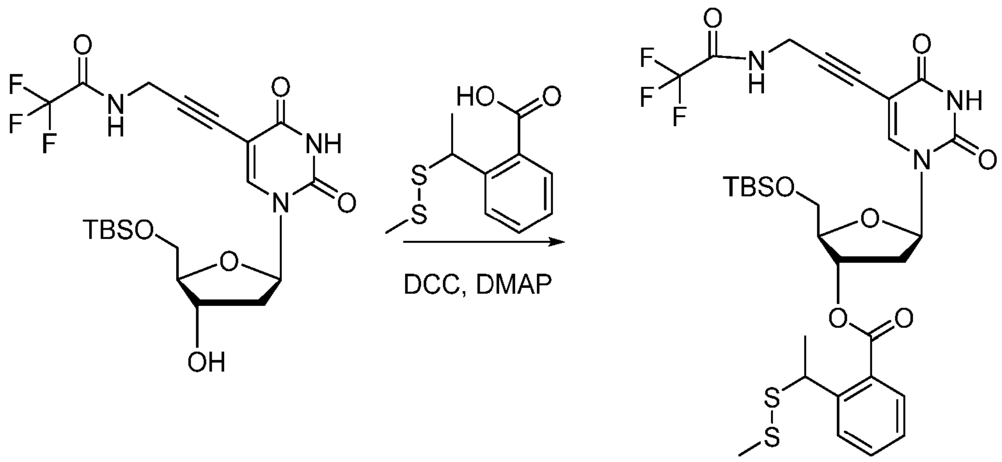


The nucleoside from Step 1 (300mg) was dissolved in 10mL of DMF, followed by adding DCC (1.2 eq) and DMAP (10% mol). The mixture was stirred for 30 minutes, added with disulfaneyl carboxylic acid substrate (OKeanos Tech, cat. No. OK20A420)(1.5 eq). The mixture was stirred for 12 hours and subjected directly to column chromatography to provide the product 359mg, as a white solid.

MS[ES(-)],m/z 700.3. $^1$H NMR (400 MHz, DMSO-$d_6$) δ 11.74 (s, 1H), 10.03 (t, J = 5.5 Hz, 1H), 7.98 (s, 1H), 7.90 - 7.82 (m, 1H), 7.68 - 7.58 (m, 2H), 7.47 - 7.40 (m, 1H), 6.23 - 6.18 (m, 1H), 5.45 -5.42 (m, 1H), 5.20 - 5.12 (m, 1H), 4.38 - 4.16 (m, 3H), 3.98 - 3.87 (m, 2H), 2.60 - 2.53 (m, 1H), 2.40 - 2.31 (m, 1H), 2.06 (d, J = 0.6 Hz, 3H), 1.65 (dd, J = 7.0, 1.0 Hz, 3H), 0.90 (s, 9H), 0.13 (d, J = 1.2 Hz, 6H).

(3) Step 3

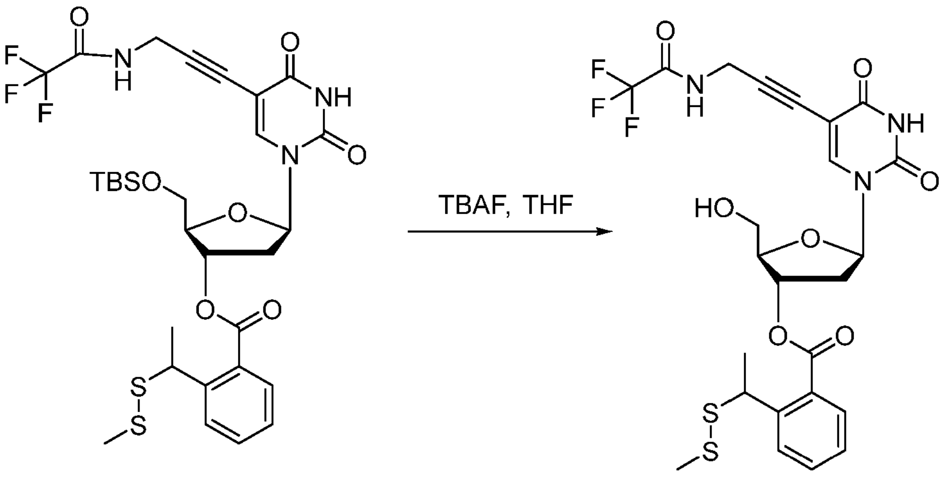


The nucleoside from Step 2 (300mg) was dissolved in 10mL of THF, followed by adding TBAF (2 eq, 1M in THF) at 0°C. The mixture was stirred at 0°C for 30 minutes, and allowed to warm back to room temperature with stirring for 4 hours. The mixture was subjected directly to column chromatography to provide the product 200mg, as a white solid.

MS[ES(-)],m/z 587.2. $^1$H NMR (400 MHz, DMSO-$d_6$) δ 11.68 (d, J = 3.2 Hz, 1H), 10.06 (t, J = 5.6 Hz, 1H), 8.23 (d, J = 3.2 Hz, 1H), 7.88 - 7.78 (m, 1H), 7.66 - 7.56 (m, 2H), 7.43 - 7.39 (m, 1H), 6.25 - 6.13 (m, 1H), 5.48 - 5.45 (m, 1H), 5.31 (t, J = 5.2 Hz, 1H), 5.18 - 5.12 (m, 1H), 4.28 - 4.16 (m, 3H), 3.76 - 3.67 (m, 2H), 2.50 - 2.40 (m, 2H), 2.04 (d, J = 9.2 Hz, 3H), 1.68 - 1.59 (m, 3H).

(4) Step 4

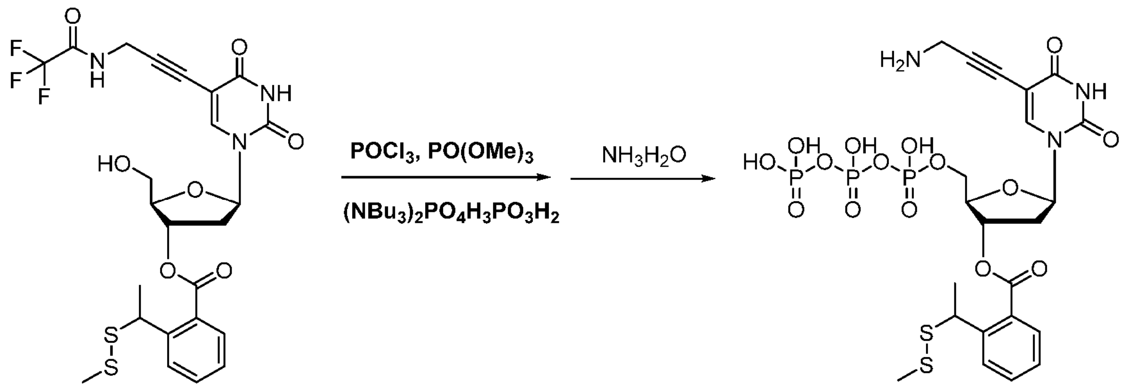


The nucleoside from Step 3 (200mg) was dissolved in 5mL of trimethyl phosphate. The mixture was added with phosphorus oxychloride (1.5 eq) at 0°C with stirring at 0°C for 120minutes. The reaction mixture was added to a DMF solution (5 mL) of tributyl ammonium pyrophosphate (2 eq) with continuously stirring at 0°C for 3h. The reaction was quenched with 0.1 M TEAB buffer and separated by preparative HPLC reversed phase column (C18, mobile phase: 0.1 M TEAB-acetonitrile). After concentration, the residue was added to 3 mL of concentrated aqueous ammonia and allowed to react for 2h, followed by preparative HPLC reversed phase column chromatography(C18, mobile phase: 0.1 M TEAB-acetonitrile)to provide the product 120mg as a white solid. MS[ES(-)],m/z 745.5. $^1$H NMR (400 MHz, $D_2O$) δ 8.51 (s, 1H), 7.90 (dd, J = 7.9, 1.4 Hz, 1H), 7.73 (d, J = 7.8 Hz, 1H), 7.67 (td, J = 7.6, 1.4 Hz, 1H), 7.48 (t, J = 7.5 Hz, 1H), 6.45 (td, J = 9.2, 5.7 Hz, 1H), 5.75 (t, J = 4.7 Hz, 1H), 5.15 - 5.07 (m, 1H), 4.72 - 4.64 (m, 1H), 4.39 (d, J = 3.3 Hz, 2H), 4.07 (s, 2H), 2.82 - 2.76 (m, 1H), 2.64 - 2.54 (m, 1H), 2.02 (d, J = 6.4 Hz, 3H), 1.73 (dd, J = 7.1, 2.2 Hz, 3H); 31P NMR (162 MHz, D2O) δ -9.79 (dd, J = 20.3, 9.6 Hz, 1P), -11.68 (d, J = 19.2 Hz, 1P), -22.82 (td, J = 19.2, 6.1 Hz, 1P).

(5) Step 5

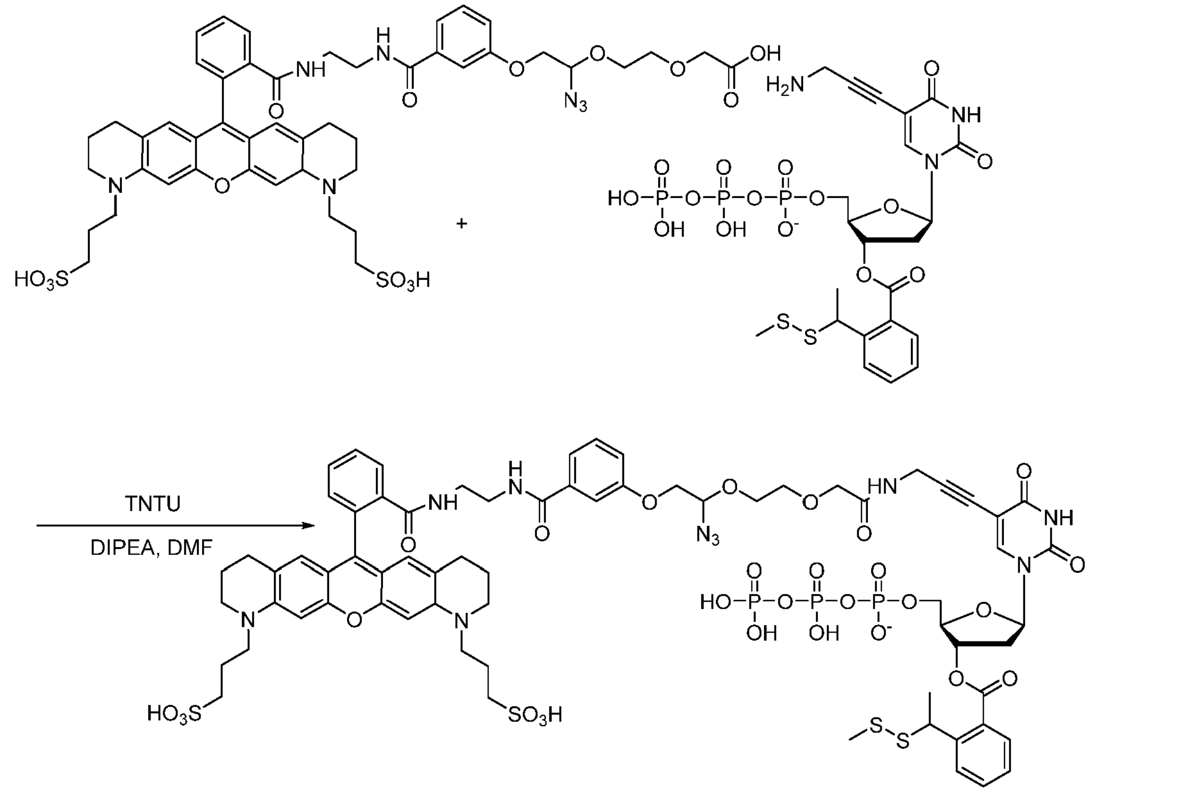


To a dried 20mL round bottom flask equipped with a magnetic stirrer was added dye-linker solid (Okeanos Tech, OK-F-20211)(9.3 mg, 1 eq.), dissolved with appropriate amount of DMF(3 ml), followed by adding in sequence TNTU solid (7 mg, 0.02 mmol, 2 eq.) and DIPEA (2.6 mg, 0.02 mmol, 2 eq.), wherein DIPEA was diluted with 0.5 ml of DMF in a 1.5 ml PE tube and added into the mixture dropwise with stirring for about 1 hour. The mixture was sampled and dissolved in acetonitrile, and monitored with HPLC and MS for AF532-V4 raw material depletion. To the mixture was added solid hot T-mSSEB substrate (15 mg, 0.02 mmol, 1 eq.) at room temperature with stirring for 1 hour, and sampled and dissolved in acetonitrile, and monitored with HPLC and MS for reaction progress. The reaction was allowed to proceed overnight until a complete consumption of nhs ester, quenched with 0.1 M TEAB buffer, and separated with preparative HPLC reverse phase column chromatography (C18, mobile phase: 0.1 M TEAB-acetonitrile). The desired fractions were concentrated, and added into 3 mL of concentrated aqueous ammonia to react for 2h, followed by purification with preparative HPLC reverse phase column chromatography (C18, mobile phase: 0.1 M TEAB-acetonitrile) to provide a solid 10mg. MS[ES(-)],m/z 1815.0. $^1$H NMR (400 MHz, DMSO-$d_6$) δ 9.06 (d, *J* = 6.7 Hz, 1H), 8.80 (d, *J* = 47.0 Hz, 1H), 8.28 (t, *J* = 7.2 Hz, 1H), 8.18 - 8.00 (m, 1H), 7.86 (d, *J* = 7.7 Hz, 1H), 7.75 - 7.66 (m, 2H), 7.66 - 7.58 (m, 3H), 7.58 - 7.40 (m, 4H), 7.39 - 7.33 (m, 1H), 7.17 - 7.07 (m, 1H), 6.98 (s, 2H), 6.79 (s, 2H), 6.31 - 6.17 (m, 1H), 5.58 (d, *J* = 3.5 Hz, 1H), 5.21 - 5.14 (m, 2H), 4.34 (d, *J* = 19.3 Hz, 1H), 4.26 (dd, *J* = 10.5, 3.9 Hz, 1H), 4.18 (dd, *J* = 10.6, 4.5 Hz, 2H), 4.09 (d, *J* = 3.5 Hz, 2H), 3.99 (s, 2H), 3.95 - 3.82 (m, 2H), 3.68 (s, 6H), 3.52 (s, 4H), 3.33 - 3.24 (m, 2H), 3.22 - 3.15 (m, 2H), 3.14 - 3.06 (m, 2H), 2.65 (t, *J* = 7.6 Hz, 4H), 2.57 (t, *J* = 7.1 Hz, 5H), 2.46 - 2.37 (m, 1H), 2.05 (d, *J* = 16.8 Hz, 3H), 2.01 - 1.93 (m, 4H), 1.86 - 1.81 (m, 3H), 1.77 - 1.72 (m, 1H), 1.65 (d, *J* = 6.9 Hz, 3H).

2. Sequencing performed with nucleotide analogues containing the hot T-SSEB synthesized above

Nucleotide substrates: fluorescent labeled standard hot dNTP (available from MGISEQ-2000RS high-throughput sequencing kit (FCL SE50), MGI Tech Co., Ltd., cat. no. 1000012551), wherein hot dTTP was replaced by hot T-SSEB synthesized above, with structures showed below; and nucleotide analogues cold dNTP of the invention (four kinds, named as SSEB) with structures showed below.

cold dNTP (SSEB)

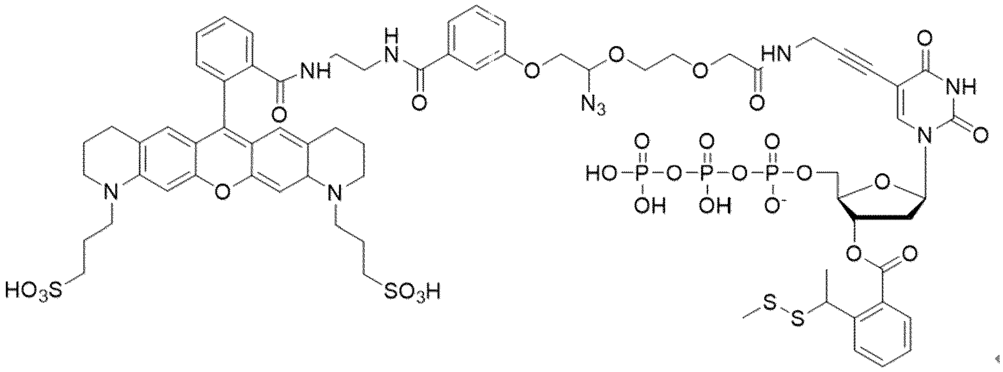

hot dTTP(hot T-SSEB)

Sequencing procedure was same as that above in Sequencing Example 1. Results of Basecall analysis were showed in Table 5.

Table 5. Results of Basecall analysis

| | |
|---|---|
| Nucleotide analogues | hot T-SSEB + cold dNTP-SSEB, standard hot dNTP for others |
| Reference | Ecoli.fa |
| Cycle Number | 50 |
| Q30(%) | 87.24 |
| Lag(%) | 0.19 |
| Runon(%) | 0.24 |
| ESR(%) | 74.7 |
| Mapping Rate, % | 98.44 |
| AvgError Rate, % | 0.27 |

Sequencing Example 5: Sequencing with SS-hot G

1. Synthesis of nucleotide analogues dGTP carrying fluorescent group (named as SS-hot G)

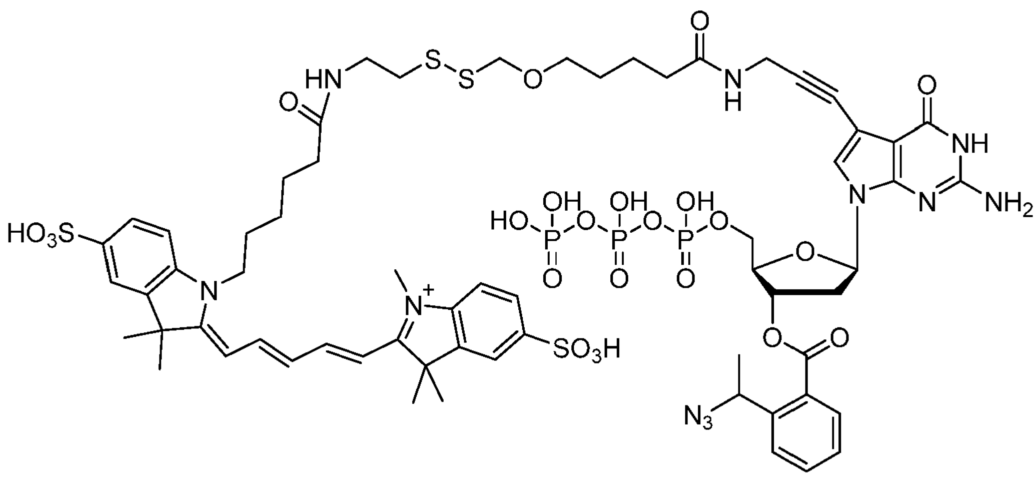

SS-hot G

The product was synthesized similarly to that in Sequencing Example 4, wherein an iodinated G-nucleoside substrate was used in place of the iodinated T-nucleoside substrate, and azdio carboxylic acid substrate was used in place of the disulfaneyl carboxylic acid substrate, both available from OKeanos Tech Co., Ltd. New dye-linker was required and available from MyChem LLC Co., Ltd. (cat. no. 110920Cy5). 10mg of final product was obtained. MS[ES(-)],m/z 1575. $^1$H NMR (400 MHz, DMSO-$d_6$) δ 10.54 (s, 1H), 8.49 (s, 1H), 8.34 (t, *J* = 13.1 Hz, 2H), 8.07 (t, *J* = 5.5 Hz, 1H), 7.92 (dd, *J* = 7.2, 4.6 Hz, 2H), 7.80 (d, *J* = 1.7 Hz, 2H), 7.70 (d, *J* = 7.4 Hz, 1H), 7.67 - 7.62 (m, 4H), 7.49 (t, *J* = 7.4 Hz, 2H), 7.30 (d, *J* = 8.3 Hz, 3H), 6.58 (t, *J* = 12.3 Hz, 1H), 6.41 (s, 2H), 6.36 - 6.27 (m, 3H), 6.26 - 6.12 (m, 2H), 5.60 (dd, *J* = 13.1, 6.4 Hz, 3H), 4.86 (s, 2H), 4.48 (dd, *J* = 29.1, 16.7 Hz, 2H), 4.35 - 4.27 (m, 2H), 4.07 (d, *J* = 4.8 Hz, 4H), 3.59 (s, 2H), 3.50 (d, *J* = 5.7 Hz, 2H), 3.34 - 3.29 (m, 2H), 2.78 (t, *J* = 6.8 Hz, 2H), 2.13 (t, *J* = 6.7 Hz, 2H), 2.07 (t, *J* = 7.2 Hz, 2H), 1.68 (s, 12H), 1.53 - 1.48 (m, 8H), 1.35 (dt, *J* = 15.8, 8.1 Hz, 4H).

2. Sequencing performed with nucleotide analogues containing the SS-hot G synthesized above

Nucleotide substrates: fluorescent labeled standard hot dNTP (available from MGISEQ-2000RS high-throughput sequencing kit (FCL SE50), MGI Tech Co., Ltd., cat. no. 1000012551), wherein hot dGTP was replaced by SS-hot G synthesized above; nucleotide analogues cold dNTP of the invention (four kinds, named as SS-cold) with structures showed below.

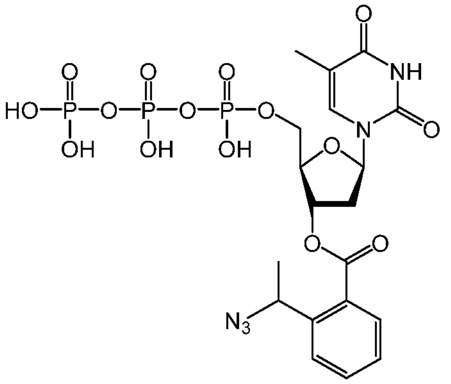
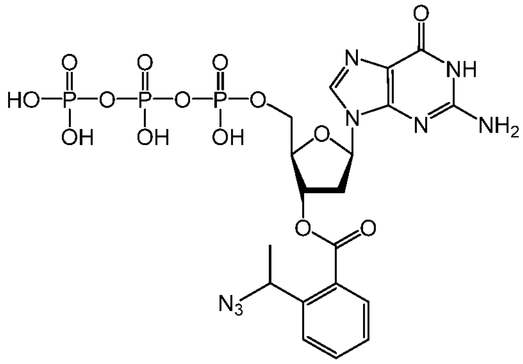
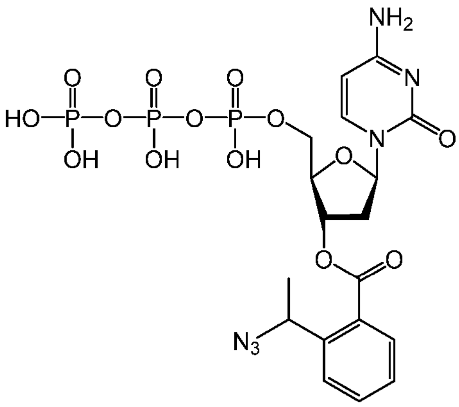
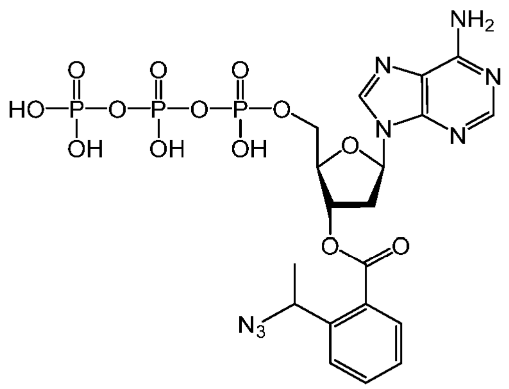

cold dNTP (SS-cold)

Sequencing procedure was same as that above in Sequencing Example 1. Results of Basecall analysis were showed in Table 6.

Table 6: Results of Basecall analysis

| nucleotide analogues | SS-hot G + SS-cold dNTP, standard hot dNTP for others |
|---|---|
| Reference | Ecoli.fa |
| Cycle Number | 50 |
| Q30(%) | 53.08 |
| Lag(%) | 0.24 |

(continued)

| | |
|---|---|
| nucleotide analogues | SS-hot G + SS-cold dNTP, standard hot dNTP for others |
| Runon(%) | 0.32 |
| ESR(%) | 66.96 |
| Mapping Rate, % | 0.13 |
| AvgError Rate, % | 5.14 |

## Claims

**1.** A compound of formula (A) or a salt thereof,

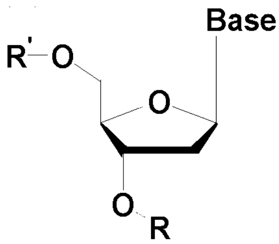

formula (A)

wherein:

R is a reversible blocking group, and R is selected from

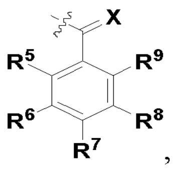

,

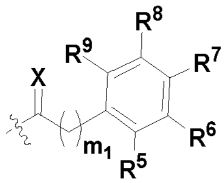

;

each X is independently selected from O, NH, S;
preferably, X is O;
$R^5$ is

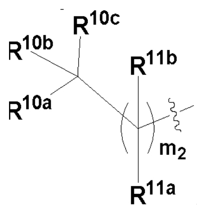

,

and $R^6$, $R^7$, $R^8$, and $R^9$ are each independently selected from H, nitro, $C_1$-$C_6$ alkyl, $C_1$-$C_6$ alkoxy, $C_1$-$C_6$ alkyl-C(=O)-$NH_2$-;
preferably, $R^5$ is

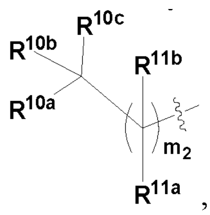,

and $R^6$, $R^7$, $R^8$, and $R^9$ are each independently selected from H nitro, methoxy, acetamido;
most preferably, $R^5$ is

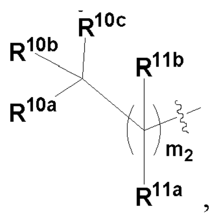,

$R^7$ is selected from H, nitro, methoxy, acetamido, and $R^6$, $R^8$, and $R^9$ are H;
any one of $R^{10a}$, $R^{10b}$, and $R^{10c}$ is $-N_3$ or -SS-$C_1$-$C_6$ alkyl (e.g., -SS-methyl, -SS-ethyl, -SS-isopropyl, -SS-tertbutyl or -SS-isobutyl), and the other two are each independently selected from H, $C_1$-$C_6$ alkyl, such as methyl, ethyl, isopropyl, tertbutyl;
preferably, any one of $R^{10a}$, $R^{10b}$, and $R^{10c}$ is $-N_3$, -SS-methyl, -SS-ethyl, -SS-isopropyl, -SS-tertbutyl or -SS-isobutyl, and the other two are each independently selected from H or methyl;
$R^{11a}$ and $R^{11b}$ are each independently selected from H or $C_1$-$C_6$ alkyl, such as methyl, ethyl, isopropyl, tertbutyl; preferably, $R^{11a}$ and $R^{11b}$ are H;
each $m_1$ is independently selected from 1, 2, 3, 4, 5, and 6; preferably, $m_1$ is 1;
each $m_2$ is independently selected from 0, 1, 2, 3, 4, 5, and 6; preferably, each $m_2$ is independently selected from 0 or 1;
most preferably,

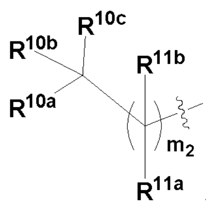,

as a whole, is selected from

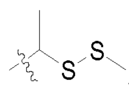,

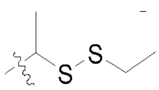, 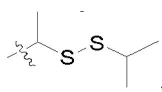, 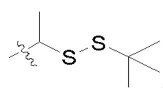, 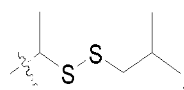, 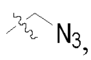, 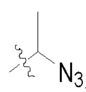, 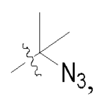,

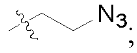;

R' is selected from H, monophosphate group

( 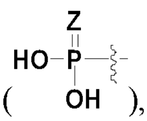 ),

diphosphate group

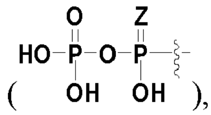,

triphosphate group

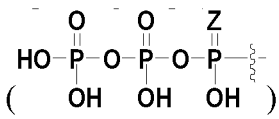

or tetraphosphate group

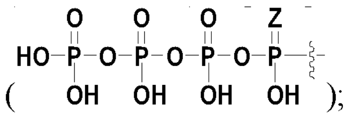;

preferably, R' is a triphosphate group

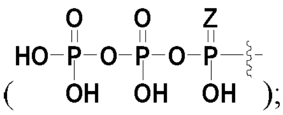;

each Z is independently selected from O, S, BH; preferably, Z is O;
Base is selected from a base, a deaza base or a tautomer thereof, for example, Base is selected from adenine, 7-deazaadenine, thymine, uracil, cytosine, guanine, 7-deazaguanine or a tautomer thereof;
preferably, Base is selected from

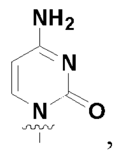, 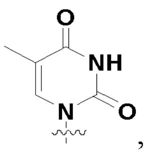, 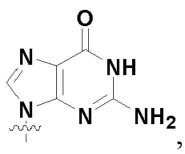, 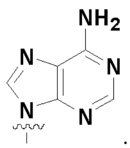.

**2.** The compound or a salt thereof according to claim 1, wherein the compound is selected from the followings:

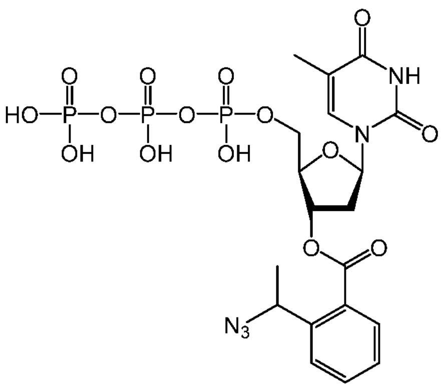 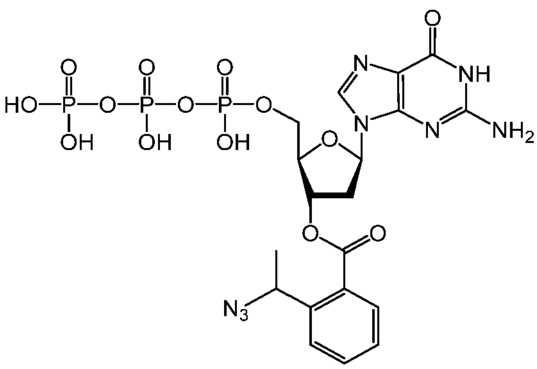

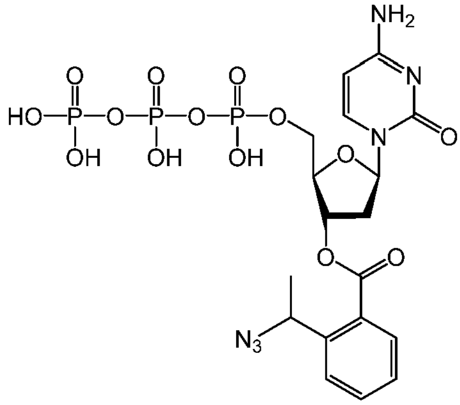

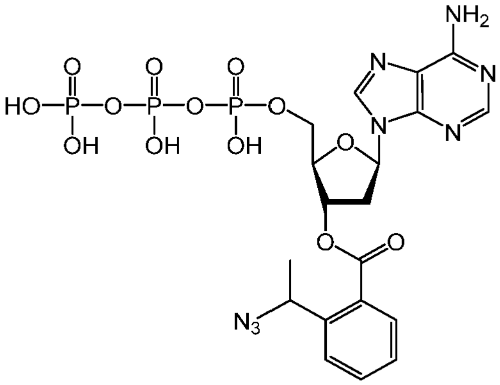

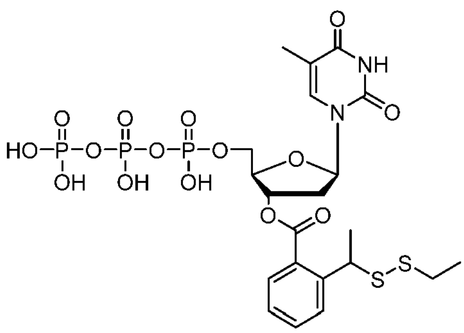

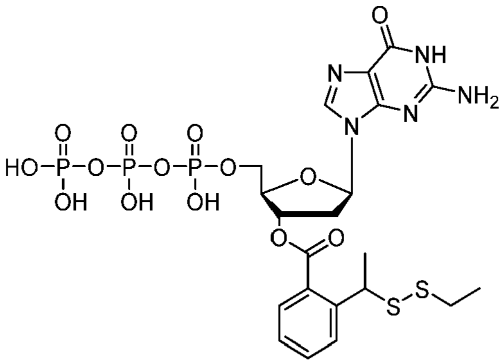

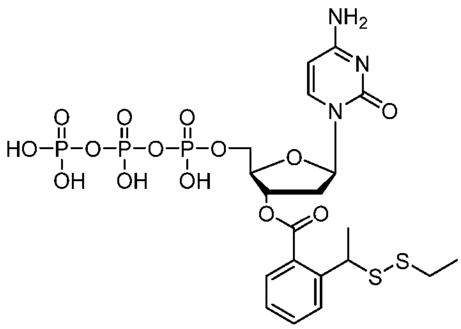

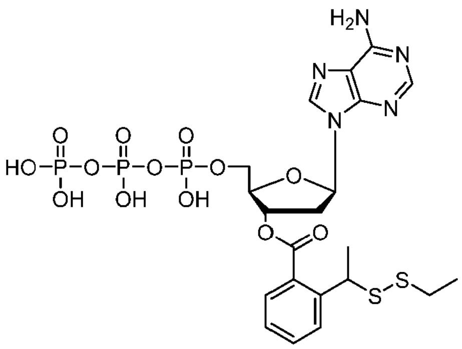

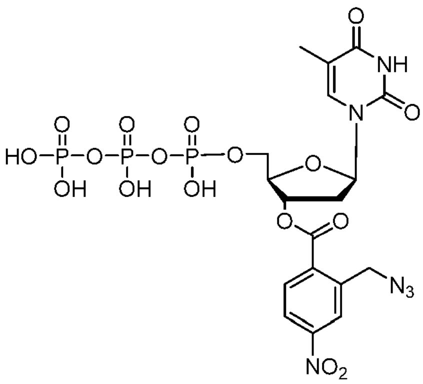

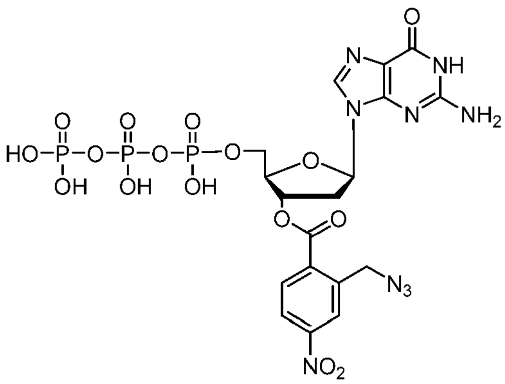

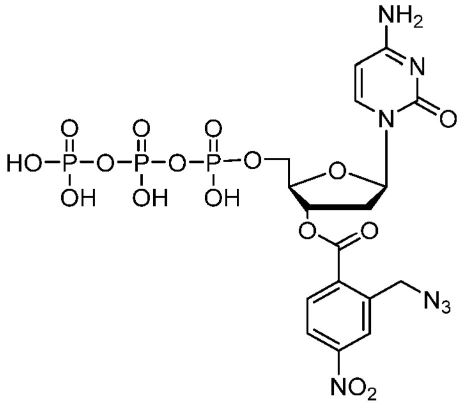

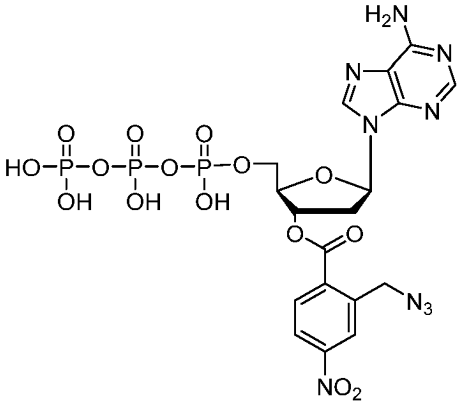

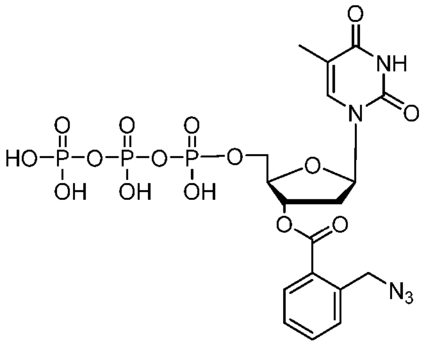

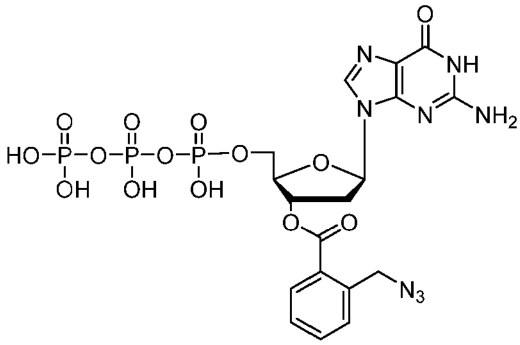

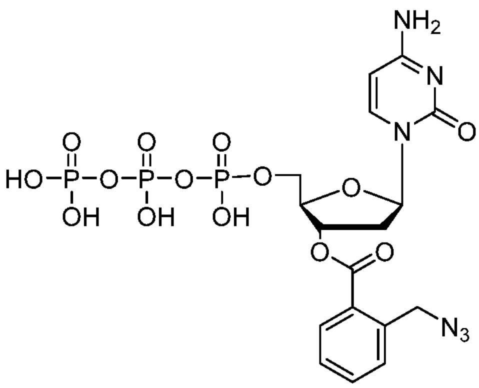

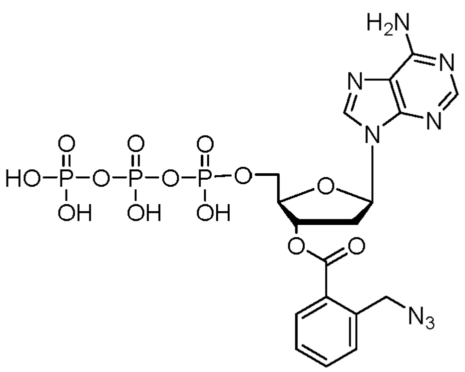

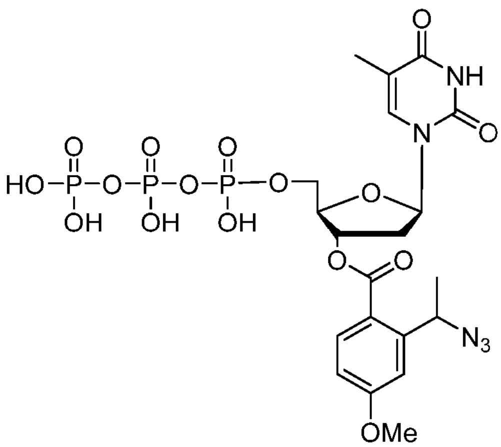

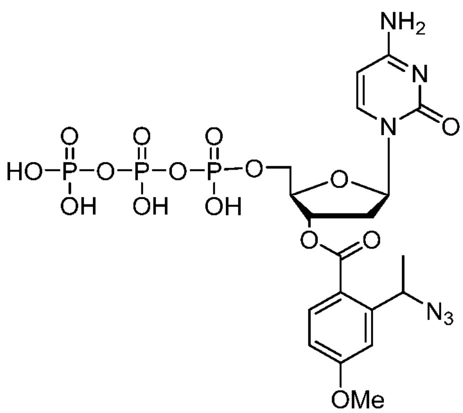

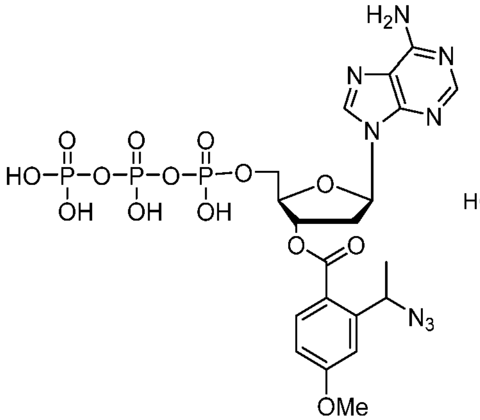

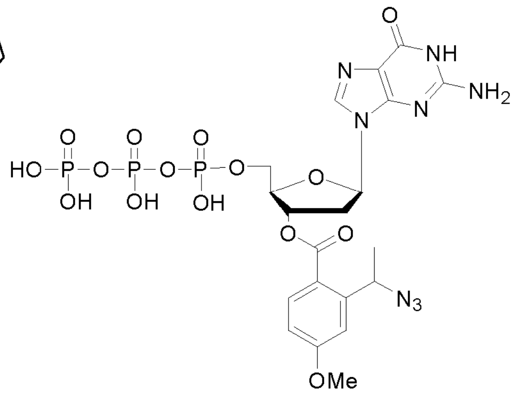

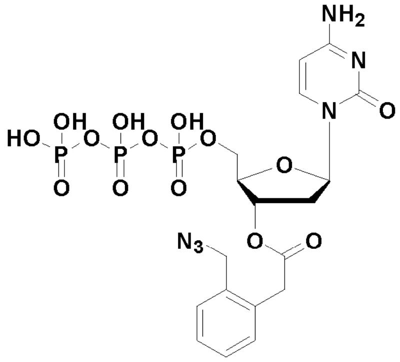

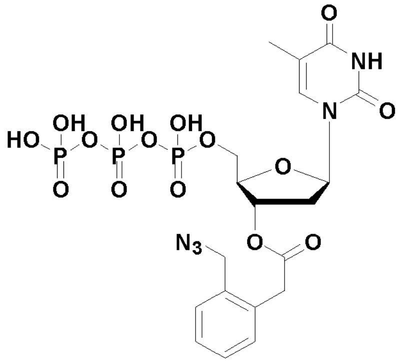

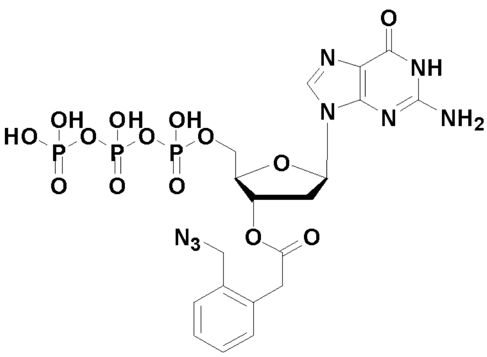

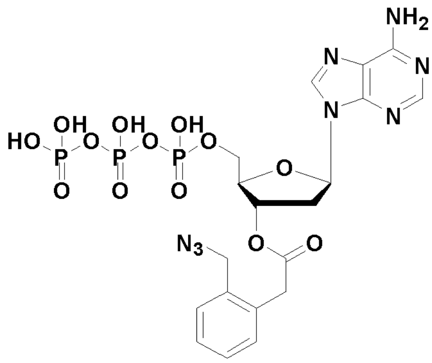

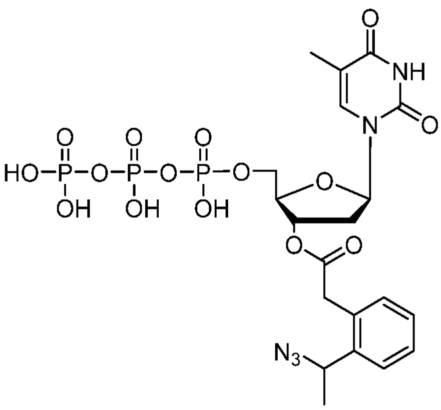

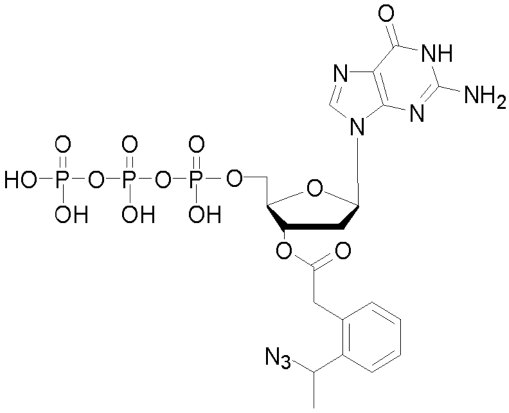

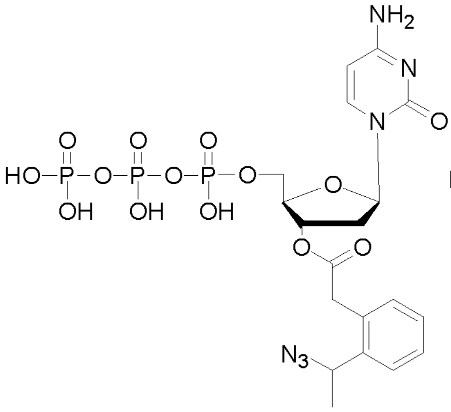

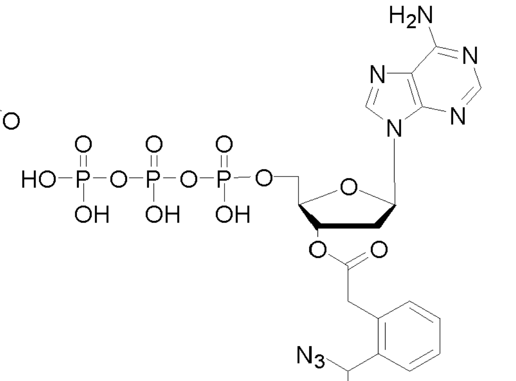

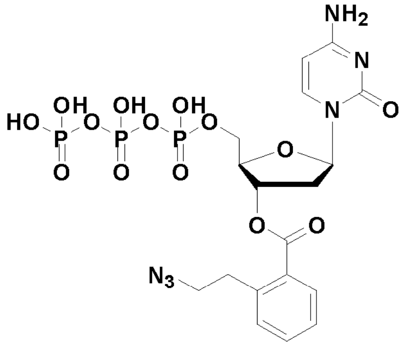

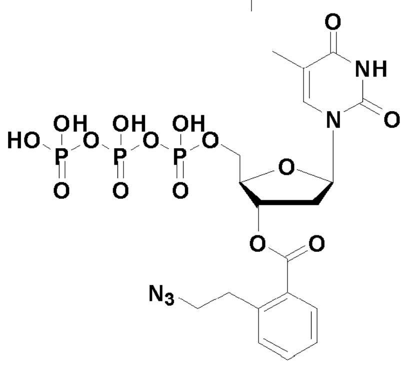

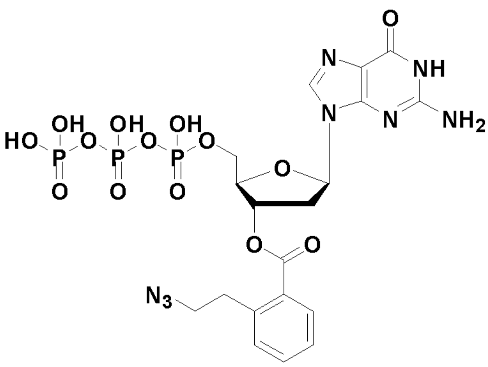

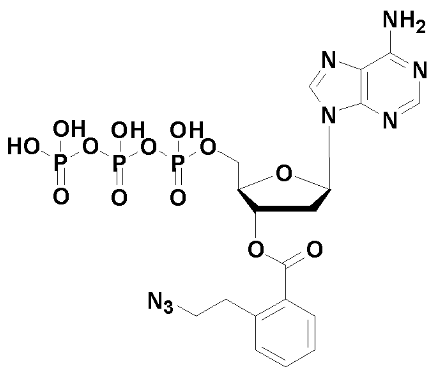

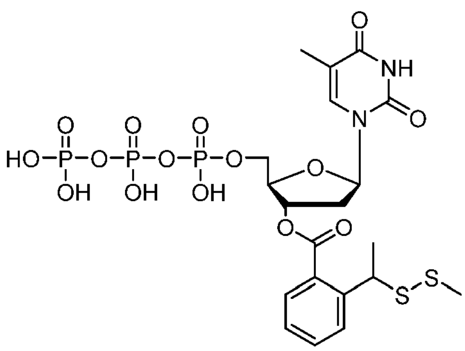

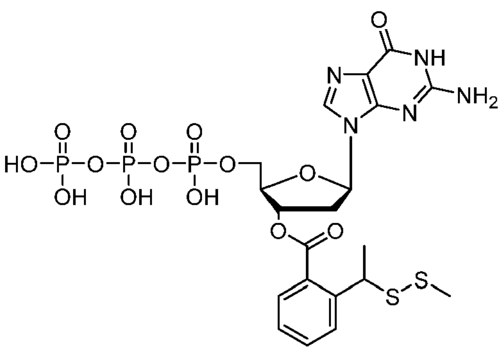

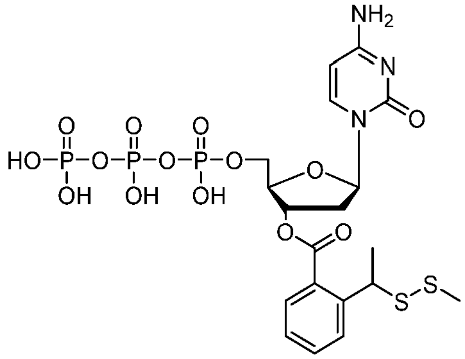

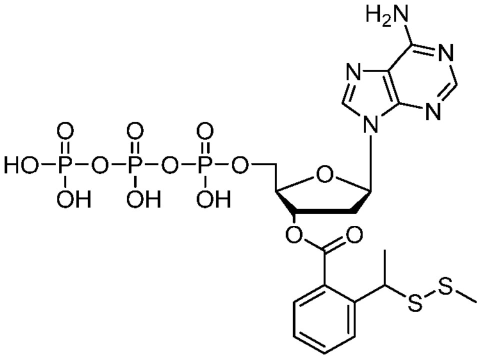

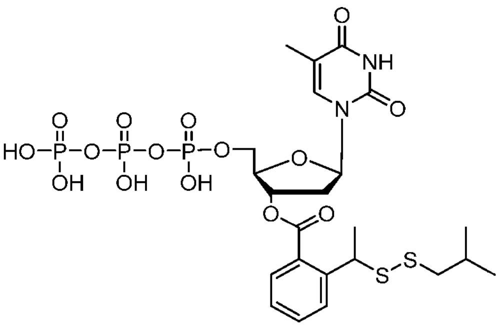

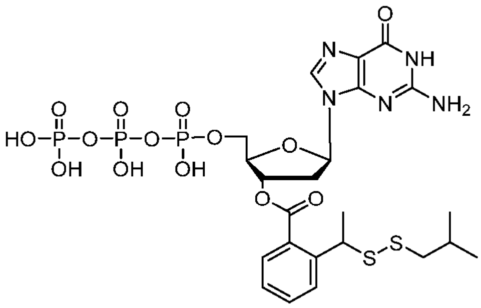

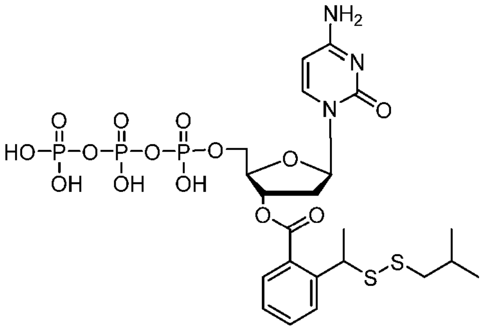

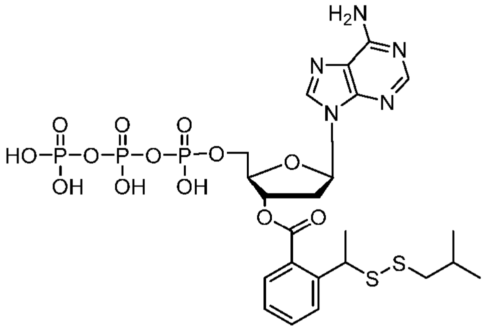

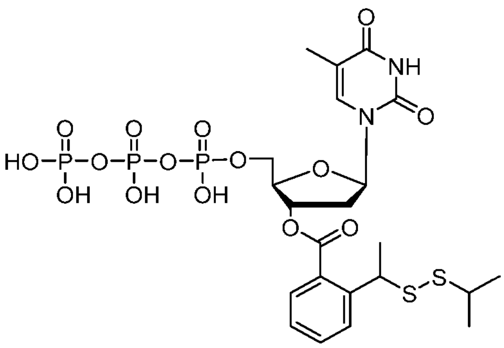

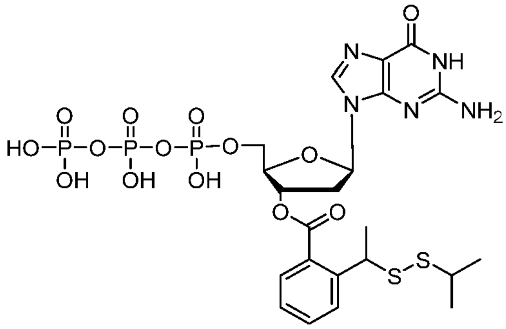

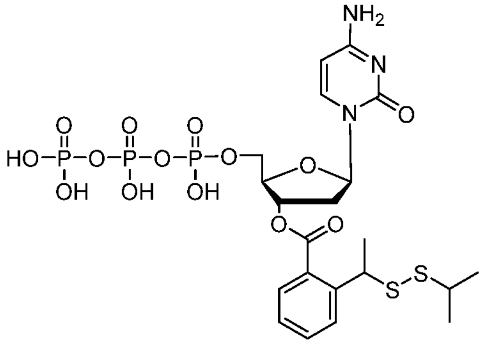

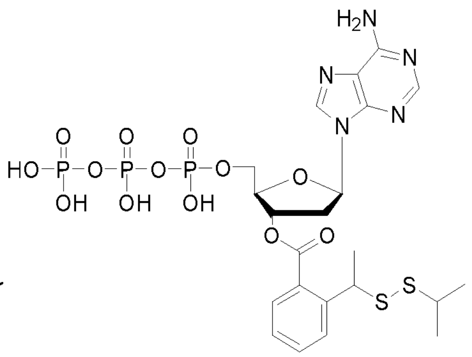

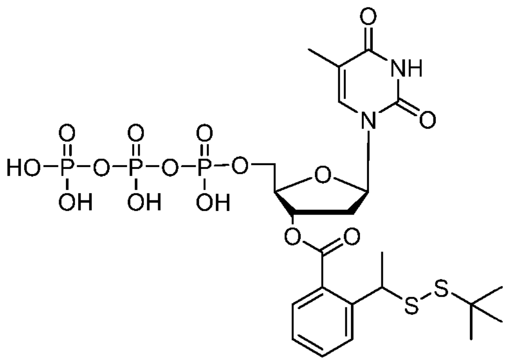

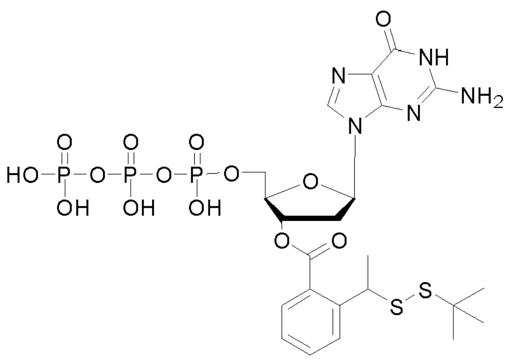

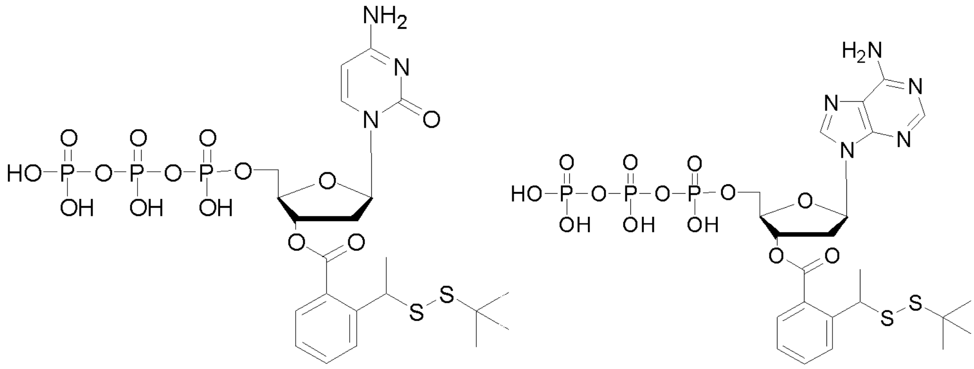

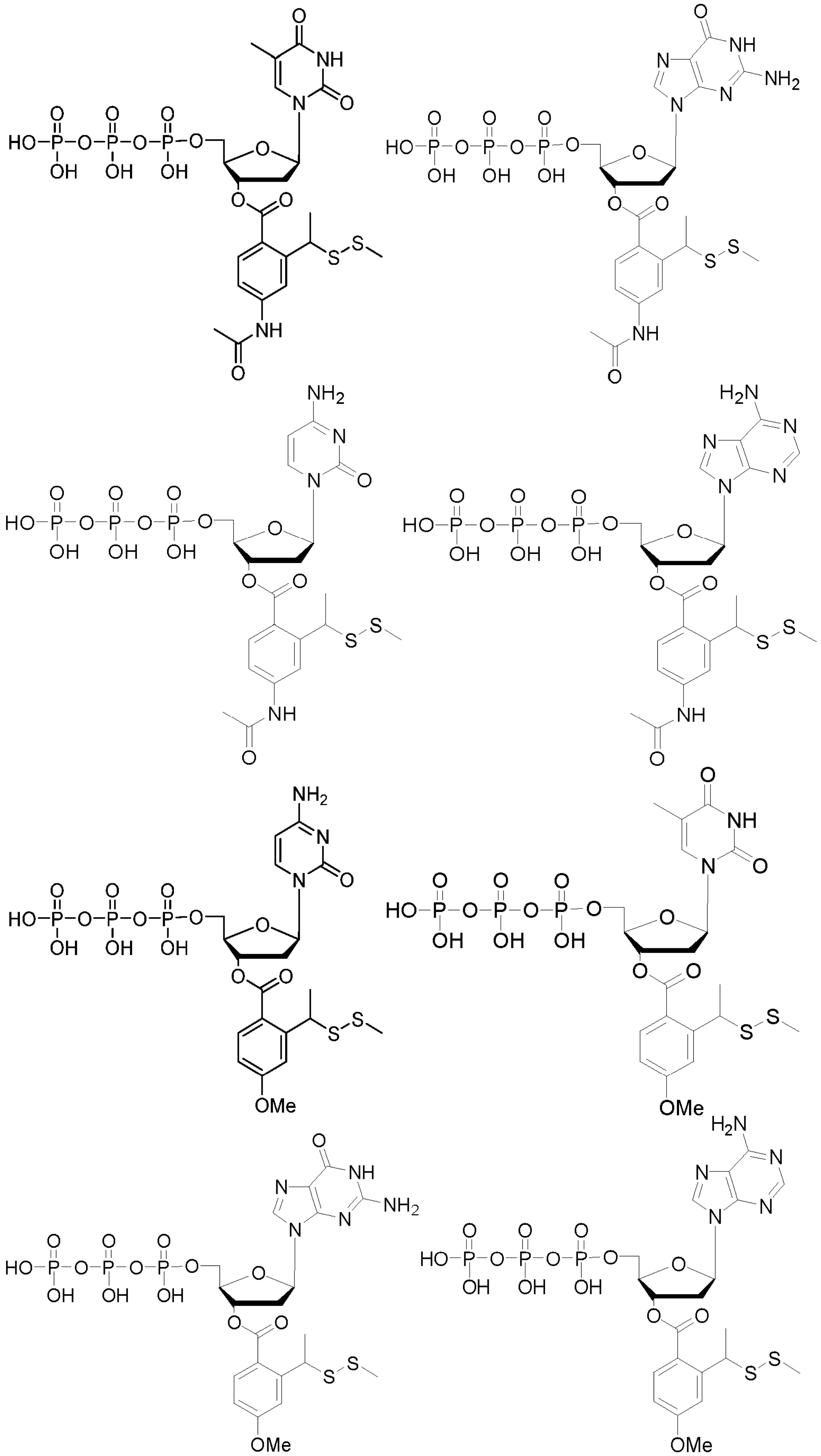

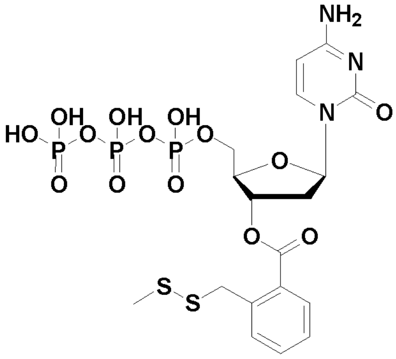

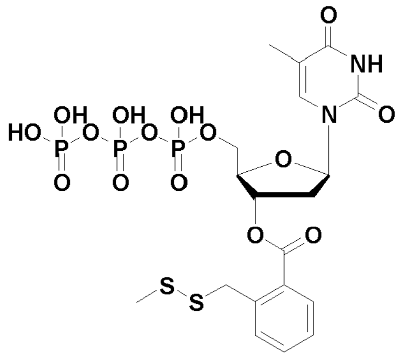

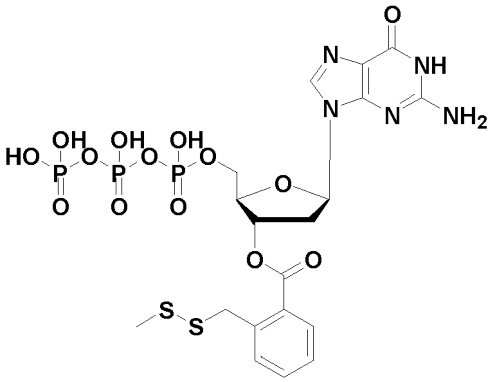

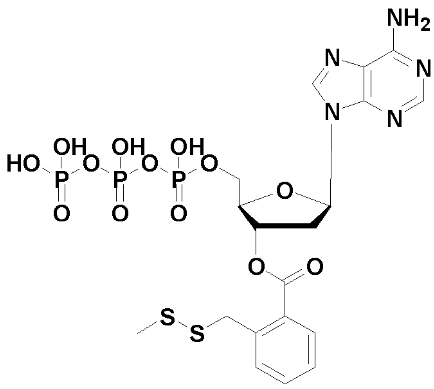

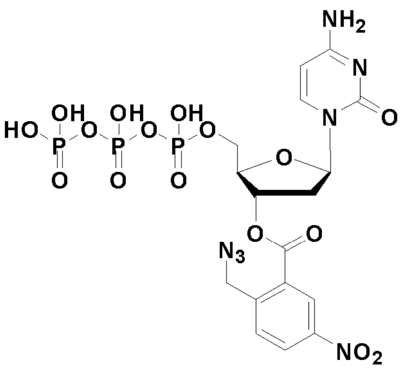

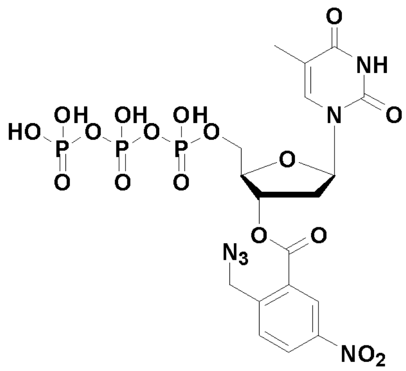

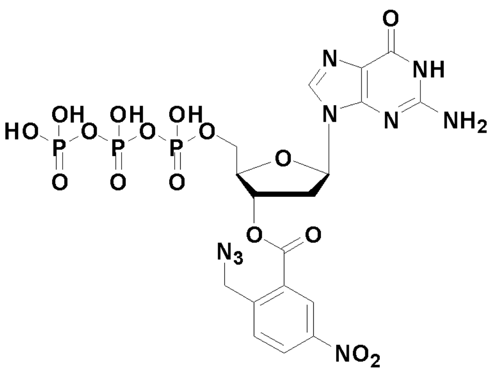

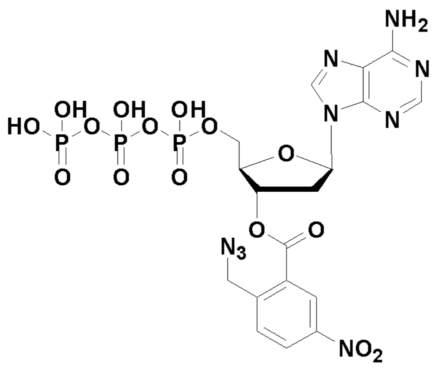

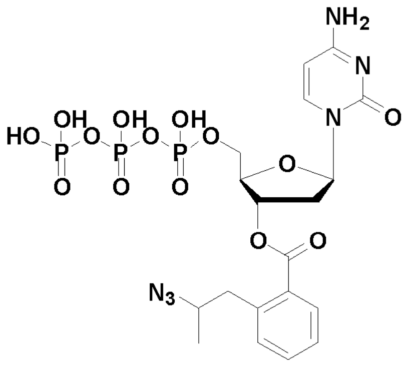
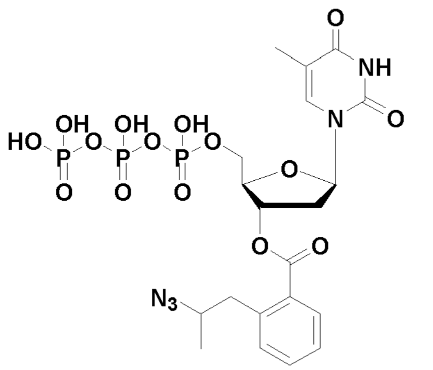
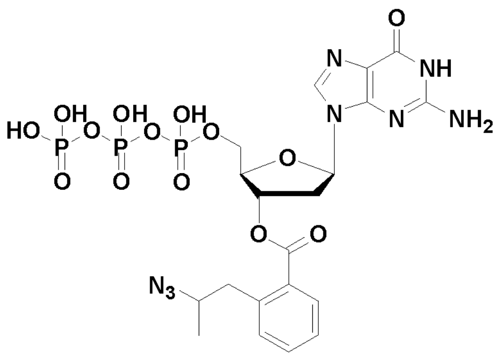
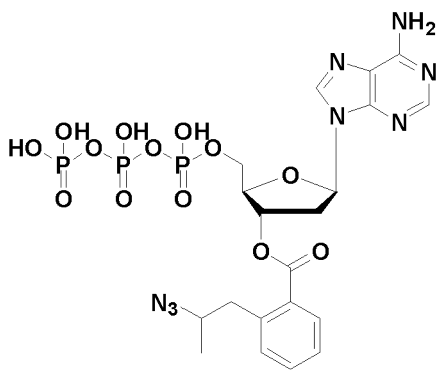
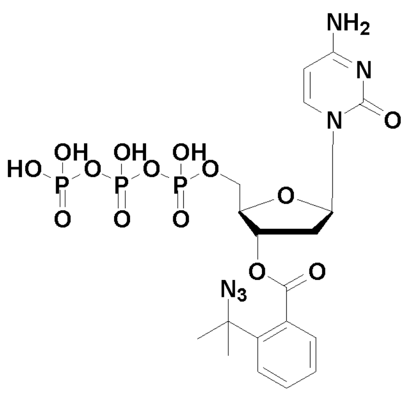
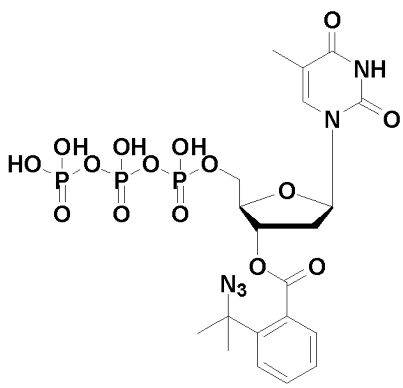
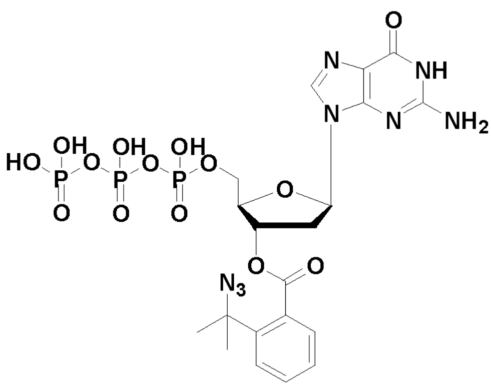
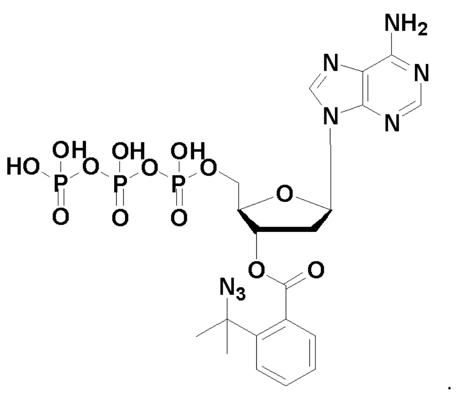

3. The compound or a salt thereof according to claim 1 or 2, which carries an additional detectable label, wherein the detectable label is a fluorescent label;

the additional detectable label carried by the compound or a salt thereof is introduced by an affinity reagent, which is selected from antibody, aptamer, Affimer, and Knottin, wherein the affinity reagent carries the detectable label, and the affinity reagent can specifically recognize and bind to an epitope of the compound or a salt thereof;
the additional detectable label is linked to the Base of the compound or a salt thereof optionally via a linker;
preferably, the linker is a cleavable linker or a non-cleavable linker;
preferably, the cleavable linker is selected from the group consisting of a linker capable of being cleaved by electrophilic reaction, a linker capable of being cleaved by nucleophilic reaction, a linker capable of being cleaved by photolysis, a linker capable of being cleaved under reductive conditions, a linker capable of being cleaved under oxidative conditions, a safety-catch linker, a linker capable of being cleaved by elimination mechanisms, or any combination thereof;
preferably, the detectable label is selected from the followings:

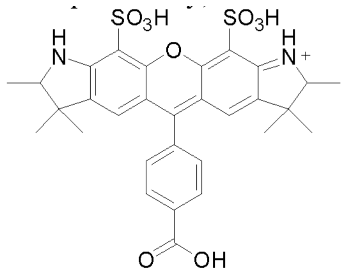

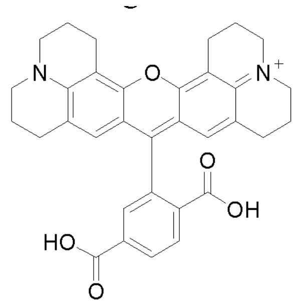

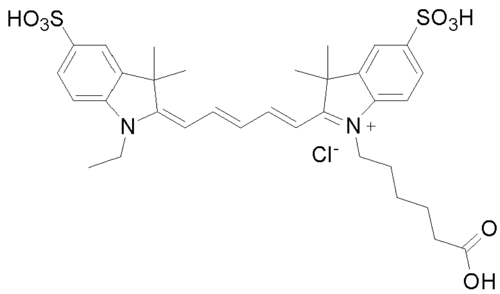

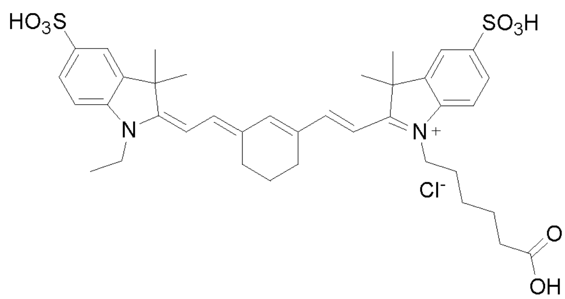

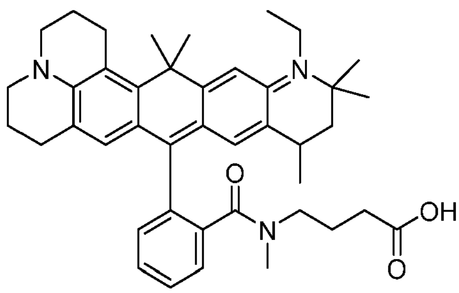

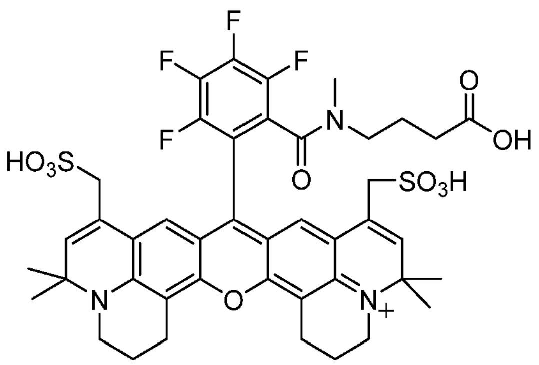

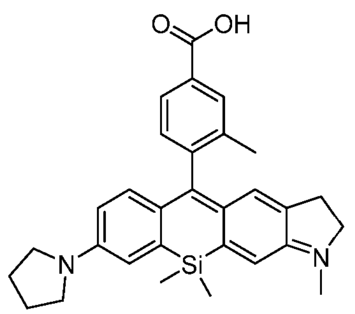

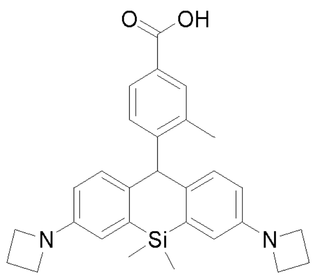

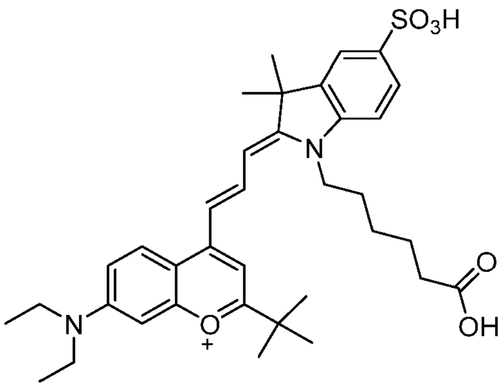

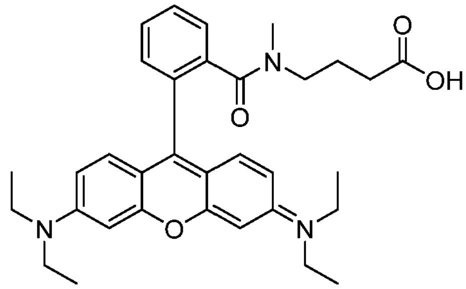

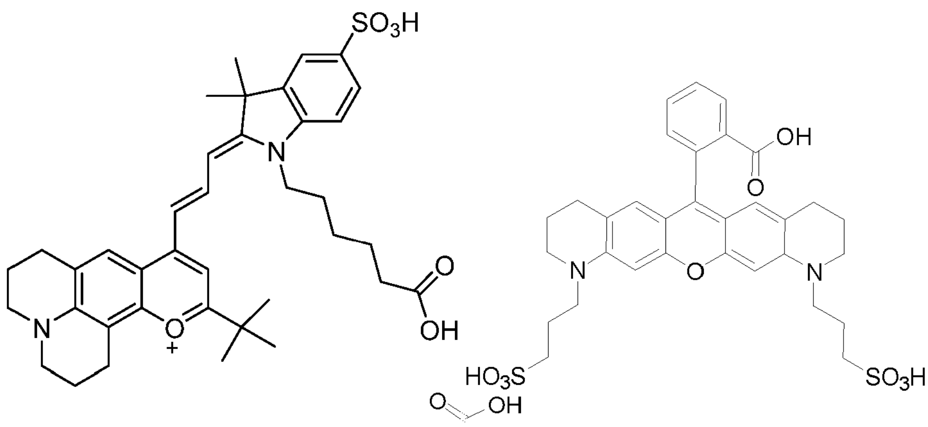

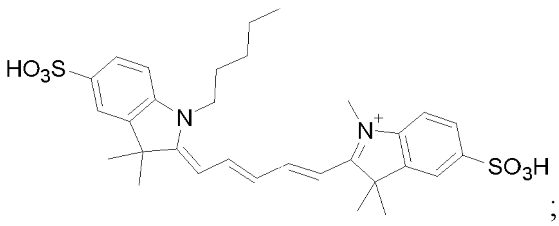

;

preferably, if Base is different, the detectable label varies.

4. A method for terminating a nucleic acid synthesis, which comprises incorporating the compound or a salt thereof according to any one of claims 1-3 into a nucleic acid molecule to be terminated;

preferably, the incorporation of the compound or a salt thereof is achieved by a terminal transferase, a terminal polymerase or a reverse transcriptase;
preferably, the method comprises incorporating the compound or a salt thereof into the nucleic acid molecule to be terminated by using a polymerase;
preferably, the method comprises performing a nucleotide polymerization reaction using a polymerase under conditions where the polymerase is allowed to carry out the nucleotide polymerization reaction, thereby incorporating the compound or a salt thereof into the 3' end of the nucleic acid molecule to be terminated.

5. A method for preparing a growing polynucleotide complementary to a target single-stranded polynucleotide in a sequencing reaction, which comprises incorporating the compound or a salt thereof according to any one of claims 1-3 into the growing complementary polynucleotide, wherein the incorporation of the compound or a salt thereof prevents any subsequent nucleotides from being introduced into the growing complementary polynucleotide;

preferably, the incorporation of the compound or a salt thereof is achieved by a terminal transferase, a terminal polymerase or a reverse transcriptase;
preferably, the method comprises incorporating the compound or a salt thereof into the growing complementary

polynucleotide by using a polymerase;
preferably, the method comprises performing a nucleotide polymerization reaction using a polymerase under conditions where the polymerase is allowed to carry out the nucleotide polymerization reaction, thereby incorporating the compound or a salt thereof into the 3' end of the growing complementary polynucleotide.

**6.** A method for determining the sequence of a target single-stranded polynucleotide, which comprises:

1) monitoring the incorporation of nucleotides complementary to the target single-stranded polynucleotide in a growing nucleic acid strand, wherein at least one complementary nucleotide incorporated is the compound or a salt thereof according to any one of claims 1 to 3, and the compound or a salt thereof carries an additional detectable label, wherein the detectable label is a fluorescent label, and,
2) detecting the detectable label to determine the incorporated nucleotide;
preferably, the additional detectable label is linked to the compound or a salt thereof optionally via a linker;
preferably, the linker is as defined in claim 3;
preferably, the additional detectable label is as defined in claim 3;
preferably, if Base is different, the detectable label carried by the compound or a salt thereof varies;
preferably, the reversible blocking group (R) in the compound or a salt thereof and the detectable label are removed prior to introducing a next complementary nucleotide;
preferably, the reversible blocking group and the detectable label are removed simultaneously; or
preferably, the reversible blocking group and the detectable label are removed sequentially; for example, after the detectable label is removed, the reversible blocking group is removed, or, after the reversible blocking group is removed, the detectable label is removed.

**7.** The method according to claim 6, which comprises the following steps of:

(a) providing a plurality of different nucleotides, wherein the plurality of different nucleotides are the compound or a salt thereof according to any one of claims 1-3, wherein each nucleotide carries an additional detectable label that can be distinguished from an additional detectable label carried by another nucleotide during detection;
(b) incorporating the plurality of different nucleotides into a sequence complementary to a target single-stranded polynucleotide;
(c) detecting the additional detectable labels carried by the nucleotides in step (b) to determine the types of nucleotides incorporated;
(d) removing the reversible blocking groups and the detectable labels carried by the nucleotides in step (b); and
(e) optionally repeating steps (b)-(d) one or more times;

whereby the sequence of the target single-stranded polynucleotide is determined.

**8.** The method according to claim 6, which comprises the following steps of:

(1)providing a first nucleotide, a second nucleotide, a third nucleotide and a fourth nucleotide, wherein at least one of the four nucleotides is the compound or a salt thereof according to any one of claims 1-3, the Bases contained in the four nucleotides are different from each other, and the four nucleotides carry an additional detectable label, preferably, the additional detectable label carried by the four nucleotides is introduced by an affinity reagent, which is selected from antibody, aptamer, Affimer, and Knottin, wherein the affinity reagent carries the detectable label, and the affinity reagent can specifically recognize and bind to an epitope of each nucleotide, or preferably, the four nucleotides are linked to the additional detectable label optionally via a linker, or more preferably, the Base of the four nucleotides is linked to the additional detectable label optionally via a linker, most preferably, the additional detectable labels carried by the four nucleotides are different from each other;
(2)contacting the four nucleotides with a target single-stranded polynucleotide; removing the nucleotides that are not incorporated into the growing nucleic acid strand; detecting the detectable labels carried by the nucleotides that are incorporated into the growing nucleic acid strand; removing the reversible blocking groups and the detectable labels carried by the nucleotides that are incorporated into the growing nucleic acid strand;

optionally, it also includes (3): repeat the step (2) one or more times.

**9.** The method according to claim 6, which comprises the following steps of:

(a)providing a mixture comprising a duplex, at least one compound or a salt thereof according to any one of claims

1-3, polymerase and an excision reagent; wherein the duplex comprises a growing nucleic acid strand and a nucleic acid strand to be sequenced; wherein the compound or a salt thereof carries an additional detectable label, preferably, the additional detectable label carried by the compound or a salt thereof is introduced by an affinity reagent, wherein the affinity reagent carries the detectable label, and the affinity reagent can specifically recognize and bind to an epitope of the compound or a salt thereof, or preferably, the compound or a salt thereof is linked to the additional detectable label optionally via a linker, or more preferably, the Base of the compound or a salt thereof is linked to the additional detectable label optionally via a linker;
(b)carrying out a reaction comprising the following steps (i), (ii) and (iii), optionally, repeating the steps for one or more times:

step (i): incorporating the compound or a salt thereof into the growing nucleic acid strand using a polymerase to form a nucleic acid intermediate containing the reversible blocking group and the detectable label;
step (ii): detecting the detectable label contained in the nucleic acid intermediate;
step (iii): removing the reversible blocking group and/or the detectable label contained in the nucleic acid intermediate by using the excision reagent;
preferably, the removal of the reversible blocking group and the removal of the detectable label are performed simultaneously, or, the removal of the reversible blocking group and the removal of the detectable label are performed sequentially (for example, the reversible blocking group is removed first, or the detectable label is removed first);
preferably, the excision reagent used for the removal of the reversible blocking group is the same as that used for the removal of the detectable label;
preferably, the excision reagent used for the removal of the reversible blocking group is different from that used for the removal of the detectable label.

**10.** The method according to claim 9, wherein the duplex is linked to a support;

preferably, the growing nucleic acid strand is a primer;
preferably, the primer is annealed to the nucleic acid strand to be sequenced to form the duplex;
preferably, the duplex, the compound or a salt thereof, and the polymerase together form a reaction system containing a solution phase and a solid phase;
preferably, the Bases contained in the compounds or salts thereof are different from each other;
preferably, the additional detectable label carried by the compound or a salt thereof are different from each other;
preferably, the compound or a salt thereof is incorporated into the growing nucleic acid strand using a polymerase under conditions where the polymerase is allowed to carry out a nucleotide polymerization reaction, thereby forming a nucleic acid intermediate containing reversible blocking group and the detectable label;
preferably, the polymerase is selected from KOD polymerase or its mutants thereof (e.g., KOD POL151, KOD POL157, KOD POL171, KOD POL174, KOD POL376, KOD POL391);
preferably, before any step of detecting the detectable label contained in the nucleic acid intermediate, the solution phase of the reaction system in the previous step is removed, and the duplex linked to the support is retained;
preferably, the excision reagent is in contact with the duplex or the growing nucleic acid strand in the reaction system containing a solution phase and a solid phase;
preferably, the excision reagent can remove the reversible blocking group and the additional detectable label carried by the compound that is incorporated into the growing nucleic acid strand, without affecting the phosphodiester bond on the backbone of the duplex;
preferably, after any step of removing the reversible blocking group and/or additional detectable label contained in the nucleic acid intermediate, the solution phase of the reaction system in this step is removed;
preferably, a washing operation is performed after any step comprising a removal operation;
preferably, after step (ii), the method further comprises: determining the type of compound incorporated into the growing nucleic acid strand in step (i) according to the signal detected in step (ii), and determining the type of nucleotide at a corresponding position in the nucleic acid strand to be sequenced based on the principle of base complementary pairing.

**11.** A kit comprising at least one compound or a salt thereof according to any one of claims 1-3;

preferably, the kit comprises a first compound, a second compound, a third compound and a fourth compound, the first, second, third and fourth compounds are each independently the compound or a salt thereof according to any one of claims 1-3;

preferably, in the first compound, the Base is selected from adenine, 7-deazaadenine or a tautomer thereof (e.g.,

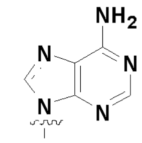

); in the second compound, the Base is selected from thymine, uracil or a tautomer thereof (e.g.,

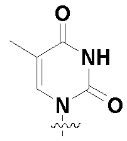

); in the third compound, the Base is selected from cytosine or a tautomer thereof (e.g.,

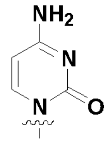

); in the fourth compound, the Base is selected from guanine, 7-deazaguanine or a tautomer thereof (e.g.,

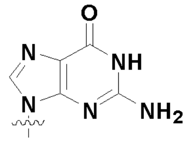

);
preferably, the first, second, third and fourth compounds carry additional detectable labels;
preferably, the additional detectable labels carried by the first, second, third and fourth compounds are introduced by an affinity reagent, which is selected from antibody, aptamer, Affimer, and Knottin, wherein the affinity reagent carries the detectable label, and the affinity reagent can specifically recognize and bind to the epitope of the first, second, third or fourth compound;
preferably, the first, second, third and fourth compounds are linked to the additional detectable label optionally via a linker;
preferably, the Base of the first, second, third or fourth compound is linked to the additional detectable label optionally via a linker;
preferably, the Bases contained in the first, second, third and fourth compounds are different from each other;
preferably, the additional detectable labels carried by the first, second, third and fourth compounds are different from each other;
preferably, the linker is as defined in claim 3;
preferably, the detectable label is as defined in claim 3.

**12.** The kit according to claim 11, wherein the kit further comprises an reagent for pretreating the nucleic acid molecule; a support for linking nucleic acid molecule to be sequenced; a reagent for linking the nucleic acid molecule to be sequenced to the support (for example, covalently or non-covalently linking); a primer for initiating a nucleotide polymerization reaction; a polymerase for carrying out the nucleotide polymerization reaction; one or more buffer solutions; one or more washing solutions; or any combination thereof.

**13.** Use of the compound or a salt thereof according to any one of claims 1-3 or the kit according to any one of claims 11-12 for determining the sequence of a target single-stranded polynucleotide.

**Patentansprüche**

**1.** Verbindung der Formel (A) oder ein Salz davon,

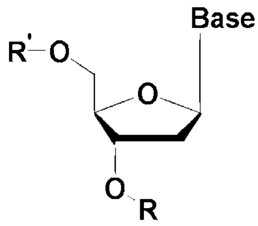

Formel (A)

wobei:

R eine reversible Blockierungsgruppe ist und R ausgewählt ist aus

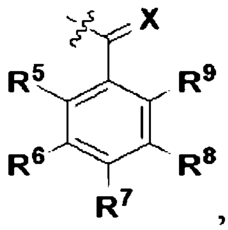

,

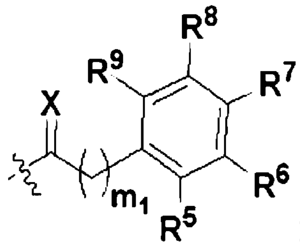

;

jedes X unabhängig ausgewählt ist aus O, NH, S;
X vorzugsweise O ist;
$R^5$

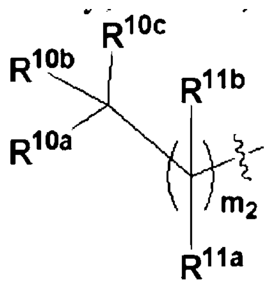

ist und $R^6$, $R^7$, $R^8$ und $R^9$ jeweils unabhängig ausgewählt sind aus H, Nitro, $C_1$-$C_6$-Alkyl, $C_1$-$C_6$-Alkoxy, $C_1$-$C_6$-Alkyl-C(=O)-$NH_2$-;
$R^5$ vorzugsweise

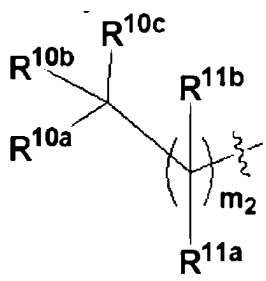

ist und $R^6$, $R^7$, $R^8$ und $R^9$ jeweils unabhängig ausgewählt sind aus H, Nitro, Methoxy, Acetamido;
$R^5$ am bevorzugtesten

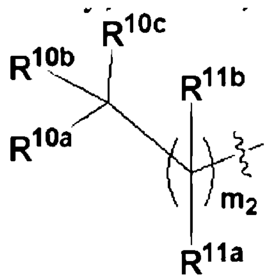

ist, $R^7$ ausgewählt ist aus H, Nitro, Methoxy, Acetamido und $R^6$, $R^8$ und $R^9$ H sind;
ein beliebiges von $R^{10a}$, $R^{10b}$ und $R^{10c}$ $-N_3$ oder -SS-$C_1$-$C_6$-Alkyl (z. B. -SS-Methyl, -SS-Ethyl, -SS-Isopropyl, -SS-Tertbutyl oder -SS-Isobutyl) ist und die anderen beiden jeweils unabhängig ausgewählt sind aus H, $C_1$-$C_6$-Alkyl, wie z. B. Methyl, Ethyl, Isopropyl, Tertbutyl;
ein beliebiges von $R^{10a}$, $R^{10b}$ und $R^{10c}$ vorzugsweise $-N_3$, -SS-Methyl, -SS-Ethyl, -SS-Isopropyl, -SS-Tertbutyl oder -SS-Isobutyl ist und die anderen beiden jeweils unabhängig ausgewählt sind aus H oder Methyl;
$R^{11a}$ und $R^{11b}$ jeweils unabhängig ausgewählt sind aus H oder $C_1$-$C_6$-Alkyl, wie z. B. Methyl, Ethyl, Isopropyl, Tertbutyl;
$R^{11a}$ und $R^{11b}$ vorzugsweise H sind;
jedes $m_1$ unabhängig ausgewählt ist aus 1, 2, 3, 4, 5 und 6;
$m_1$ vorzugsweise 1 ist;
jedes $m_2$ unabhängig ausgewählt ist aus 0, 1, 2, 3, 4, 5 und 6;
jedes $m_2$ vorzugsweise unabhängig ausgewählt ist aus 0 oder 1;

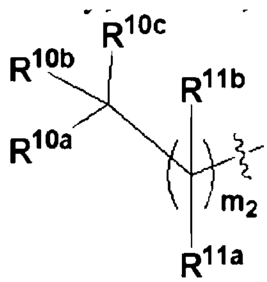

als Ganzes am bevorzugtesten ausgewählt ist aus

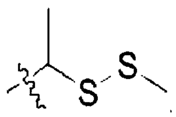,

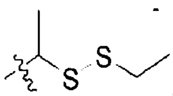, 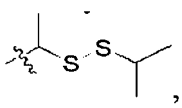, 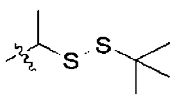, 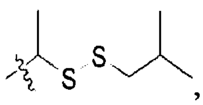, 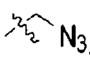, 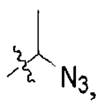, 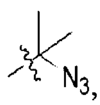,

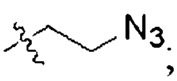;

R' ausgewählt ist aus H, einer Monophosphatgruppe

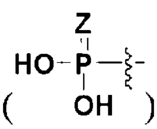,

einer Diphosphatgruppe

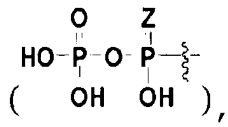,

einer Triphosphatgruppe

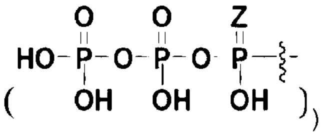

oder einer Tetraphosphatgruppe

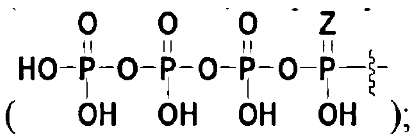;

R' vorzugsweise eine Triphosphatgruppe

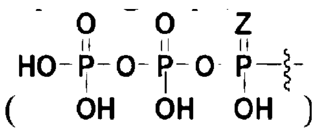

ist;
jedes Z unabhängig ausgewählt ist aus O, S, BH;
Z vorzugsweise O ist;
die Base ausgewählt ist aus einer Base, einer Deaza-Base oder einem Tautomer davon;
die Base zum Beispiel ausgewählt ist aus Adenin, 7-Deazaadenin, Thymin, Uracil, Cytosin, Guanin, 7-Deazaguanin oder einem Tautomer davon;
die Base vorzugsweise ausgewählt ist aus

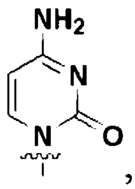, 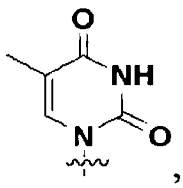, 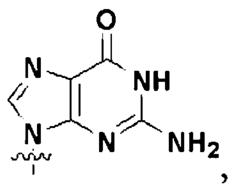, 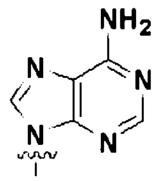.

**2.** Verbindung oder ein Salz davon nach Anspruch 1, wobei die Verbindung aus den Folgenden ausgewählt ist:

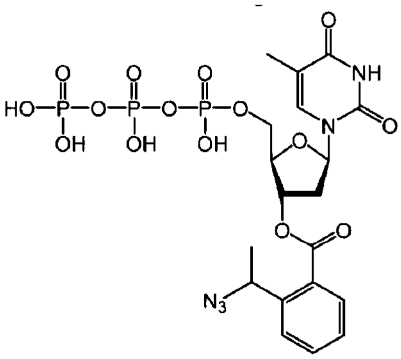 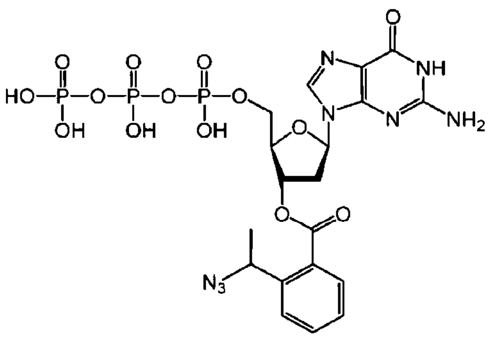

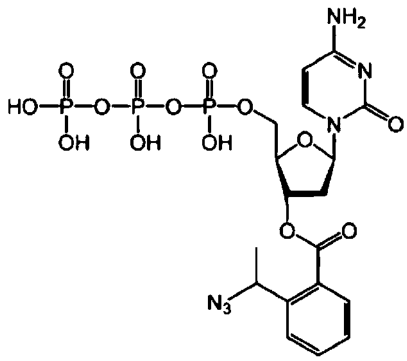

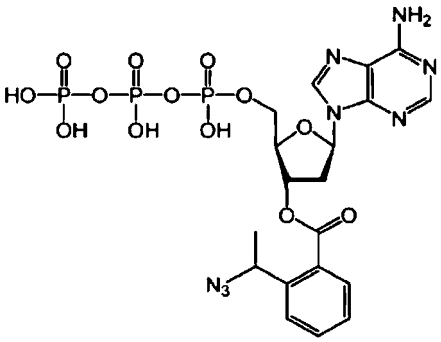

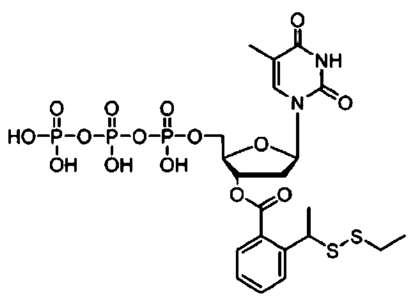

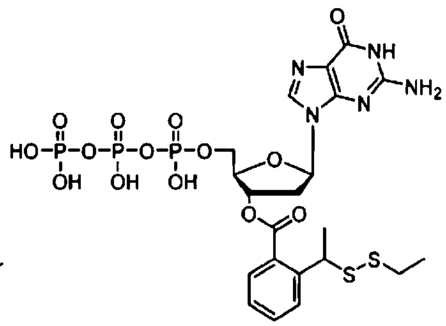

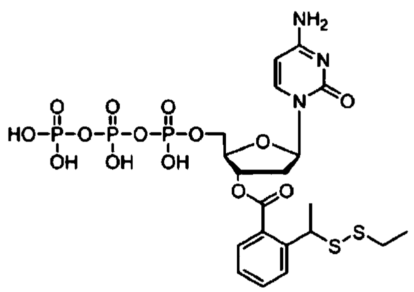

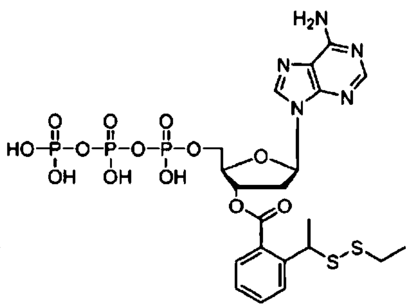

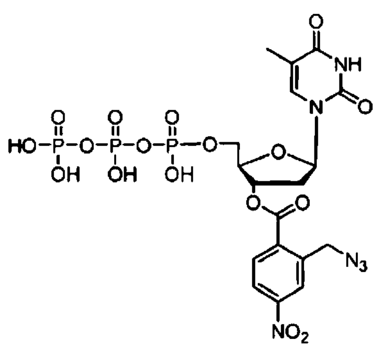

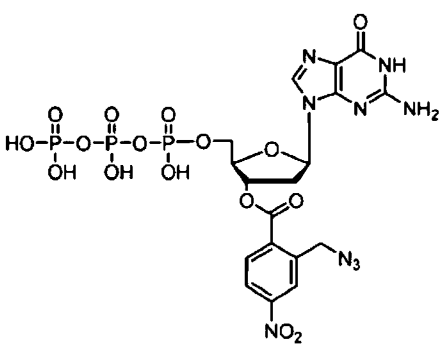

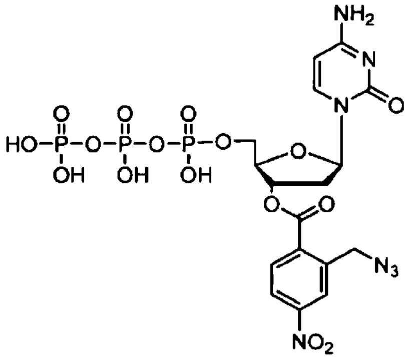
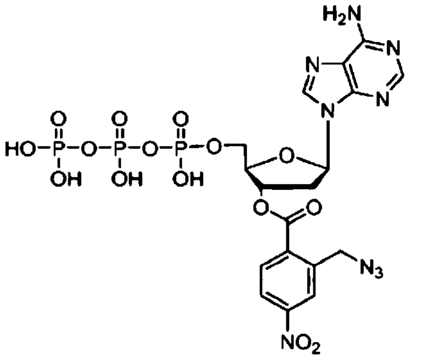

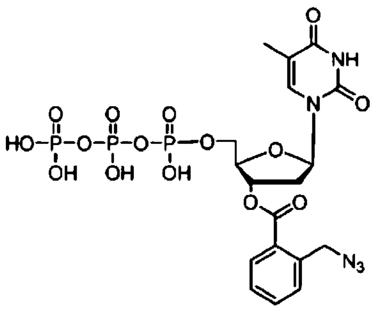
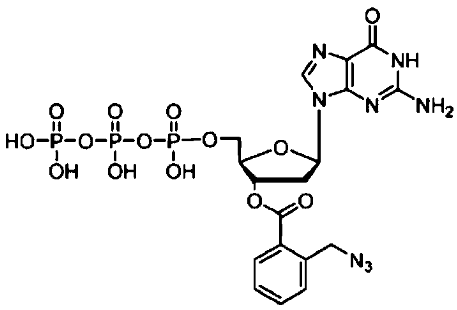

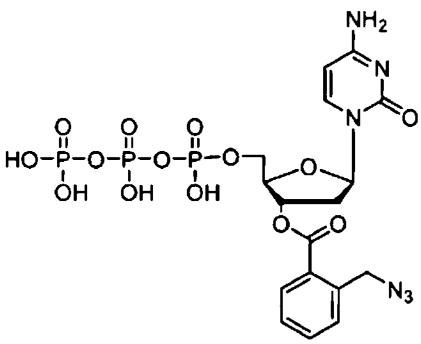
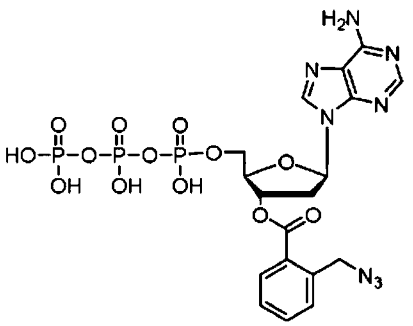

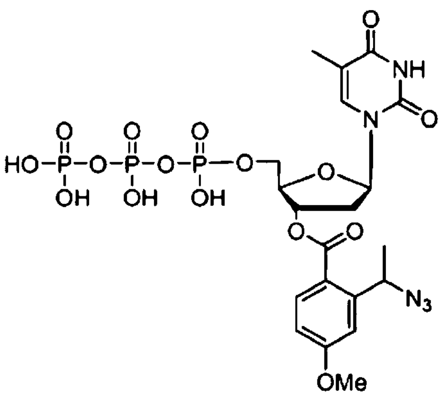
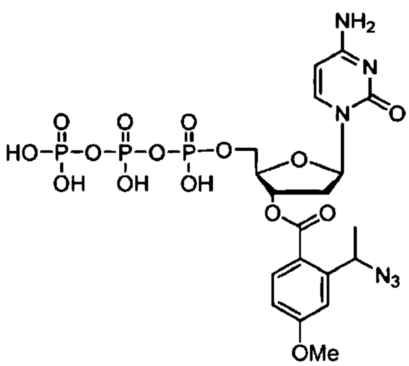

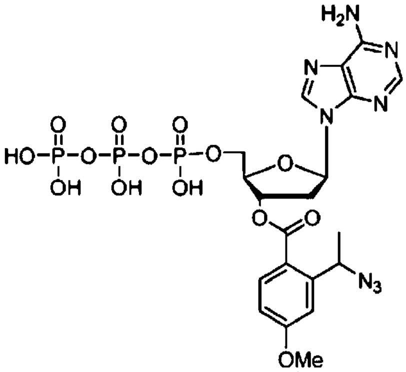

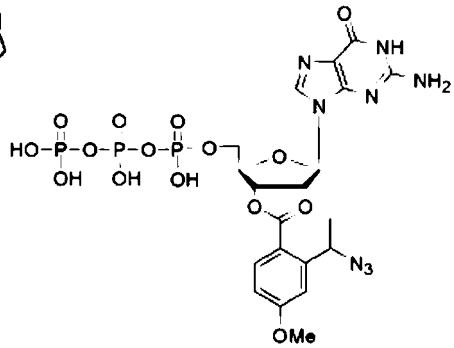

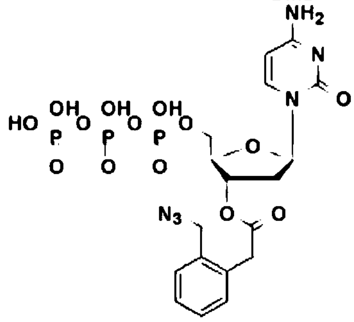

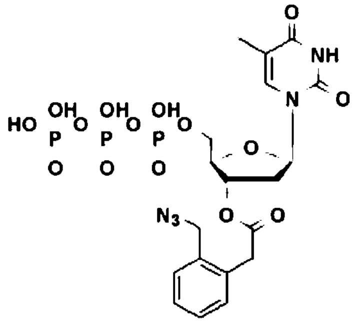

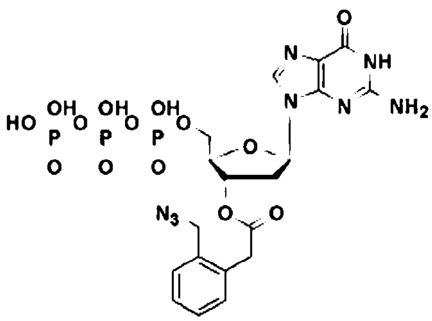

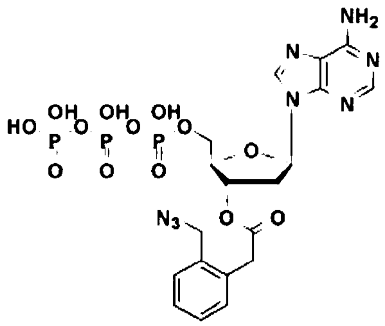

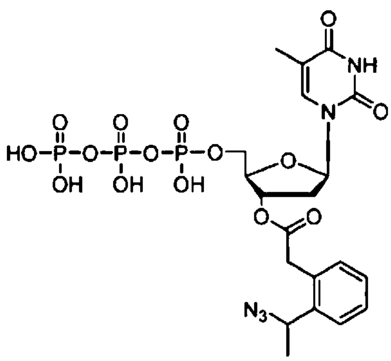

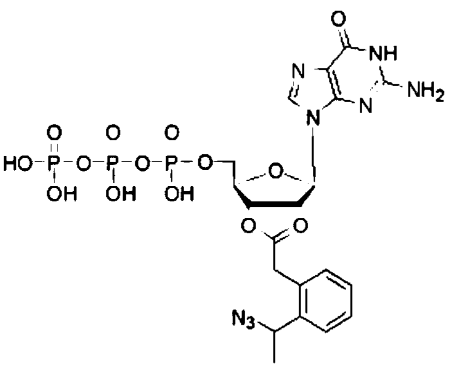

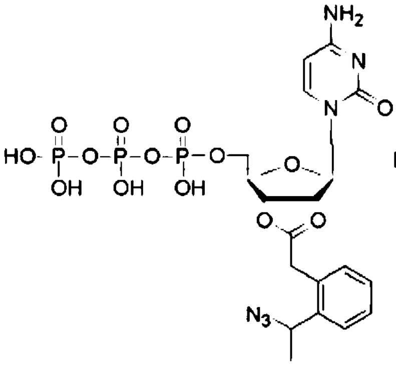

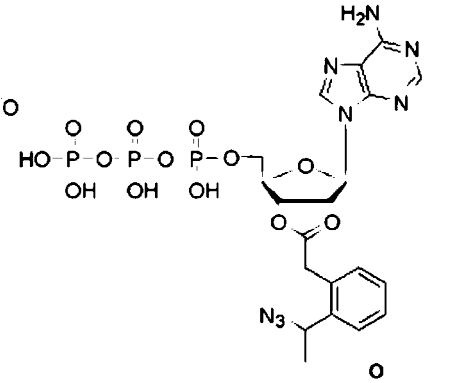

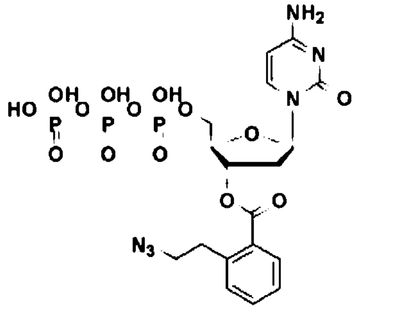

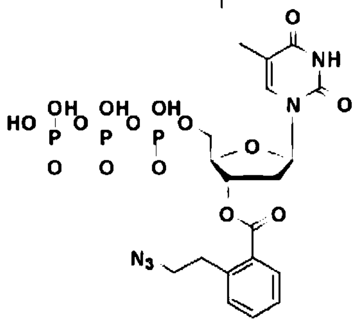

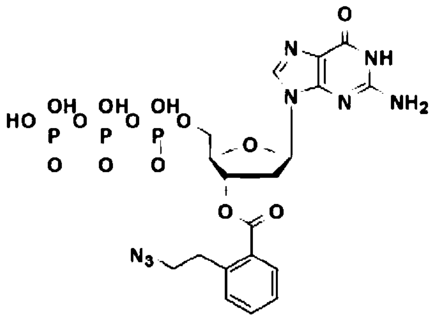

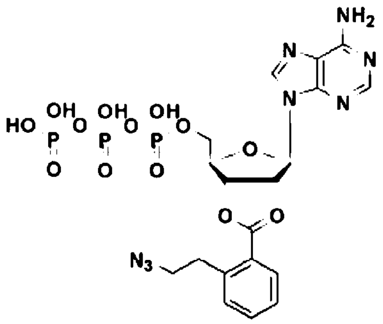

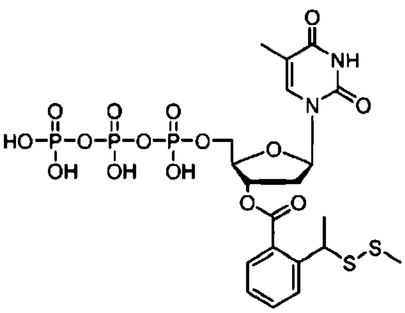

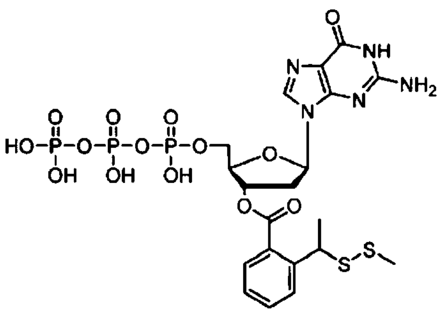

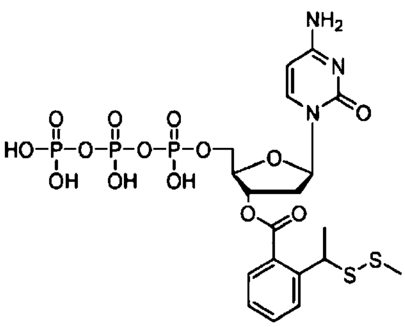

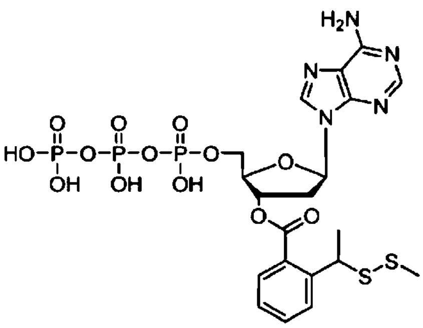

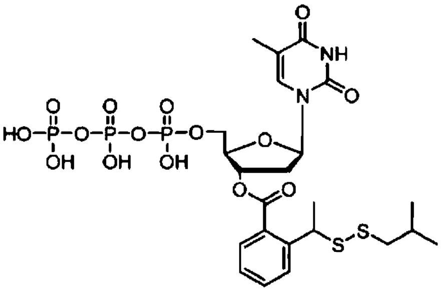

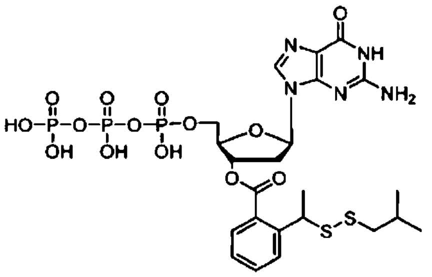

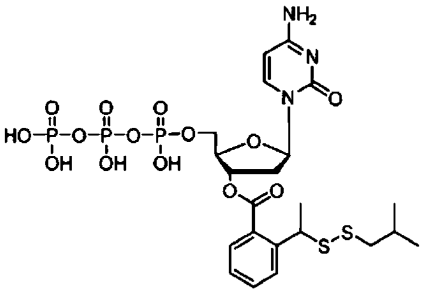

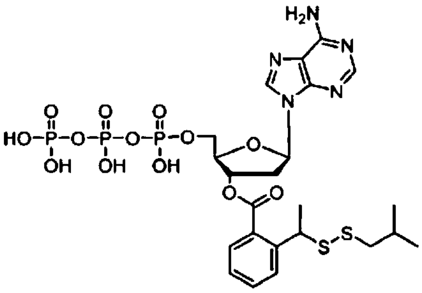

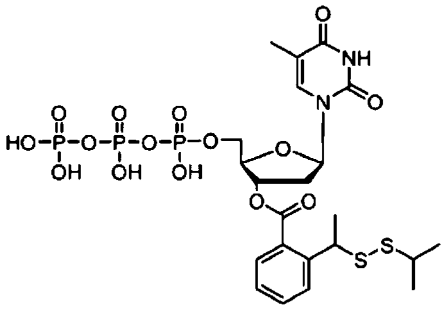

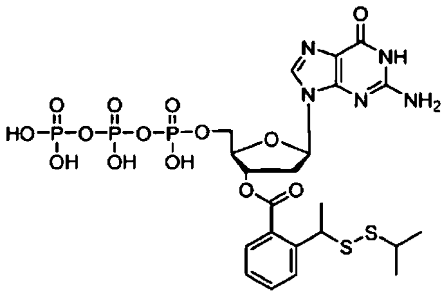

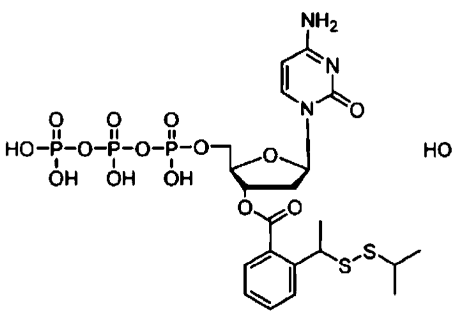

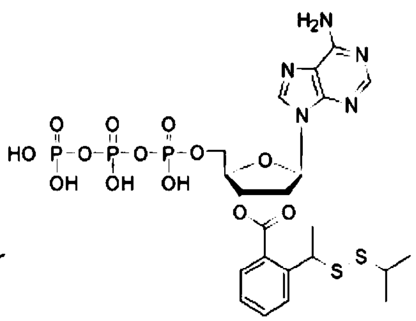

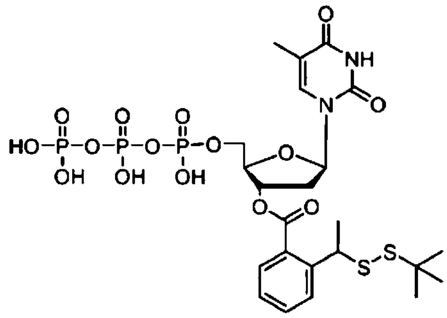

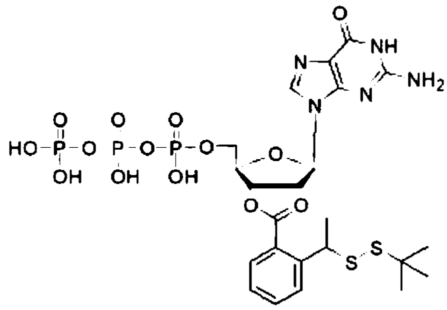

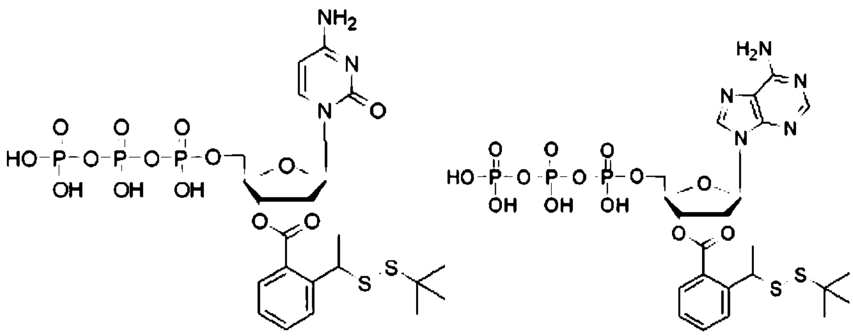

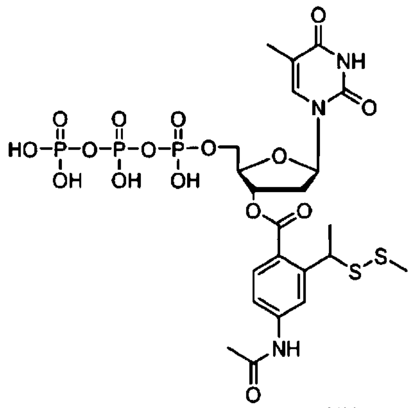
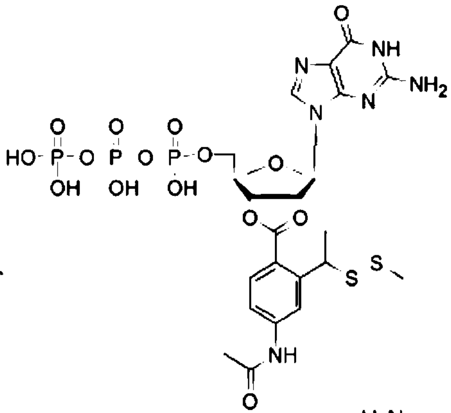

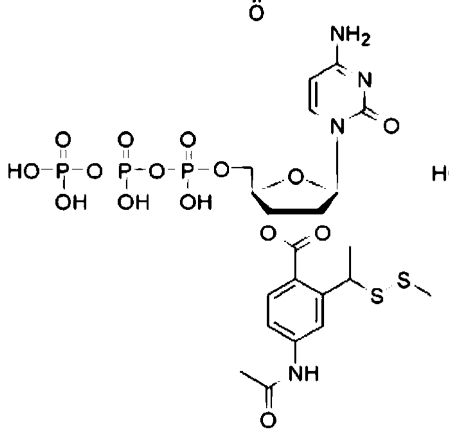
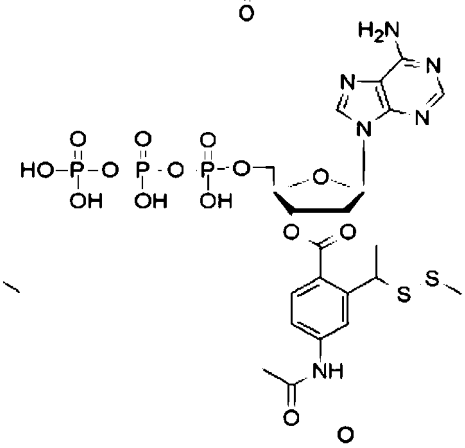

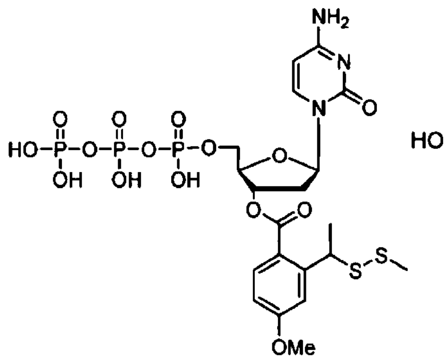
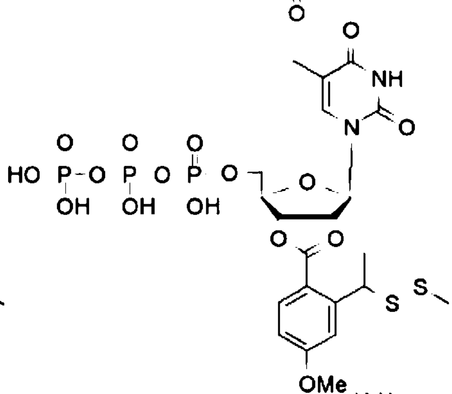

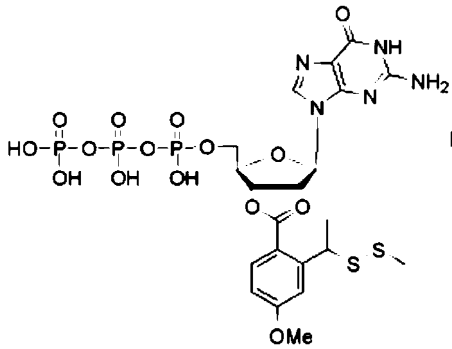
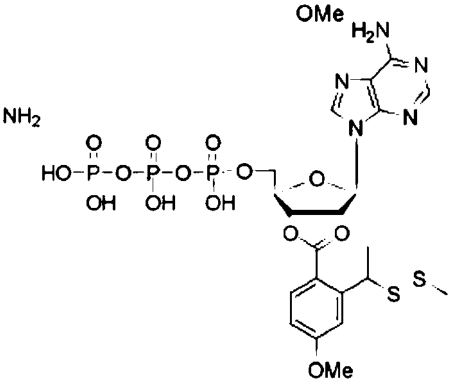

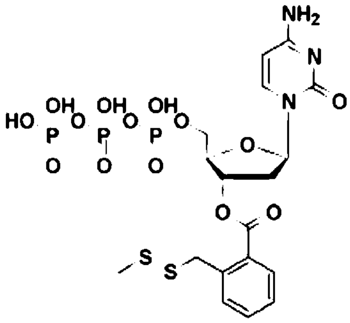

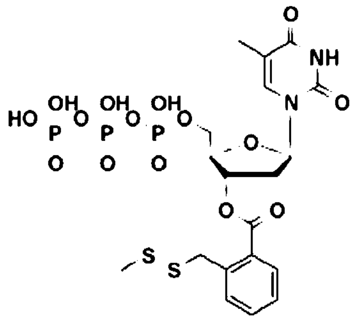

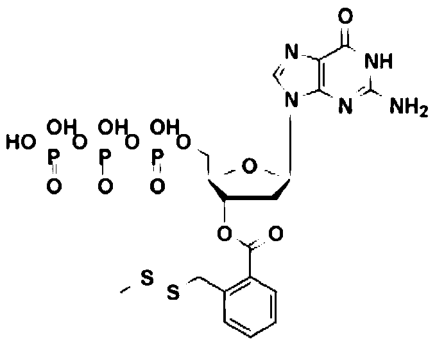

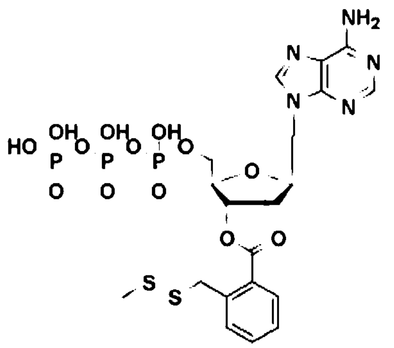

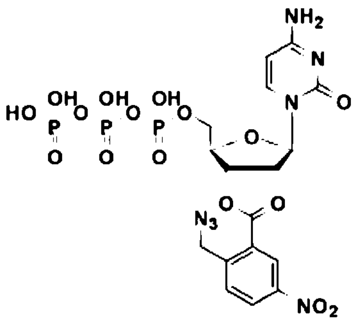

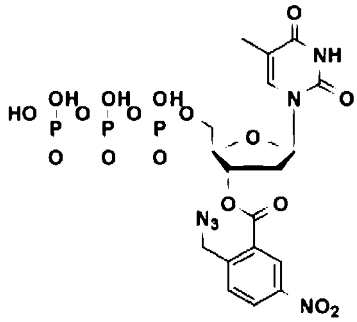

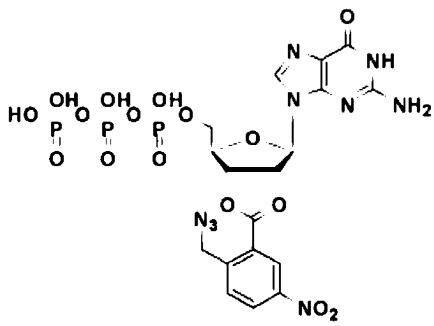

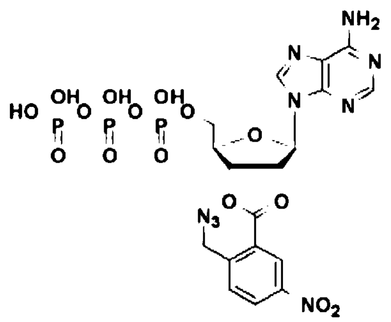

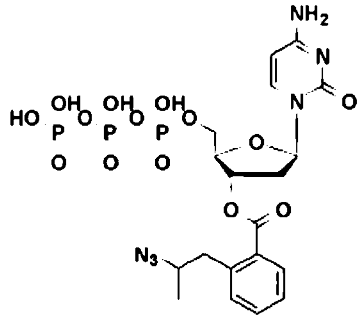

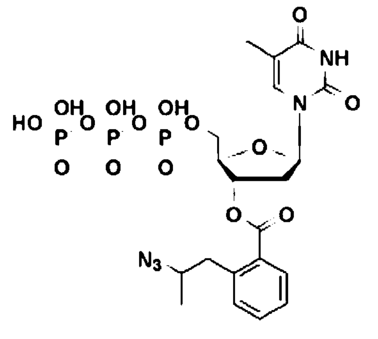

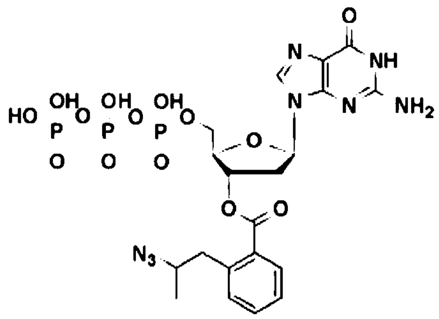

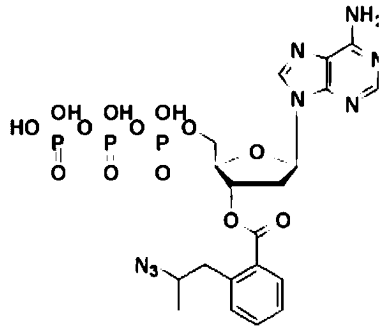

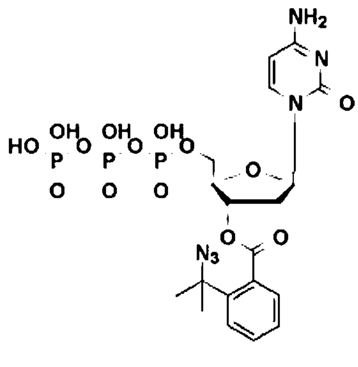

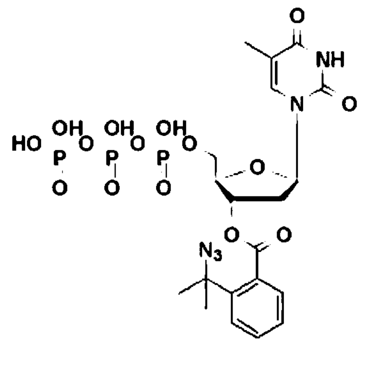

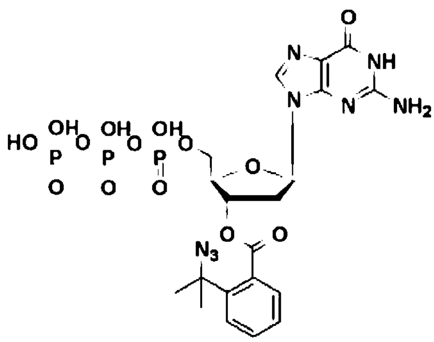

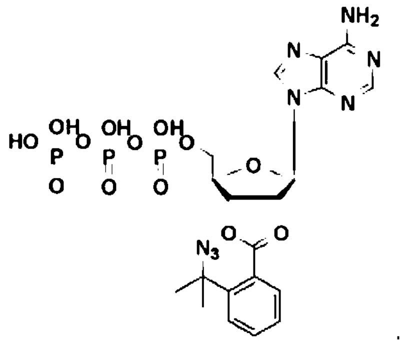

3. Verbindung oder ein Salz davon nach Anspruch 1 oder 2, welche/s eine zusätzliche nachweisbare Markierung trägt, wobei es sich bei der nachweisbaren Markierung um eine fluoreszierende Markierung handelt;

wobei die zusätzliche nachweisbare Markierung, die von der Verbindung oder einem Salz davon getragen wird, durch ein Affinitätsreagens eingeführt wird, welches aus einem Antikörper, einem Aptamer, einem Affimer und einem Knottin ausgewählt ist, wobei das Affinitätsreagens die nachweisbare Markierung trägt und das Affinitätsreagens spezifisch ein Epitop der Verbindung oder eines Salzes davon erkennen und daran binden kann; wobei die zusätzliche nachweisbare Markierung optional über einen Linker mit der Base der Verbindung oder

eines Salzes davon verknüpft ist;
wobei der Linker vorzugsweise ein spaltbarer Linker oder ein nicht spaltbarer Linker ist;
wobei der spaltbare Linker vorzugsweise ausgewählt ist aus der Gruppe bestehend aus einem Linker, der durch eine elektrophile Reaktion gespalten werden kann, einem Linker, der durch eine nukleophile Reaktion gespalten werden kann, einem Linker, der durch Photolyse gespalten werden kann, einem Linker, der unter reduktiven Bedingungen gespalten werden kann, einem Linker, der unter oxidativen Bedingungen gespalten werden kann, einem Safety-Catch-Linker, einem Linker, der durch Eliminationsmechanismen gespalten werden kann oder einer beliebigen Kombination daraus;
wobei die nachweisbare Markierung vorzugsweise ausgewählt ist aus den Folgenden:

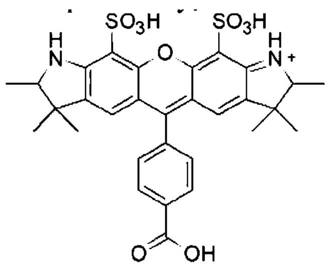

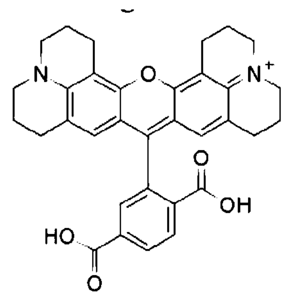

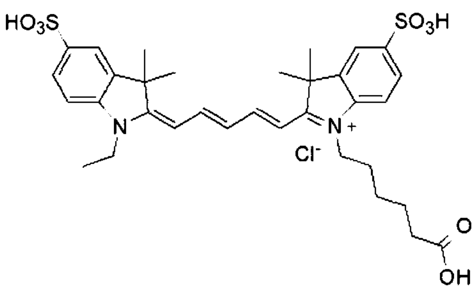

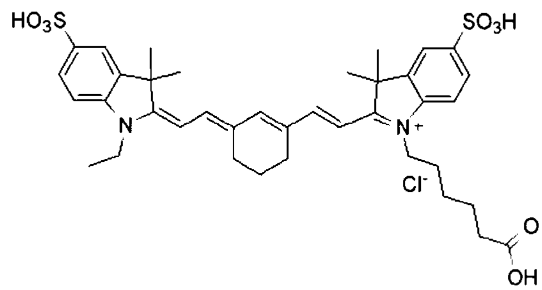

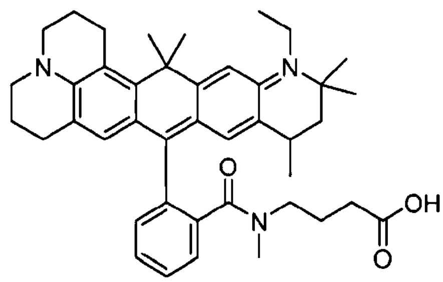

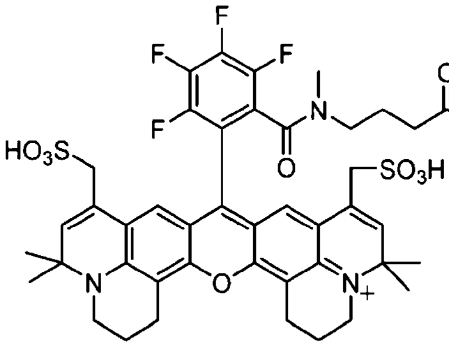

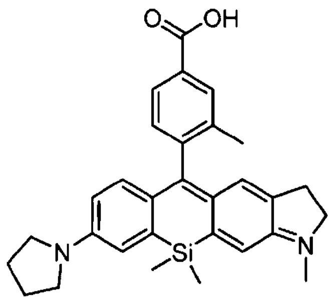

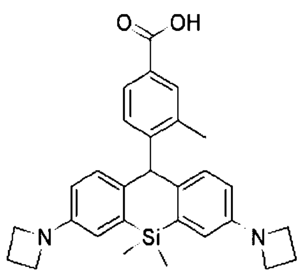

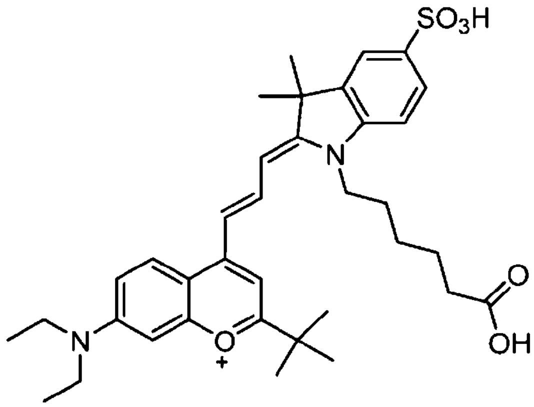

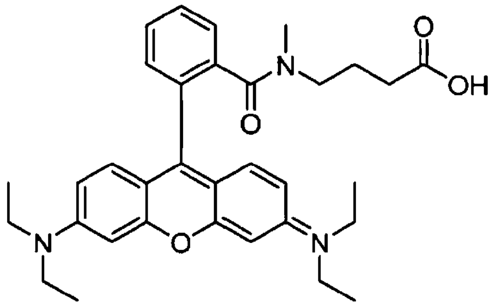

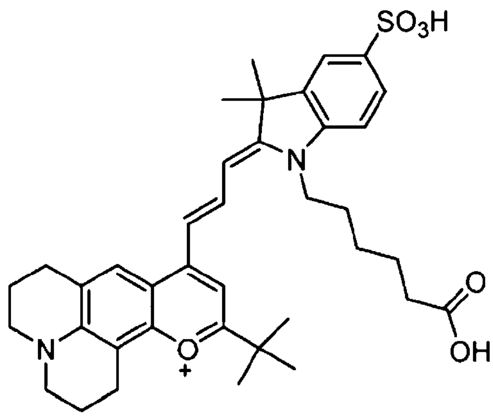

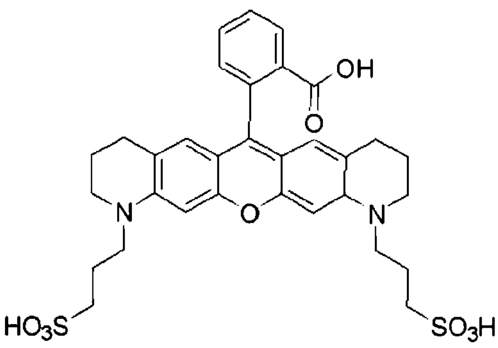

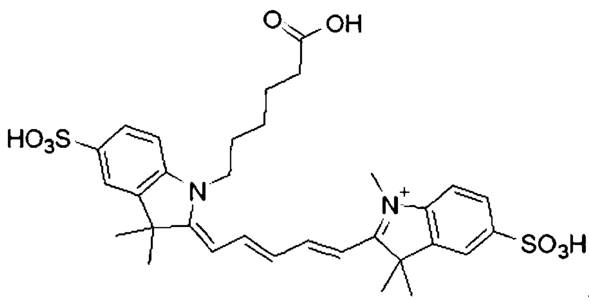

;

wobei vorzugsweise, wenn die Base unterschiedlich ist, die nachweisbare Markierung variiert.

4. Verfahren zum Beenden einer Nukleinsäuresynthese, welches das Einbinden der Verbindung oder eines Salzes davon nach einem der Ansprüche 1-3 in ein zu terminierendes Nukleinsäuremolekül umfasst,

wobei die Einbindung der Verbindung oder eines Salzes davon vorzugsweise durch eine terminale Transferase, eine terminale Polymerase oder eine reverse Transkriptase erreicht wird;
wobei das Verfahren vorzugsweise das Einbinden der Verbindung oder eines Salzes davon in das zu terminierende Nukleinsäuremolekül durch Verwendung einer Polymerase umfasst;
wobei das Verfahren vorzugsweise das Durchführen einer Nukleotidpolymerisationsreaktion unter Verwendung einer Polymerase unter Bedingungen umfasst, unter welchen der Polymerase gestattet wird, die Nukleotidpolymerisationsreaktion durchzuführen, wodurch die Verbindung oder ein Salz davon in das 3'-Ende des zu terminierenden Nukleinsäuremoleküls eingebunden wird.

5. Verfahren zur Herstellung eines wachsenden Polynukleotids, das komplementär zu einem Ziel-Einzelstrang-Polynukleotid in einer Sequenzierungsreaktion ist, welches das Einbinden der Verbindung oder eines Salzes davon nach einem der Ansprüche 1-3 in das wachsende komplementäre Polynukleotid umfasst, wobei die Einbindung der Verbindung oder eines Salzes davon verhindert, dass jegliche nachfolgenden Nukleotide in das wachsende komplementäre Polynukleotid eingebunden werden;

wobei die Einbindung der Verbindung oder eines Salzes davon vorzugsweise durch eine terminale Transferase, eine terminale Polymerase oder eine reverse Transkriptase erreicht wird;
wobei das Verfahren vorzugsweise das Einbinden der Verbindung oder eines Salzes davon in das wachsende komplementäre Polynukleotid durch Verwendung einer Polymerase umfasst;
wobei das Verfahren vorzugsweise das Durchführen einer Nukleotidpolymerisationsreaktion unter Verwendung einer Polymerase unter Bedingungen umfasst, unter welchen der Polymerase gestattet wird, die Nukleotidpolymerisationsreaktion durchzuführen, wodurch die Verbindung oder ein Salz davon in das 3'-Ende des wachsenden komplementären Polynukleotids eingebunden wird.

**6.** Verfahren zur Bestimmung der Sequenz eines Ziel-Einzelstrang-Polynukleotids, welches Folgendes umfasst:

1) Überwachen der Einbindung von Nukleotiden, die komplementär zu dem Ziel-Einzelstrang-Polynukleotid sind, in einen wachsenden Nukleinsäurestrang, wobei mindestens ein eingebundenes komplementäres Nukleotid die Verbindung oder ein Salz davon nach einem der Ansprüche 1 bis 3 ist und die Verbindung oder ein Salz davon eine zusätzliche nachweisbare Markierung trägt, wobei es sich bei der nachweisbaren Markierung um eine fluoreszierende Markierung handelt, und
2) Nachweisen der nachweisbaren Markierung zum Bestimmen des eingebundenen Nukleotids;
wobei die zusätzliche nachweisbare Markierung vorzugsweise optional über einen Linker mit der Verbindung oder einem Salz davon verknüpft ist;
wobei der Linker vorzugsweise wie in Anspruch 3 definiert ist;
wobei die zusätzliche nachweisbare Markierung vorzugsweise wie in Anspruch 3 definiert ist;
wobei, wenn die Base unterschiedlich ist, vorzugsweise die nachweisbare Markierung, die durch die Verbindung oder ein Salz davon getragen wird, variiert;
wobei die reversible Blockierungsgruppe (R) in der Verbindung oder einem Salz davon und die nachweisbare Markierung vorzugsweise entfernt werden, bevor ein nächstes komplementäres Nukleotid eingebunden wird;
wobei die reversible Blockierungsgruppe und die nachweisbare Markierung vorzugsweise gleichzeitig entfernt werden; oder
wobei die reversible Blockierungsgruppe und die nachweisbare Markierung vorzugsweise nacheinander entfernt werden, zum Beispiel wird die reversible Blockierungsgruppe entfernt, nachdem die nachweisbare Markierung entfernt wurde oder die nachweisbare Markierung wird entfernt, nachdem die reversible Blockierungsgruppe entfernt wurde.

**7.** Verfahren nach Anspruch 6, welches die folgenden Schritte umfasst:

(a) Bereitstellen mehrerer unterschiedlicher Nukleotide, wobei es sich bei den mehreren unterschiedlichen Nukleotiden um die Verbindung oder ein Salz davon nach einem der Ansprüche 1-3 handelt, wobei jedes Nukleotid eine zusätzliche nachweisbare Markierung trägt, die während des Nachweises von einer zusätzlichen nachweisbaren Markierung, die von einem anderen Nukleotid getragen wird, unterschieden werden kann;
(b) Einbinden der mehreren unterschiedlichen Nukleotide in eine Sequenz, die komplementär zu einem Ziel-Einzelstrang-Polynukleotid ist;
(c) Nachweisen der zusätzlichen nachweisbaren Markierungen, die durch die Nukleotide in Schritt (b) getragen werden, zum Bestimmen der Arten der eingebundenen Nukleotide;
(d) Entfernen der reversiblen Blockierungsgruppen und der nachweisbaren Markierungen, durch die Nukleotide in Schritt (b) getragen werden; und
(e) wahlweise ein- oder mehrmaliges Wiederholen der Schritte (b)-(d);

wodurch die Sequenz des Ziel-Einzelstrang-Polynukleotids bestimmt wird.

**8.** Verfahren nach Anspruch 6, welches die folgenden Schritte umfasst:

(1) Bereitstellen eines ersten Nukleotids, eines zweiten Nukleotids, eines dritten Nukleotids und eines vierten Nukleotids, wobei es sich bei mindestens einem der vier Nukleotide um die Verbindung oder ein Salz davon nach einem der Ansprüche 1-3 handelt, wobei sich die in den vier Nukleotiden enthaltenen Basen voneinander unterscheiden und die vier Nukleotide eine zusätzliche nachweisbare Markierung tragen, wobei die zusätzliche nachweisbare Markierung, die durch die vier Nukleotide getragen wird, vorzugsweise durch ein Affinitätsreagens eingeführt wird, welches aus einem Antikörper, einem Aptamer, einem Affimer und einem Knottin ausgewählt ist, wobei das Affinitätsreagens die nachweisbare Markierung trägt und das Affinitätsreagens spezifisch ein Epitop jedes Nukleotids erkennen und daran binden kann oder die vier Nukleotide vorzugsweise optional über einen

Linker mit der zusätzlichen nachweisbaren Markierung verknüpft sind oder bevorzugter die Base der vier Nukleotide optional über einen Linker mit der zusätzlichen nachweisbaren Markierung verknüpft ist und sich am bevorzugtesten die zusätzlichen nachweisbaren Markierungen, die durch die vier Nukleotide getragen werden, voneinander unterscheiden;
(2) in Kontakt bringen der vier Nukleotide mit einem Ziel-Einzelstrang-Polynukleotid; Entfernen der Nukleotide, die nicht in den wachsenden Nukleinsäurestrang eingebunden wurden; Nachweisen der nachweisbaren Markierungen, die durch die Nukleotide getragen werden, die in den wachsenden Nukleinsäurestrang eingebunden wurden; Entfernen der reversiblen Blockierungsgruppen und der nachweisbaren Markierungen, die durch die Nukleotide getragen werden, welche in den wachsenden Nukleinsäurestrang eingebunden wurden; und

wahlweise auch (3): das ein- oder mehrmalige Wiederholen von Schritt (2) umfasst.

**9.** Verfahren nach Anspruch 6, welches die folgenden Schritte umfasst:

(a) Bereitstellen einer Mischung, die einen Doppelstrang, mindestens eine Verbindung oder ein Salz davon nach einem der Ansprüche 1-3, eine Polymerase und ein Exzisionsreagens umfasst; wobei der Doppelstrang einen wachsenden Nukleinsäurestrang und einen zu sequenzierenden Nukleinsäurestrang umfasst, wobei die Verbindung oder ein Salz davon eine zusätzliche nachweisbare Markierung trägt, wobei die zusätzliche nachweisbare Markierung, die durch die Verbindung oder ein Salz davon getragen wird, vorzugsweise durch ein Affinitätsreagens eingebunden wird, wobei das Affinitätsreagens die nachweisbare Markierung trägt und das Affinitätsreagens spezifisch ein Epitop der Verbindung oder eines Salzes davon erkennen und daran binden kann oder die Verbindung oder ein Salz davon vorzugsweise optional über einen Linker mit der zusätzlichen nachweisbaren Markierung verknüpft ist oder bevorzugter die Base der Verbindung oder eines Salzes davon optional über einen Linker mit der zusätzlichen nachweisbaren Markierung verknüpft ist;
(b) Ausführen einer Reaktion, welche die folgenden Schritte (i), (ii) und (iii) umfasst, optional ein- oder mehrmaliger Wiederholen der Schritte:

Schritt (i): Einbinden der Verbindung oder eines Salzes davon in den wachsenden Nukleinsäurestrang unter Verwendung einer Polymerase zum Bilden eines Nukleinsäurezwischenprodukts, das die reversible Blockierungsgruppe und die nachweisbare Markierung enthält;
Schritt (ii): Nachweisen der nachweisbaren Markierung, die in dem Nukleinsäurezwischenprodukt enthalten ist;
Schritt (iii): Entfernen der reversiblen Blockierungsgruppe und/oder der nachweisbaren Markierung, die in dem Nukleinsäurezwischenprodukt enthalten ist/sind, durch Verwendung des Exzisionsreagens;
wobei die Entfernung der reversiblen Blockierungsgruppe und die Entfernung der nachweisbaren Markierung vorzugsweise gleichzeitig durchgeführt werden oder die Entfernung der reversiblen Blockierungsgruppe und die Entfernung der nachweisbaren Markierung nacheinander durchgeführt werden (zum Beispiel wird die reversible Blockierungsgruppe als erstes entfernt oder die nachweisbare Markierung wird als erstes entfernt);
wobei das Exzisionsreagens, das für die Entfernung der reversiblen Blockierungsgruppe verwendet wird, vorzugsweise das gleiche wie das ist, das für die Entfernung der nachweisbaren Markierung verwendet wird;
wobei sich das Exzisionsreagens, das für die Entfernung der reversiblen Blockierungsgruppe verwendet wird, vorzugsweise von dem unterscheidet, das für die Entfernung der nachweisbaren Markierung verwendet wird.

**10.** Verfahren nach Anspruch 9, wobei der Doppelstrang mit einem Träger verknüpft ist;

wobei der wachsende Nukleinsäurestrang vorzugsweise ein Primer ist;
wobei der Primer vorzugsweise mit dem zu sequenzierenden Nukleinsäurestrang verschmolzen ist, um den Doppelstrang zu bilden;
wobei der Doppelstrang, die Verbindung oder ein Salz davon und die Polymerase zusammen vorzugsweise ein Reaktionssystem bilden, das eine Lösungsphase und eine feste Phase enthält;
wobei sich die Basen, die in den Verbindungen oder Salzen davon enthalten sind, vorzugsweise voneinander unterscheiden;
wobei sich die zusätzlichen nachweisbaren Markierungen, die durch die Verbindung oder ein Salz davon getragen werden, vorzugsweise voneinander unterscheiden;
wobei die Verbindung oder ein Salz davon vorzugsweise unter Verwendung einer Polymerase in den wachsenden Nukleinsäurestrang eingebunden wird, und zwar unter Bedingungen, unter welchen es der Polymerase

gestattet wird, eine Nukleotidpolymerisationsreaktion auszuführen, wodurch ein Nukleinsäurezwischenprodukt gebildet wird, das eine reversible Blockierungsgruppe und die nachweisbare Markierung enthält;
wobei die Polymerase vorzugsweise ausgewählt ist aus KOD-Polymerase oder ihren Mutanten davon (z. B. KOD POL151, KOD POL157, KOD POL171, KOD POL174, KOD POL376, KOD POL391);
wobei vor jeglichem Schritt des Nachweisens der nachweisbaren Markierung, die in dem Nukleinsäurezwischenprodukt enthalten ist, vorzugsweise die Lösungsphase des Reaktionssystems im vorhergehenden Schritt entfernt wird und der mit dem Träger verknüpfte Doppelstrang beibehalten wird;
wobei das Exzisionsreagens vorzugsweise mit dem Doppelstrang oder dem wachsenden Nukleinsäurestrang in dem Reaktionssystem, das eine Lösungsphase und eine feste Phase enthält, in Kontakt steht;
wobei das Exzisionsreagens vorzugsweise die reversible Blockierungsgruppe und die zusätzliche nachweisbare Markierung, die durch die Verbindung getragen wird, die in den wachsenden Nukleinsäurestrang eingebunden wurde, entfernen kann, ohne die Phosphodiesterbindung an der Hauptkette des Doppelstrangs zu beeinträchtigen;
wobei nach jeglichem Schritt des Entfernens der reversiblen Blockierungsgruppe und/oder der zusätzlichen nachweisbaren Markierung, die in dem Nukleinsäurezwischenprodukt enthalten ist/sind, in diesem Schritt vorzugsweise die Lösungsphase des Reaktionssystems entfernt wird;
wobei nach jeglichem Schritt, der einen Entfernungsvorgang umfasst, vorzugsweise ein Waschvorgang durchgeführt wird;
wobei das Verfahren nach Schritt (ii) vorzugsweise ferner Folgendes umfasst: Bestimmen der Art der Verbindung, die in Schritt (i) in den wachsenden Nukleinsäurestrang eingebunden wurde, gemäß dem Signal, das in Schritt (ii) nachgewiesen wird, und Bestimmen der Art des Nukleotids an einer entsprechenden Position in dem zu sequenzierenden Nukleinsäurestrang basierend auf dem Prinzip der komplementären Basenpaarung.

**11.** Kit, welches mindestens eine Verbindung oder ein Salz davon nach einem der Ansprüche 1-3 umfasst;

wobei das Kit vorzugsweise eine erste Verbindung, eine zweite Verbindung, eine dritte Verbindung und eine vierte Verbindung umfasst, wobei die erste, die zweite, die dritte und die vierte Verbindung jeweils unabhängig die Verbindung oder ein Salz davon nach einem der Ansprüche 1-3 sind;
wobei in der ersten Verbindung die Base vorzugsweise ausgewählt ist aus Adenin, 7-Deazaadenin oder einem Tautomer davon (z. B.

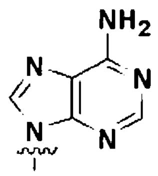

); wobei in der zweiten Verbindung die Base vorzugsweise ausgewählt ist aus Thymin, Uracil oder einem Tautomer davon (z. B.

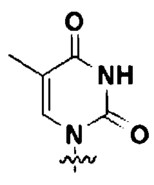

); wobei in der dritten Verbindung die Base vorzugsweise ausgewählt ist aus Cytosin oder einem Tautomer davon (z. B.

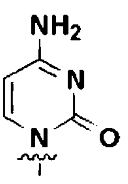

); wobei in der vierten Verbindung die Base vorzugsweise ausgewählt ist aus Guanin, 7-Deazaguanin oder einem

Tautomer davon (z. B.

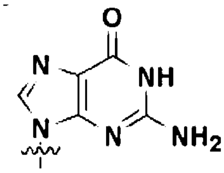

);

wobei die erste, die zweite, die dritte und die vierte Verbindung vorzugsweise zusätzliche nachweisbare Markierungen tragen;

wobei die zusätzlichen nachweisbaren Markierungen, die durch die erste, die zweite, die dritte und die vierte Verbindung getragen werden, vorzugsweise durch ein Affinitätsreagens eingeführt werden, welches aus einem Antikörper, einem Aptamer, einem Affimer und einem Knottin ausgewählt ist, wobei das Affinitätsreagens die nachweisbare Markierung trägt und das Affinitätsreagens spezifisch das Epitop der ersten, der zweiten, der dritten oder der vierten Verbindung erkennen und daran binden kann;

wobei die erste, die zweite, die dritte und die vierte Verbindung vorzugsweise optional über einen Linker mit der zusätzlichen nachweisbaren Markierung verknüpft sind;

wobei die Base der ersten, der zweiten, der dritten oder der vierten Verbindung vorzugsweise optional über einen Linker mit der zusätzlichen nachweisbaren Markierung verknüpft ist;

wobei sich die Basen, die in der ersten, der zweiten, der dritten und der vierten Verbindung enthalten sind, vorzugsweise voneinander unterscheiden;

wobei sich die zusätzlichen nachweisbaren Markierungen, die durch die erste, die zweite, die dritte und die vierte Verbindung getragen werden, voneinander unterscheiden;

wobei der Linker vorzugsweise wie in Anspruch 3 definiert ist;

wobei die nachweisbare Markierung vorzugsweise wie in Anspruch 3 definiert ist.

**12.** Kit nach Anspruch 11, wobei das Kit ferner ein Reagens zum Vorbehandeln des Nukleinsäuremoleküls; einen Träger zum Verknüpfen des zu sequenzierenden Nukleinsäuremoleküls; ein Reagens zum Verknüpfen des zu sequenzierenden Nukleinsäuremoleküls mit dem Träger (zum Beispiel kovalentes oder nicht-kovalentes Verknüpfen); einen Primer zum Initiieren einer Nukleotidpolymerisationsreaktion; eine Polymerase zum Ausführen der Nukleotidpolymerisationsreaktion; eine oder mehrere Pufferlösungen; eine oder mehrere Waschlösungen oder jegliche Kombination daraus umfasst.

**13.** Verwendung der Verbindung oder eines Salzes davon nach einem der Ansprüche 1-3 oder des Kits nach einem der Ansprüche 11-12 zum Bestimmen der Sequenz eines Ziel-Einzelstrang-Polynukleotids.

## Revendications

**1.** Composé de formule (A) ou un sel de celui-ci,

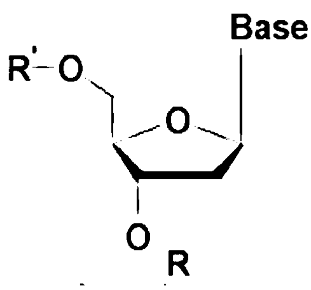

formule (A)

dans lequel :

R est un groupe bloquant réversible, et R est choisi parmi

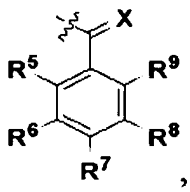 , 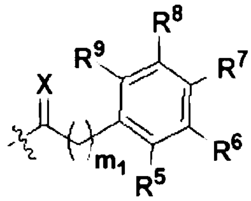 ;

chaque X est choisi indépendamment parmi O, NH, S ;
de préférence, X est O ;
$R^5$ est

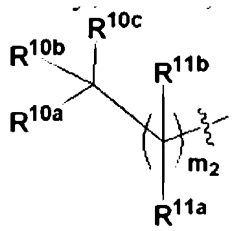 ,

et $R^6$, $R^7$, $R^8$ et $R^9$ sont choisis chacun indépendamment parmi H, un groupe nitro, un groupe alkyle en $C_1$ à $C_6$, un groupe alcoxy en $C_1$ à $C_6$, un groupe alkyle en $C_1$ à $C_6$-C(=O)-$NH_2$- ;
de préférence, $R^5$ est

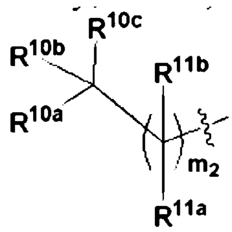 ,

et $R^6$, $R^7$, $R^8$ et $R^9$ sont choisis chacun indépendamment parmi H, un groupe nitro, un groupe méthoxy, un groupe acétamido ;
de manière préférée entre toutes, $R^5$ est

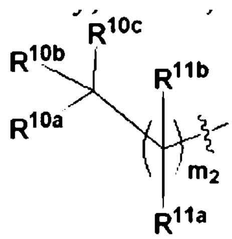 ,

$R^7$ est choisi parmi H, un groupe nitro, un groupe méthoxy, un groupe acétamido, et $R^6$, $R^8$ et $R^9$ sont H ;
l'un quelconque parmi $R^{10a}$, $R^{10b}$ et $R^{10c}$ est -$N_3$ ou -SS-alkyle en $C_1$ à $C_6$ (par ex. -SS-méthyle, -SS-éthyle, - SS-isopropyle, -SS-tertbutyle ou -SS-isobutyle) et les deux autres sont choisis chacun indépendamment parmi H, un groupe alkyle en $C_1$ à $C_6$, tel qu'un groupe méthyle, un groupe éthyle, un groupe isopropyle, un groupe tertbutyle ;
de préférence, l'un quelconque parmi $R^{10a}$, $R^{10b}$ et $R^{10c}$ est -$N_3$, -SS-méthyle, -SS-éthyle, -SS-isopropyle, -SS-tertbutyle ou -SS-isobutyle, et les deux autres sont choisis chacun indépendamment parmi H ou un groupe méthyle ;
$R^{11a}$ et $R^{11b}$ sont choisis chacun indépendamment parmi H ou un groupe alkyle en $C_1$ à $C_6$, tel qu'un groupe méthyle, un groupe éthyle, un groupe isopropyle, un groupe tertbutyle ;
de préférence, $R^{11a}$ et $R^{11b}$ sont H ;
chaque $m_1$ est choisi indépendamment parmi 1, 2, 3, 4, 5 et 6 ; de préférence $m_1$ est 1 ;
chaque $m_2$ est choisi indépendamment parmi 0, 1, 2, 3, 4, 5 et 6 ; de préférence chaque $m_2$ est choisi

indépendamment parmi 0 ou 1 ;
de manière préférée entre toutes,

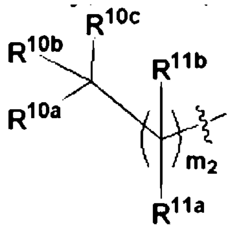
,

dans sa globalité, est choisi parmi

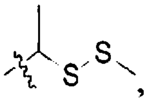
,

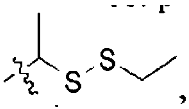, 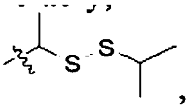, 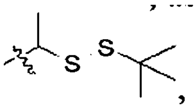, 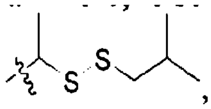, 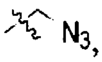, 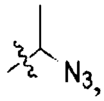,

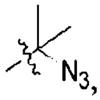, 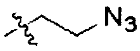 ;

R' est choisi parmi H, un groupe monophosphate

( 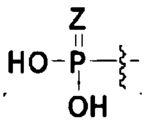 ) ,

un groupe disphosphate

( 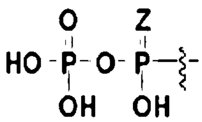 ) ,

un groupe triphosphate

( 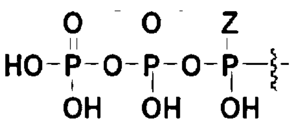 )

ou un groupe tétraphosphate

( 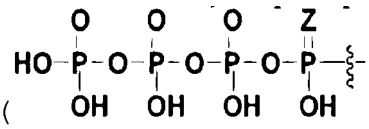 ) ;

de préférence, R' est un groupe triphosphate

(

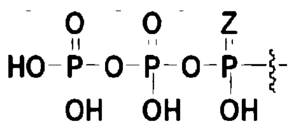 ) ;

chaque Z est choisi indépendamment parmi O, S, BH ; de préférence , Z est O ;
Base est choisie parmi une base, une base déaza ou un tautomère de celles-ci, par exemple Base est choisie parmi l'adénine, la 7-déazaadénine, la thymine, l'uracile, la cytosine, la guanine, la 7-déazaguanine ou un tautomère de celles-ci ;
de préférence, Base est choisie parmi

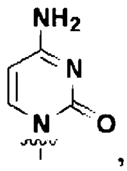 , 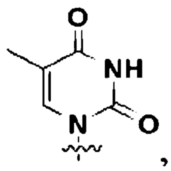 , 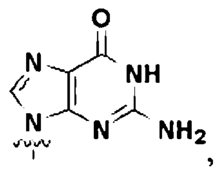 , 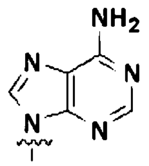 .

2. Composé ou un sel de celui-ci selon la revendication 1, dans lequel le composé est choisi parmi les suivants :

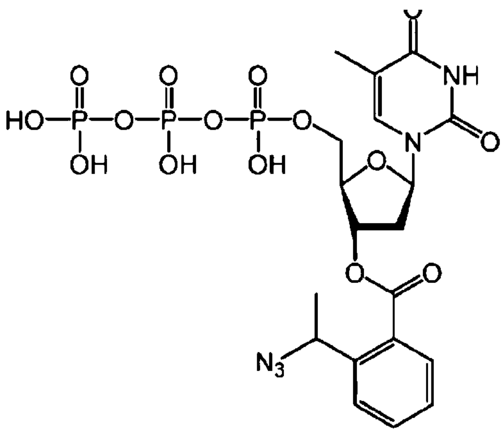 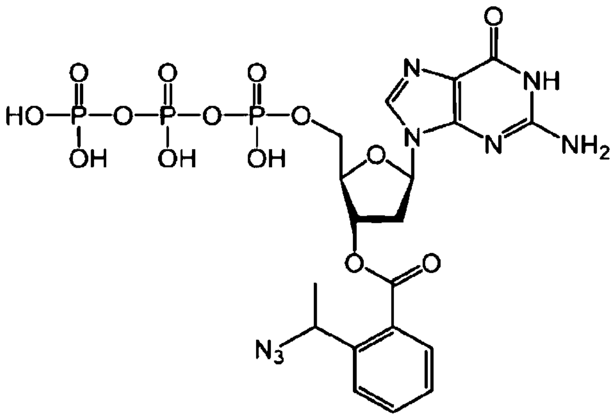

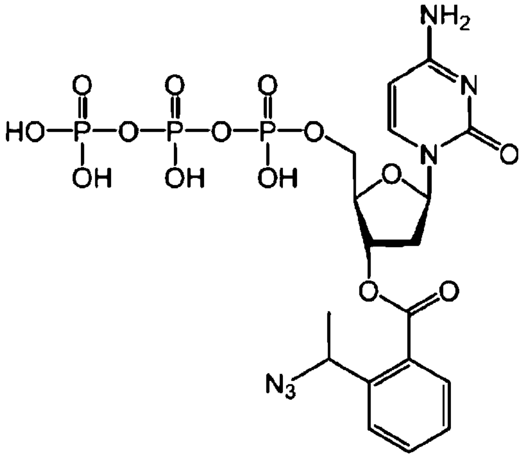

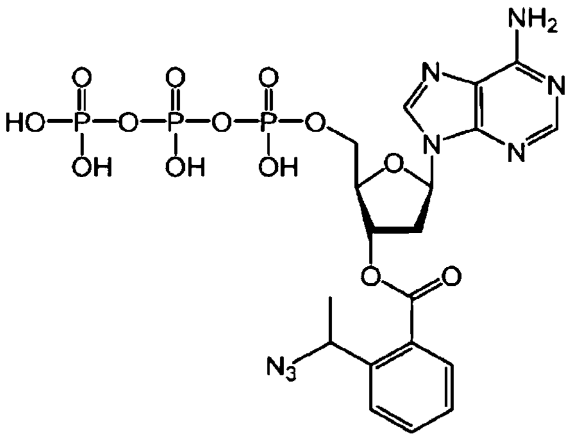

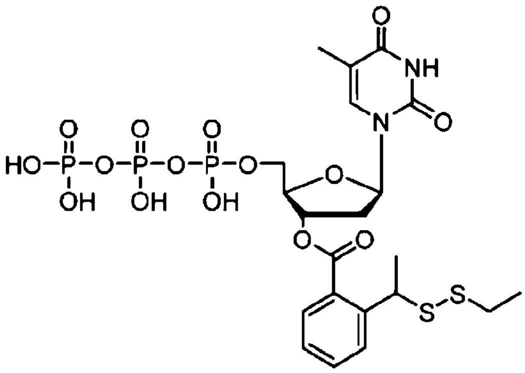

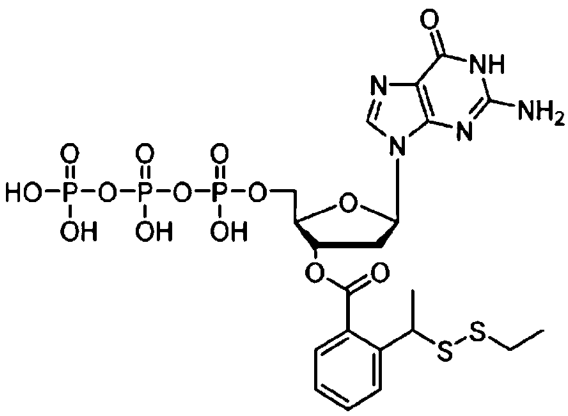

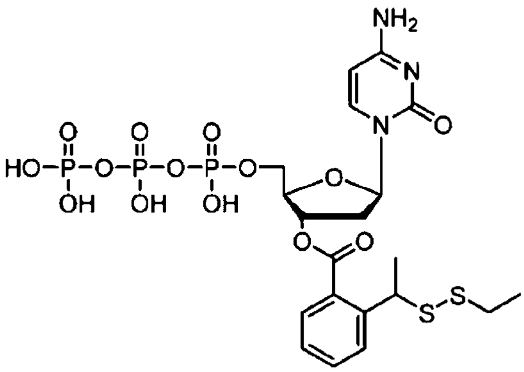

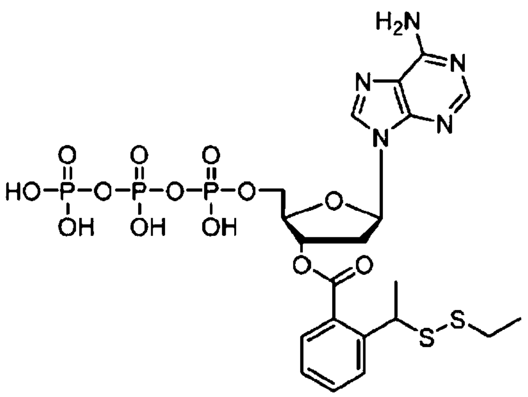

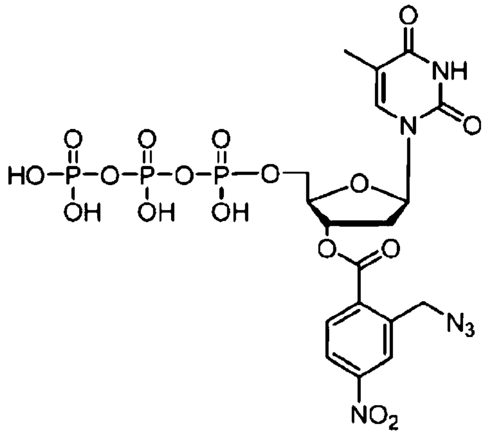

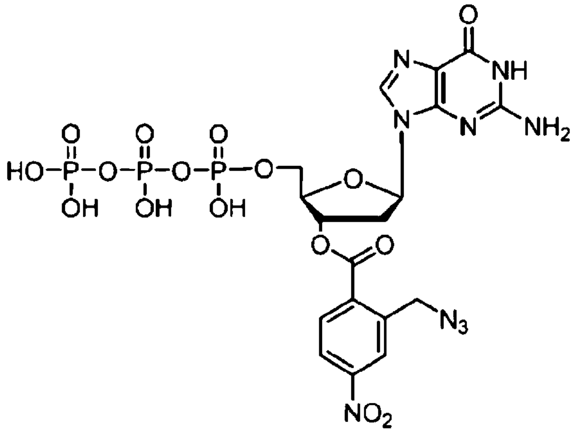

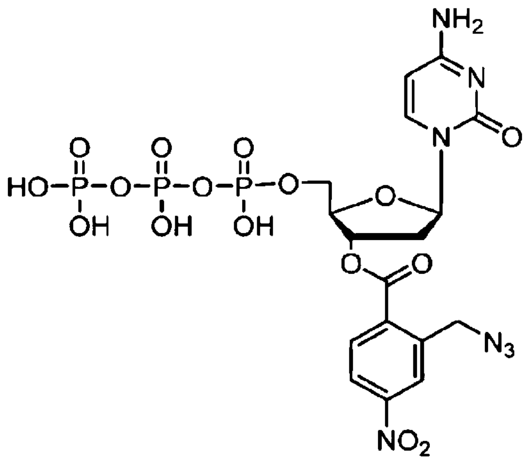

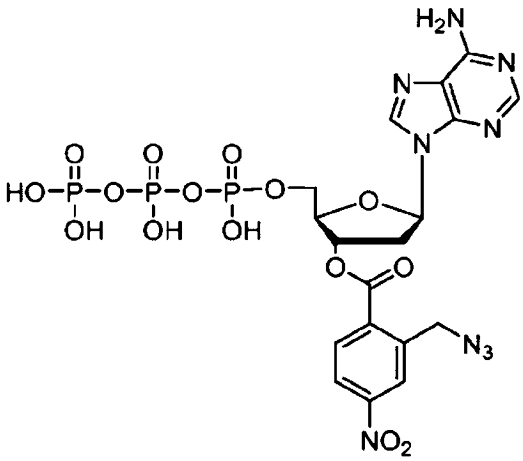

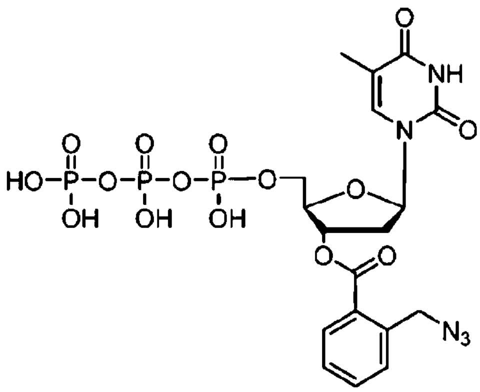

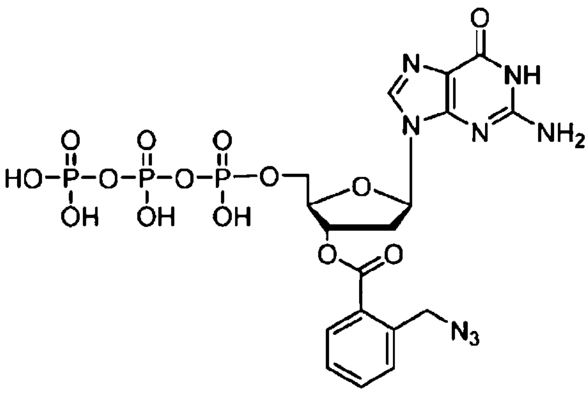

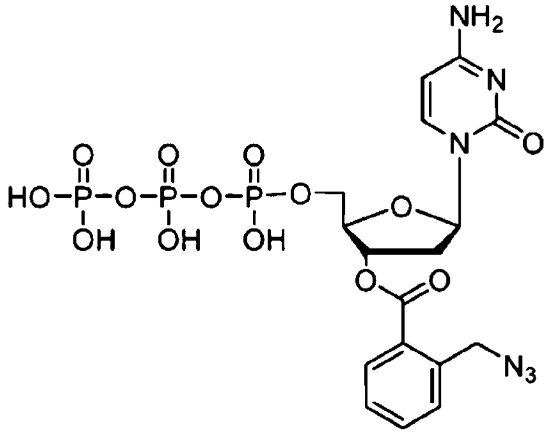

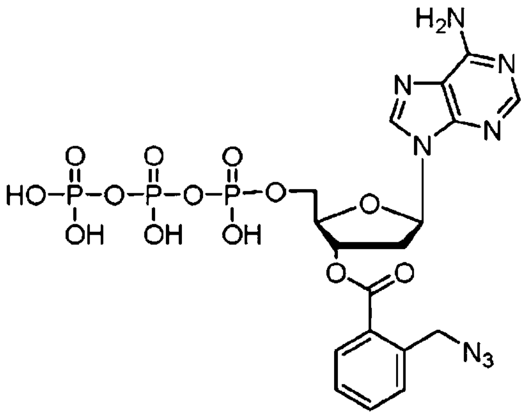

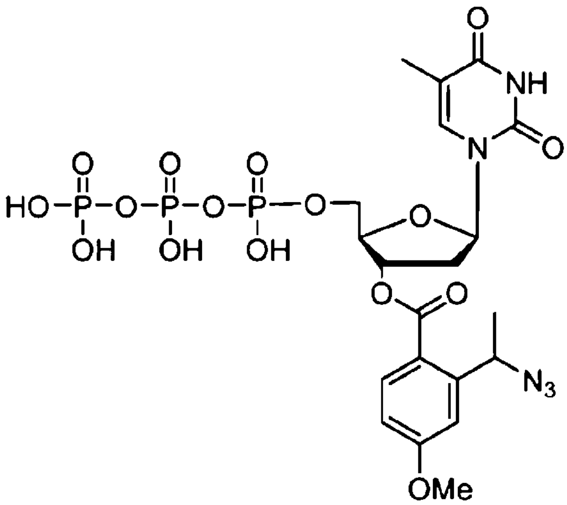

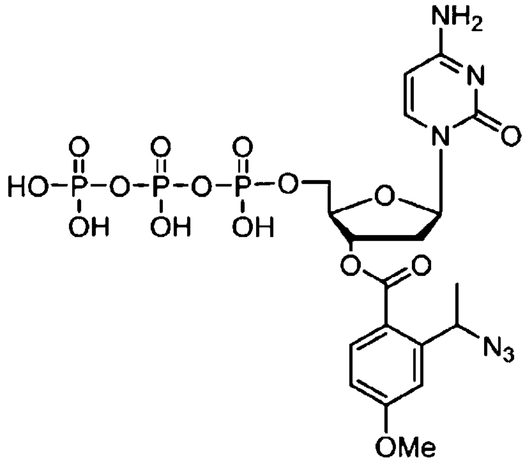

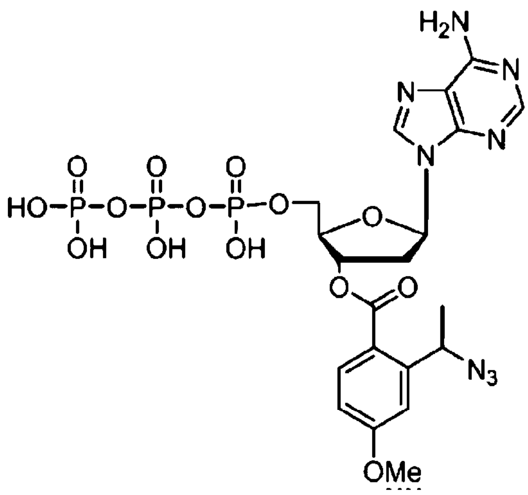

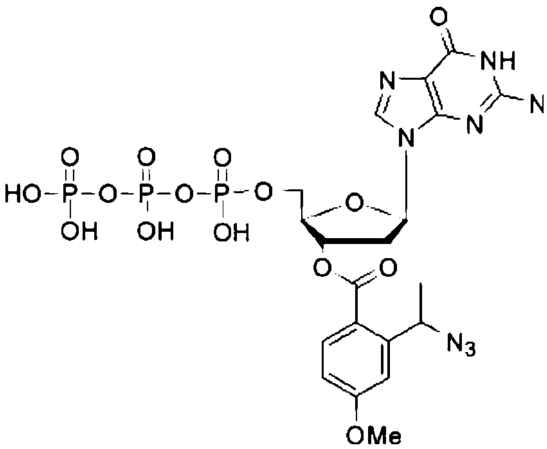

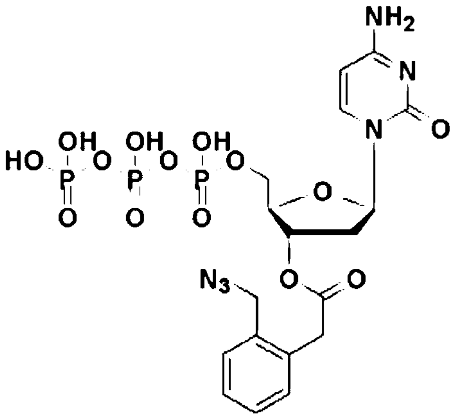

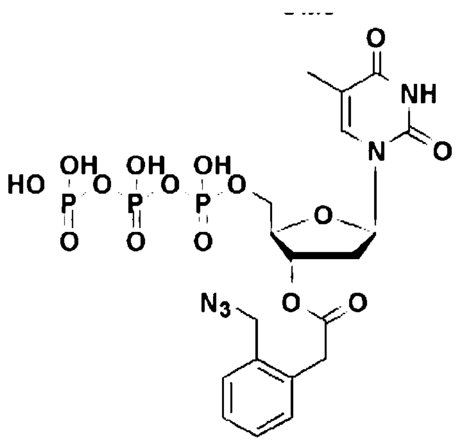

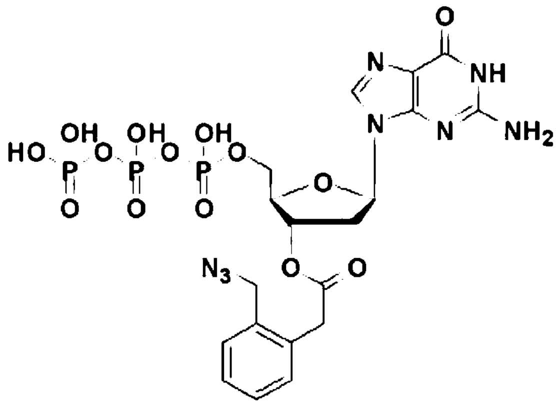

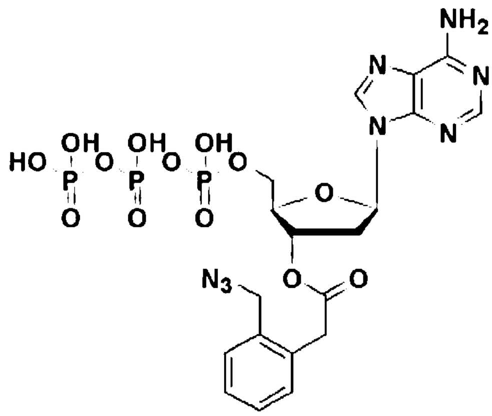

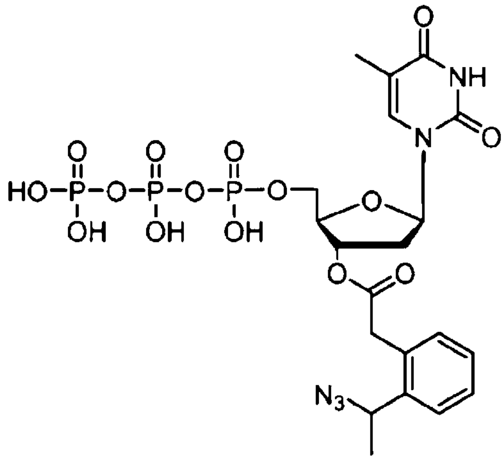

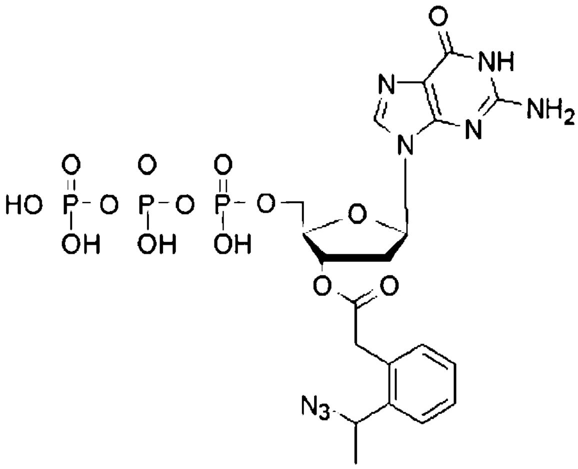

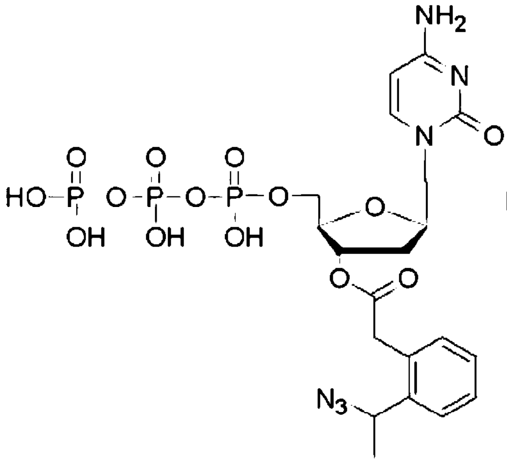

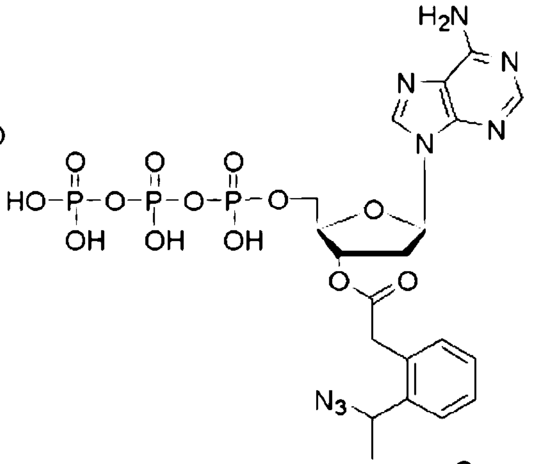

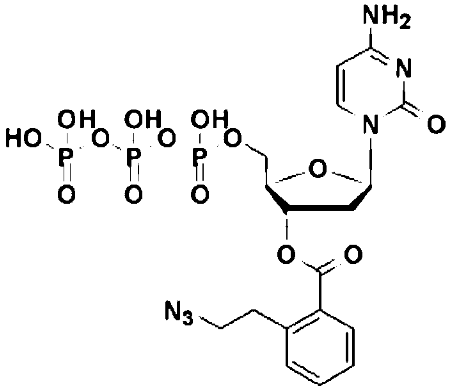

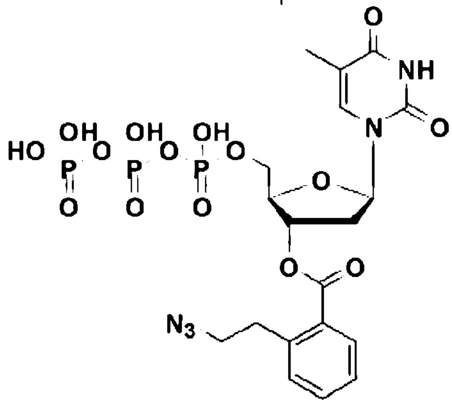

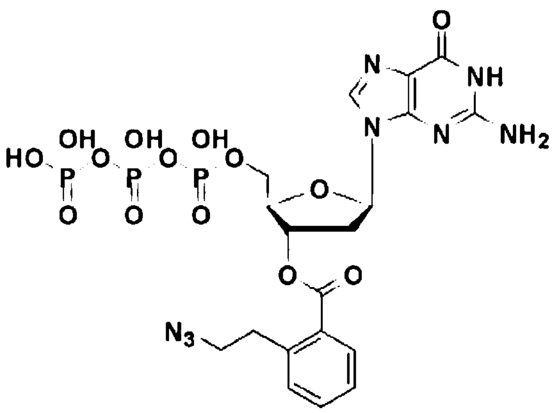

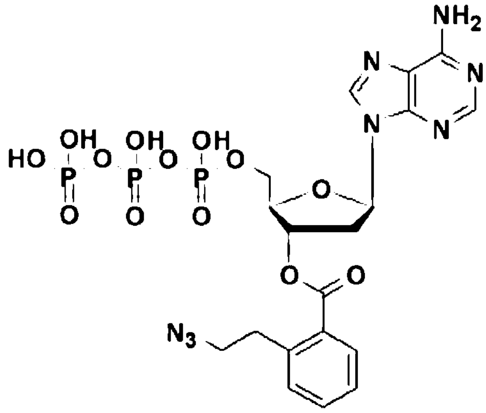

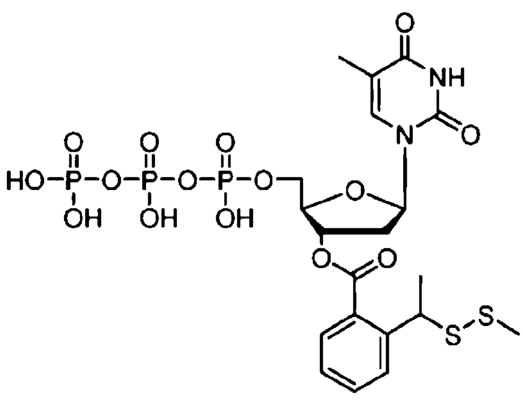

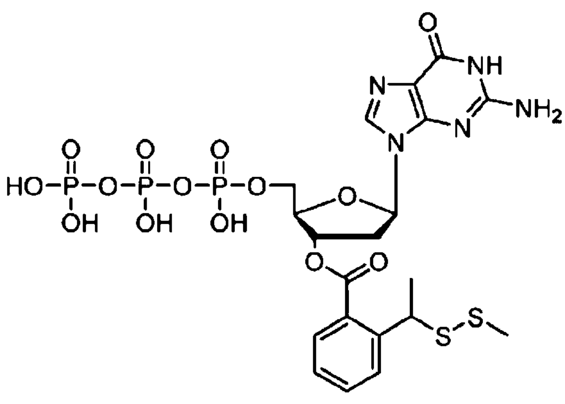

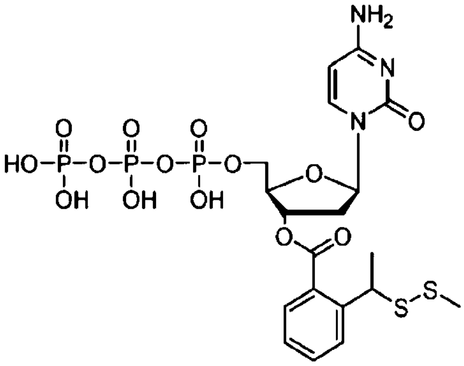

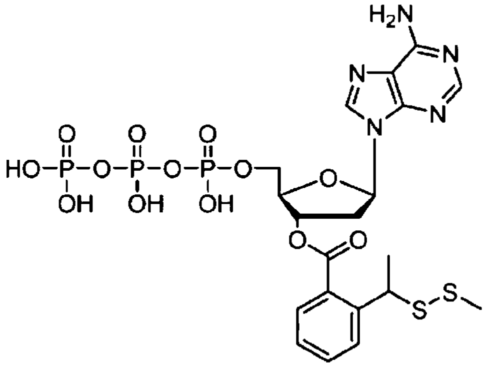

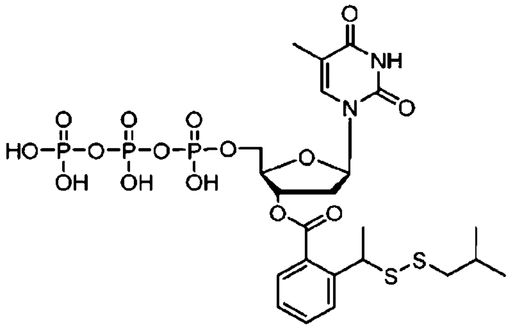

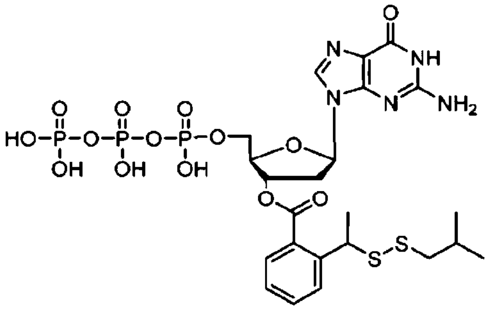

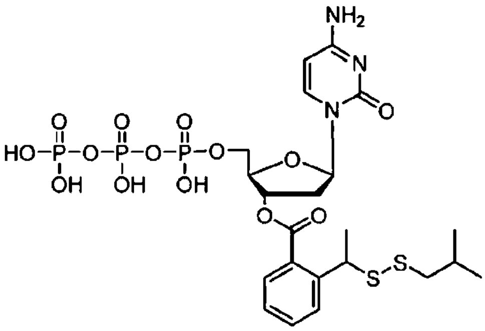

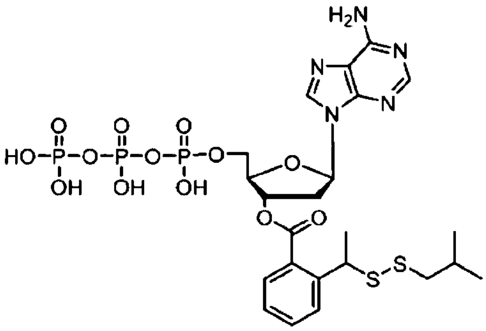

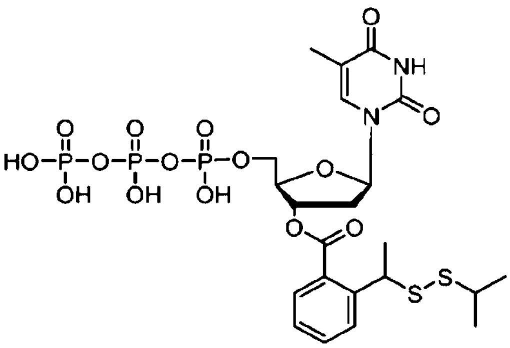

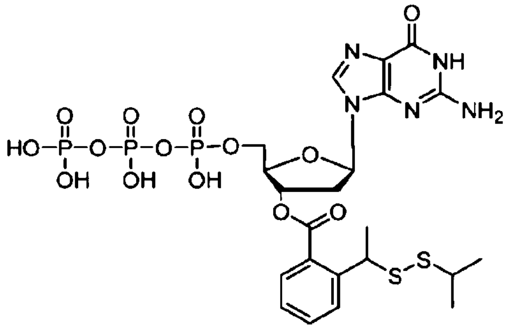

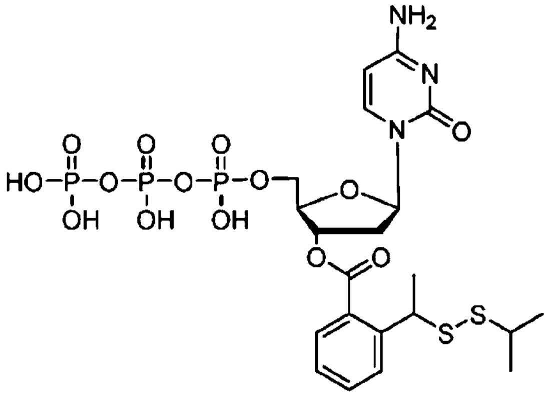

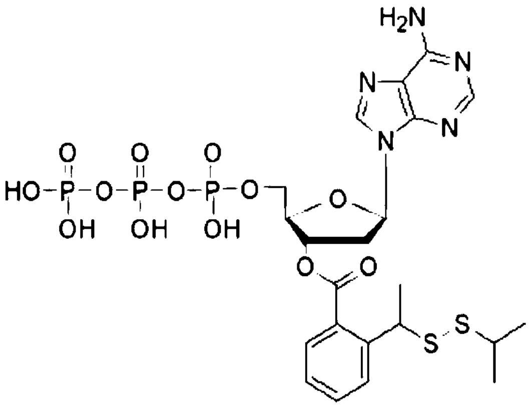

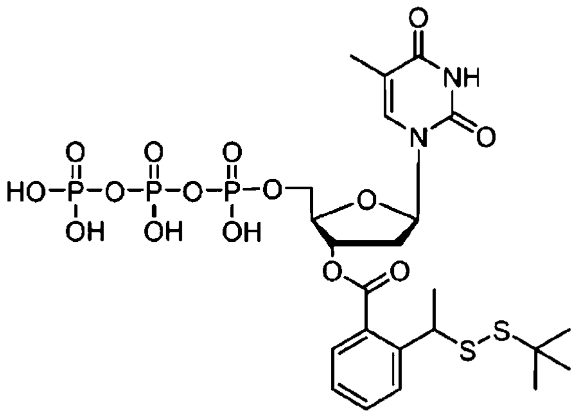

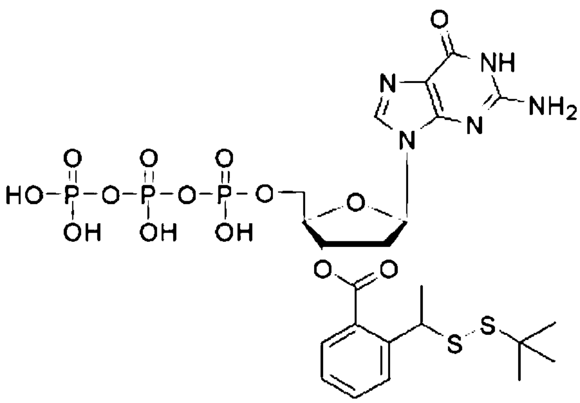

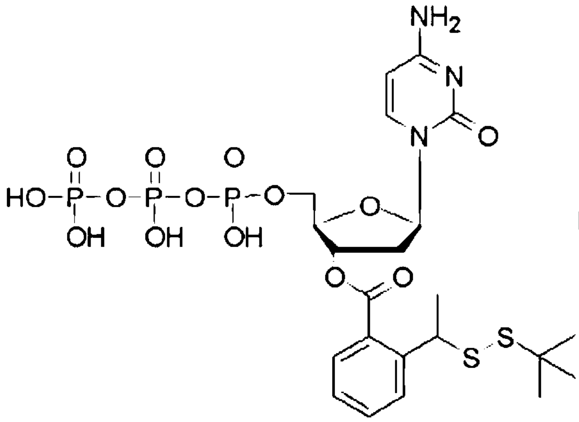

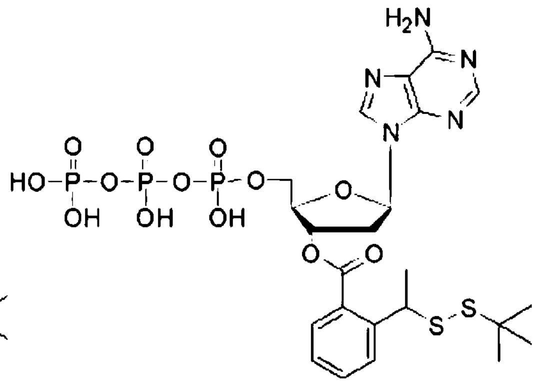

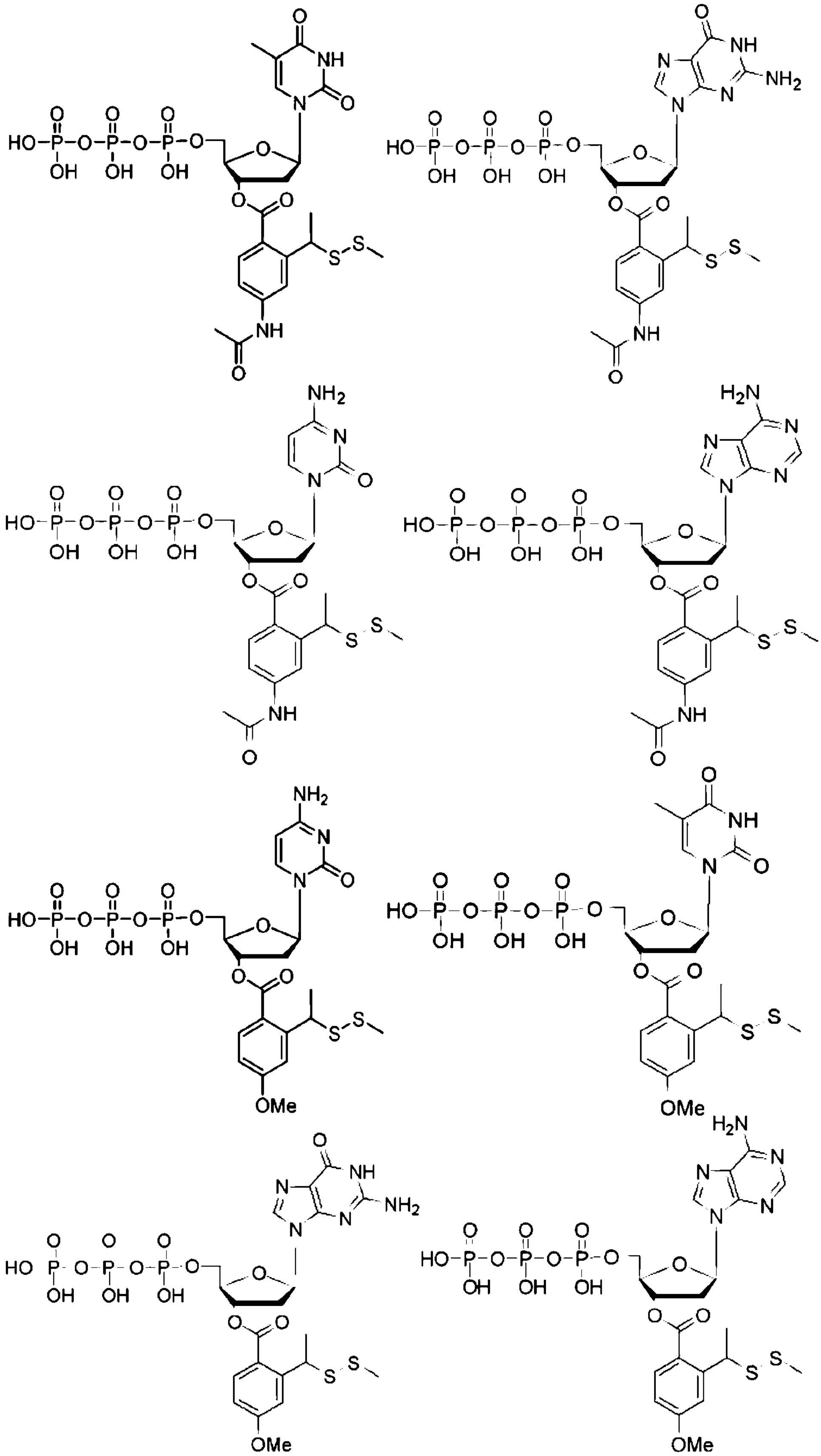

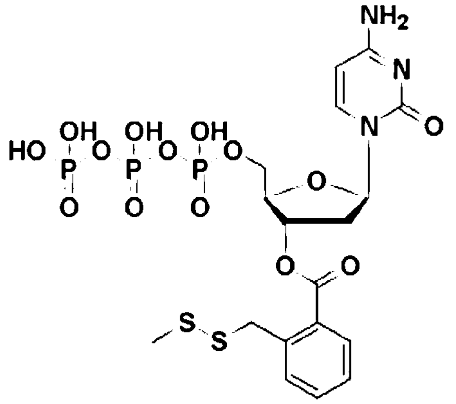

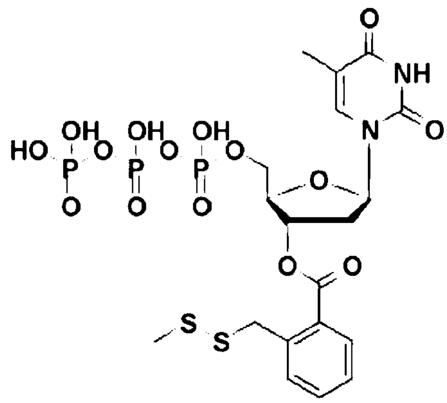

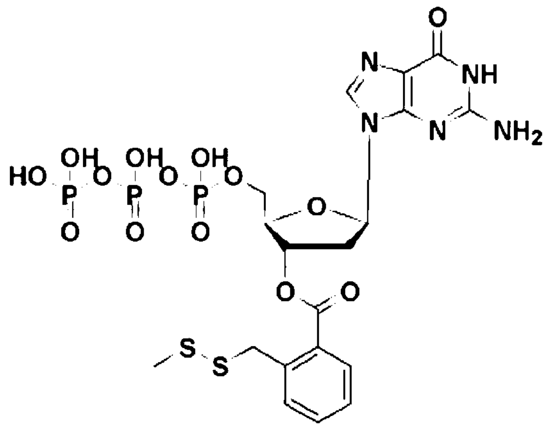

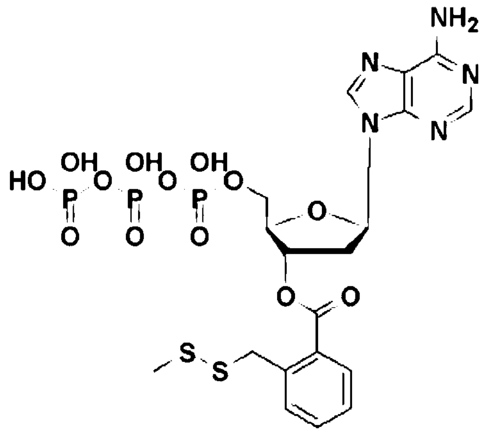

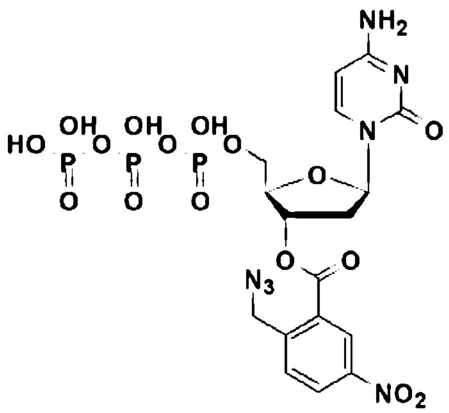

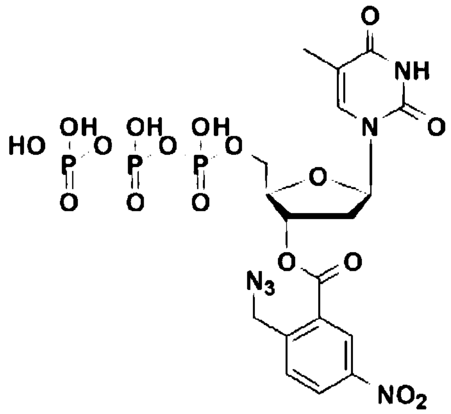

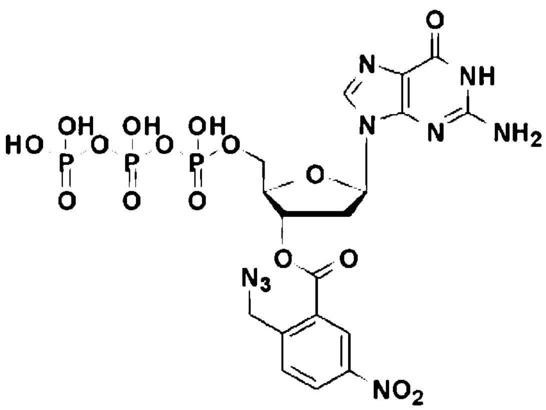

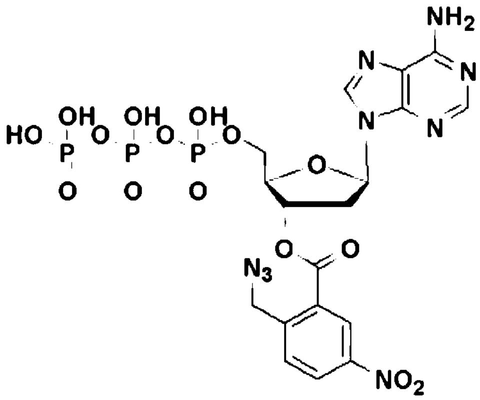

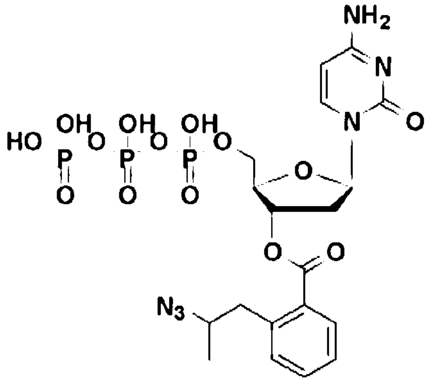

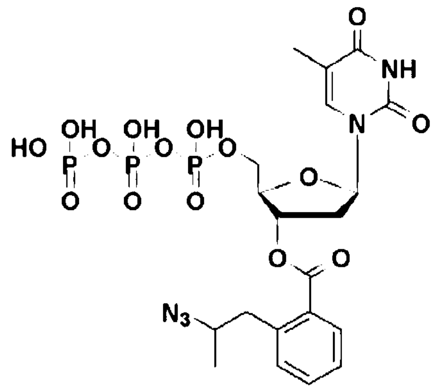

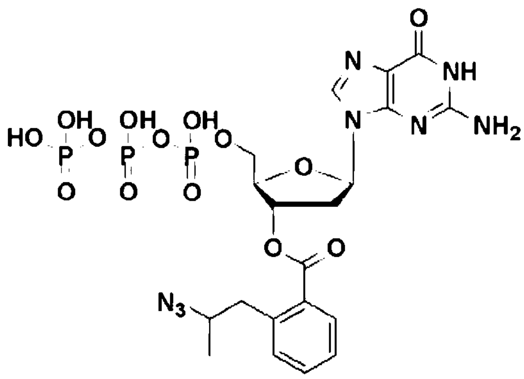

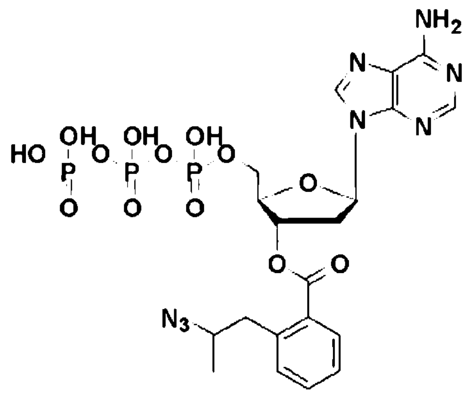

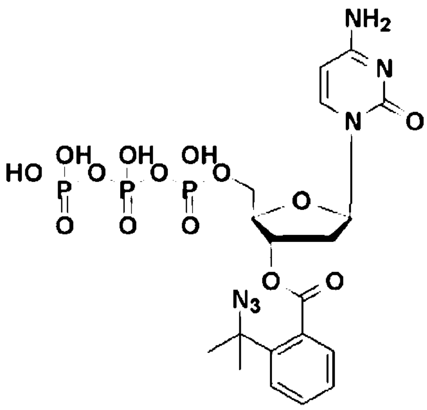

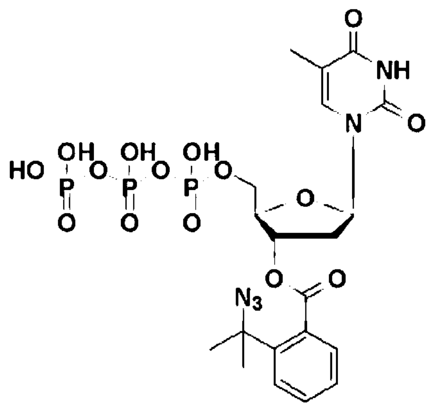

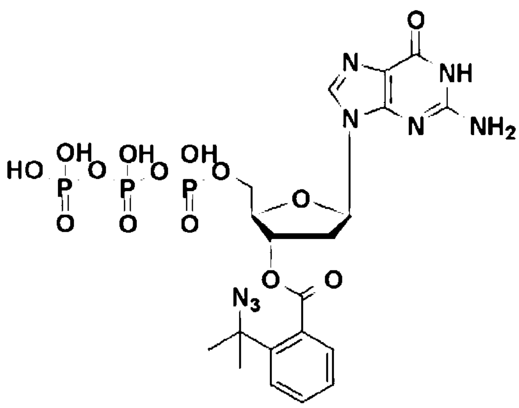

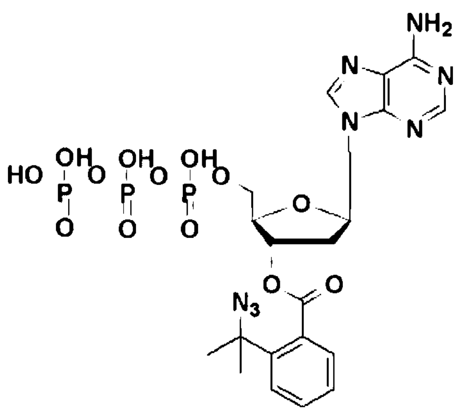

**3.** Composé ou un sel de celui-ci selon la revendication 1 ou 2, qui porte un marqueur détectable supplémentaire, dans lequel le marqueur détectable est un marqueur fluorescent ;

le marqueur détectable supplémentaire porté par le composé ou un sel de celui-ci est introduit par un réactif d'affinité, qui est choisi parmi un anticorps, un aptamère, un Affimer et une knottine, dans lequel le réactif d'affinité porte le marqueur détectable, et le réactif d'affinité peut reconnaître et se lier spécifiquement à un épitope du composé ou d'un sel de celui-ci ;

le marqueur détectable supplémentaire est lié à la Base du composé ou d'un sel de celui-ci éventuellement par le biais d'un lieur ;

de préférence, le lieur est un lieur clivable ou un lieur non clivable ;

de préférence, le lieur clivable est choisi dans le groupe constitué par un lieur capable d'être clivé par réaction électrophile, un lieur capable d'être clivé par réaction nucléophile, un lieur capable d'être clivé par photolyse, un lieur capable d'être clivé dans des conditions réductrices, un lieur capable d'être clivé dans des conditions oxydantes, un lieur à sécurité intégrée, un lieur capable d'être clivé dans par des mécanismes d'élimination, ou une quelconque combinaison de ceux-ci ;

de préférence, le marqueur détectable est choisi parmi les suivants :

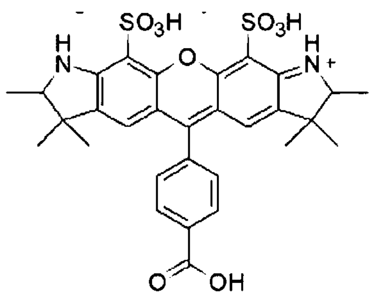

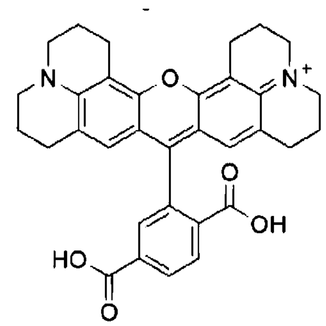

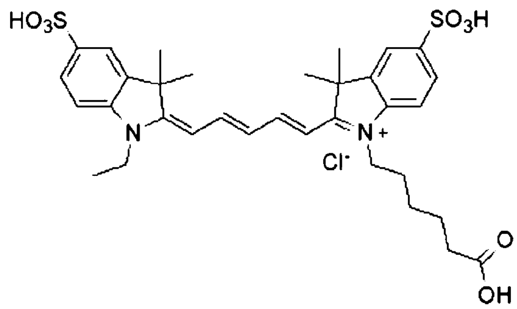

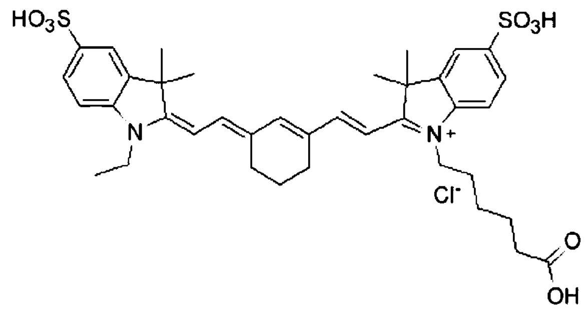

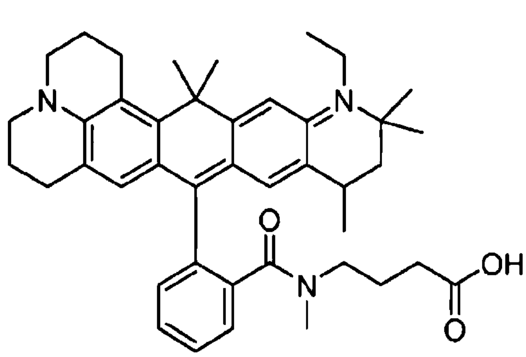

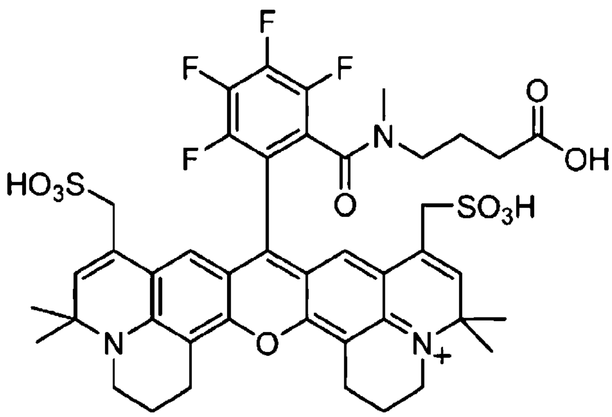

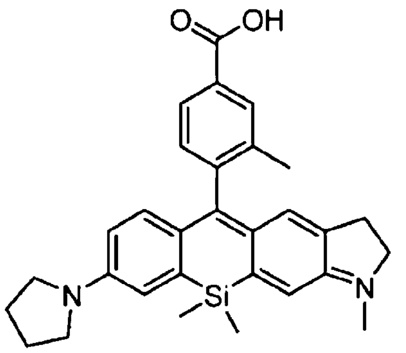

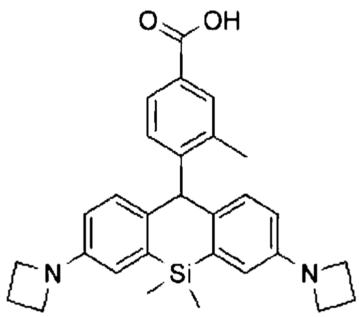

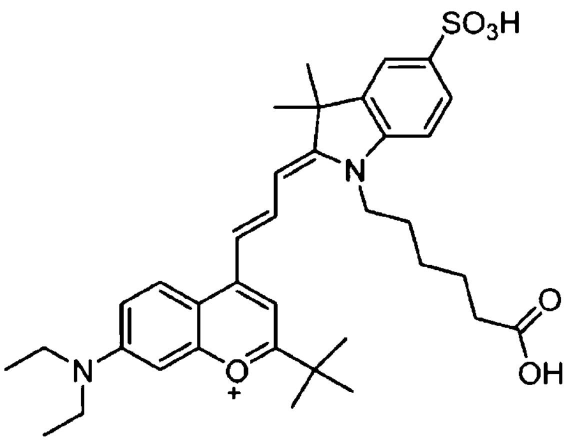

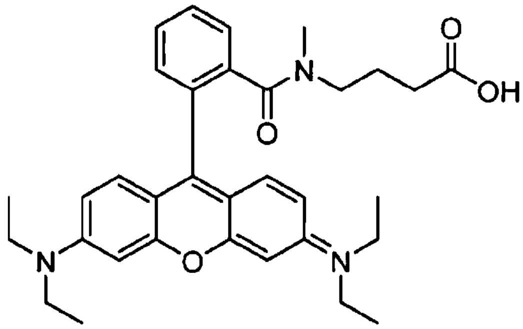

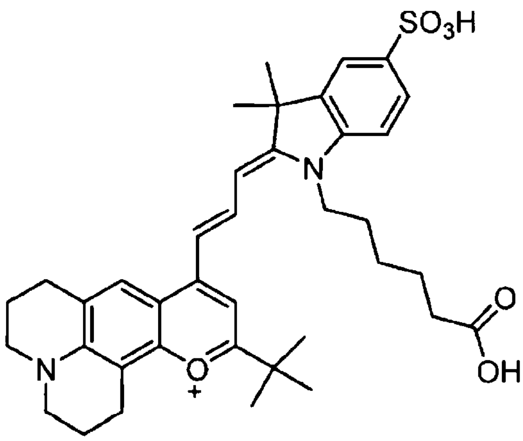

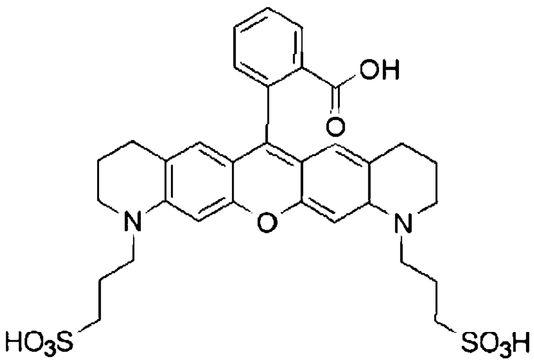

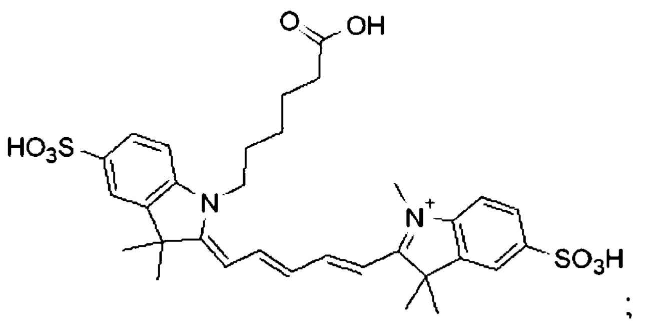

;

de préférence, si Base est différente, le marqueur détectable varie.

4. Procédé pour terminer une synthèse d'acide nucléique, comprenant l'incorporation du composé ou d'un sel de celui-ci selon l'une quelconque des revendications 1 à 3 dans une molécule d'acide nucléique à terminer ;

de préférence, l'incorporation du composé ou d'un sel de celui-ci est réalisée par une transférase terminale, une polymérase terminale ou une transcriptase inverse ;
de préférence, le procédé comprend l'incorporation du composé ou d'un sel de celui-ci dans la molécule d'acide nucléique à terminer à l'aide d'une polymérase ;
de préférence, le procédé comprend la réalisation d'une réaction de polymérisation nucléotidique à l'aide d'une polymérase dans des conditions dans lesquelles la polymérase permet de réaliser la réaction de polymérisation nucléotidique, incorporant ainsi le composé ou un sel de celui-ci dans l'extrémité 3' de la molécule d'acide nucléique à terminer.

**5.** Procédé de préparation d'un polynucléotide en cours d'élongation complémentaire d'un polynucléotide simple brin cible lors d'une réaction de séquençage, comprenant l'incorporation du composé ou d'un sel de celui-ci selon l'une quelconque des revendications 1 à 3 dans le polynucléotide complémentaire en cours d'élongation, dans lequel l'incorporation du composé ou d'un sel de celui-ci empêche l'introduction de quelconques nucléotides ultérieurs dans le polynucléotide complémentaire en cours d'élongation ;

de préférence, l'incorporation du composé ou d'un sel de celui-ci est réalisée par une transférase terminale, une polymérase terminale ou une transcriptase inverse ; de préférence, le procédé comprend l'incorporation du composé ou d'un sel de celui-ci dans le polynucléotide complémentaire en cours d'élongation à l'aide d'une polymérase ;
de préférence, le procédé comprend la réalisation d'une réaction de polymérisation nucléotidique à l'aide d'une polymérase dans des conditions dans lesquelles la polymérase permet de réaliser la réaction de polymérisation nucléotidique, incorporant ainsi le composé ou un sel de celui-ci dans l'extrémité 3' du polynucléotide complémentaire en cours d'élongation.

**6.** Procédé de détermination de la séquence d'un polynucléotide simple brin cible, comprenant :

1) le suivi de l'incorporation de nucléotides complémentaires au polynucléotide simple brin cible dans un brin d'acide nucléique en cours d'élongation, dans lequel l'au moins un nucléotide complémentaire incorporé est le composé ou un sel de celui-ci selon l'une quelconque des revendications 1 à 3, et le composé ou un sel de celui-ci porte un marqueur détectable supplémentaire, dans lequel le marqueur détectable est un marqueur fluorescent, et,
2) la détection du marqueur détectable pour déterminer le nucléotide incorporé ;
de préférence, le marqueur détectable supplémentaire est lié au composé ou à un sel de celui-ci éventuellement par le biais d'un lieur ;
de préférence, le lieur est tel que défini dans la revendication 3 ;
de préférence, le marqueur détectable supplémentaire est tel que défini dans la revendication 3 ;
de préférence, si Base est différente, le marqueur détectable porté par le composé ou un sel de celui-ci varie ;
de préférence, le groupe bloquant réversible (R) du composé ou d'un sel de celui-ci et le marqueur détectable sont éliminés avant l'introduction d'un nucléotide complémentaire suivant ;
de préférence, le groupe bloquant réversible et le marqueur détectable sont éliminés simultanément ; ou
de préférence, le groupe bloquant réversible et le marqueur détectable sont éliminés séquentiellement ;
par exemple, après l'élimination du marqueur détectable, le groupe bloquant réversible est éliminé, ou, après l'élimination du groupe bloquant réversible, le marqueur détectable est éliminé.

**7.** Procédé selon la revendication 6, qui comprend les étapes suivantes consistant à :

(a) fournir une pluralité de nucléotides différents, dans lequel la pluralité de nucléotides différents sont le composé ou un sel de celui-ci selon l'une quelconque des revendications 1 à 3, dans lequel chaque nucléotide porte un marqueur détectable supplémentaire qui peut être distinct d'un marqueur détectable supplémentaire porté par un autre nucléotide pendant la détection ;
(b) incorporer la pluralité de nucléotides différents dans une séquence complémentaire à un polynucléotide simple brin cible ;
(c) détecter les marqueurs détectables supplémentaires portés par les nucléotides de l'étape (b) pour déterminer les types de nucléotides incorporés ;
(d) éliminer les groupes bloquants réversibles et les marqueurs détectables portés par les nucléotides à l'étape (b) ; et
(e) éventuellement, répéter les étapes (b) à (d) une ou plusieurs fois ;

ce par quoi la séquence du polynucléotide simple brin cible est déterminée.

**8.** Procédé selon la revendication 6, comprenant les étapes suivantes consistant à :

(1) fournir un premier nucléotide, un deuxième nucléotide, un troisième nucléotide et un quatrième nucléotide, dans lequel au moins l'un des quatre nucléotides est le composé ou un sel de celui-ci selon l'une quelconque des revendications 1 à 3, les Bases contenues dans les quatre nucléotides sont différentes les unes des autres, et les quatre nucléotides portent un marqueur détectable supplémentaire, de préférence, le marqueur détectable supplémentaire porté par les quatre nucléotides est introduit par un réactif d'affinité, qui est choisi parmi un anticorps, un aptamère, un Affimer et une knottine, dans lequel le réactif d'affinité porte le réactif détectable, et le marqueur d'affinité peut reconnaître et se lier spécifiquement à un épitope de chaque nucléotide, ou de préférence, les quatre nucléotides sont liés au marqueur détectable supplémentaire éventuellement par le biais d'un lieur, ou plus préférablement, la Base des quatre nucléotides est liée au marqueur détectable supplémentaire éventuellement par le biais d'un lieur, de manière préférée entre toutes, les marqueurs détectables supplémentaires portés par les quatre nucléotides sont différents les uns des autres ;
(2) mettre en contact les quatre nucléotides avec un polynucléotide simple brin cible ; éliminer les nucléotides qui ne sont pas incorporés dans le brin d'acide nucléique en cours d'élongation ; détecter les marqueurs détectables portés par les nucléotides qui sont incorporés dans le brin d'acide nucléique en cours d'élongation ; éliminer les groupes bloquants réversibles et les marqueurs détectables portés par les nucléotides qui sont incorporés dans le brin d'acide nucléique en cours d'élongation ;

éventuellement, il comprend également (3) : répéter l'étape (2) une ou plusieurs fois.

**9.** Procédé selon la revendication 6, qui comprend les étapes suivantes consistant à :

(a) fournir un mélange comprenant un duplex, au moins un composé ou un sel de celui-ci selon l'une quelconque des revendications 1 à 3, une polymérase et un réactif d'excision ; dans lequel le duplex comprend un brin d'acide nucléique en cours d'élongation et un brin d'acide nucléique à séquencer ; dans lequel le composé ou un sel de celui-ci porte un marqueur détectable supplémentaire, de préférence, le marqueur détectable supplémentaire porté par le composé ou un sel de celui-ci est introduit par un réactif d'affinité, dans lequel le réactif d'affinité porte le marqueur détectable, et le réactif d'affinité peut reconnaître et de se lier spécifiquement à un épitope du composé ou d'un sel de celui-ci, ou de préférence, le composé ou un sel de celui-ci est lié au marqueur détectable supplémentaire éventuellement par le biais d'un lieur, ou plus préférablement, la Base du composé ou d'un sel de celui-ci est liée au marqueur détectable supplémentaire éventuellement par le biais d'un lieur ;
(b) réaliser une réaction comprenant les étapes (i), (ii) et (iii) suivantes, éventuellement, répéter les étapes une ou plusieurs fois :

étape (i) : incorporer le composé ou un sel de celui-ci dans le brin d'acide nucléique en cours d'élongation à l'aide d'une polymérase pour former un intermédiaire d'acide nucléique contenant le groupe bloquant réversible et le marqueur détectable ;
étape (ii) : détecter le marqueur détectable contenu dans l'intermédiaire d'acide nucléique ;
étape (iii) : éliminer le groupe bloquant réversible et/ou le marqueur détectable contenus dans l'intermédiaire d'acide nucléique à l'aide du réactif d'excision ;
de préférence, l'élimination du groupe bloquant réversible et l'élimination du marqueur détectable sont effectuées simultanément, ou, l'élimination du groupe bloquant réversible et l'élimination du marqueur détectable sont effectuées séquentiellement (par exemple, le groupe bloquant réversible est éliminé en premier, ou le marqueur détectable est éliminé en premier) ;
de préférence, le réactif d'excision utilisé pour l'élimination du groupe bloquant réversible est le même que celui utilisé pour l'élimination du marqueur détectable ;
de préférence, le réactif d'excision utilisé pour l'élimination du groupe bloquant réversible est différent de celui utilisé pour l'élimination du marqueur détectable.

**10.** Procédé selon la revendication 9, dans lequel le duplex est lié à un support ;

de préférence, le brin d'acide nucléique en cours d'élongation est une amorce ;
de préférence, l'amorce est hybridée au brin d'acide nucléique à séquencer pour former le duplex ;
de préférence, le duplex, le composé ou un sel de celui-ci, et la polymérase forment ensemble un système réactionnel contenant une phase en solution et une phase solide ;

de préférence, les Bases contenues dans les composés ou des sels de ceux-ci sont différentes les unes des autres ;
de préférence, le marqueur détectable supplémentaire porté par le composé ou un sel de celui-ci est différent les uns des autres ;
de préférence, le composé ou un sel de celui-ci est incorporé dans le brin d'acide nucléique en cours d'élongation à l'aide d'une polymérase dans des conditions dans lesquelles la polymérase permet de réaliser une réaction de polymérisation nucléotidique, formant ainsi un intermédiaire d'acide nucléique contenant un groupe bloquant réversible et le marqueur détectable ;
de préférence, la polymérase est choisie parmi une polymérase KOD ou des mutants de celle-ci (par ex., KOD POL151, KOD POL157, KOD POL171, KOD POL174, KOD POL376, KOD POL391) ;
de préférence, avant une quelconque étape de détection du marqueur détectable contenu dans l'intermédiaire d'acide nucléique, la phase en solution du système réactionnel de l'étape précédente est éliminée et le duplex lié au support est conservé ;
de préférence, le réactif d'excision est en contact avec le duplex ou le brin d'acide nucléique en cours d'élongation dans le système réactionnel contenant une phase en solution et une phase solide ;
de préférence, le réactif d'excision peut éliminer le groupe bloquant réversible et le marqueur détectable supplémentaire portés par le composé qui est incorporé dans le brin d'acide nucléique en cours d'élongation, sans affecter la liaison phosphodiester sur le squelette du duplex ;
de préférence, après une quelconque étape d'élimination du groupe bloquant réversible et/ou du marqueur détectable supplémentaire contenus dans l'intermédiaire d'acide nucléique, la phase en solution du système réactionnel dans cette étape est éliminée ;
de préférence, une opération de lavage est effectuée après une quelconque étape comprenant une opération d'élimination ;
de préférence, après l'étape (ii), le procédé comprend en outre : la détermination du type de composé incorporé dans le brin d'acide nucléique en cours d'élongation de l'étape (i) en fonction du signal détecté à l'étape (ii), et la détermination du type de nucléotide au niveau d'une position correspondante dans le brin d'acide nucléique à séquencer en se basant sur le principe d'appariement complémentaire des bases.

**11.** Kit comprenant au moins un composé ou un sel de celui-ci selon l'une quelconque des revendications 1 à 3 ;

de préférence, le kit comprend un premier composé, un deuxième composé, un troisième composé et un quatrième composé, les premier, deuxième, troisième et quatrième composés sont indépendamment le composé ou un sel de celui-ci selon l'une quelconque des revendications 1 à 3 ;
de préférence, dans le premier composé, la Base est choisie parmi l'adénine, la 7-déazaadénine ou un tautomère de celles-ci (par ex.

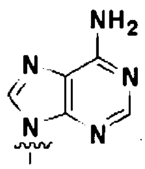

) ; dans le deuxième composé, la Base est choisie parmi la thymine, l'uracile ou un tautomère de ceux-ci (par ex.

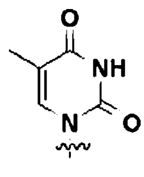

) ; dans le troisième composé, la Base est choisie parmi la cytosine ou un tautomère de celle-ci (par ex.

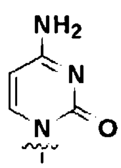

) ; dans le quatrième composé, la Base est choisie parmi la guanine, la 7-déazaguanine ou un tautomère de celles-ci (par ex.

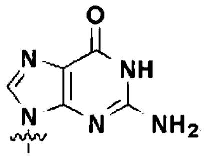

) ;
de préférence, les premier, deuxième, troisième et quatrième composés portent des marqueurs détectables supplémentaires ;
de préférence, les marqueurs détectables supplémentaires portés par les premier, deuxième, troisième et quatrième composés sont introduits par un réactif d'affinité, qui est choisi parmi un anticorps, un aptamère, un Affimer et une knottine, dans lequel le réactif d'affinité porte le marqueur détectable, et le réactif d'affinité peut reconnaître et de se lier spécifiquement à l'épitope du premier, du deuxième, du troisième ou du quatrième composé ;
de préférence, les premier, deuxième, troisième et quatrième composés sont liés au marqueur détectable supplémentaire éventuellement par le biais d'un lieur ;
de préférence, la Base du premier, du deuxième, du troisième ou du quatrième composé est liée au marqueur détectable supplémentaire éventuellement par le biais d'un lieur ;
de préférence, les Bases contenues dans les premier, deuxième, troisième et quatrième composés sont différentes les unes des autres ;
de préférence, les marqueurs détectables supplémentaires portés par les premier, deuxième, troisième et quatrième composés sont différents les uns des autres ;
de préférence, le lieur est tel que défini dans la revendication 3 ;
de préférence, le marqueur détectable est tel que défini dans la revendication 3.

**12.** Kit selon la revendication 11, dans lequel le kit comprend en outre un réactif pour prétraiter la molécule d'acide nucléique ; un support pour lier la molécule d'acide nucléique à séquencer ; un réactif pour lier la molécule d'acide nucléique à séquencer au support (par ex., liaison covalente ou non covalente) ; une amorce pour initier une réaction de polymérisation nucléotidique ; une polymérase pour réaliser la réaction de polymérisation nucléotidique ; une ou plusieurs solutions tampons ; une ou plusieurs solutions de lavage ; ou une quelconque combinaison de ceux-ci.

**13.** Utilisation du composé ou d'un sel de celui-ci selon l'une quelconque des revendications 1 à 3 ou du kit selon l'une quelconque des revendications 11 à 12 pour la détermination de la séquence d'un polynucléotide simple brin cible.

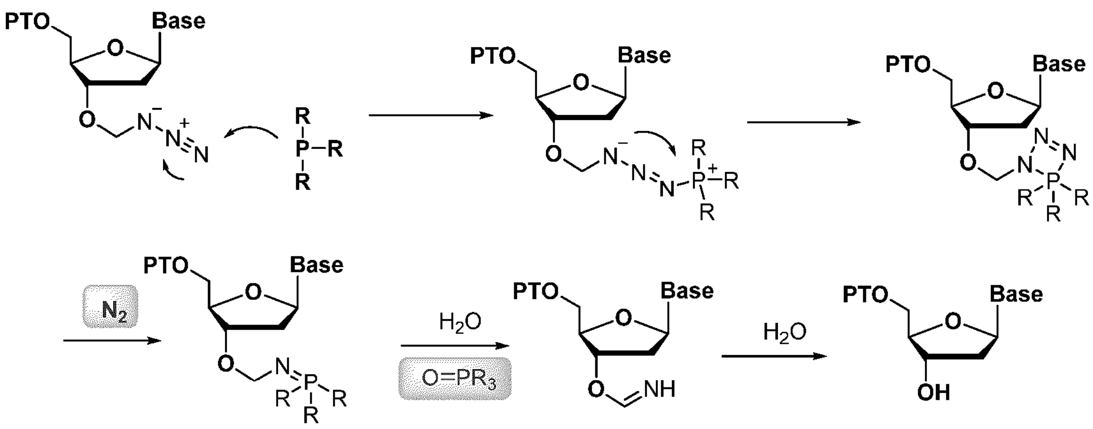


Figure 1

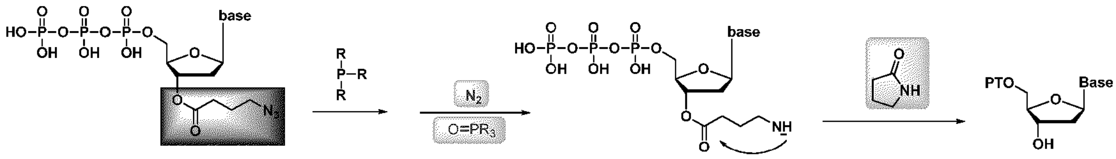


Figure 2

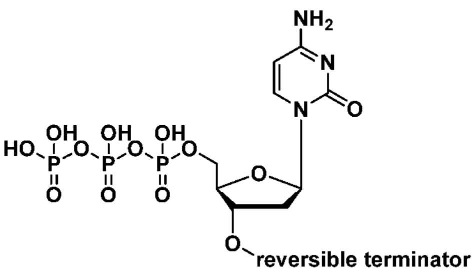

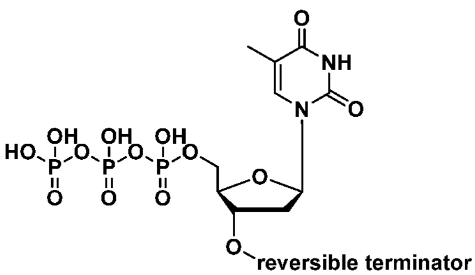

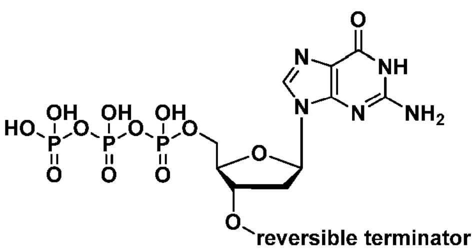

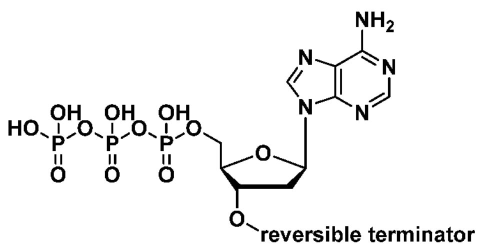

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020159447 A
- WO 0229003 A
- WO 9423064 A
- WO 9607669 A
- WO 2019071474 A
- WO 2018107350 A
- US 20160010150 A
- WO 2008037568 A
- WO 2014139596 A
- WO 2018129214 A1


### Non-patent literature cited in the description


- **CANARD BRUNO et al.** *Gene*, 02 June 1994, vol. 148 (1994), 1-6
- **SHOSHANI LLANA et al.** *Nucleosides and Nucleotides*, 01 September 1994, vol. 13 (9), 1977-1989
- **J. SAMBROOK et al.** Molecular Cloning: A Laboratory Manual. Cold Spring Harbor Laboratory Press, 1989
- **F.M. AUSUBEL et al.** Short Protocols in Molecular Biology. John Wiley&Sons, Inc., 1995
- **WELCH et al.** *Chem. Eur. J.*, 1999, vol. 5 (3), 951-960
- **ZHU et al.** *Cytometry*, 1997, vol. 28, 206-211
- **PROBER et al.** *Science*, 1987, vol. 238, 336-341
- **CONNELL et al.** *BioTechniques*, 1987, vol. 5 (4), 342-384
- **ANSORGE et al.** *Nucl. Acids Res.*, 1987, vol. 15 (11), 4593-4602
- **SMITH et al.** *Nature*, 1986, vol. 321, 674
- *Tet. Let.*, 2000, vol. 46, 8867-8871
- *J. Am. Chem. Soc.*, 2001, vol. 123, 8101-8108
- **EMPODOCLES et al.** *Nature*, 1999, vol. 399, 126-130
- **REICHERT et al.** *Anal. Chem.*, 2000, vol. 72, 6025-6029
- **LACOSTE et al.** *Proc. Natl. Acad. Sci USA*, 2000, vol. 97 (17), 9461-9466
- **GREENE ; WUTS**. Protective Groups in Organic Synthesis. John Wiley & Sons
- **GUILLIER et al.** *Chem. Rev.*, 2000, vol. 100, 2092-2157
- **LEE et al.** *J. Org. Chem.*, 1999, vol. 64, 3454-3460
- **BURGESS et al.** *J. Org. Chem.*, 1997, vol. 62, 5165-5168